# EUROPÄISCHE PATENTSCHRIFT

(11) **EP 4 158 703 B1**
(45) Veröffentlichungstag und Bekanntmachung des Hinweises auf die Patenterteilung: **25.06.2025**
(21) Anmeldenummer: 21727481.0
(22) Anmeldetag: 25.05.2021
(51) Int. Cl.: H10K 85/60, C07C 211/00, C07D 307/91, C07D 333/76, H10K 50/18

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 27.05.2020 EP 20176926
(43) Veröffentlichungstag der Anmeldung: 05.04.2023
(73) Patentinhaber: Merck Patent GmbH, 64293 Darmstadt (DE)
(72) Erfinder: ENGELHART, Jens, 64293 DARMSTADT (DE); MONTENEGRO, Elvira, 64293 DARMSTADT (DE); SIEMIANOWSKI, Simon, 64293 DARMSTADT (DE)
(74) Vertreter: Merck Patent Association
(86) Internationale Anmeldenummer: PCT/EP2021/063873
(87) Internationale Veröffentlichungsnummer: WO 2021/160898

(56) Entgegenhaltungen:
- EP-A1- 3 305 782
- WO-A1-2017/142310
- CN-A- 110 317 139
- US-A1- 2017 133 590

## Beschreibung

Die vorliegende Anmeldung betrifft aromatische und heteroaromatische Verbindungen, deren Herstellung, Mischungen und Formulierungen enthaltend die Verbindungen sowie elektronische Vorrichtungen enthaltend die Verbindungen oder die Mischungen.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter organische Elektrolumineszenzvorrichtungen verstanden, wobei OLEDs (organische lichtemittierende Dioden) ganz besonders bevorzugte organische Elektrolumineszenzvorrichtungen darstellen. Unter der Bezeichnung OLEDs werden organische Elektrolumineszenzvorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an einer Verbesserung der Leistungsdaten. In diesen Punkten konnte noch keine vollständig zufriedenstellende Lösung gefunden werden.

Einen großen Einfluss auf die Leistungsdaten von elektronischen Vorrichtungen haben Emissionsschichten und Schichten mit lochtransportierender Funktion. Dazu gehören neben der Emissionsschicht insbesondere auch die Lochtransportschicht (HTL), die Elektronenblockierschicht (EBL) und die Lochinjektionsscicht (HIL). Zur Verwendung in diesen Schichten werden weiterhin neue Verbindungen gesucht, insbesondere lochtransportierende Verbindungen und Verbindungen, die als lochleitenden Elektronenblockiermaterial in einer Elektronenblockierschicht, als Lochleiter in einer Lochtransportschicht oder als lochtransportierendes Matrixmaterial, insbesondere für phosphoreszierende Emitter, in einer emittierenden Schicht dienen können. Hierzu werden insbesondere Verbindungen gesucht, die eine hohe Glasübergangstemperatur, eine hohe Stabilität, und eine hohe Leitfähigkeit für Löcher aufweisen. Eine hohe Stabilität der Verbindung ist eine Voraussetzung, um eine lange Lebensdauer der elektronischen Vorrichtung zu erreichen.

Im Stand der Technik sind insbesondere Triarylaminverbindungen als Elektronenblockier- und Lochtransportmaterialien sowie lochtransportierende Matrixmaterialien für elektronische Vorrichtungen bekannt. Zu den Triarylaminverbindungen, die für die Verwendung in elektronischen Vorrichtungen bekannt sind, zählen auch Fluorenylamin-Verbindungen, d. h. Triarylaminverbindungen, in denen wenigstens eine Arylgruppe eine Fluorenylgruppe ist. Verbindungen, die sich zur Verwendung in organischen Elektrolumineszenzvorrichtungen eignen, sind beispielsweise in US 2017/133590 A1, CN 110 317 139 A1, EP 3 305 782 A1 und WO 2017/142310 A1 offenbart.

Es besteht jedoch weiterhin Bedarf an alternativen Verbindungen, die zur Verwendung in elektronischen Vorrichtungen geeignet sind, insbesondere an Verbindungen, die eine oder mehrere der oben genannten vorteilhaften Eigenschaften aufweisen. Es besteht weiterhin Verbesserungsbedarf bei den erzielten Leistungsdaten bei der Verwendung der Verbindungen in elektronischen Vorrichtungen, insbesondere bei Lebensdauer, Betriebsspannung und auch Effizienz der Vorrichtungen.

Es wurde gefunden, dass sich bestimmte, weiter unten näher genannte Fluorenyl-Aminverbindungen, die zwischen einer Fluorenylgruppe und einer Amingruppe bestimmte Linker L enthalten und die am Amin in spezifischer Weise mit aromatischen und heteroaromatischen Gruppen substituiert sind, hervorragend zur Verwendung in organischen elektronischen Vorrichtungen eignen, insbesondere zur Verwendung in OLEDs, nochmals insbesondere zur Verwendung als Lochtransportmaterialien, zur Verwendung als lochtransportierende Matrixmaterialien, insbesondere für phosphoreszierende Emitter und ganz besonders zur Verwendung als Elektronenblockiermaterialien (EBM) in einer Elektronenblockierschicht (EBL). Leistungsdaten der Vorrichtungen enthaltend die Verbindungen sind deutlich verbessert gegenüber dem Stand der Technik. Insbesondere die Lebensdauern und Bestriebsspannungen der Vorrichtungen weisen deutlich verbesserte Werte auf.

Außerdem weisen die Verbindungen selbst eine hohe Stabilität auf, insbesondere gegenüber Luft und Licht. Die Verbindungen zeichnen sich durch eine hohe Lagerstabilität aus. Weiterhin weisen die Verbindungen eine hohe Glasübergangstemperatur und eine hohe Leitfähigkeit für Löcher auf.

Die erfindungsgemäßen Verbindungen betreffen solche der folgenden Formel (3) 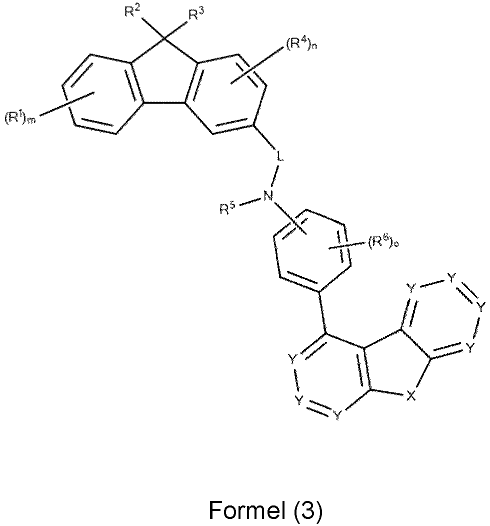 wobei für die auftretenden Variablen gilt:
X ist entweder O oder S, wobei in einer ganz bevorzugten Ausführungsform
X gleich O ist; in einer anderen bevorzugten Ausführungsform ist X gleich S;
Y ist gleich oder verschieden bei jedem Auftreten gleich CR⁷ oder N, bevorzugt ist, wenn Y gleich CR⁷ ist;
L ist ein divalentes aromatisches Ringsystem mit 6 bis 40 aromatischen Ringatomen; vorzugsweise ist L ist eine divalente Gruppe ausgewählt aus den folgenden Formeln (L-1), (L-2), (L-3), (L-4) oder (L-5) 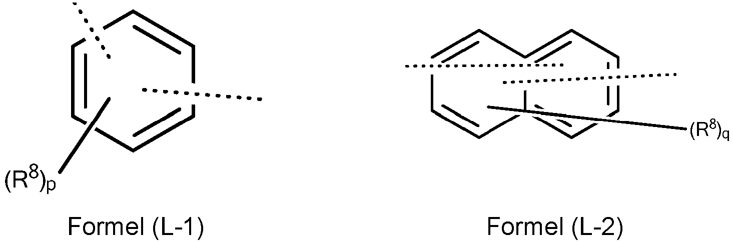 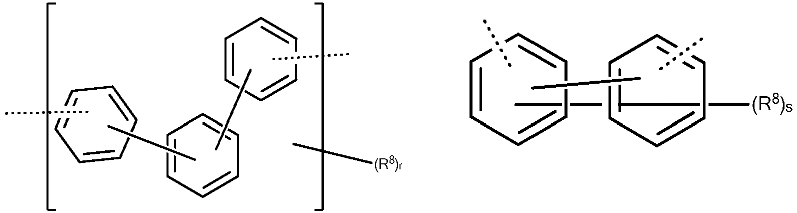 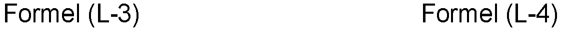 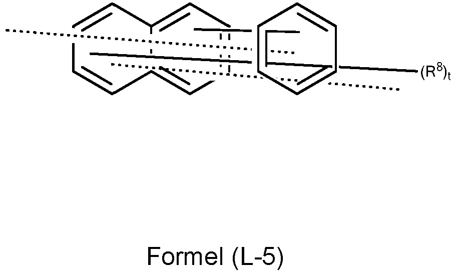
wobei einer der beiden gestrichelten Linien die Bindung der Gruppe L zum Stickstoff einerseits und die andere gestrichelte Linie die Bindung zur Fluorenylgruppe andererseits kennzeichnet und wobei es sich bei den Gruppen der Formeln (L-1), (L-2), (L-3), (L-4) und (L-5) um eine mit einem odere mehreren Resten R⁸ substituierte oder um eine unsubstituierte Phenylen-, Naphthylen-, Terpheylen-, Biphenylen beziehungsweise um eine Naphthyl-Penylgruppe handelt.

Die Schreibweise für die Gruppe R⁸ in Formel (L-2) und in Formel (L-3) bedeutet, dass der oder die Reste R⁸ in beiden Ringen der Formel (L-2) bzw. in allen drei Ringen der Formel (L-3) auftreten kann.
R¹, R⁴, R⁶, R⁷ und R⁸ sind bei jedem Auftreten gleich oder verschieden gewählt aus H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ keinen Ring miteinander bilden und/oder zwei oder mehr Reste R⁴ miteinander verknüpft sein können und einen Ring bilden können und/oder zwei oder mehr Reste R⁶ miteinander verknüpft sein können und einen Ring bilden können und/oder zwei oder mehr Reste R⁷ miteinander verknüpft sein können und einen Ring bilden können und/oder zwei oder mehr Reste R⁸ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; vorzugsweise bilden zwei oder mehr Reste R⁴ keinen Ring miteinander, und/oder zwei oder mehr Reste R⁶ keinen Ring miteinander und/oder zwei oder mehr Reste R⁷ keinen Ring miteinander und/oder zwei oder mehr Reste R⁸ keinen Ring miteinander;
R² und R³ sind bei jedem Auftreten gleich oder verschieden gewählt aus geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die beiden Reste R² and R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden;
R⁵ ist ein aromatisches Ringsystem mit 6 bis 40 aromatischen Ringatomen, das mit einem oder mehreren Resten R¹¹ substituiert sei kann, oder ein heteroaromatisches Ringsystem mit 5 bis 40 aromatischen Ringatomen, das mit einem oder meheren Resten R¹¹ substituiert sein kann, wobei der Rest R⁵ kein kondensiertes heteroaromatisches Ringsystem mit mehr als 12 aromatischen Kohlenstoffatomen als Ringatome im heteroaromatischen Ringsystem enthält;
R¹¹ ist bei jedem Auftreten gleich oder verschieden gewählt aus H, D, F, Cl, Br, I, C(=O)_{R}¹², CN, Si(R¹²)₃, N(R¹²)₂, P(=O)(R¹²)₂, OR¹², S(=O)R¹², S(=O)₂R¹², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹² substituiert sind; und wobei eine oder mehrere CH₂-Gruppen in den genannten Alkyl-, Alkoxy-, Alkenyl- und Alkinylgruppen durch -R¹²C=CR¹²-, -C=C-, Si(R¹²)₂, C=O, C=NR¹², -C(=O)O-, -C(=O)NR¹²-, NR¹², P(=O)(R¹²), -O-, -S-, SO oder SO₂ ersetzt sein können; wobei zwei oder mehr Reste R¹¹ miteinander keinen Ring bilden können;
R¹² ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;
m ist 0, 1, 2, 3 oder 4, bevorzugt ist, wenn m gleich 0 oder 1 ist, ganz bevorzugt ist, wenn m gleich 1 ist und ganz besonders bevorzugt ist, wenn m gleich 0 ist;
n ist 0,1, 2 oder 3, bevorzugt ist, wenn n gleich 0 oder 1 ist, ganz bevorzugt ist, wenn n gleich 1 ist und ganz besonders bevorzugt ist, wenn n gleich 0 ist;
o ist 0,1, 2 oder 3, bevorzugt ist, wenn o gleich 0 oder 1 ist, ganz bevorzugt ist, wenn o gleich 1 ist und ganz besonders bevorzugt ist, wenn o gleich 0 ist;
p ist 0,1, 2, 3 oder 4, bevorzugt ist, wenn p gleich 0 oder 1 ist, ganz bevorzugt ist, wenn p gleich 1 ist und ganz besonders bevorzugt ist, wenn p gleich 0 ist;
q ist 0, 1, 2, 3, 4, 5 oder 6, bevorzugt ist, wenn q gleich 0 oder 1 ist, ganz bevorzugt ist, wenn q gleich 1 ist und ganz besonders bevorzugt ist, wenn q gleich 0 ist;
r ist 0, 1, 2, 3, 4, 5, 7, 8, 9, 10, 11 oder 12, bevorzugt ist, wenn r gleich 0 oder 1 ist, ganz bevorzugt ist, wenn r gleich 1 ist und ganz besonders bevorzugt ist, wenn r gleich 0 ist;.
s ist 0, 1, 2, 3, 4, 5, 7 oder 8, bevorzugt ist, wenn s gleich 0 oder 1 ist, ganz bevorzugt ist, wenn s gleich 1 ist und ganz besonders bevorzugt ist, wenn s gleich 0 ist;
t ist 0, 1, 2, 3, 4, 5, 7, 8, 9 oder 10, bevorzugt ist, wenn t gleich 0 oder 1 ist, ganz bevorzugt ist, wenn t gleich 1 ist und ganz besonders bevorzugt ist, wenn t gleich 0 ist.

In einer bevorzugten Ausführungsform enthält der Rest R¹ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R¹ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R¹ keine Carbazole, insbesondere bevorzugt enthält der Rest R¹ keine kondensierten heteroaromatischen Ringsysteme und am meisten bevorzugt enthählt der Rest R¹ weder aromatische noch heteroaromatische kondensierte Ringsysteme.

In einer bevorzugten Ausführungsform enthält der Rest R² keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R² keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R² keine Carbazole, insbesondere bevorzugt enthält der Rest R² keine kondensierten heteroaromatischen Ringsysteme und am meisten bevorzugt enthählt der Rest R² weder aromatische noch heteroaromatische kondensierte Ringsysteme.

In einer weiteren bevorzugten Ausführungsform enthält der Rest R³ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R³ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R³ keine Carbazole, insbesondere bevorzugt enthält der Rest R³ keine kondensierten heteroaromatischen Ringsysteme und am meisten bevorzugt enthählt der Rest R³ weder aromatische noch heteroaromatische kondensierte Ringsysteme.

In noch einer weiteren bevorzugten Ausführungsform enthält der Rest R⁴ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R⁴ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R⁴ keine Carbazole, insbesondere bevorzugt enthält der Rest R⁴ keine kondensierten heteroaromatischen Ringsysteme und am meisten bevorzugt enthählt der Rest R⁴ weder aromatische noch heteroaromatische kondensierte Ringsysteme.

Gemäß der Erfindung enthält der Rest R⁵ keine kondensierten aromatischen Ringsysteme mit mehr als 12 aromatischen Kohlenstoffatomen als Ringatome im aromatischen Ringsystem; in einer bevorzugten Ausführungsform enthält der Rest R⁵ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R⁵ keine Carbazole, insbesondere bevorzugt enthält der Rest R⁵ keine kondensierten heteroaromatischen Ringsysteme und am meisten bevorzugt enthählt der Rest R⁵ weder aromatische noch heteroaromatische kondensierte Ringsysteme.

In noch einer weiteren bevorzugten Ausführungsform enthält der Rest R⁶ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R⁶ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R⁶ keine Carbazole, insbesondere bevorzugt enthält der Rest R⁶ keine kondensierten heteroaromatischen Ringsysteme und am meisten bevorzugt enthählt der Rest R⁶ weder aromatische noch heteroaromatische kondensierte Ringsysteme.

In noch einer weiteren bevorzugten Ausführungsform enthält der Rest R⁷ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R⁷ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R⁷ keine Carbazole, insbesondere bevorzugt enthält der Rest R⁷ keine kondensierten heteroaromatischen Ringsysteme und am meisten bevorzugt enthählt der Rest R⁷ weder aromatische noch heteroaromatische kondensierte Ringsysteme.

In noch einer weiteren bevorzugten Ausführungsform enthält der Rest R⁸ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R⁸ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R⁸ keine Carbazole, insbesondere bevorzugt enthält der Rest R⁸ keine kondensierten heteroaromatischen Ringsysteme und am meisten bevorzugt enthählt der Rest R⁸ weder aromatische noch heteroaromatische kondensierte Ringsysteme.

In noch einer weiteren bevorzugten Ausführungsform enthält der Rest R¹¹ keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R¹¹ keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R¹¹ keine Carbazole, insbesondere bevorzugt enthält der Rest R¹¹ keine kondensierten heteroaromatischen Ringsysteme und am meisten bevorzugt enthählt der Rest R¹¹ weder aromatische noch heteroaromatische kondensierte Ringsysteme.

In noch einer weiteren bevorzugten Ausführungsform enthält der Rest R¹² keine kondensierten aromatischen und/oder heteroaromatischen Ringsysteme mit mehr als 12 aromatischen Kohlenstoffatomen als Ringatome im aromatischen oder heteroaromatischen Ringsystem; ganz bevorzugt enthält der Rest R¹² keine kondensierten aromatischen und/oder heteroaromatischen Ringsysteme mit mehr als 10 aromatischen Kohlenstoffatomen als Ringatome im aromatischen oder heteroaromatischen Ringsystem; ganz besonders bevorzugt enthält der Rest R¹² keine Carbazole, insbesondere bevorzugt enthält der Rest R¹² keine kondensierten heteroaromatischen Ringsysteme und am meisten bevorzugt enthählt der Rest R¹² weder aromatische noch heteroaromatische kondensierte Ringsysteme.

In einer besonders bevorzugten Ausführungsform enthält keiner der Reste R¹ bis R⁸ kondensierte aromatische und/oder heteroaromatische Ringsysteme mit mehr als 12 aromatischen Kohlenstoffatomen als Ringatome im aromatischen oder heteroaromatischen Ringsystem; ganz bevorzugt enthält keiner der Reste R¹ bis R⁸ kondensierte aromatische und/oder heteroaromatischen Ringsysteme mit mehr als 10 aromatischen Kohlenstoffatomen als Ringatome im aromatischen oder heteroaromatischen Ringsystem; ganz besonders bevorzugt enthält keiner der Reste R¹ bis R⁸ Carbazole, insbesondere bevorzugt enthält keiner der Reste R¹ bis R⁸ kondensierte heteroaromatische Ringsysteme und am meisten bevorzugt enthählt keiner der Reste R¹ bis R⁸ aromatische oder heteroaromatische kondensierte Ringsysteme.

In einer besonders bevorzugten Ausführungsform enthält keiner der Reste R¹ bis R⁸, R¹¹ und R¹² kondensierte aromatische und/oder heteroaromatische Ringsysteme mit mehr als 12 aromatischen Kohlenstoffatomen als Ringatome im aromatischen oder heteroaromatischen Ringsystem; ganz bevorzugt enthält keiner der Reste R¹ bis R⁸, R¹¹ und R¹² kondensierte aromatische und/oder heteroaromatischen Ringsysteme mit mehr als 10 aromatischen Kohlenstoffatomen als Ringatome im aromatischen oder heteroaromatischen Ringsystem; ganz besonders bevorzugt enthält keiner der Reste R¹ bis R⁸, R¹¹ und R¹² Carbazole, insbesondere bevorzugt enthält keiner der Reste R¹ bis R⁸, R¹¹ und R¹² kondensierte heteroaromatische Ringsysteme und am meisten bevorzugt enthählt keiner der Reste R¹ bis R⁸, R¹¹ und R¹² aromatische oder heteroaromatische kondensierte Ringsysteme.

Die folgenden Definitionen gelten für die chemischen Gruppen, die in der vorliegenden Anmeldungen verwendet werden. Sie gelten, soweit keine spezielleren Definitionen angegeben sind.

Unter einer Arylgruppe im Sinne dieser Erfindung wird entweder ein einzelner aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einzelnen aromatischen Cyclen. Unter Kondensation zwischen Cyclen ist dabei zu verstehen, dass die Cyclen mindestens eine Kante miteinander teilen. Eine Arylgruppe im Sinne dieser Erfindung enthält 6 bis 40 aromatische Ringatome, von denen keines ein Heteroatom ist.

Unter einer Heteroarylgruppe im Sinne dieser Erfindung wird entweder ein einzelner heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einzelnen aromatischen oder heteroaromatischen Cyclen, wobei wenigstens einer der aromatischen und heteroaromatischen Cyclen ein heteroaromatischer Cyclus ist. Unter Kondensation zwischen Cyclen ist dabei zu verstehen, dass die Cyclen mindestens eine Kante miteinander teilen. Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus **N, O** und S.

Unter einer Aryl- oder Heteroarylgruppe, die jeweils mit den oben genannten Resten substituiert sein kann werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Benzimidazolo[1,2-a]benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung ist ein System, welches nicht notwendigerweise nur Arylgruppen enthält, sondern welches zusätzlich einen oder mehrere nicht-aromatische Ringe enthalten kann, die mit wenigstens einer Arylgruppe kondensiert sind. Diese nicht-aromatischen Ringe enthalten ausschließlich Kohlenstoffatome als Ringatome. Beispiele für Gruppen, die von dieser Definition umfasst sind, sind Tetrahydronaphthalin, Fluoren und Spirobifluoren. Weiterhin umfasst der Begriff aromatisches Ringsystem Systeme, die aus zwei oder mehr aromatischen Ringsystemen bestehen, die über Einfachbindungen miteinander verbunden sind, beispielsweise Biphenyl, Terphenyl, 7-Phenyl-2-fluorenyl, Quaterphenyl und 3,5-Diphenyl-1-phenyl. Ein aromatisches Ringsystem im Sinne dieser Erfindung enthält 6 bis 40 C-Atome und keine Heteroatome im Ringsystem. Die Definition von "aromatisches Ringsystem" umfasst nicht Heteroarylgruppen.

Ein heteroaromatisches Ringsystem entspricht der oben genannten Definition eines aromatischen Ringsystems, mit dem Unterschied dass es mindestens ein Heteroatom als Ringatom enthalten muss. Wie es beim aromatischen Ringsystem der Fall ist, muss das heteroaromatische Ringsystem nicht ausschließlich Arylgruppen und Heteroarylgruppen enthalten, sondern es kann zusätzlich einen oder mehrere nicht-aromatische Ringe enthalten, die mit wenigstens einer Aryl- oder Heteroarylgruppe kondensiert sind. Die nicht-aromatischen Ringe können ausschließlich C-Atome als Ringatome enthalten, oder sie können zusätzlich ein oder mehrere Heteroatome enthalten, wobei die Heteroatome bevorzugt gewählt sind aus **N,** O und S. Ein Beispiel für ein derartiges heteroaromatisches Ringsystem ist Benzopyranyl. Weiterhin werden unter dem Begriff "heteroaromatisches Ringsystem" Systeme verstanden, die aus zwei oder mehr aromatischen oder heteroaromatischen Ringsystemen bestehen, die miteinander über Einfachbindungen verbunden sind, wie beispielsweise 4,6-Diphenyl-2-triazinyl. Ein heteroaromatisches Ringsystem im Sinne dieser Erfindung enthält 5 bis 40 Ringatome, die gewählt sind aus Kohlenstoff und Heteroatomen, wobei mindestens eines der Ringatome ein Heteroatom ist. Die Heteroatome des heteroaromatischen Ringsystems sind bevorzugt ausgewählt aus N, O und S.

Die Begriffe "heteroaromatisches Ringsystem" und "aromatisches Ringsystem" gemäß der Definition der vorliegenden Anmeldung unterscheiden sich damit dadurch voneinander, dass ein aromatisches Ringsystem kein Heteroatom als Ringatom aufweisen kann, während ein heteroaromatisches Ringsystem mindestens ein Heteroatom als Ringatom aufweisen muss. Dieses Heteroatom kann als Ringatom eines nicht-aromatischen heterocyclischen Rings oder als Ringatom eines aromatischen heterocyclischen Rings vorliegen.

Entsprechend der obenstehenden Definitionen ist jede Arylgruppe vom Begriff "aromatisches Ringsystem" umfasst, und jede Heteroarylgruppe ist vom Begriff "heteroatomatisches Ringsystem" umfasst.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Die Verbindung der Formel (3) ist bevorzugt ein Monoamin. Unter einem Monoamin wird eine Verbindung verstanden, die eine einzige Triarylaminogruppe enthält, und keine weiteren Triarylaminogruppen, besonders bevorzugt eine Verbindung, die eine einzige Aminogruppe enthält, und keine weiteren Aminogruppen.

In einer bezorzugten Ausführungsform ist die Gruppe L eine Gruppe der Formel (L-1).

In einer anderen bevorzugten Ausführungsform ist die Gruppe L eine Gruppe der Formel (L-2).

Und in noch einer anderen bevorzugten Ausführungsform ist die Gruppe L eine Gruppe der Formel (L-3), wobei unter den Gruppen der Formel (L-3) die folgenden Gruppen ganz bevorzugt sind.

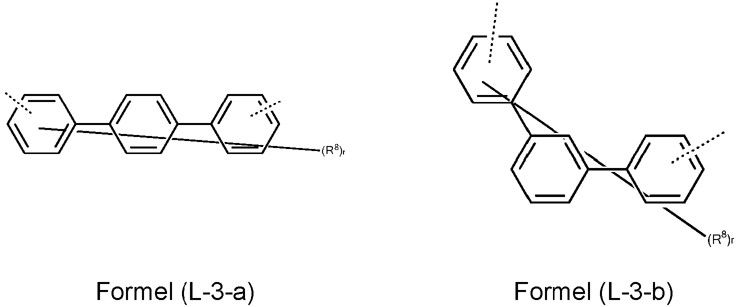

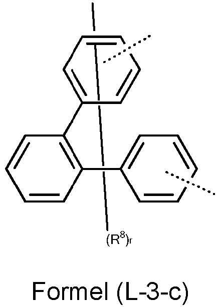

Ganz analog zu der obigen Beschreibung der Formel (L-3) bedeutet die Schreibweise in den Formeln (L-3-a) bis (L-3-c), dass die Reste R⁸ in allen drei aromatischen Ringen auftreten können.

In einer anderen bevorzugten Ausführungsform ist die Gruppe L eine Gruppe der Formel (L-4).

In einer anderen bevorzugten Ausführungsform ist die Gruppe L eine Gruppe der Formel (L-5).

L ist ganz besonders bevorzugt ausgewählt aus den folgenden Gruppen

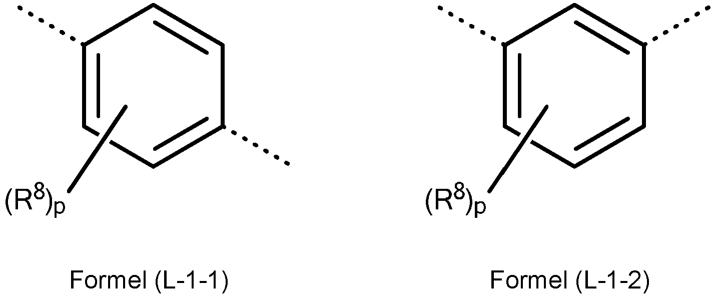

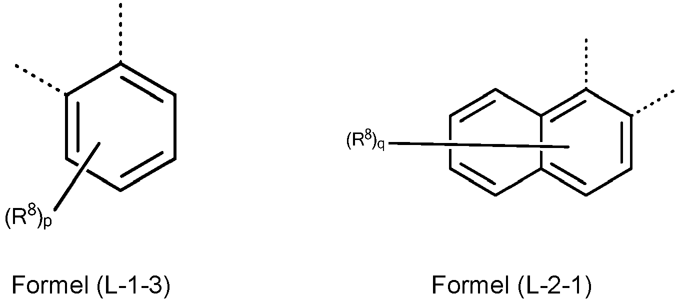

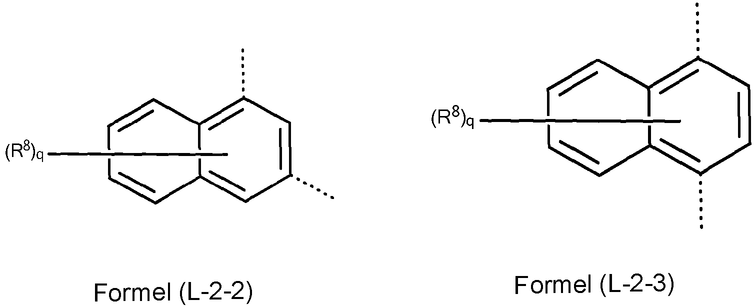

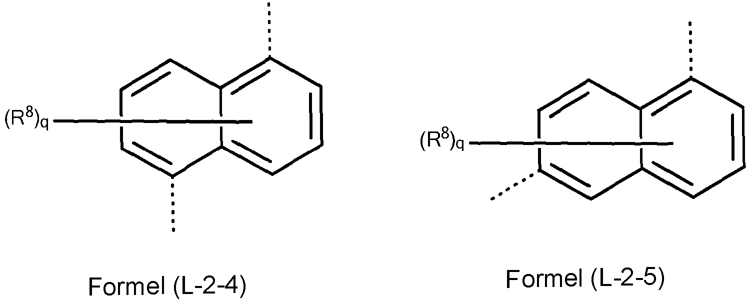

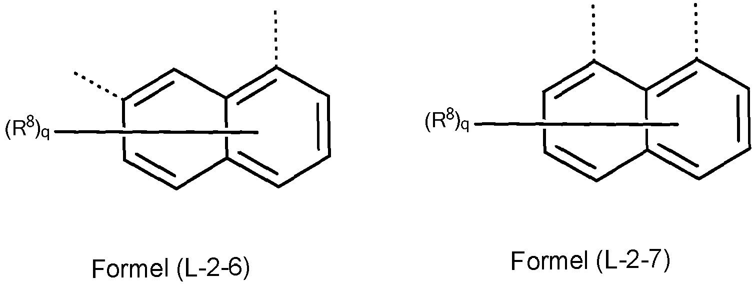

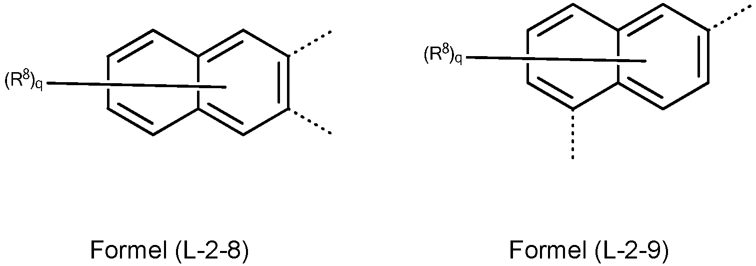

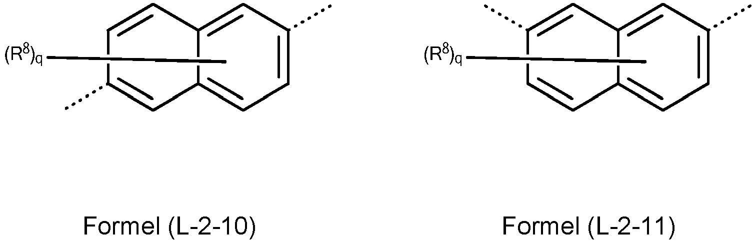

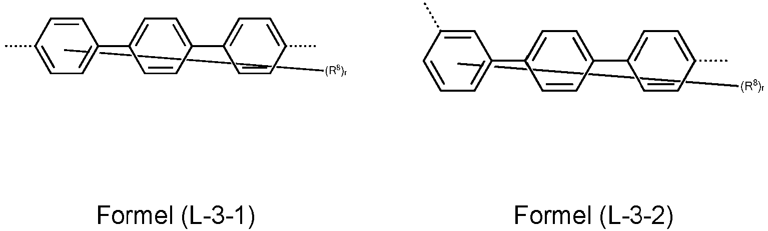

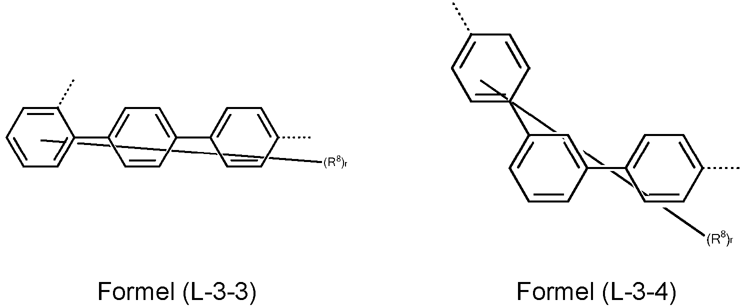

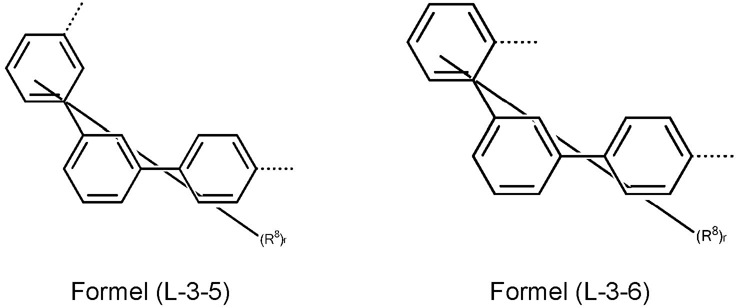

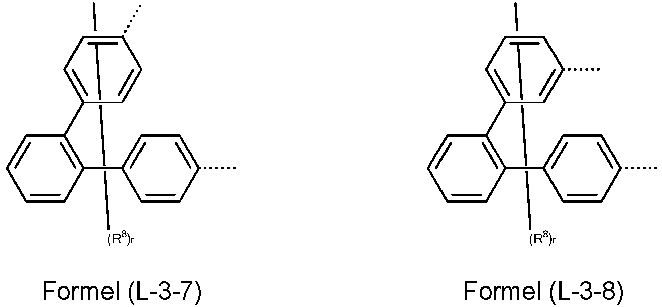

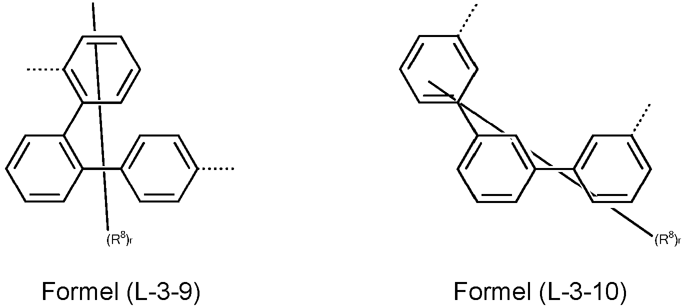

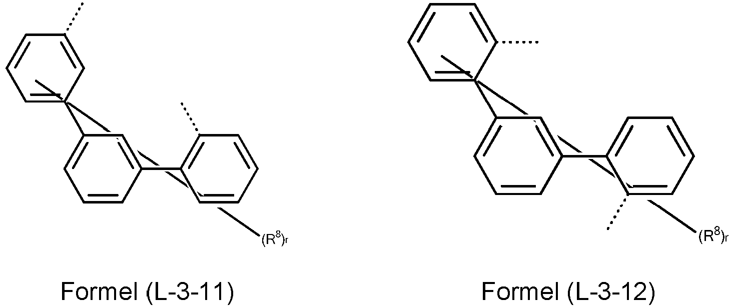

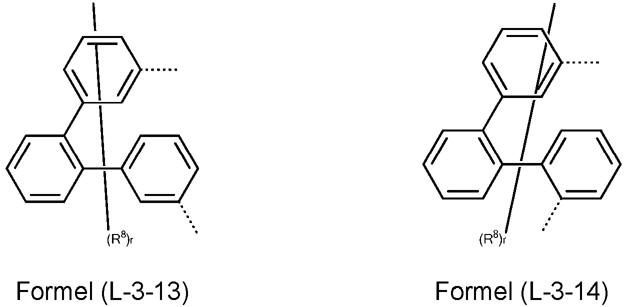

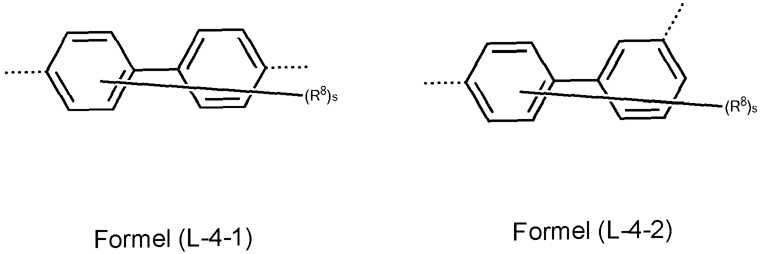

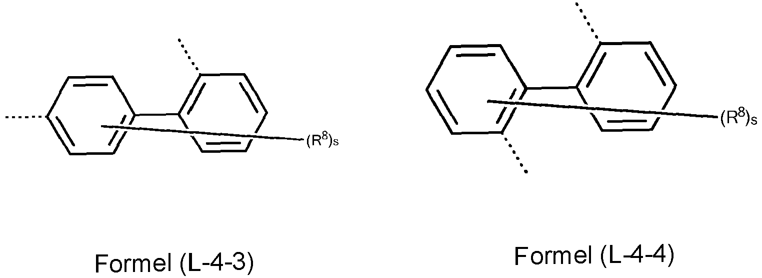

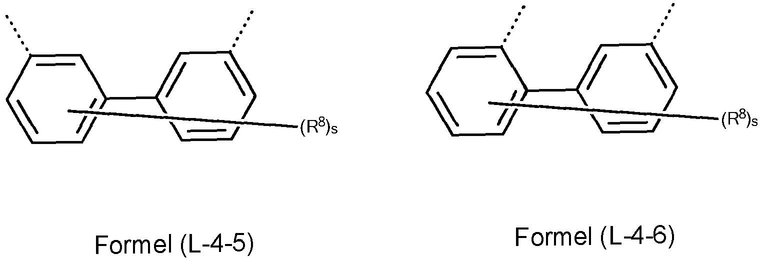

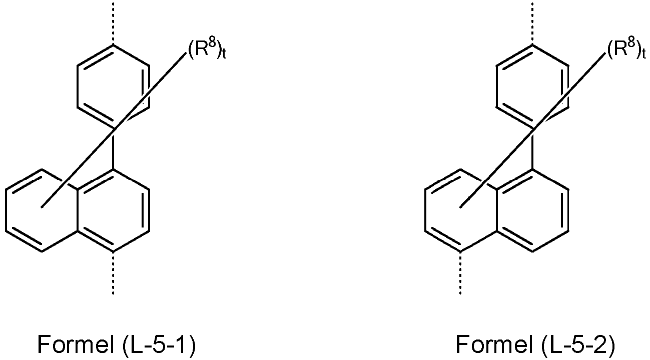

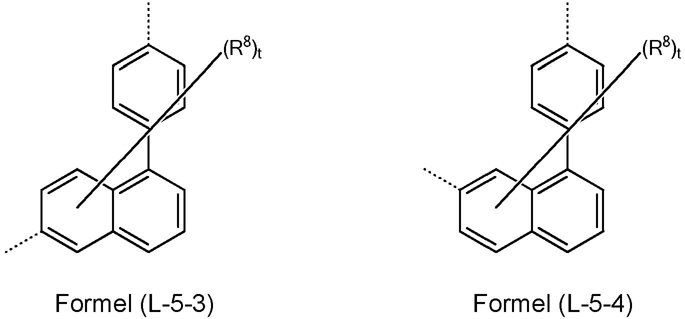

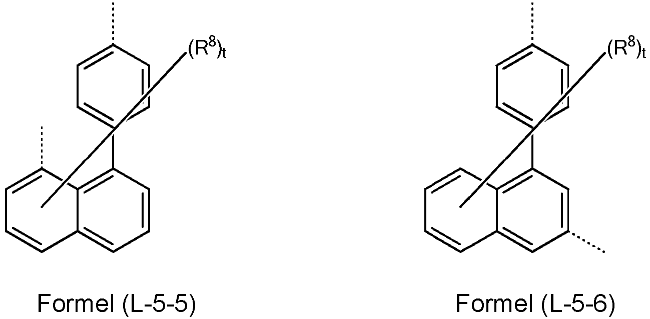

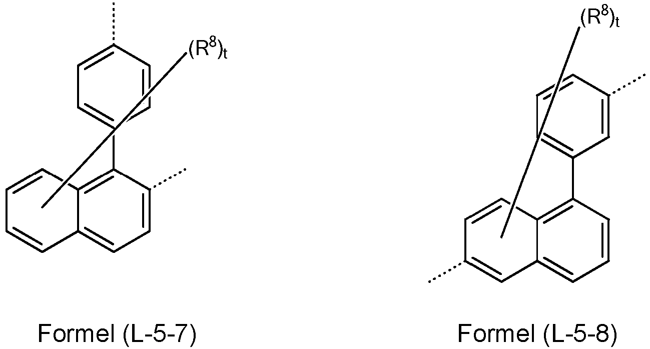

Davon sind insbesondere bevorzugte L Gruppen solche der Formeln (L-1-1) bis (L-1-3).

Weitere insbesondere bevorzugte L Gruppen solche der Formeln (L-2-1) bis (L-2-11).

Weitere insbesondere bevorzugte L Gruppen solche der Formeln (L-3-1) bis (L-3-14).

Weitere insbesondere bevorzugte L Gruppen solche der Formeln (L-4-1) bis (L-4-6).

Weitere insbesondere bevorzugte L Gruppen solche der Formeln (L-5-1) bis (L-5-8).

Eine bevorzugte erfindungsgemäße Verbindungen ist die folgende Formel (7).

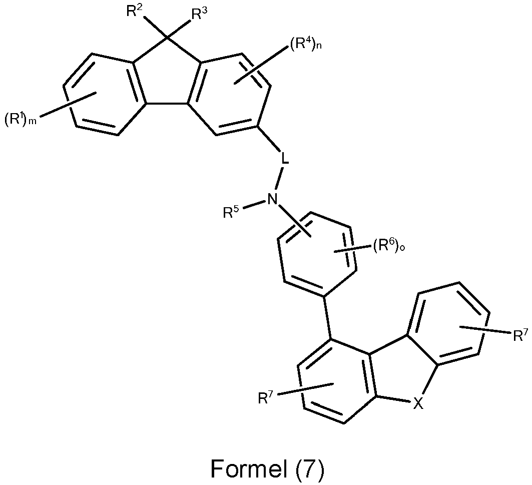

Ganz besonders bevorzugte erfindungsgemäße Verbindungen sind solche der Formeln (11) bis (13).

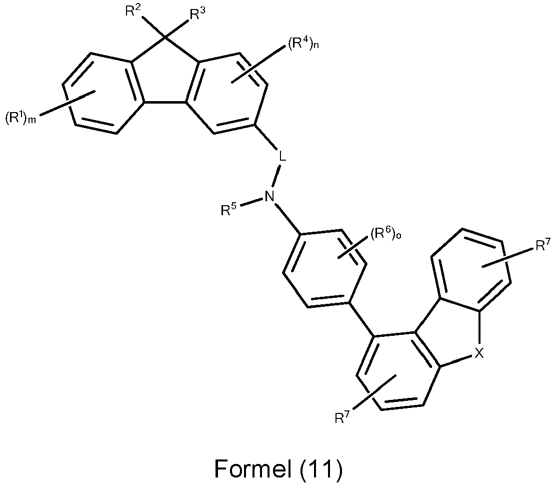

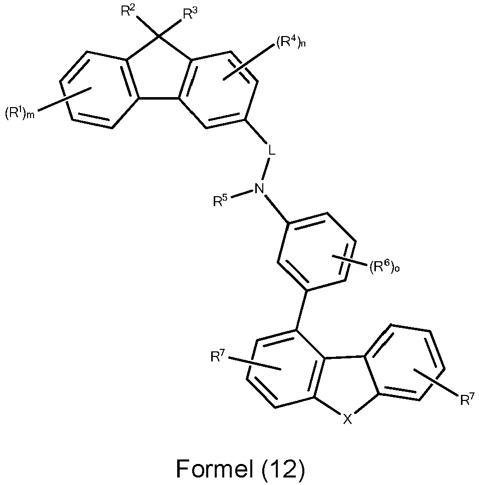

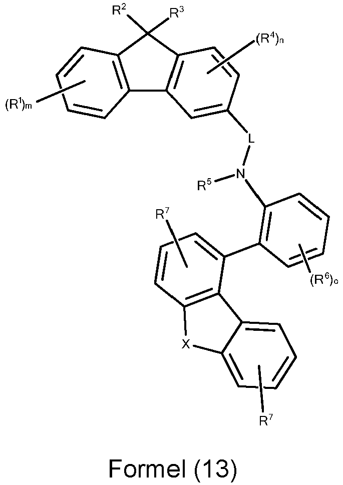

Noch mehr bevorzugte erfindungsgemäße Verbindungen sind solche der Formeln (23) bis (25).

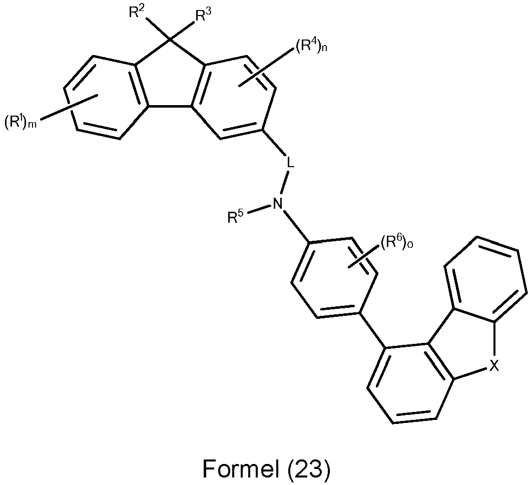

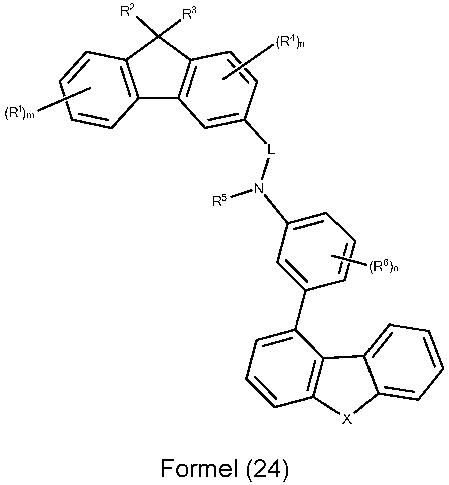

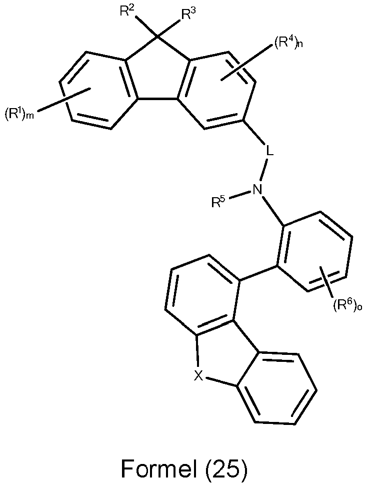

Eine bevorzugte Ausführungsform der vorliegenden Erfindung der Formel (3) betrifft Verbindungen der Formel (7), besonders bevorzugt der Formeln (11) bis (13), ganz besonders bevorzugt der Formeln (23) bis (25), insbesondere bevorzugt der Formel (11) und am meisten bevorzugt der Formel (23), wobei die Gruppe L in den genannten Verbindungen die Formel (L-1), vorzugsweise eine der Formeln (L-1-1) bis (L-1-3) und ganz bevorzugt die Formel (L-1-1) aufweist.

Ferner ist bevorzugt, wenn in den zuvor genannten Verbindungen die Reste R² und R³ gleich oder verschieden bei jedem Auftreten ausgewählt sind aus geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die beiden Reste R² and R³ miteinander verknüpft sein können und einen Ring bilden können; wobei die genannten Alkylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden.

Noch bevorzugter ist es im Sinne der vorliegenden Erfindung, wenn in den zuvor genannten Verbindungen die Reste R² und R³ gleich oder verschieden bei jedem Auftreten ausgewählt sind aus geradkettigen Alkylgruppen mit 1 bis 10 C-Atomen, verzweigten oder cyclischen Alkylgruppen mit 3 bis 10 C-Atomen, aromatischen Ringsystemen mit 6 bis 18 aromatischen Ringatomen, und heteroaromatischen Ringsystemen mit 5 bis 18 aromatischen Ringatomen; wobei die beiden Reste R² and R³ miteinander verknüpft sein können und einen Ring bilden können; wobei die genannten Alkylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden.

Noch mehr bevorzugt im Sinne der vorliegenden Erfindung ist, wenn in den zuvor genannten Verbindungen die Reste R² und R³ gleich oder verschieden bei jedem Auftreten ausgewählt sind aus geradkettigen Alkylgruppen mit 1 bis 10 C-Atomen, verzweigten oder cyclischen Alkylgruppen mit 3 bis 10 C-Atomen oder aromatischen Ringsystemen mit 6 bis 18 aromatischen Ringatomen; wobei die beiden Reste R² and R³ miteinander verknüpft sein können und einen Ring bilden können; wobei die genannten Alkylgruppen und die genannten aromatischen Ringsysteme jeweils mit Resten R¹¹ substituiert sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden.

Insbesondere bevorzugt im Sinne der vorliegenden Erfindung ist, wenn in den zuvor genannten Verbindungen die Reste R² und R³ gleich oder verschieden bei jedem Auftreten ausgewählt sind aus geradkettigen Alkylgruppen mit 1 bis 10 C-Atomen, verzweigten oder cyclischen Alkylgruppen mit 3 bis 10 C-Atomen oder aromatischen Ringsystemen mit 6 bis 18 aromatischen Ringatomen; wobei die genannten Alkylgruppen und die genannten aromatischen Ringsysteme jeweils mit Resten R¹¹ substituiert sein können; vorzugsweise liegen die beiden Reste R² und R³ unsubstituiert vor.

Insbesondere bevorzugt im Sinne der vorliegenden Erfindung ist ferner, wenn in den zuvor genannten Verbindungen die Reste R² und R³ gleich oder verschieden bei jedem Auftreten ausgewählt sind aus geradkettigen Alkylgruppen mit 1 bis 10 C-Atomen oder aromatischen Ringsystemen mit 6 bis 18 aromatischen Ringatomen; wobei die genannten Alkylgruppen und die genannten aromatischen Ringsysteme jeweils mit Resten R¹¹ substituiert sein können; vorzugsweise liegen die beiden Reste R² und R³ unsubstituiert vor.

Ganz besonders bevorzugt im Sinne der vorliegenden Erfindung ist, wenn in den zuvor genannten Verbindungen die Reste R² und R³ gleich oder verschieden, bei jedem Auftreten ausgewählt sind aus geradkettigen Alkylgruppen mit 1 bis 5 C-Atomen, wobei Methylgruppen am meisten bevorzugt sind.

Ganz besonders bevorzugt im Sinne der vorliegenden Erfindung ist, wenn in den zuvor genannten Verbindungen die Reste R² und R³ gleich oder verschieden bei jedem Auftreten ausgewählt sind aromatischen Ringsystemen mit 6 bis 12 aromatischen Ringatomen; wobei Phenylgruppen am meisten bevorzugt sind.

In einer bevorzugten Ausführungsform der vorliegenden Erfindung sind R² und R³ gleich.

In noch einer bevorzugten Ausführungsform der vorliegenden Erfindung sind R² und R³ unterschiedlich.

Bevorzugt sind Reste R⁵ gewählt aus monovalenten Gruppen abgeleitet von Benzol, Biphenyl, Terphenyl, Quaterphenyl, Naphthalin, Fluoren, insbesondere 9,9'-Dimethylfluoren und 9,9'-Diphenylfluoren, 9-Sila-Fluoren, insbesondere 9,9'-Dimethyl-9-silafluoren und 9,9'-Diphenyl-9-silafluoren, Benzofluoren, Spirobifluoren, Indenofluoren, Dibenzofuran, Dibenzothiophen, Benzocarbazol, Carbazol, Benzofuran, Benzothiophen, Indol, Chinolin, Pyridin, Pyrimidin, Pyrazin, Pyridazin, und Triazin, wobei die monovalenten Gruppen jeweils mit einem oder mehreren Resten R¹¹ substituiert sind. Alternativ bevorzugt kann die Gruppe R⁵ gewählt sein aus Kombinationen von Gruppen, die abgeleitet sind von Benzol, Biphenyl, Terphenyl, Quaterphenyl, Naphthalin, Fluoren, insbesondere 9,9'-Dimethylfluoren und 9,9'-Diphenylfluoren, 9-Sila-Fluoren, insbesondere 9,9'-Dimethyl-9-silafluoren und 9,9'-Diphenyl-9-silafluoren, Benzofluoren, Spirobifluoren, Indenofluoren, Indenocarbazol, Dibenzofuran, Dibenzothiophen, Carbazol, Benzofuran, Benzothiophen, Indol, Chinolin, Pyridin, Pyrimidin, Pyrazin, Pyridazin und Triazin, wobei die Gruppen jeweils mit einem oder mehreren Resten R¹¹ substituiert sind.

Besonders bevorzugte Gruppen R⁵ sind gewählt aus Phenyl, Biphenyl, Terphenyl, Quaterphenyl, Naphthyl, Fluorenyl, insbesondere 9,9'-Dimethylfluorenyl und 9,9'-Diphenylfluorenyl, Benzofluorenyl, Spirobifluorenyl, Indenofluorenyl, Dibenzofuranyl, Dibenzothiophenyl, Carbazolyl, Benzofuranyl, Benzothiophenyl, benzokondensiertem Dibenzofuranyl, benzokondensiertem Dibenzothiophenyl, Naphthylsubstituiertem Phenyl, Fluorenyl-substituiertem Phenyl, Spirobifluorenylsubstituiertem Phenyl, Dibenzofuranyl-substituiertem Phenyl, Dibenzothiophenyl-substituiertem Phenyl, Carbazolyl-substituiertem Phenyl, Pyridyl-substituiertem Phenyl, Pyrimidyl-substituiertem Phenyl, und Triazinyl-substituiertem Phenyl, wobei die genannten Gruppen jeweils mit einem oder mehreren Resten R¹¹ substituiert sind.

Besonders bevorzugte Gruppen für R⁵ sind gewählt aus den folgenden Formeln: 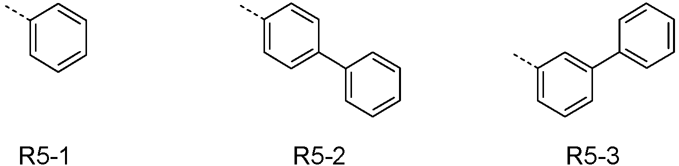 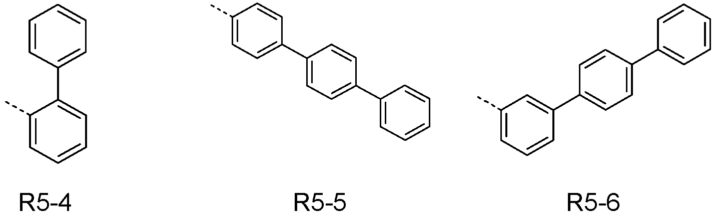 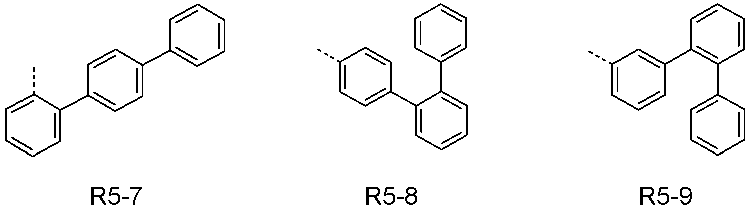 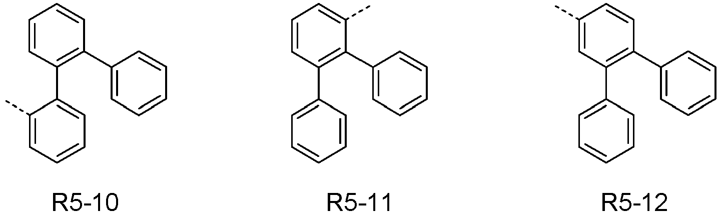 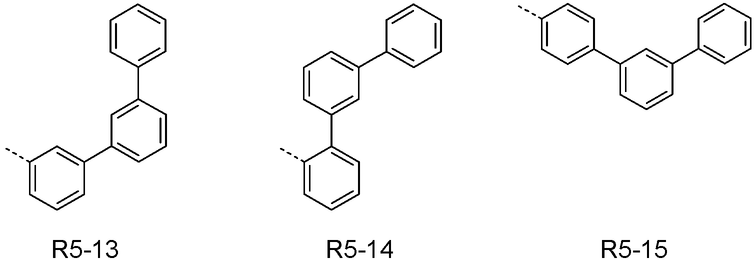 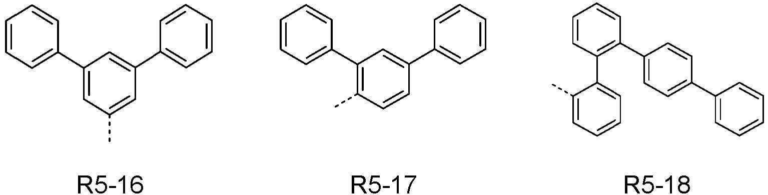 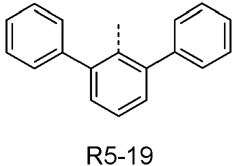 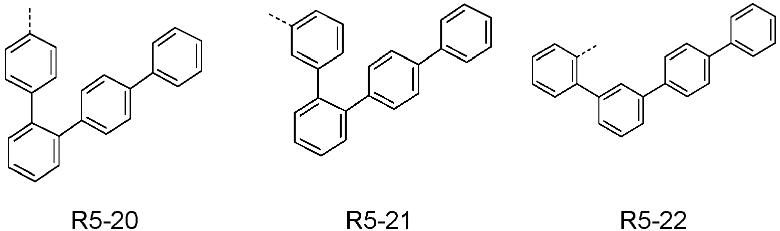 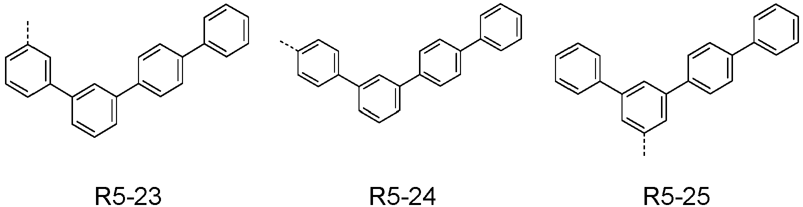 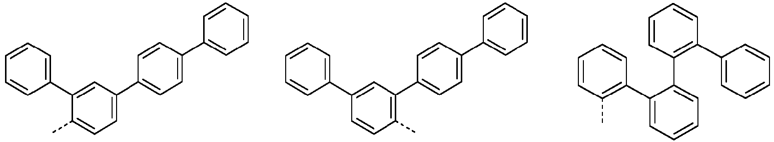 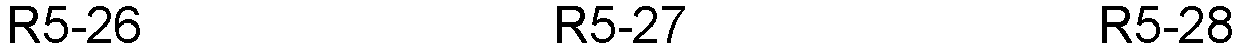 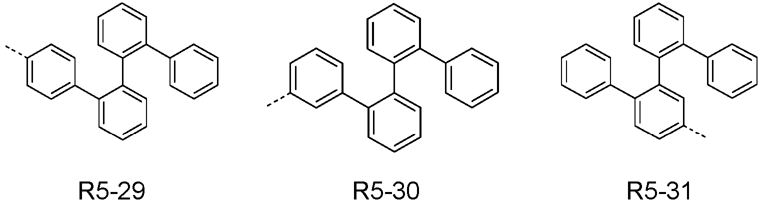 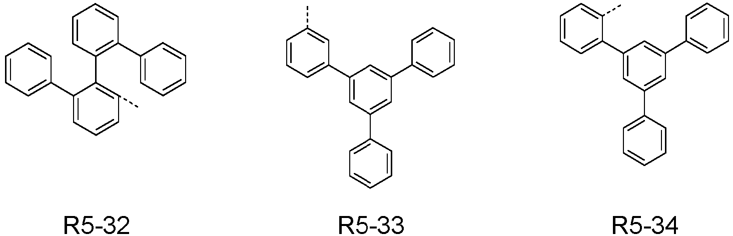 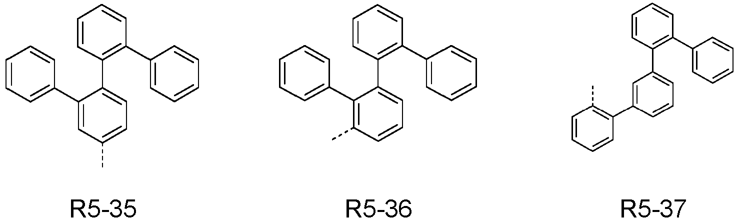 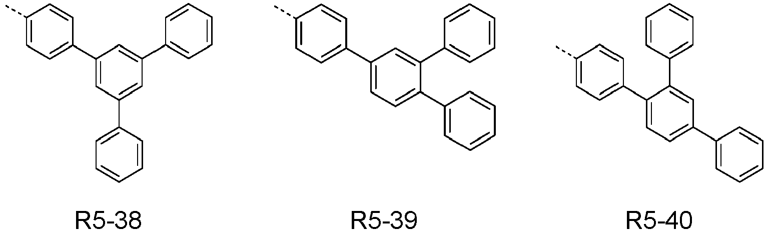 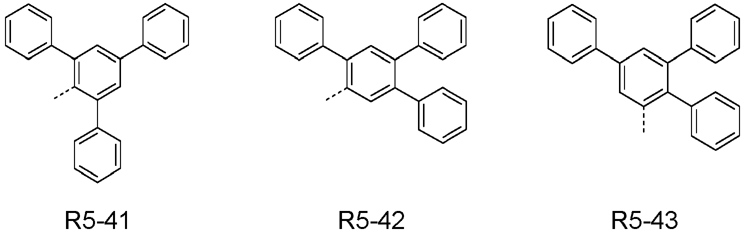 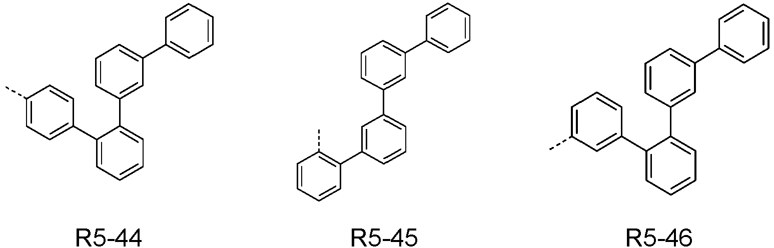 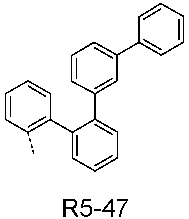 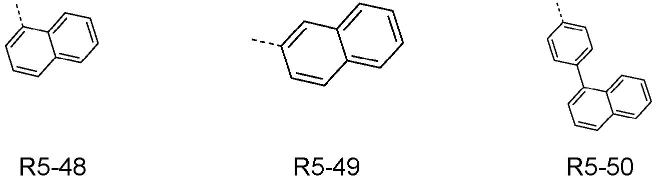 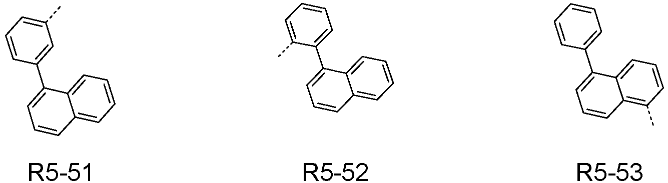 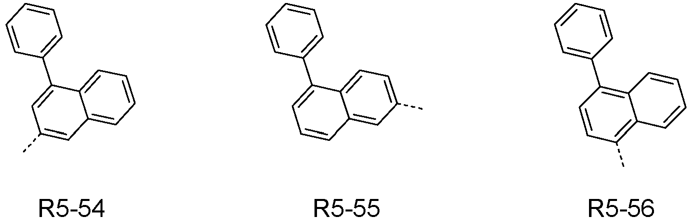 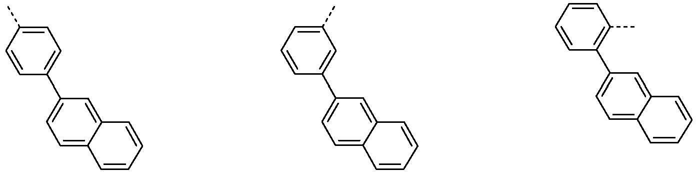 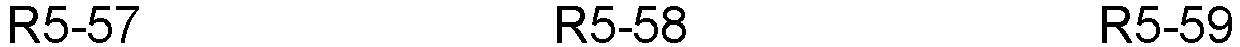 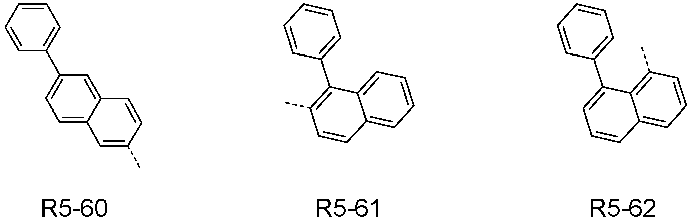 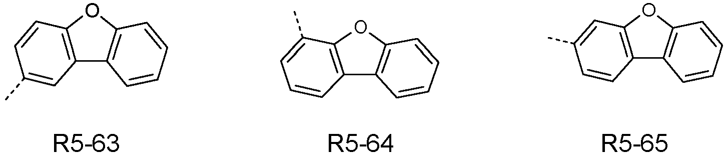 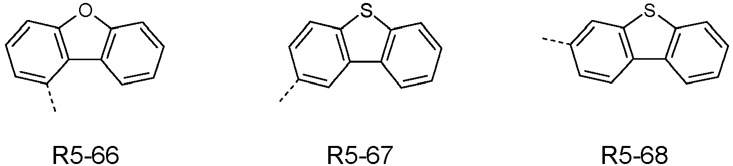 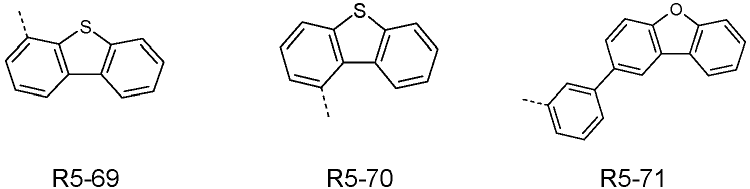 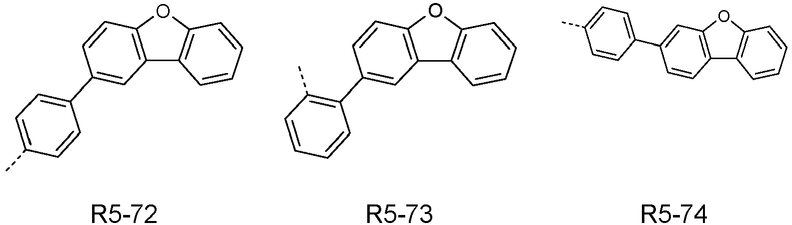 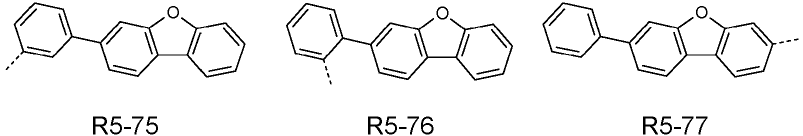 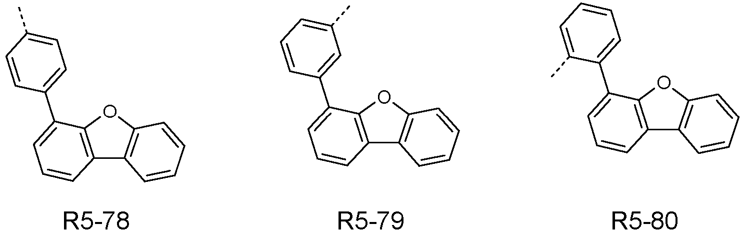 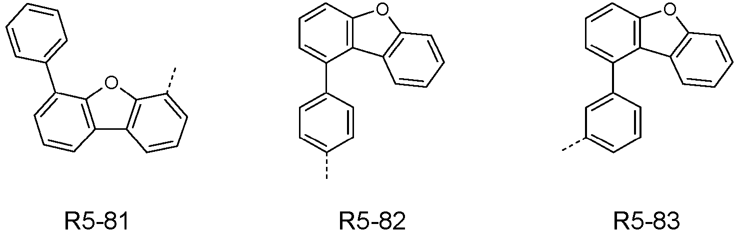 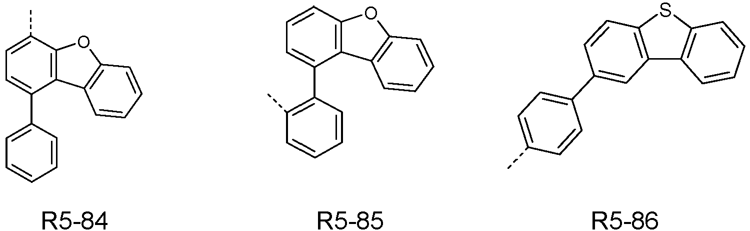 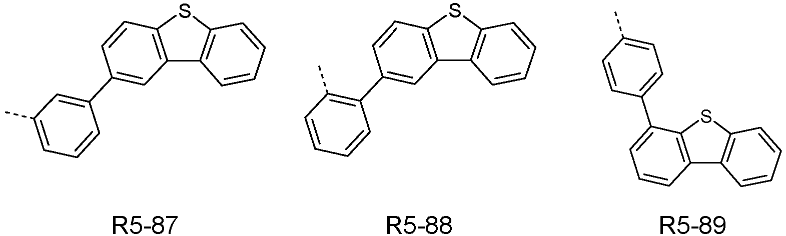 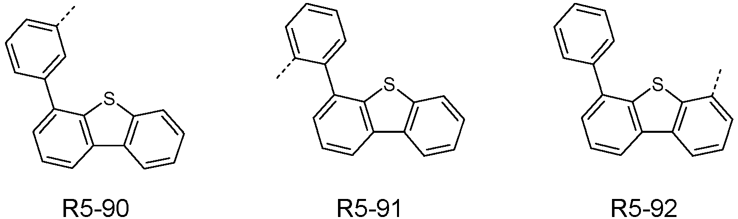 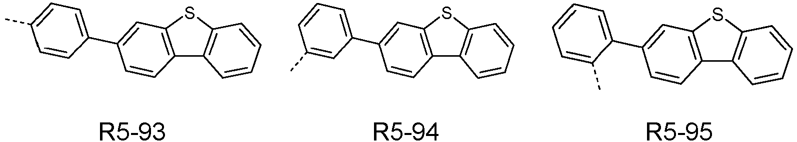 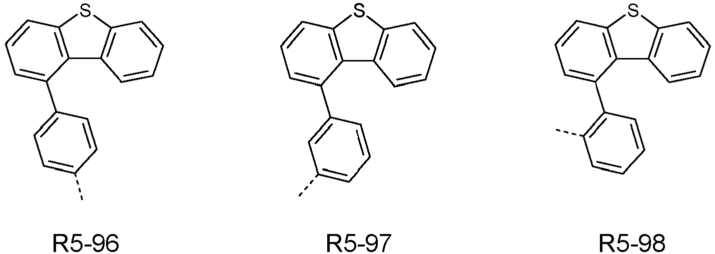 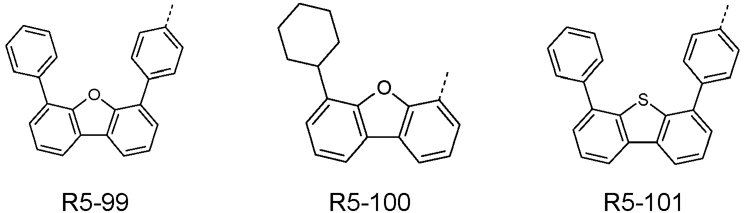 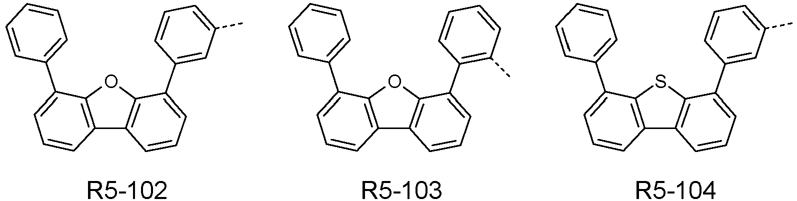 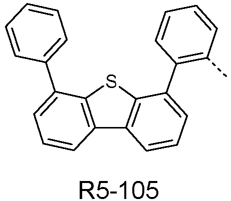 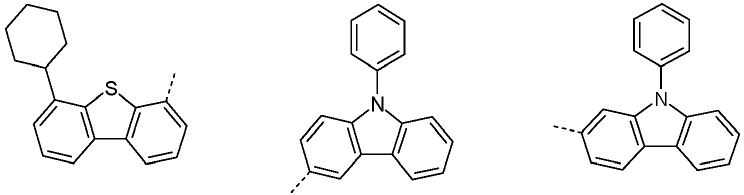 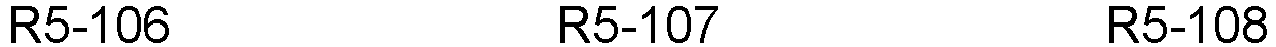 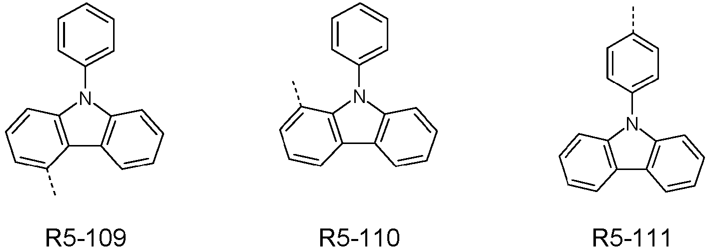 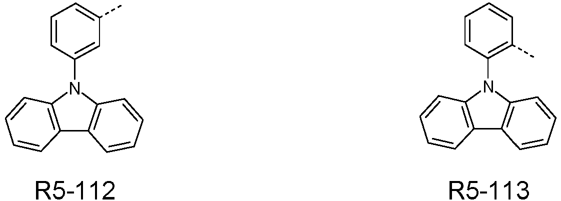 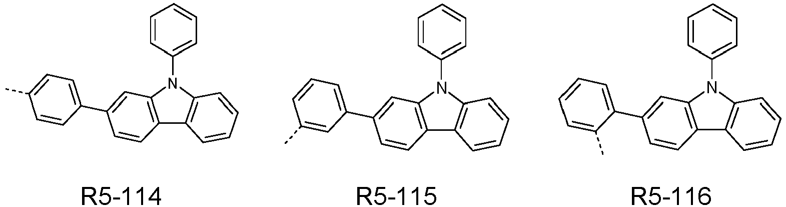 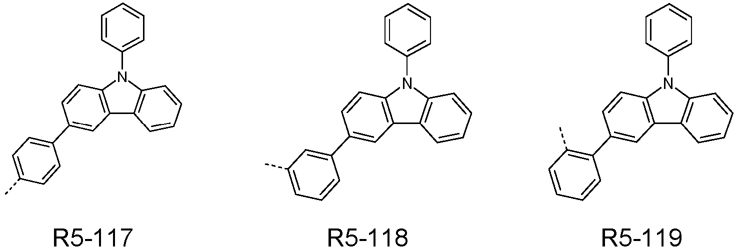 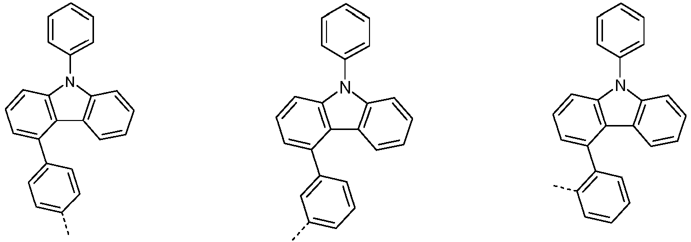 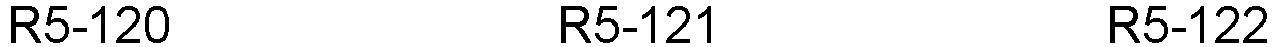 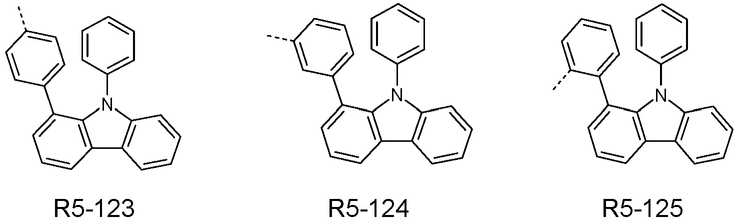 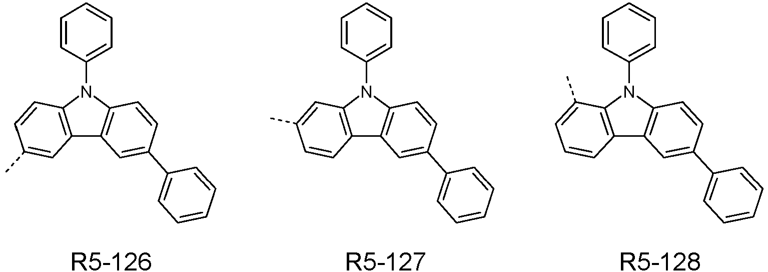 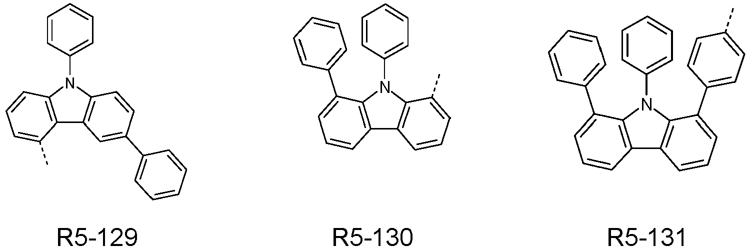 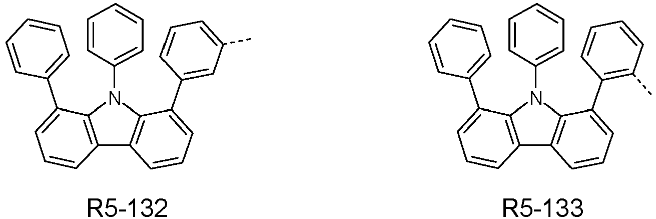 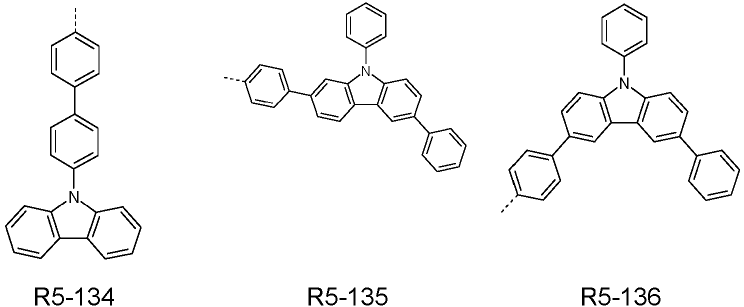 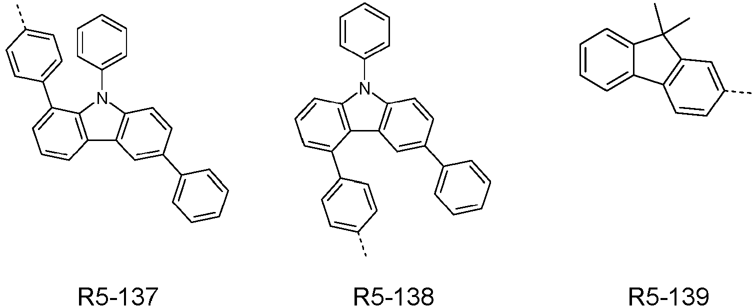 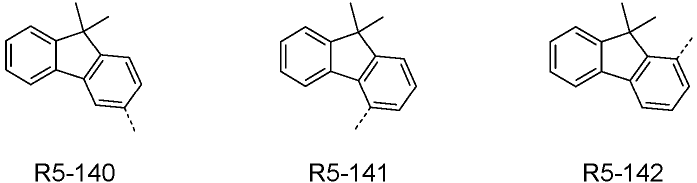 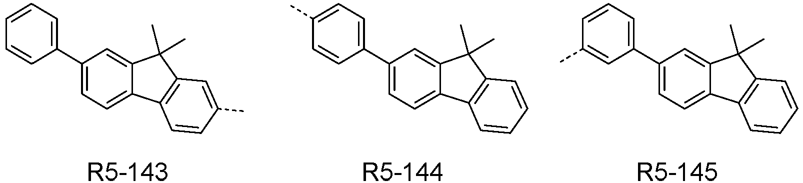 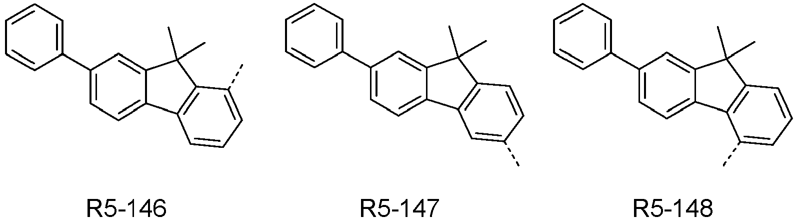 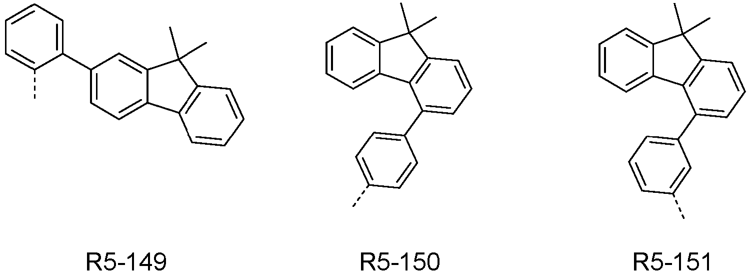 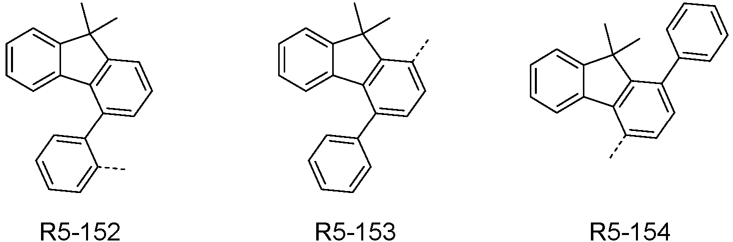 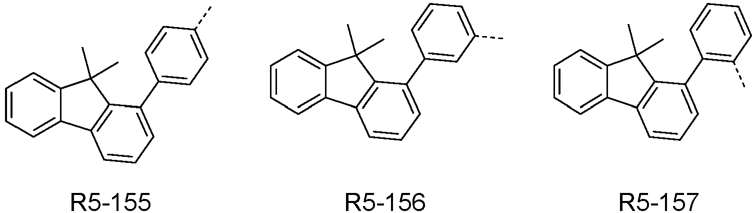 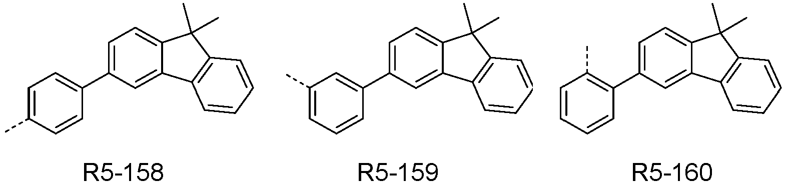 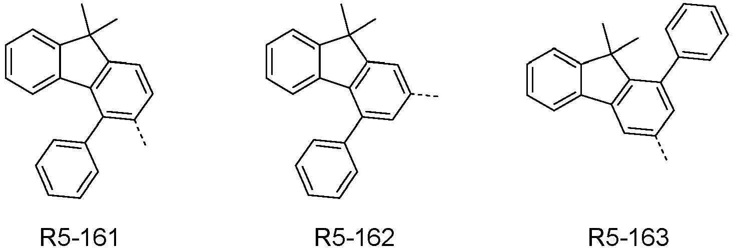 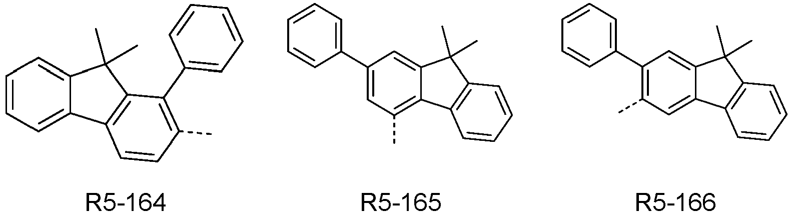 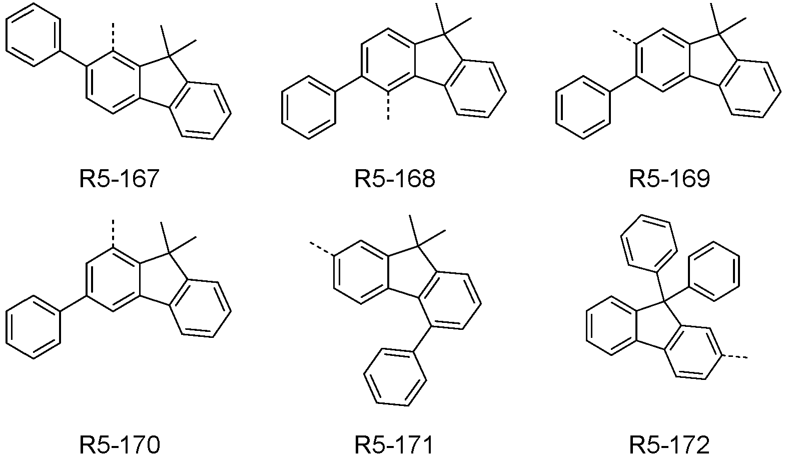 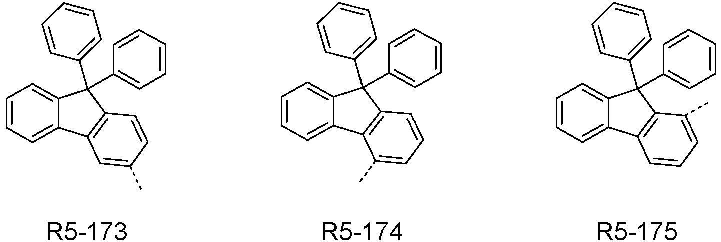 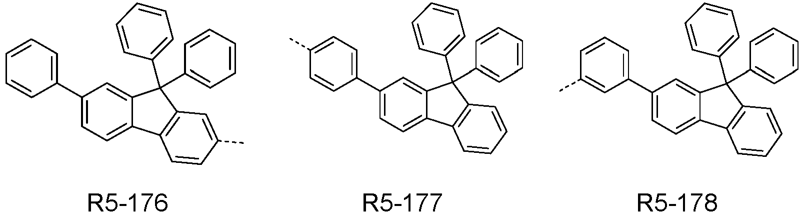 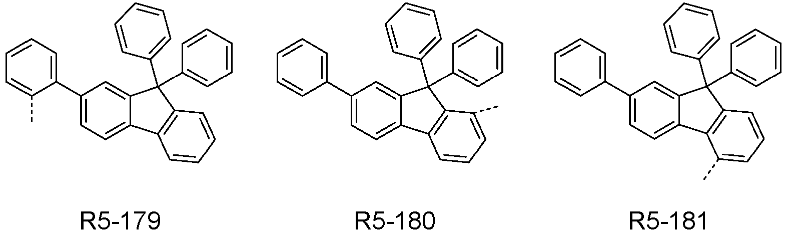 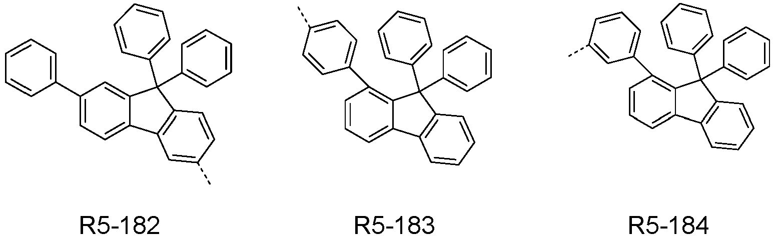 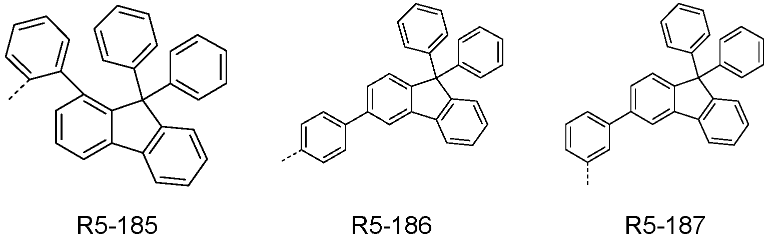 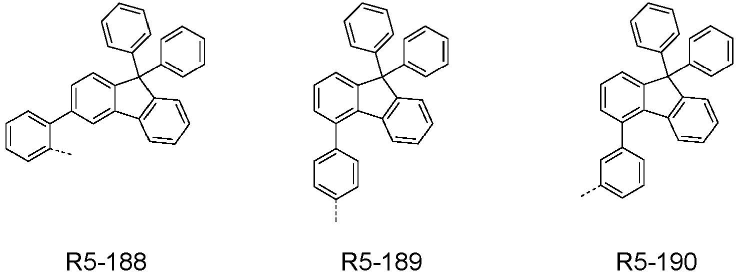 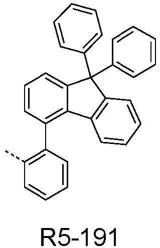 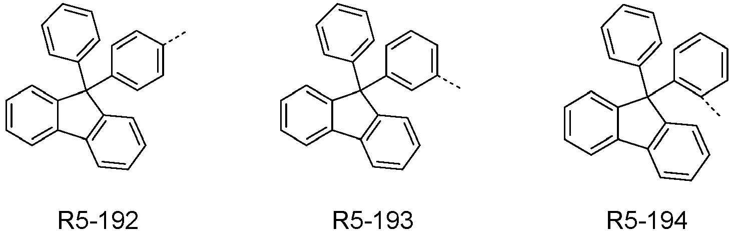 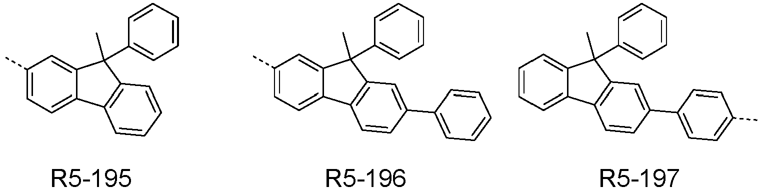 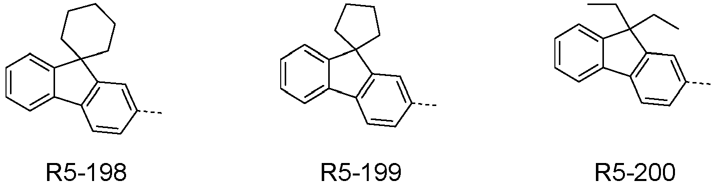 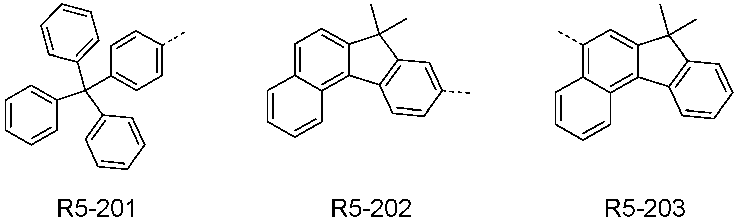 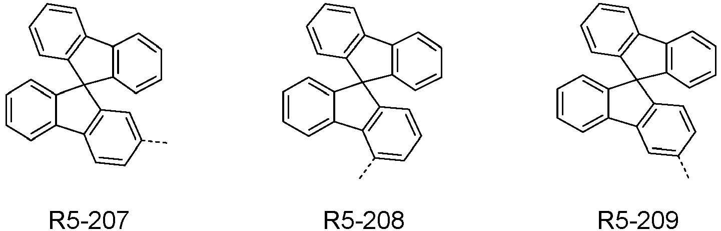 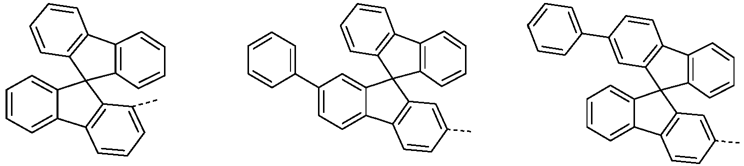 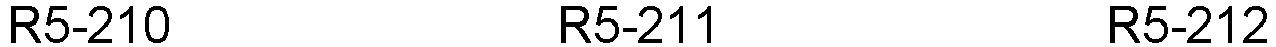 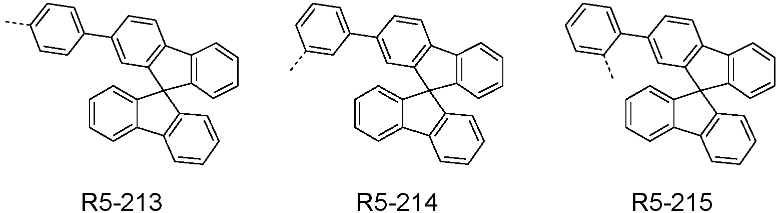 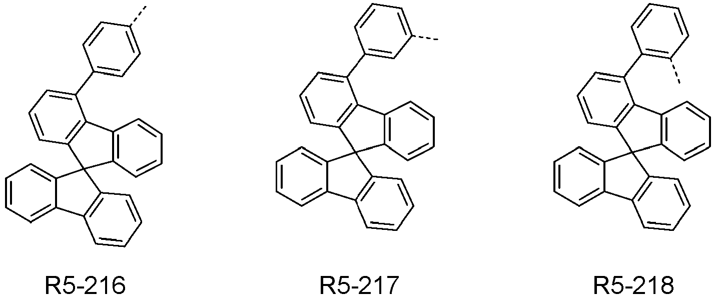 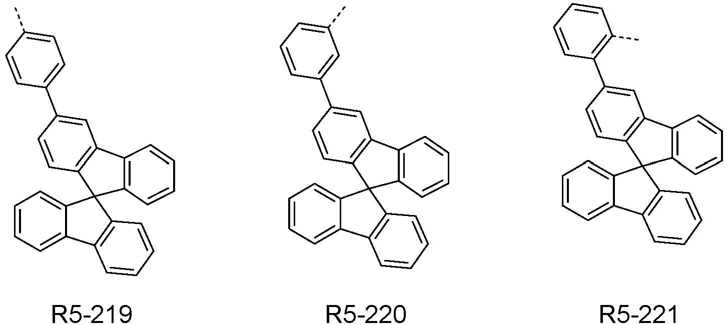 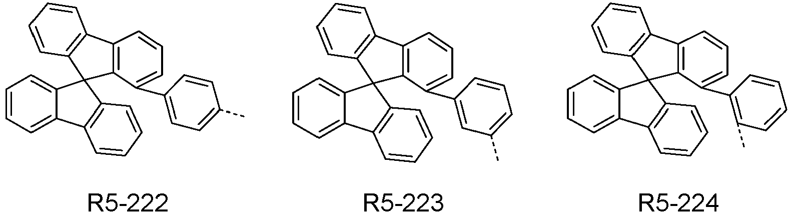 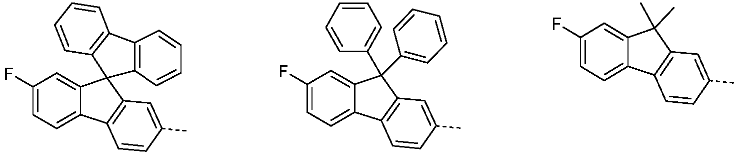 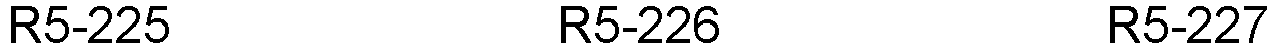 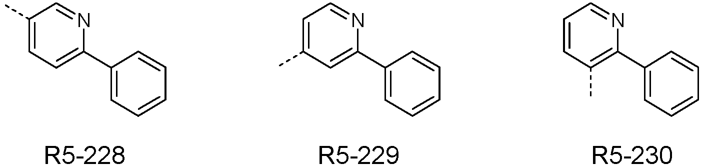 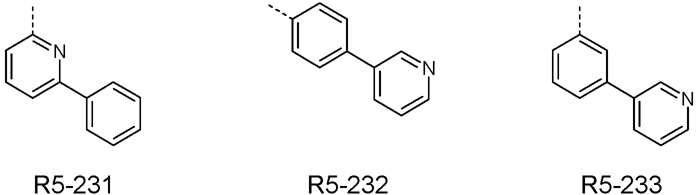 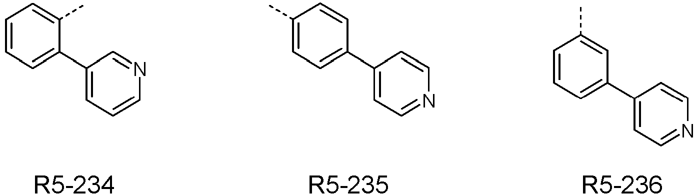 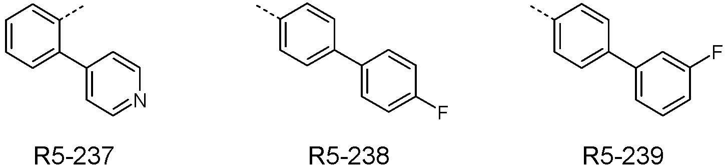 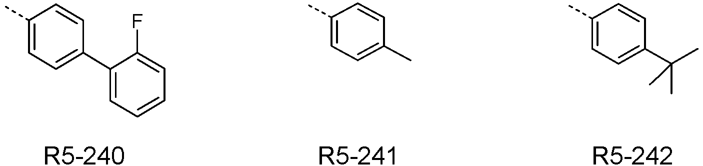 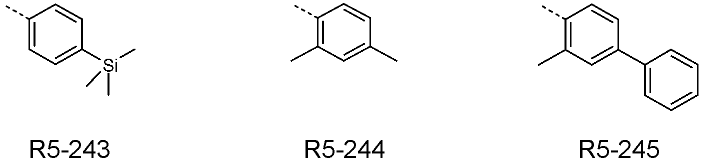 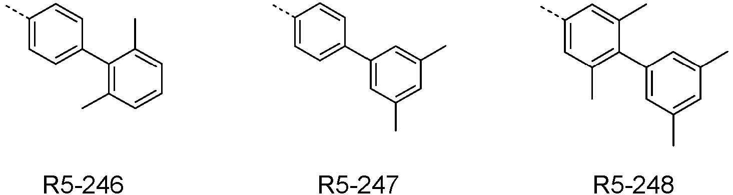 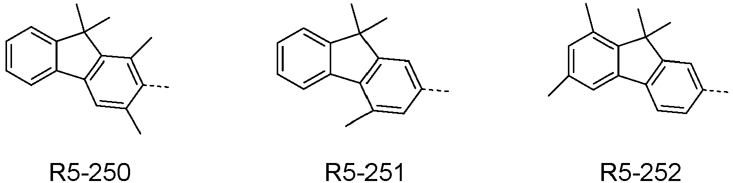 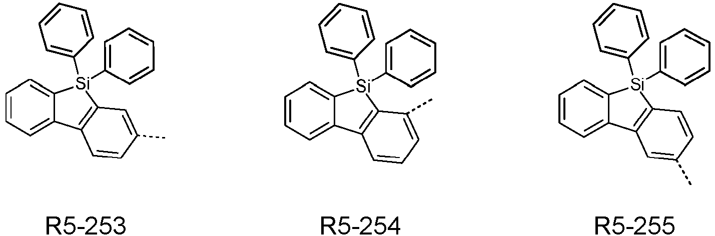 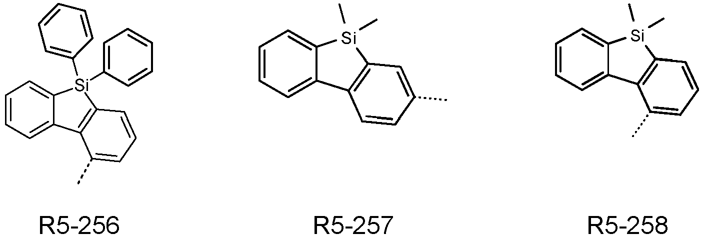 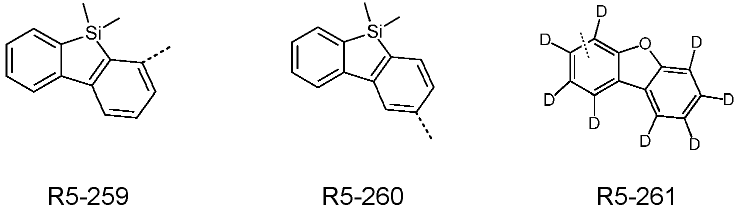 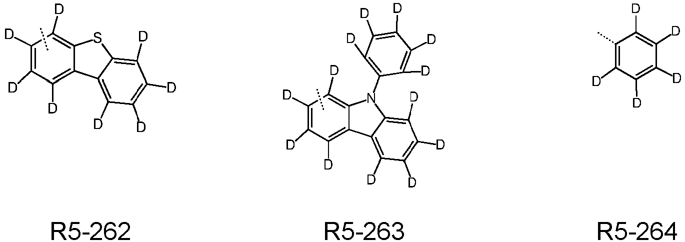 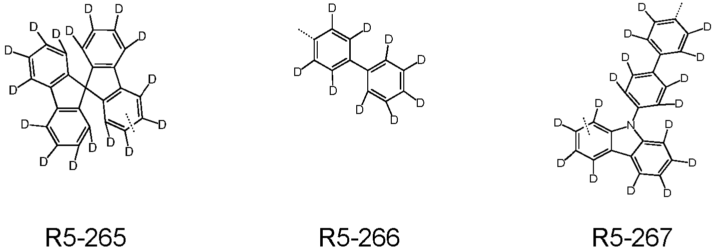 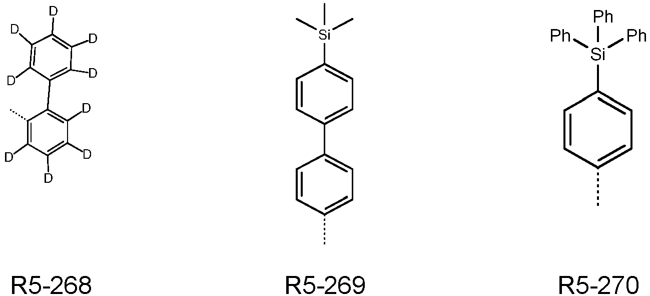 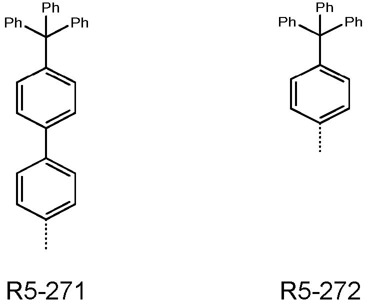 wobei die Gruppen an den unsubstituiert dargestellten Positionen mit Resten R¹¹ substituiert sein können, wobei R¹¹ in diesen Positionen bevorzugt H ist, und wobei die gestrichelte Bindung die Bindung an das Amin-Stickstoffatom ist.

Es ist ferner bevorzugt, wenn die oben genannten erfindungsgemäßen Verbindungen mit den genannten Formeln sowie die in den verschiedenen Ausführungsformen beschriebenen bevorzugten Verbindungen nur einen Rest R¹ aufweisen und vorzugsweise keinen Rest R¹ aufweisen.

Es ist ferner bevorzugt, wenn die oben genannten erfindungsgemäßen Verbindungen mit den genannten Formeln sowie die in den verschiedenen Ausführungsformen beschriebenen bevorzugten Verbindungen nur einen Rest R⁴ aufweisen und vorzugsweise keinen Rest R⁴ aufweisen.

Es ist ferner bevorzugt, wenn die oben genannten erfindungsgemäßen Verbindungen mit den genannten Formeln sowie die in den verschiedenen Ausführungsformen beschriebenen bevorzugten Verbindungen nur einen Rest R⁶ aufweisen und vorzugsweise keinen Rest R ⁶ aufweisen.

Es ist ferner bevorzugt, wenn die oben genannten erfindungsgemäßen Verbindungen mit den genannten Formeln sowie die in den verschiedenen Ausführungsformen beschriebenen bevorzugten Verbindungen nur einen Rest R⁷ aufweisen und vorzugsweise keinen Rest R⁷ aufweisen.

Schließlich ist noch mehr bevorzugt, wenn die oben genannten erfindungsgemäßen Verbindungen mit den genannten Formeln sowie die in den verschiedenen Ausführungsformen beschriebenen bevorzugten Verbindungen nur einen Rest R¹ und nur einen Rest R⁴ und nur einen Rest R⁶ und nur einen R⁷ aufweisen und vorzugsweise weisen die Verbindungen keinen der Reste R¹, R⁴, R⁶ und R⁷ auf.

In einer bevorzugten Ausführungsform ist X in den oben genannten erfindungsgemäßen Verbindungen mit den genannten Formeln sowie in den bevorzugten Verbindungen, die in den verschiedenen Ausführungsformen beschriebenen wurden, gleich O. Elektronische Vorrichtungen enthaltend diese Verbindungen weisen sehr gute Lebensdauern auf. Besonder gute Lebensdauern lassen sich in grün emittierenden organischen elektronischen Vorrichtungen beobachten.

In einer anderen bevorzugten Ausführungsform ist X in den oben genannten erfindungsgemäßen Verbindungen mit den genannten Formeln sowie in den bevorzugten Verbindungen, die in den verschiedenen Ausführungsformen beschriebenen wurden, gleich S.

Bevorzugte Ausführungsformen von erfindungsgemäßen Verbindungen der Formel (3) sind im Folgenden gezeigt:

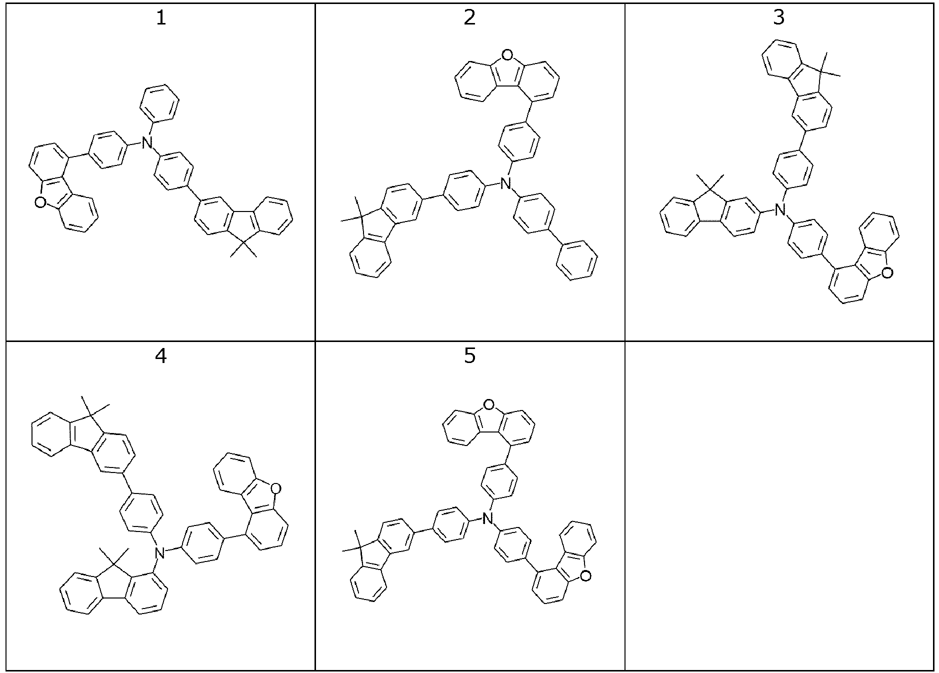

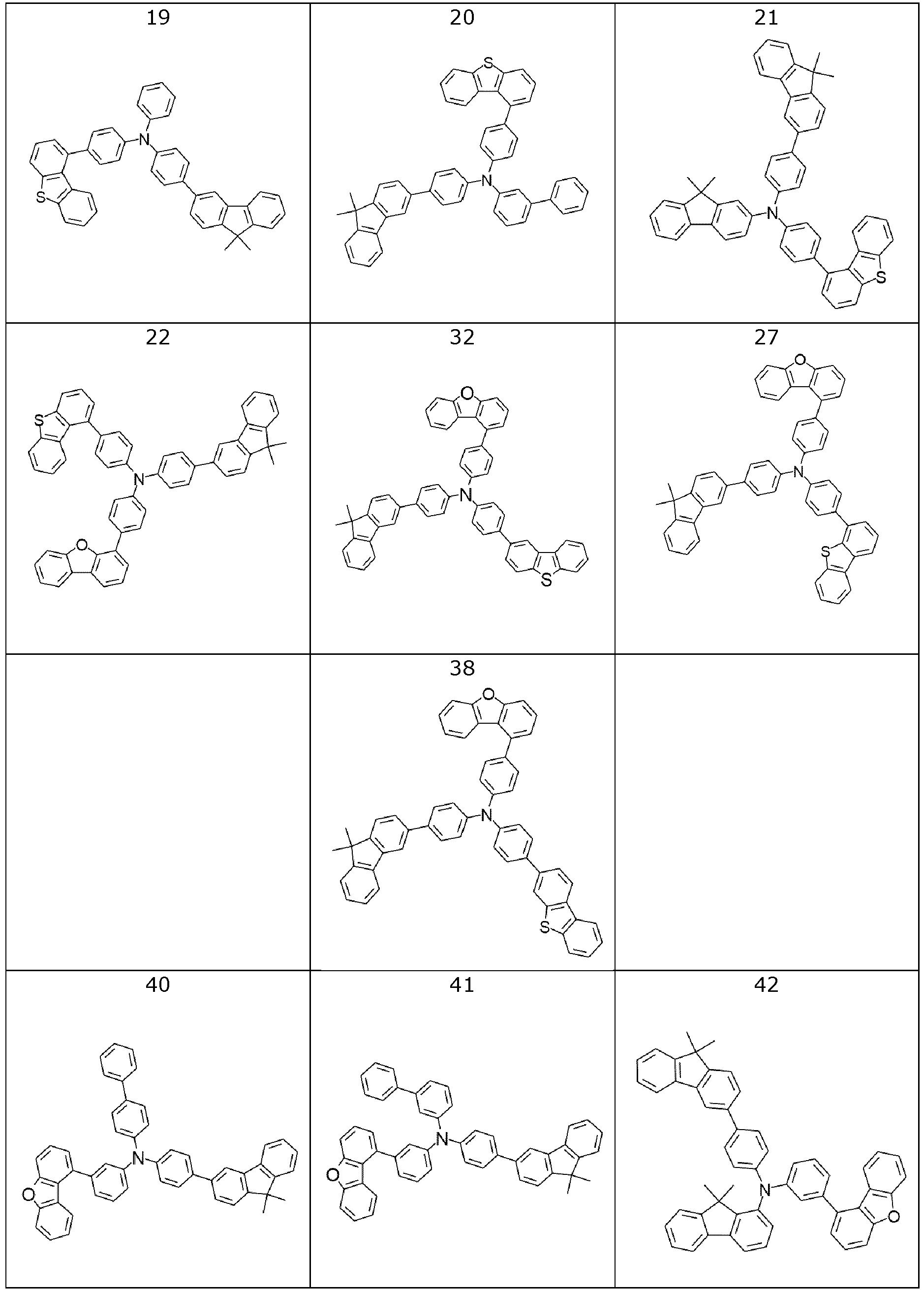

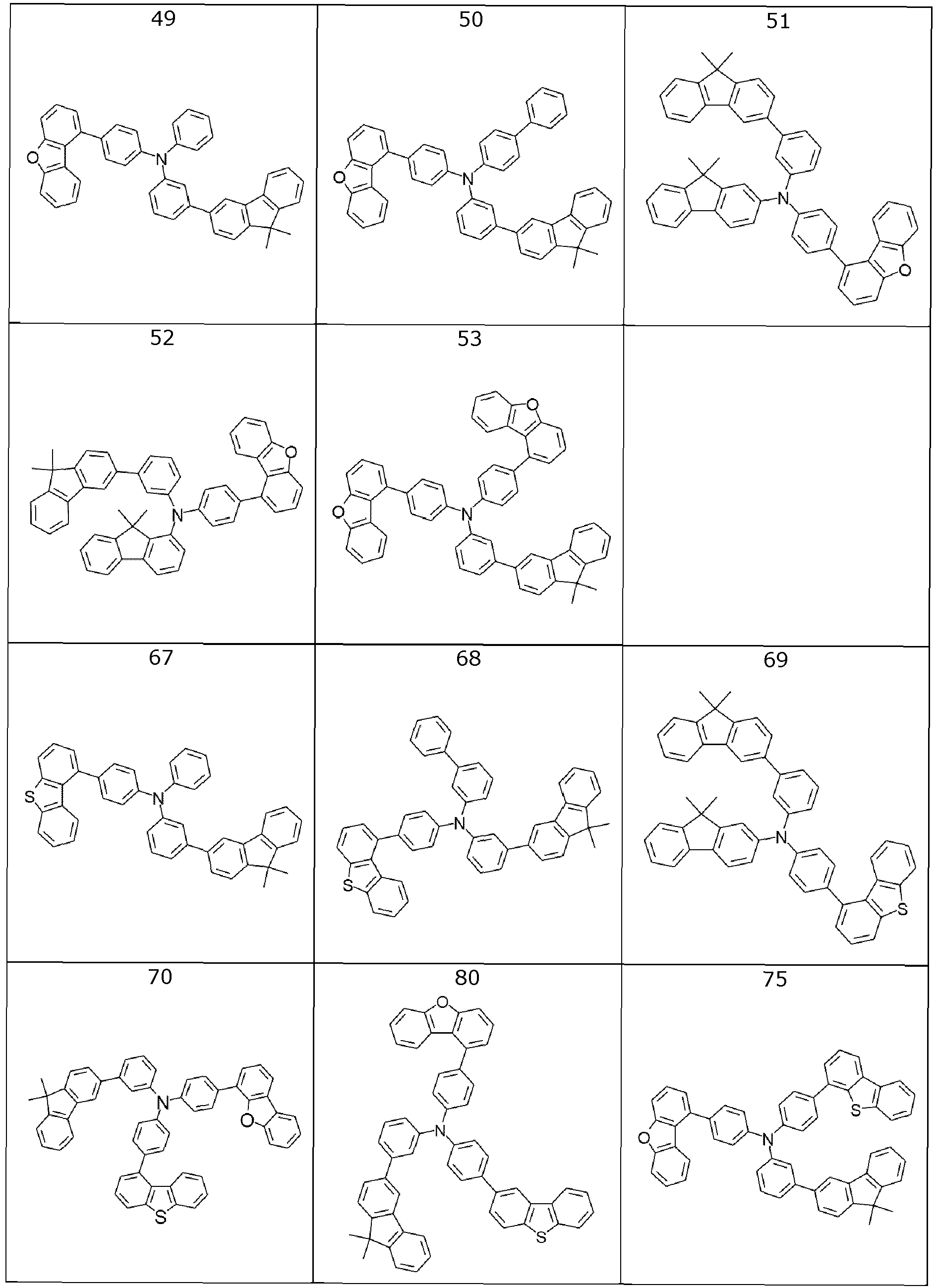

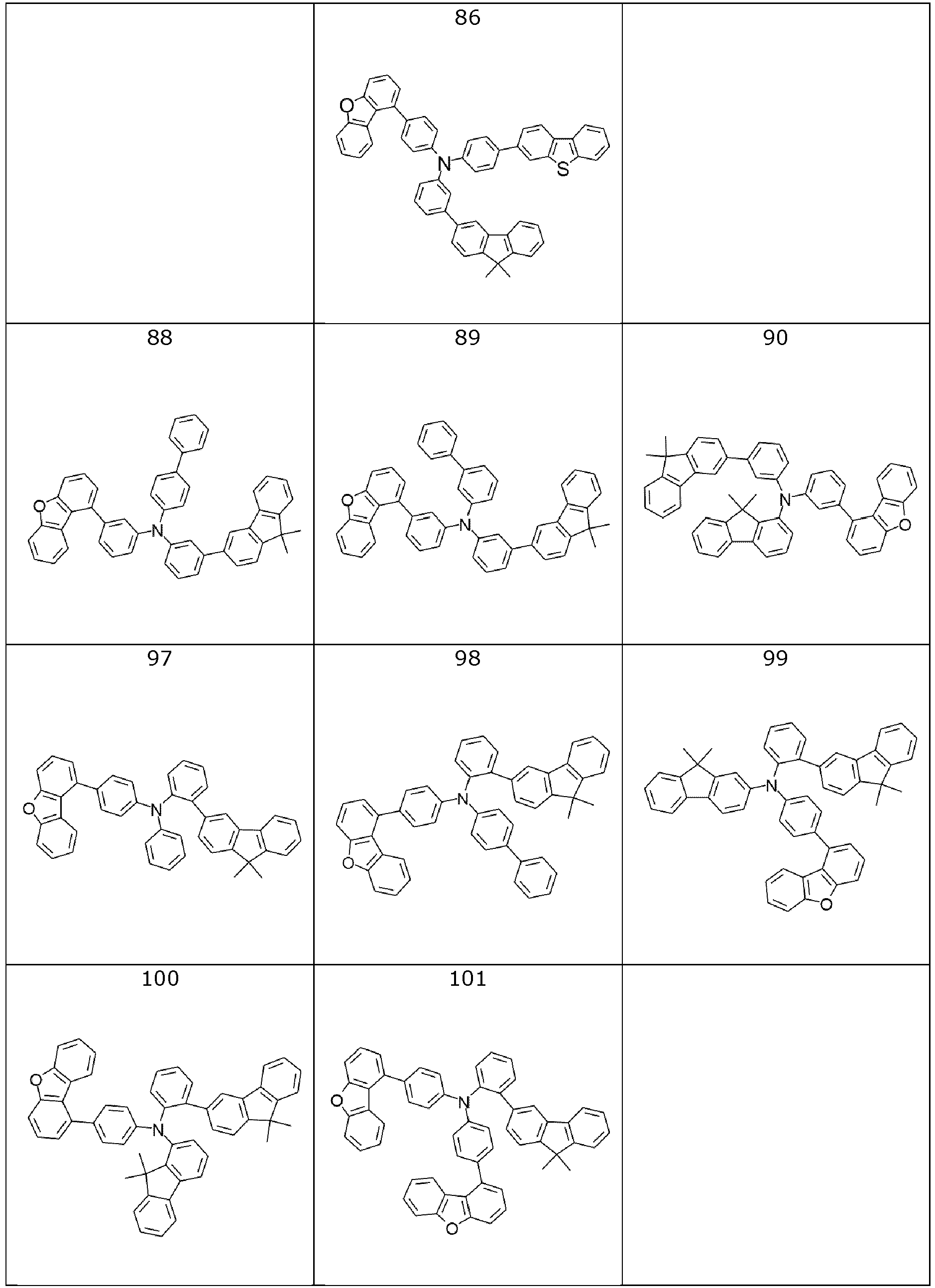

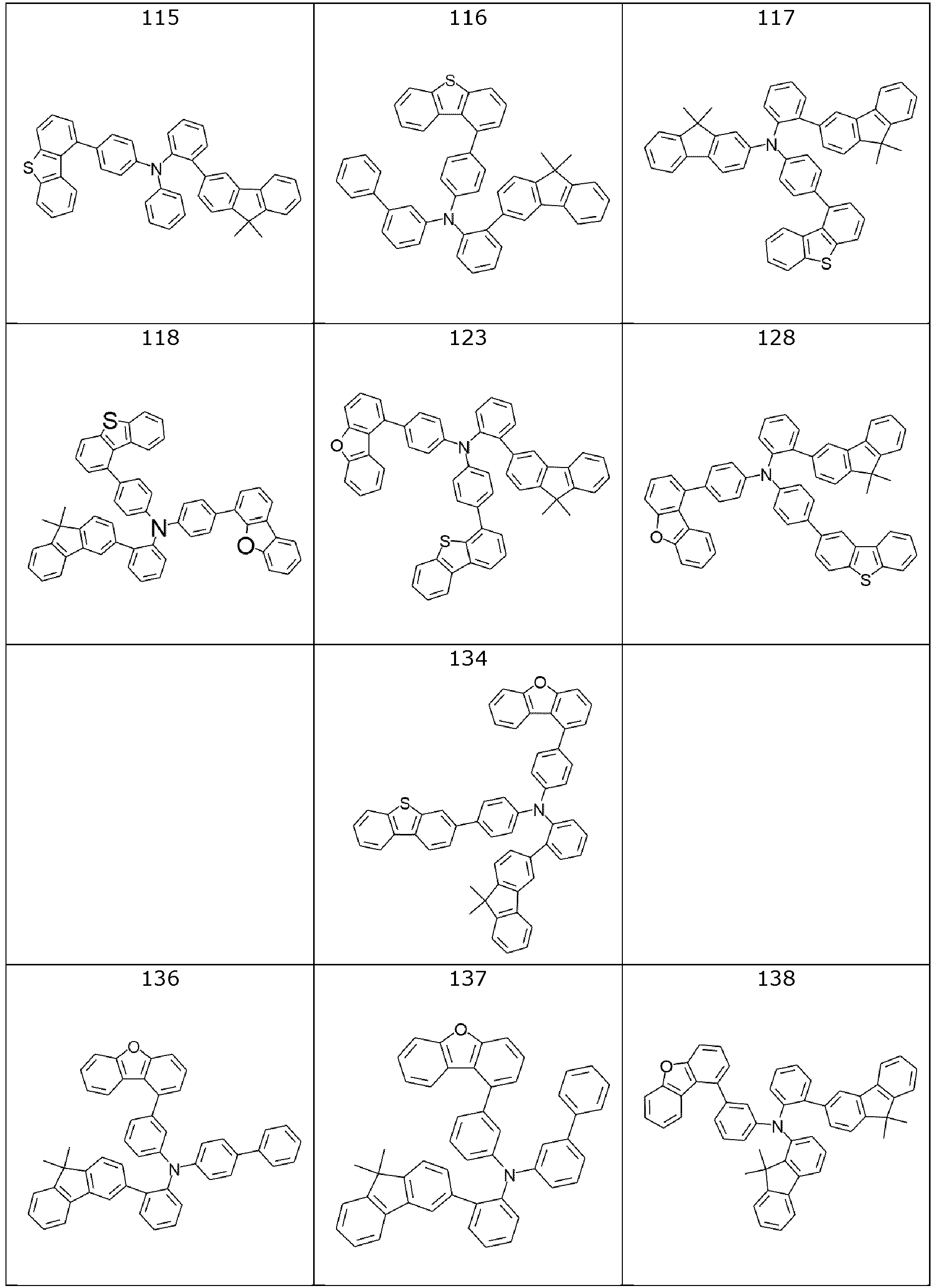

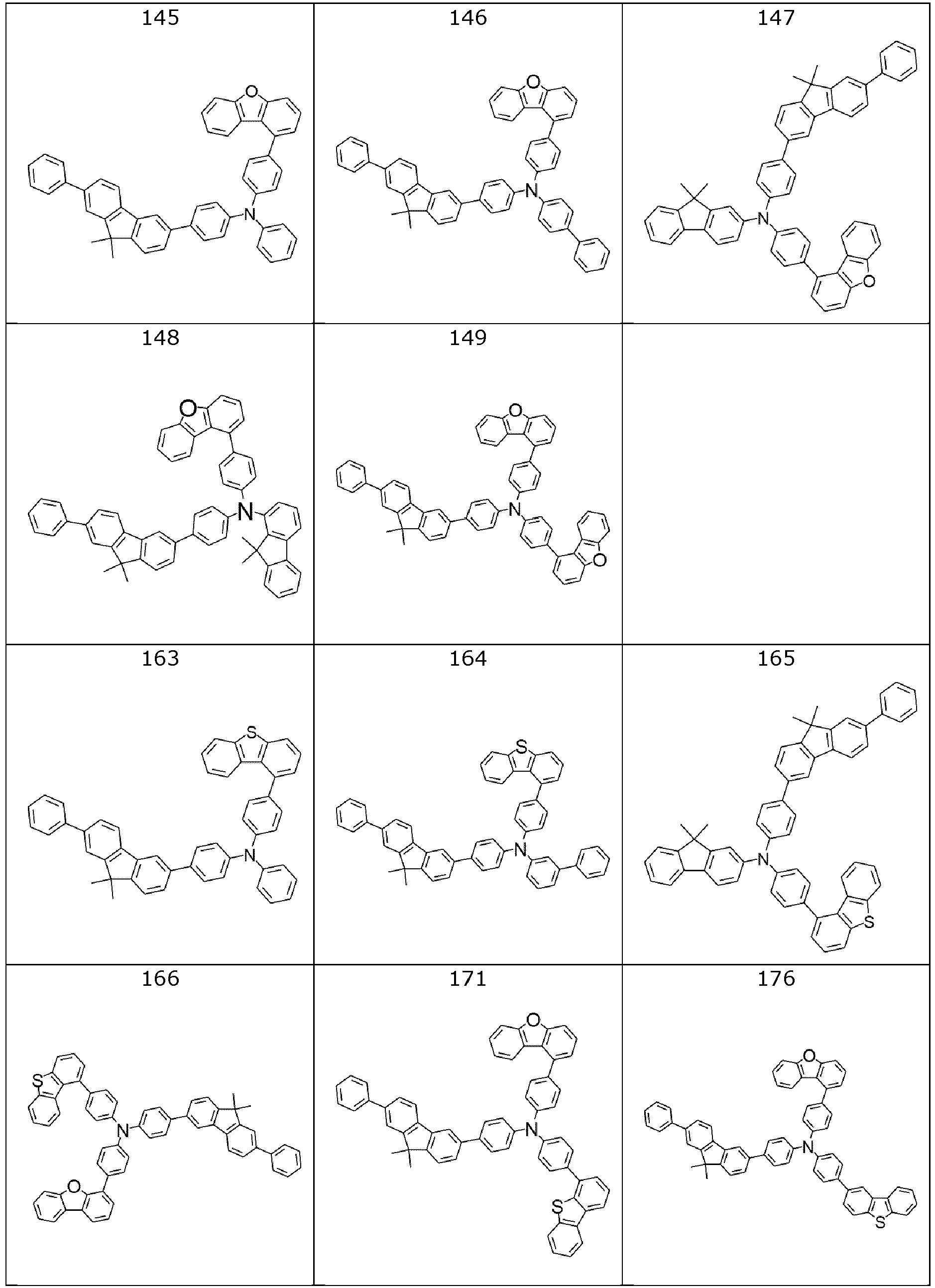

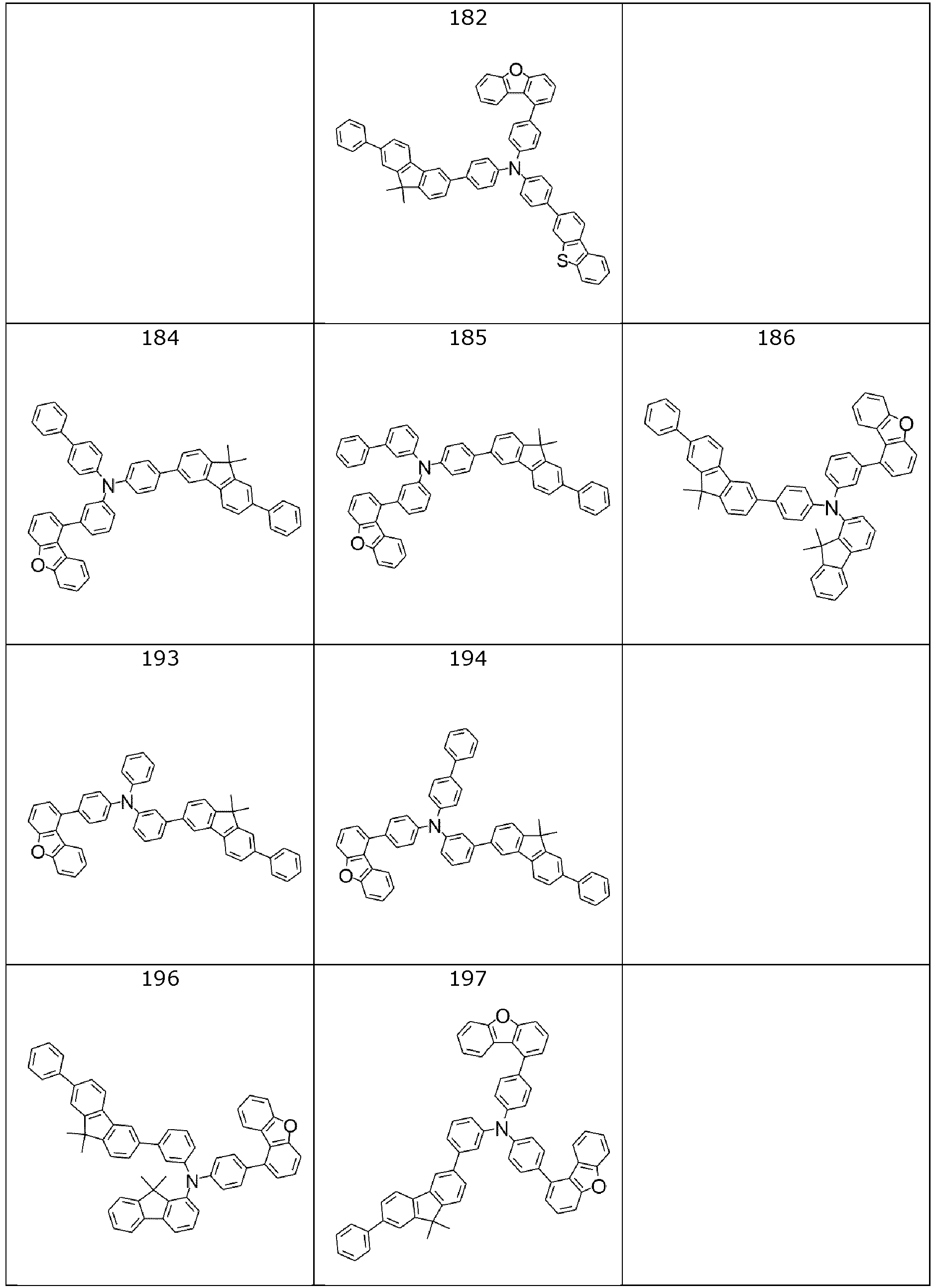

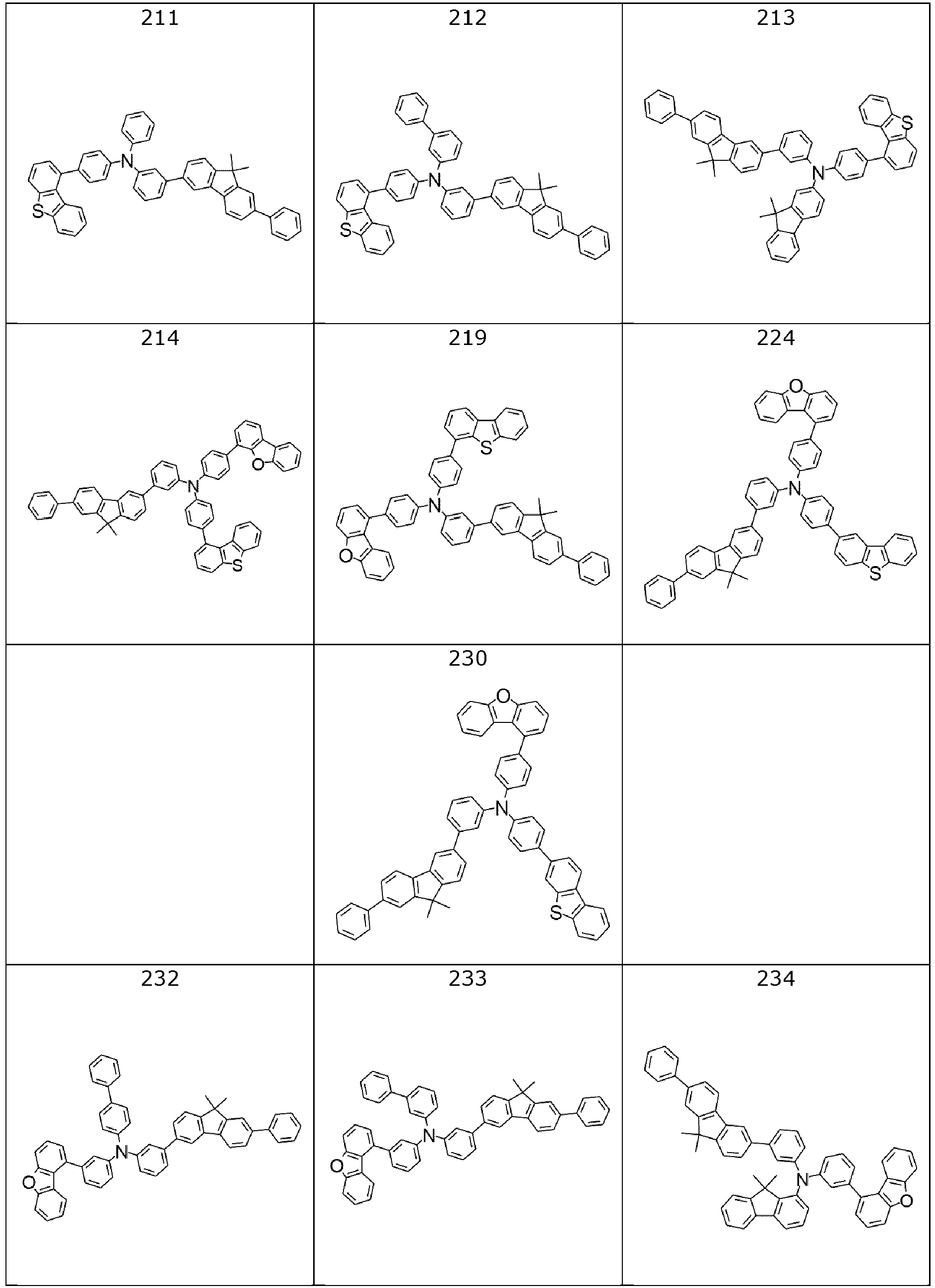

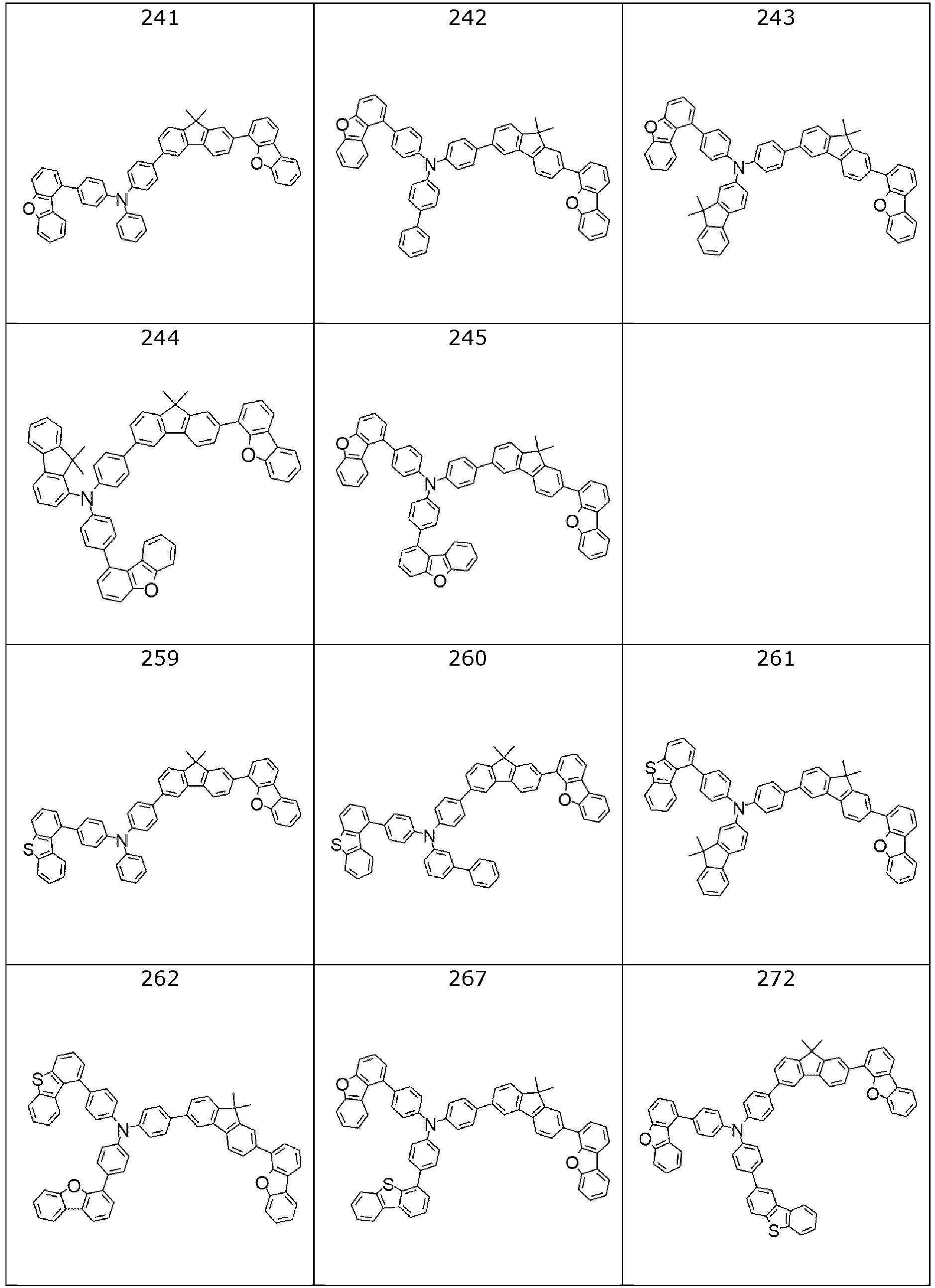

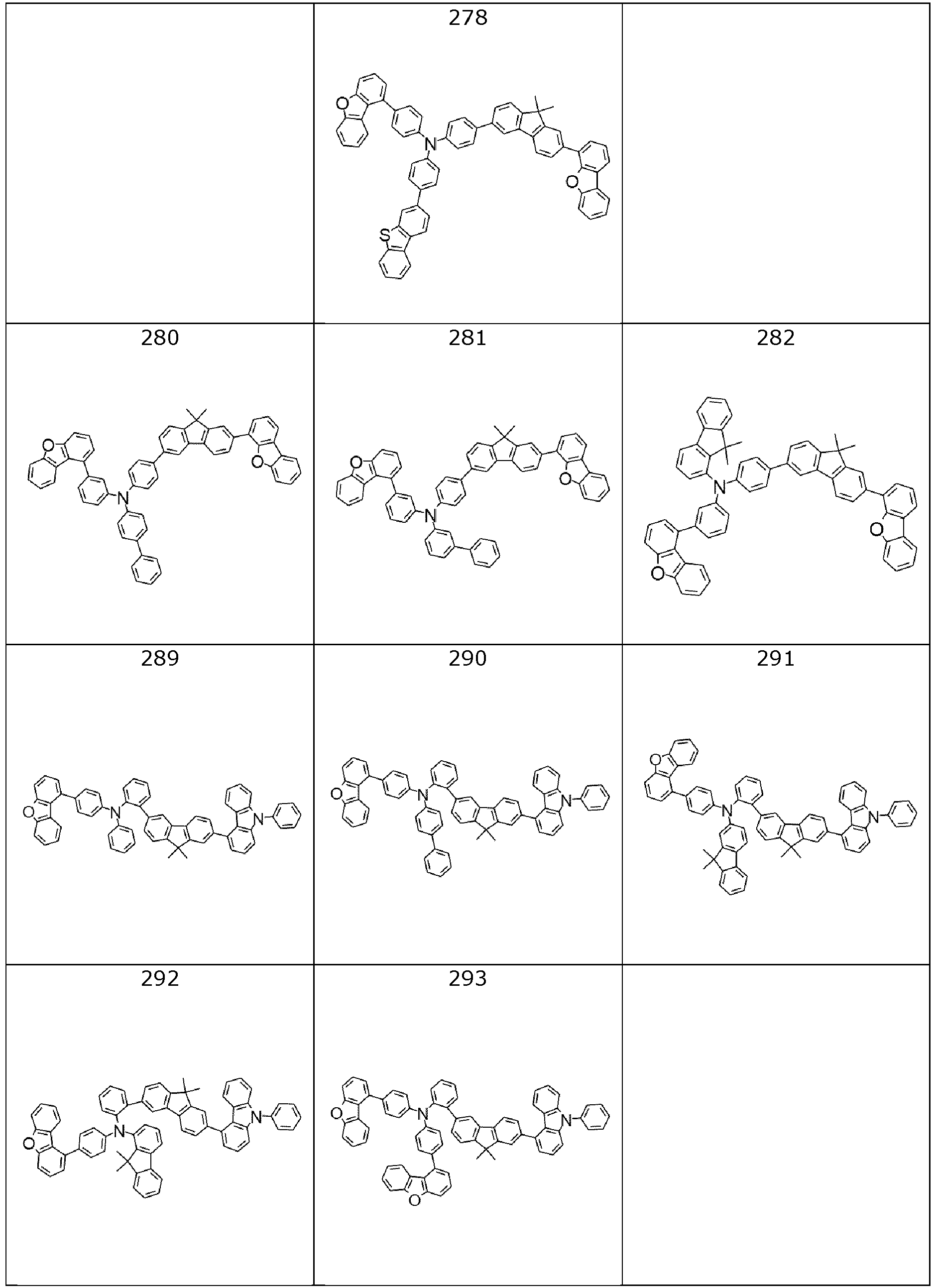

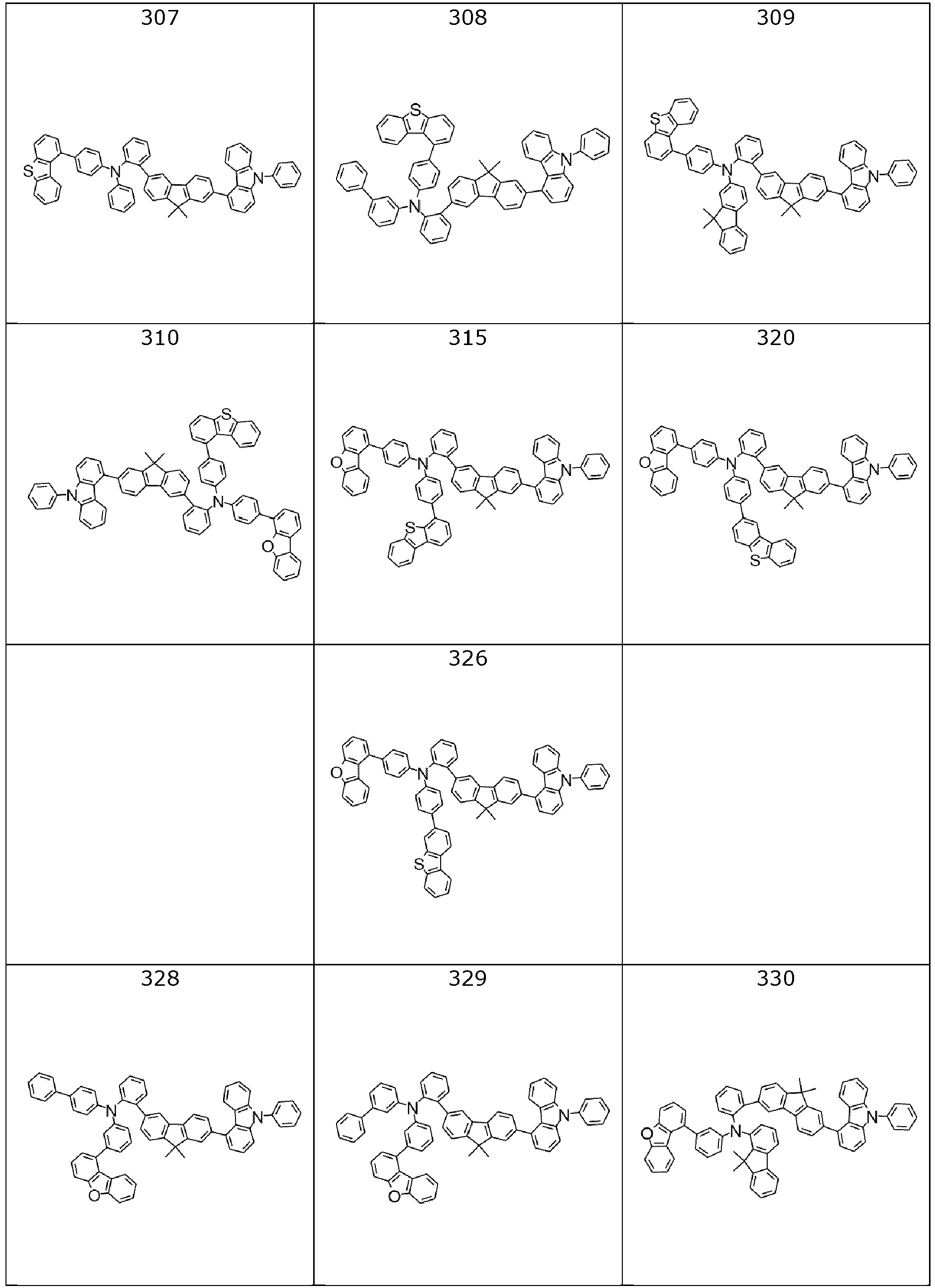

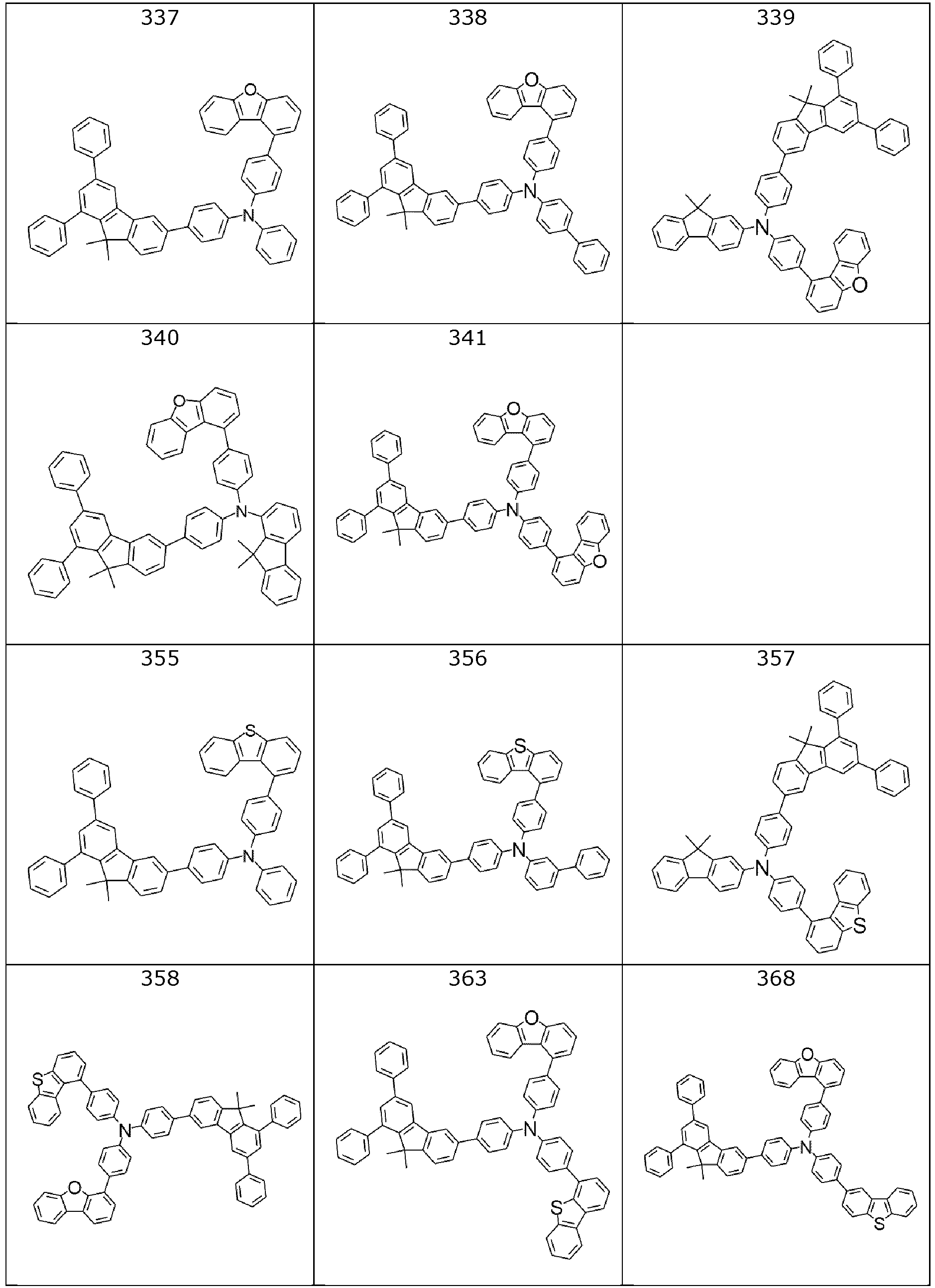

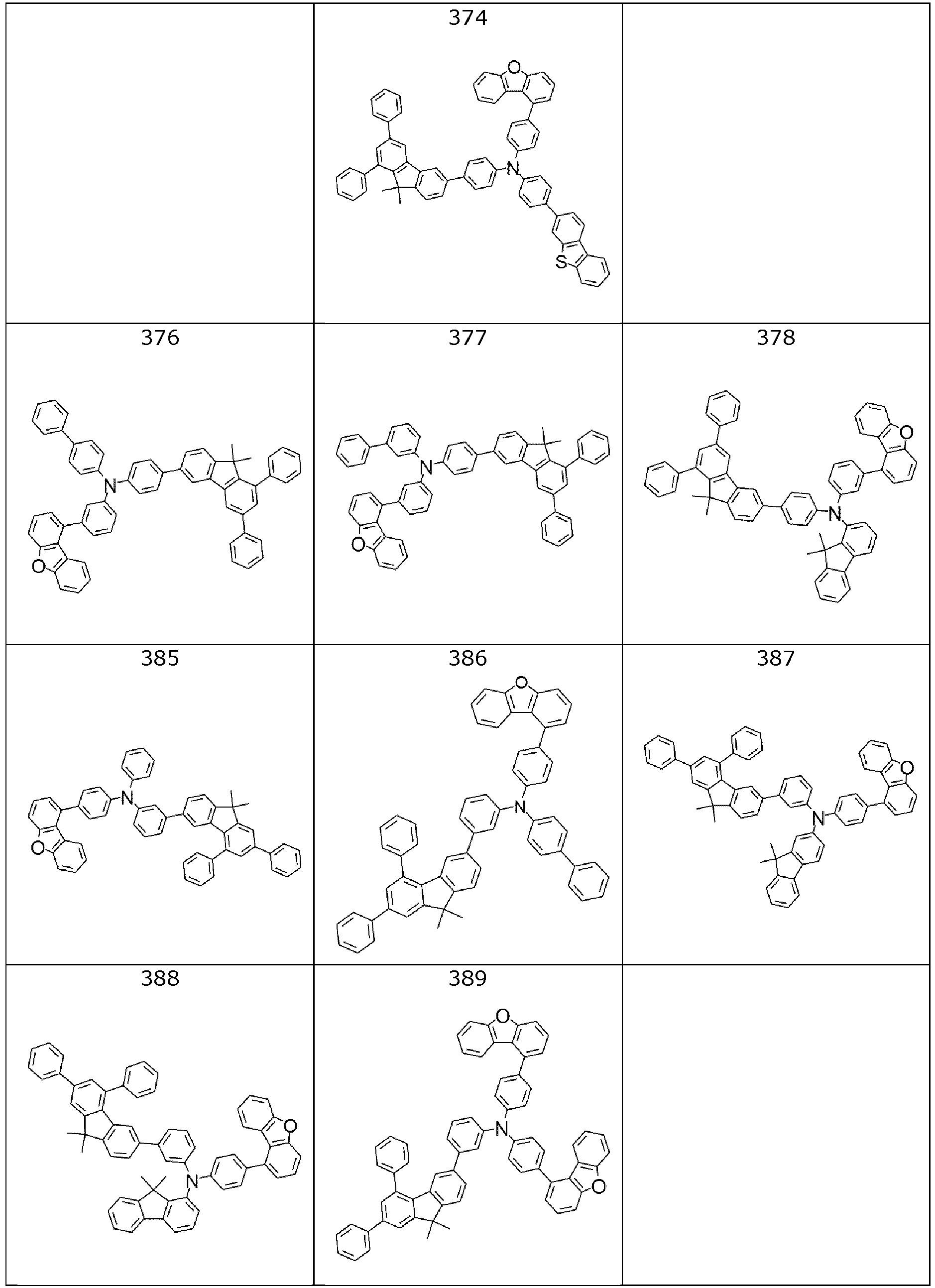

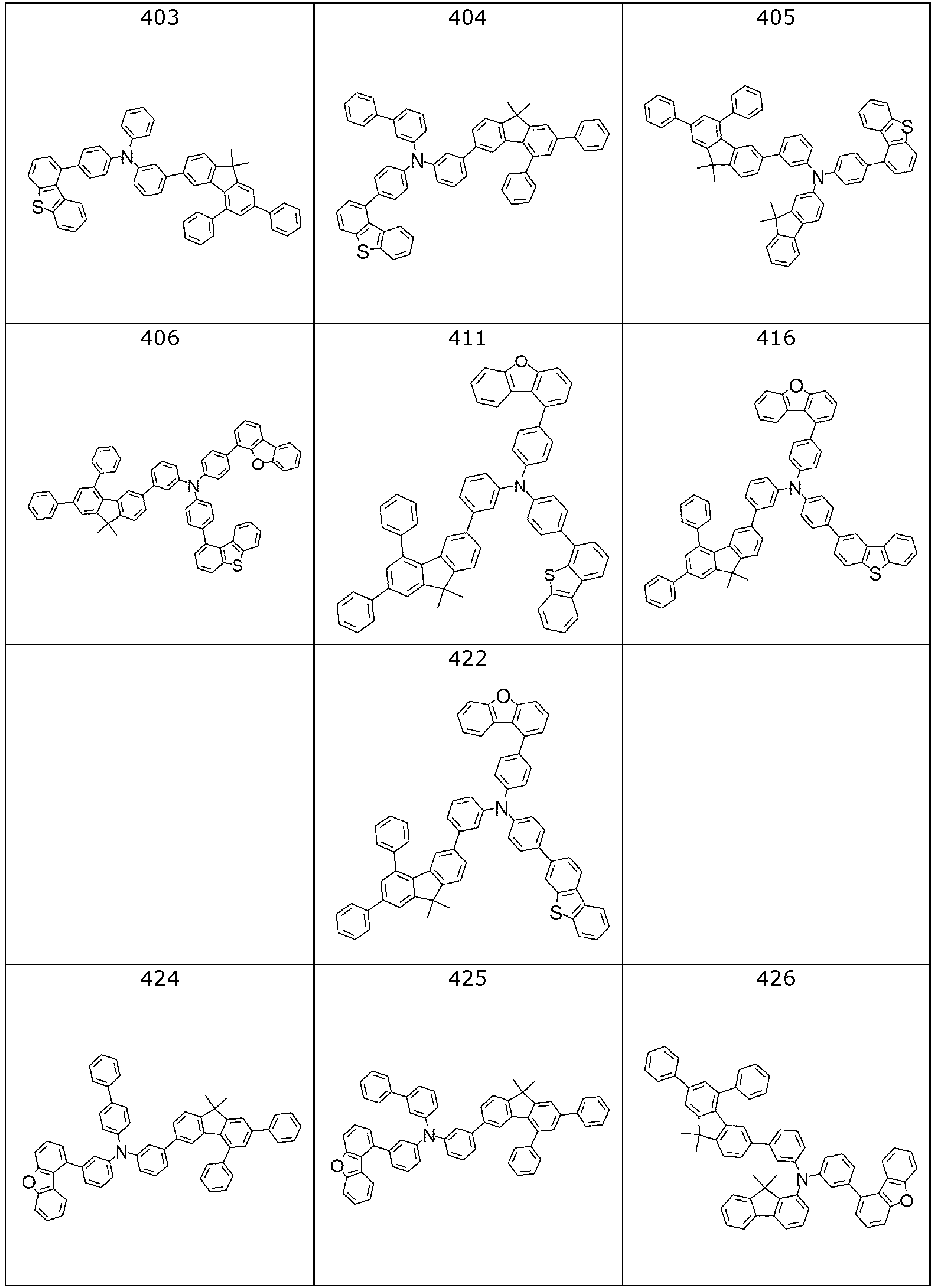

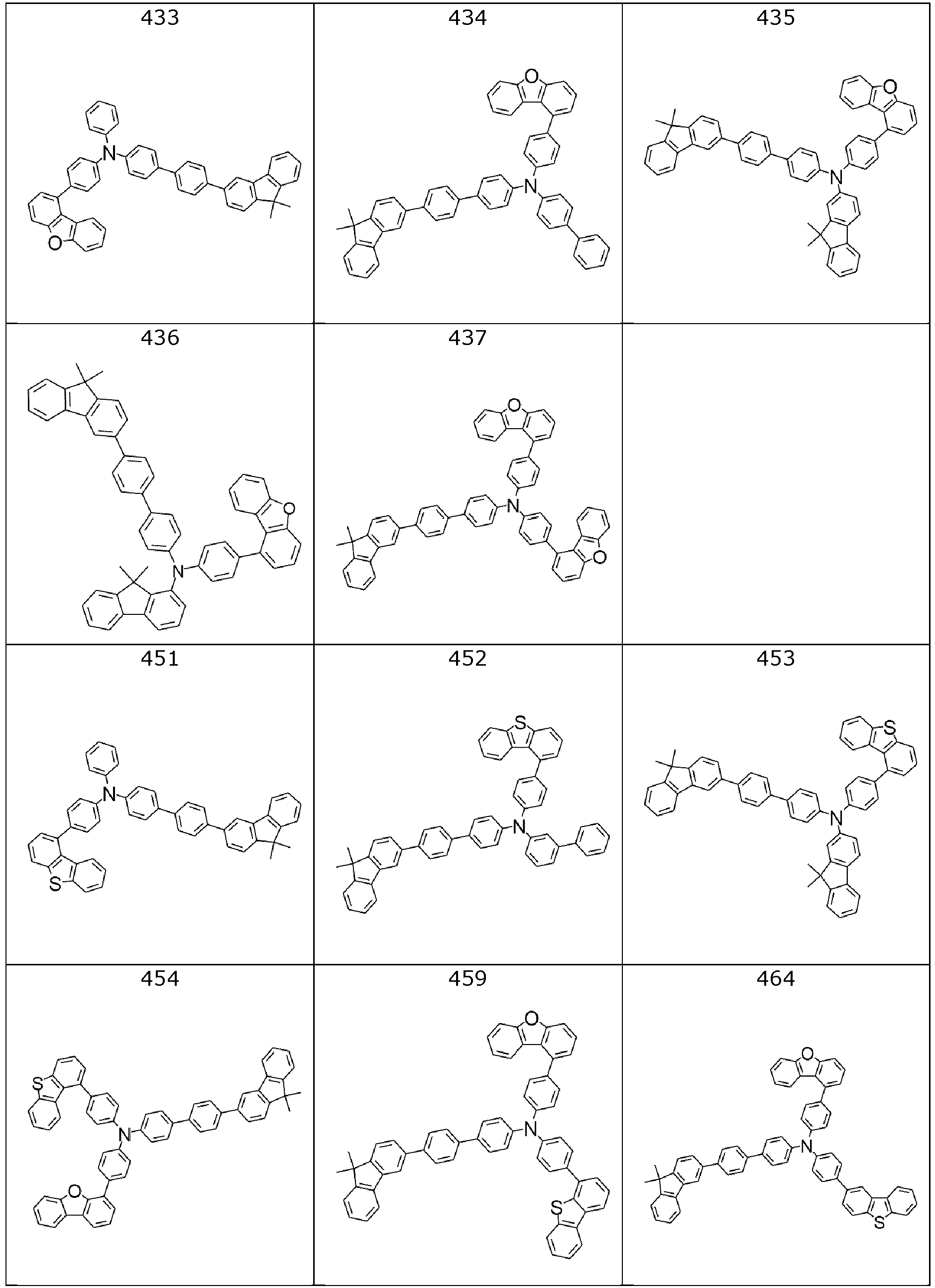

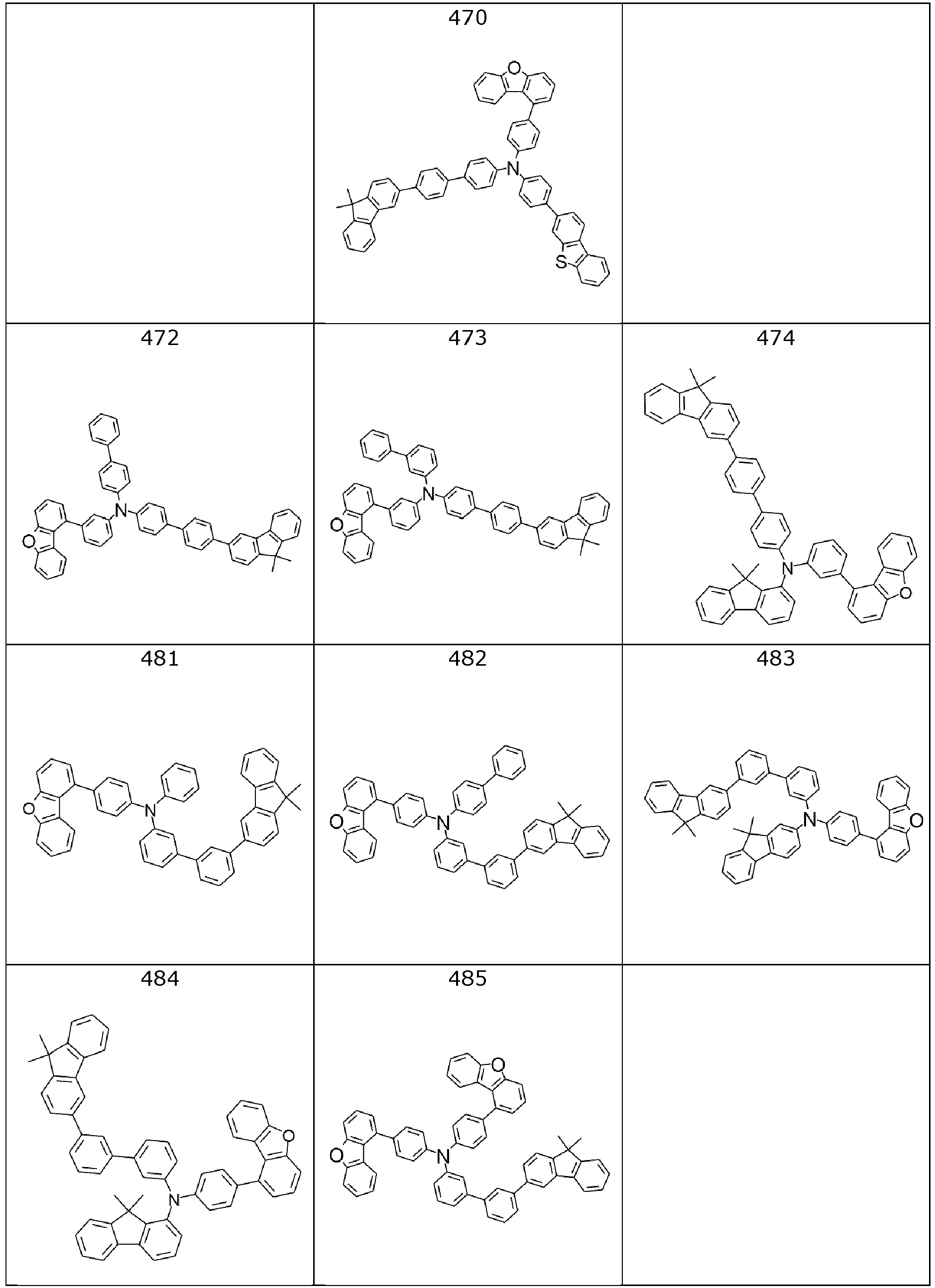

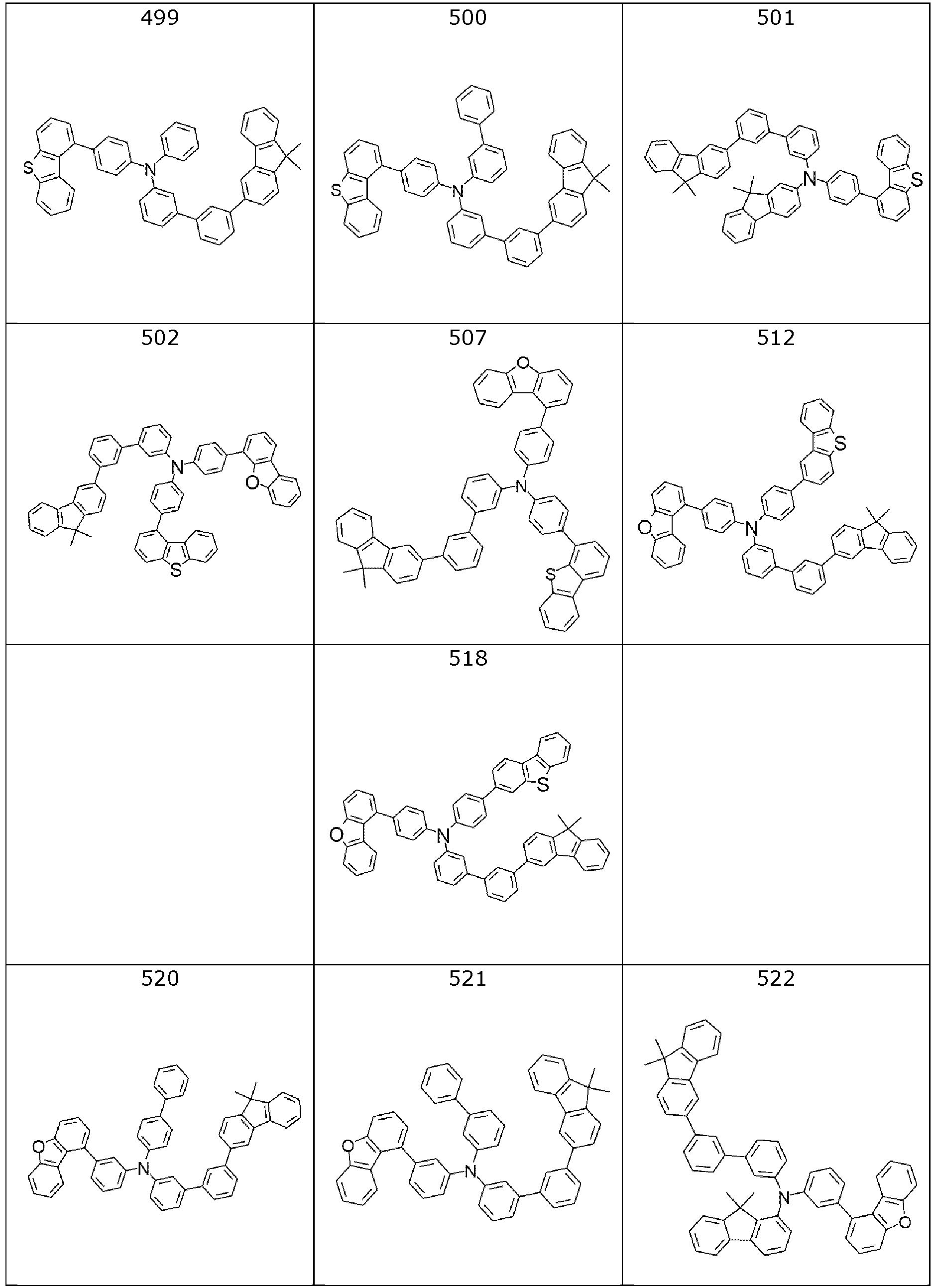

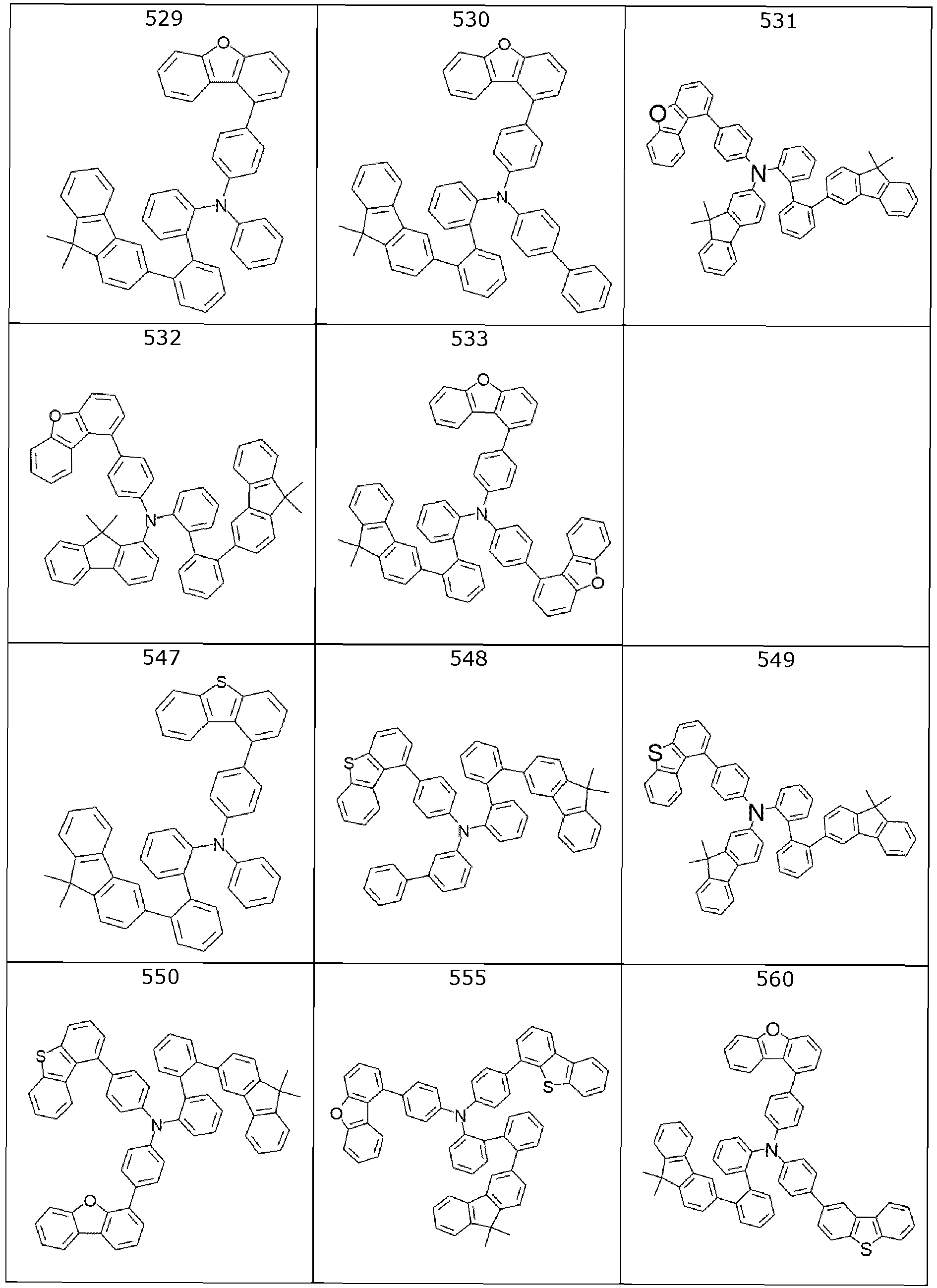

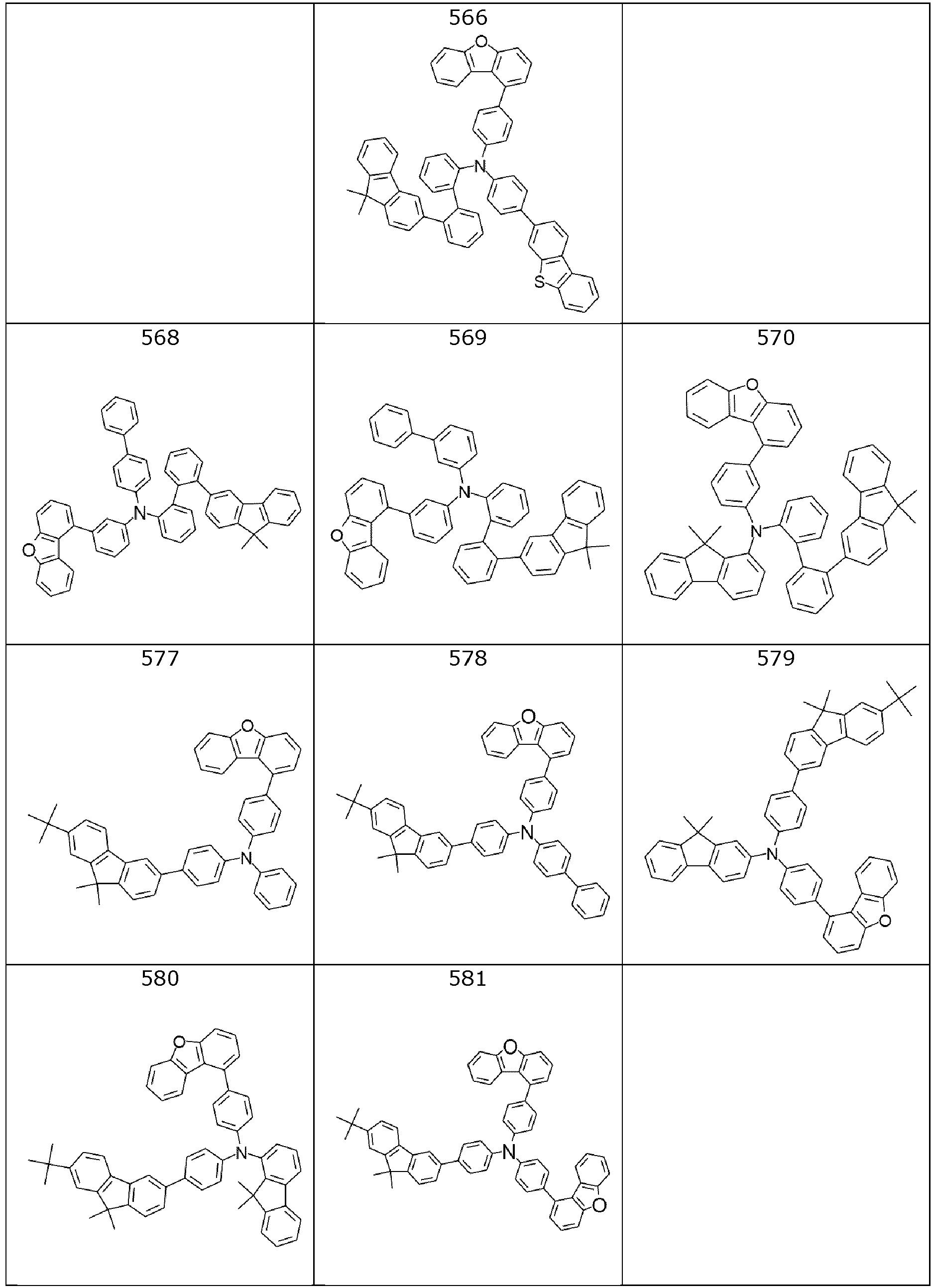

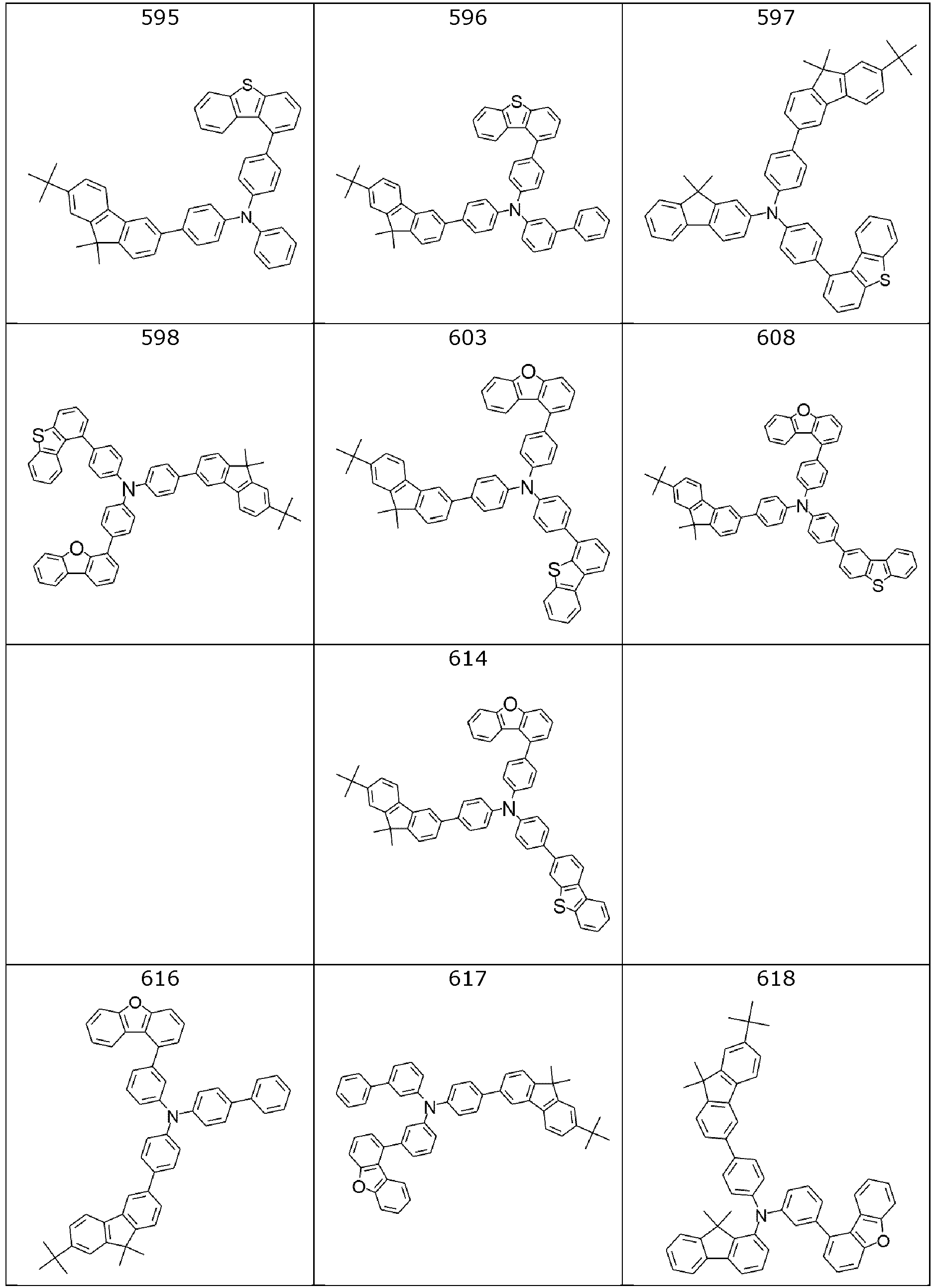

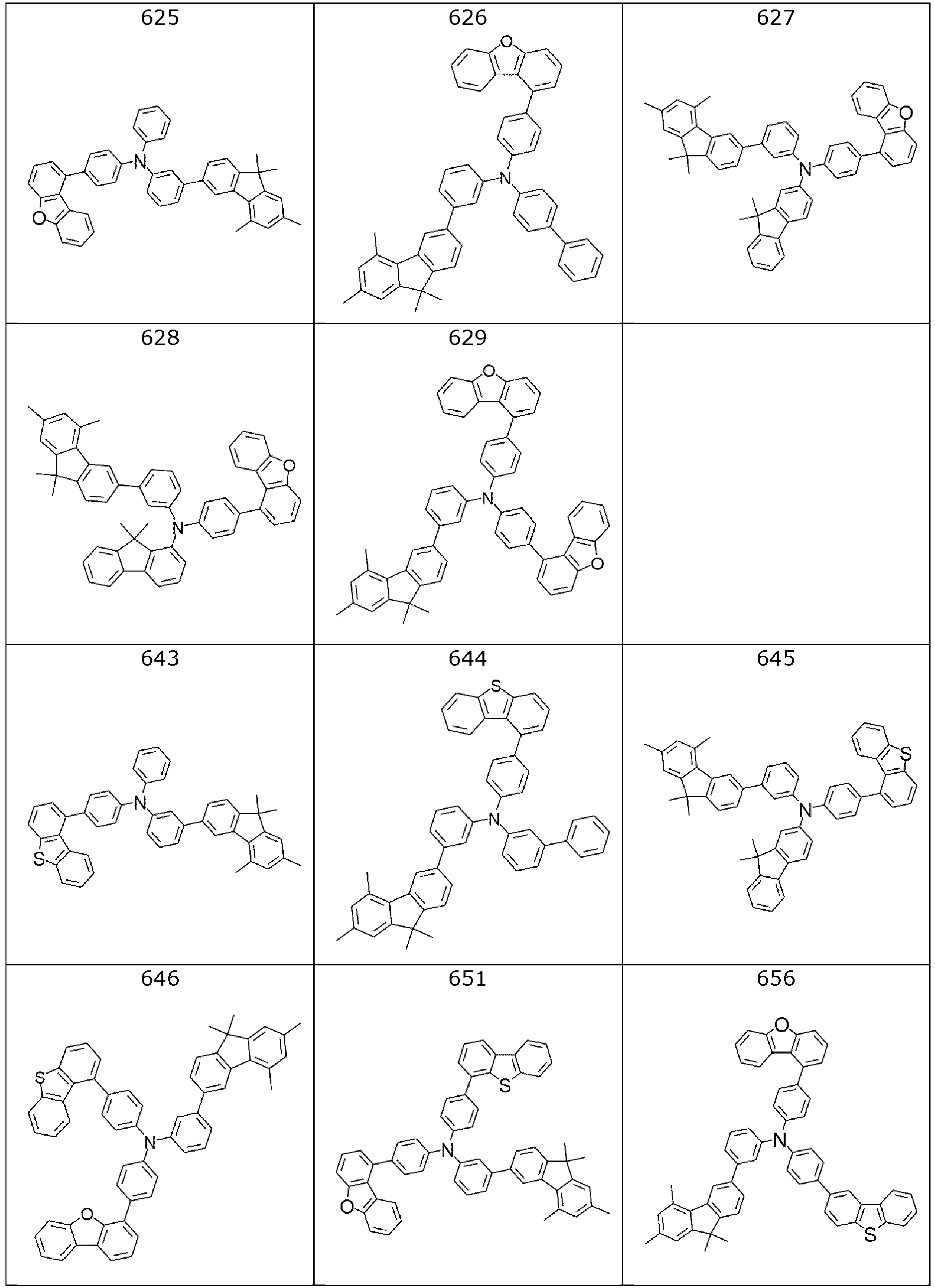

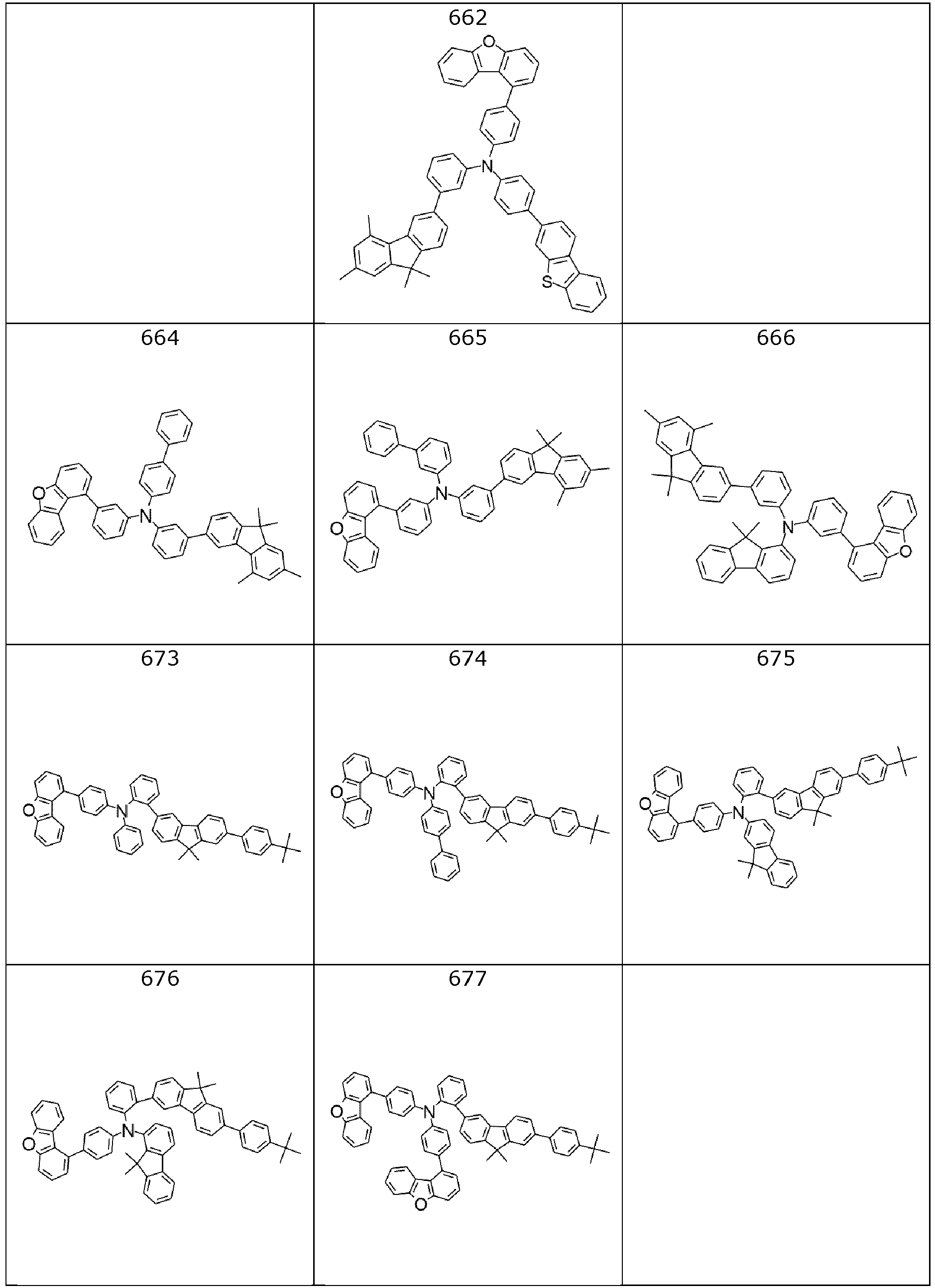

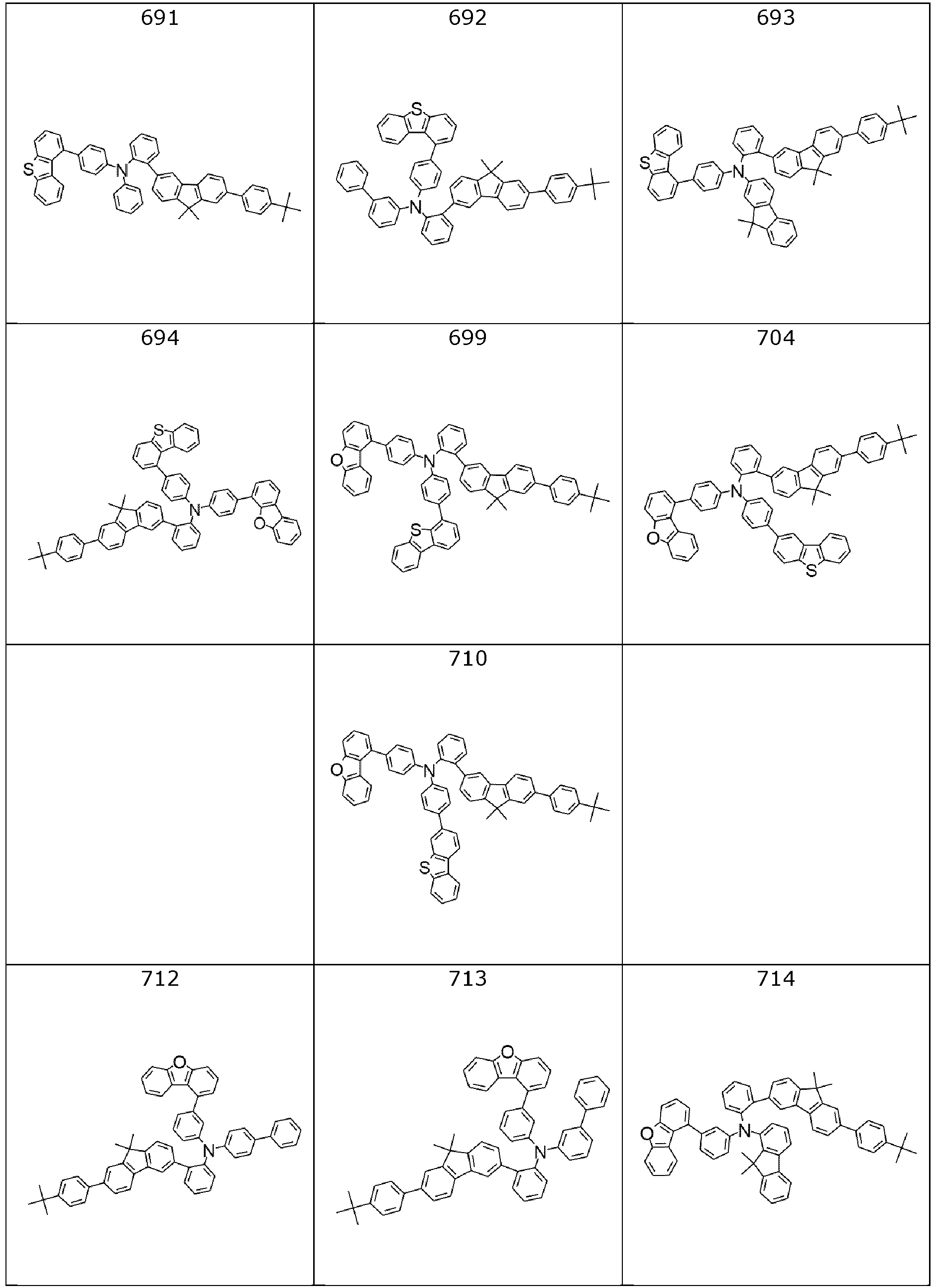

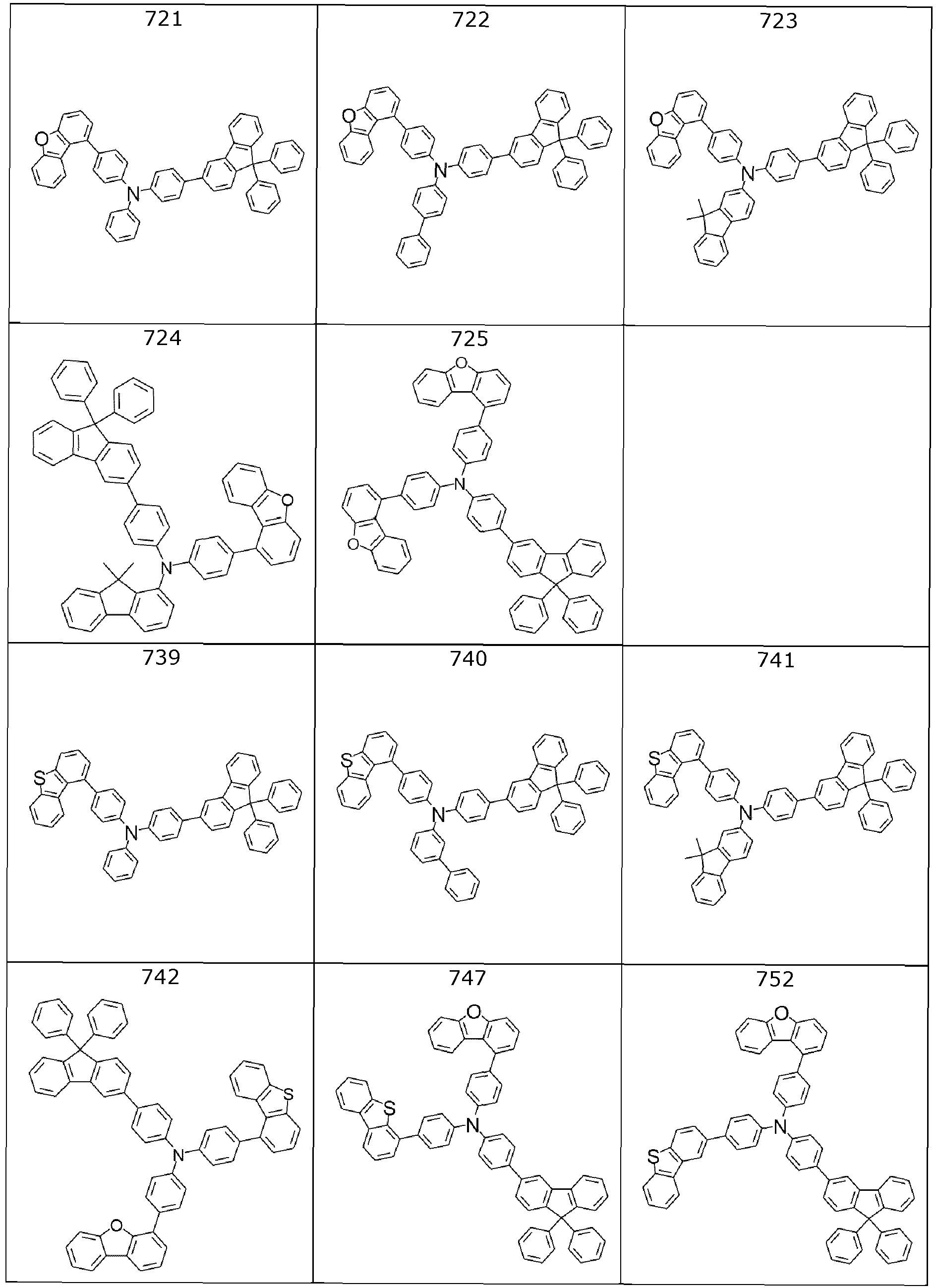

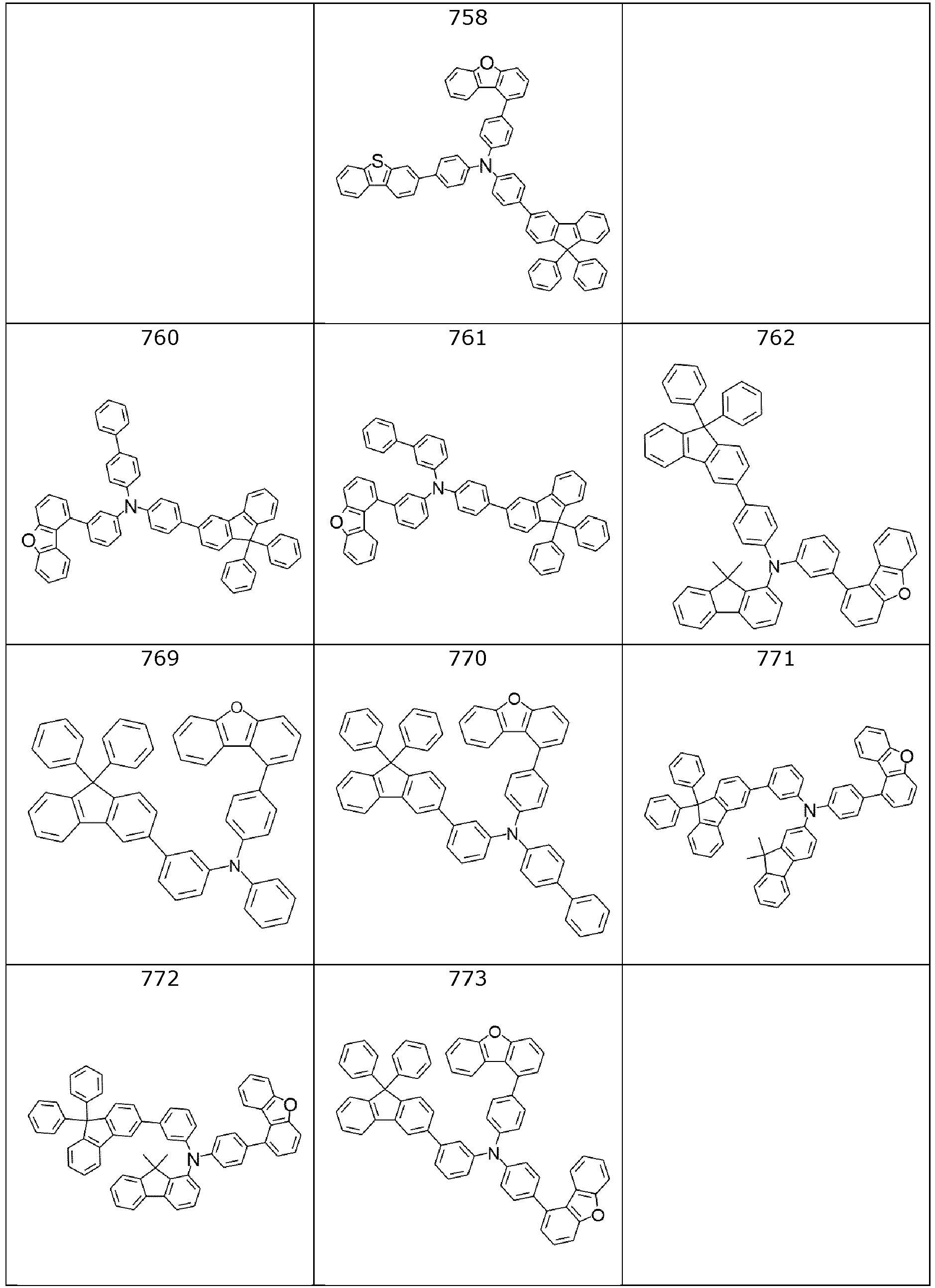

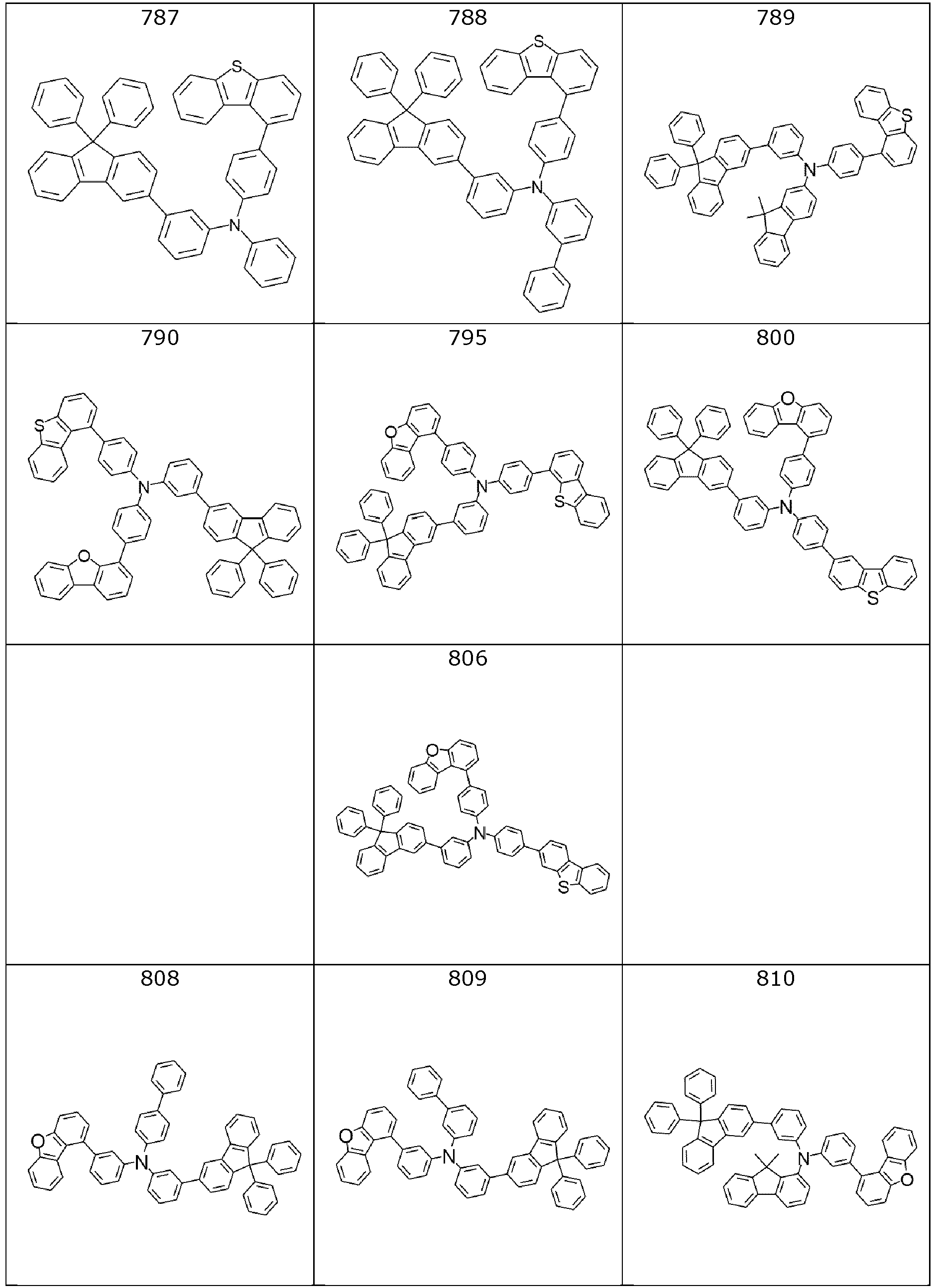

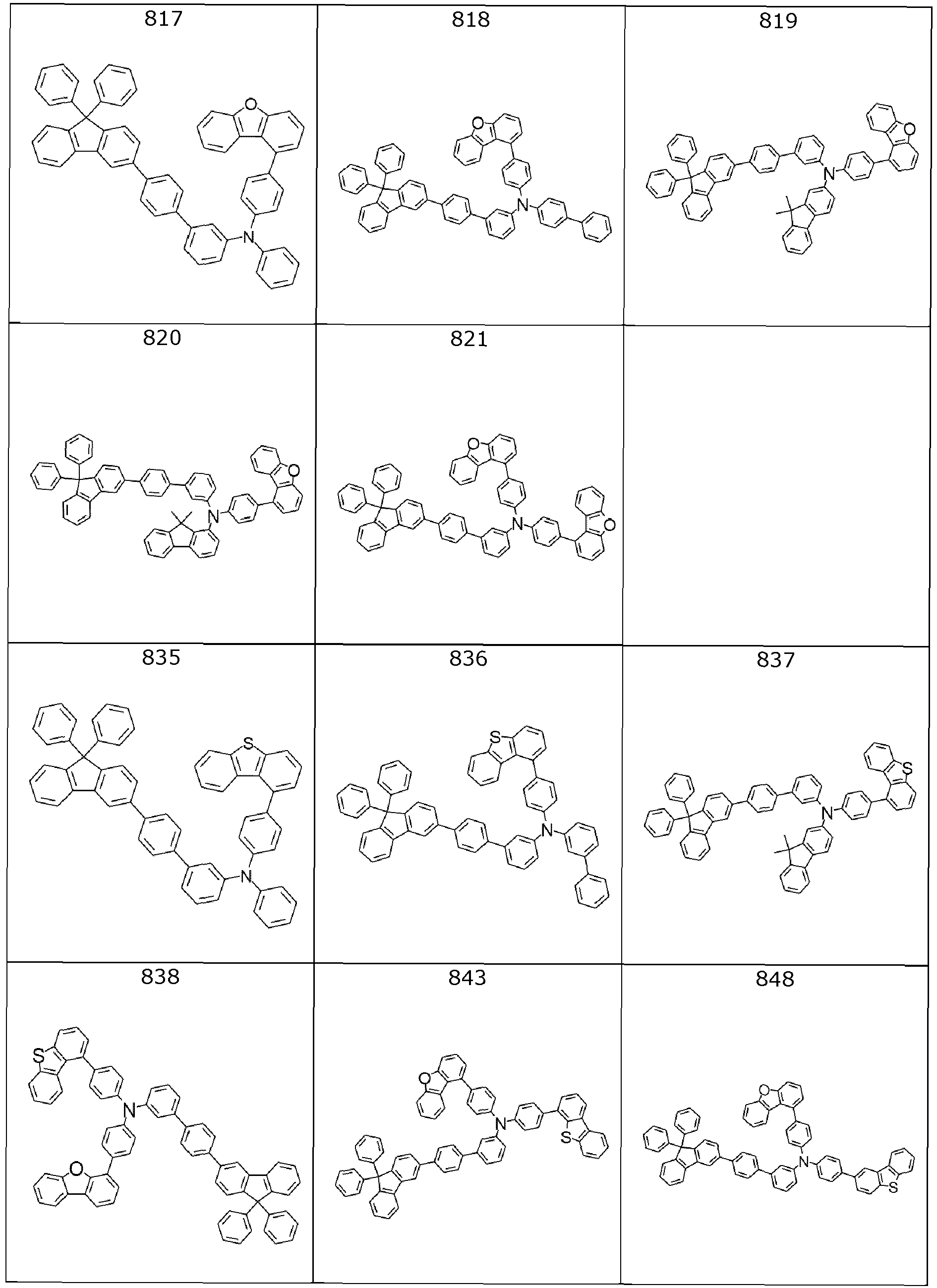

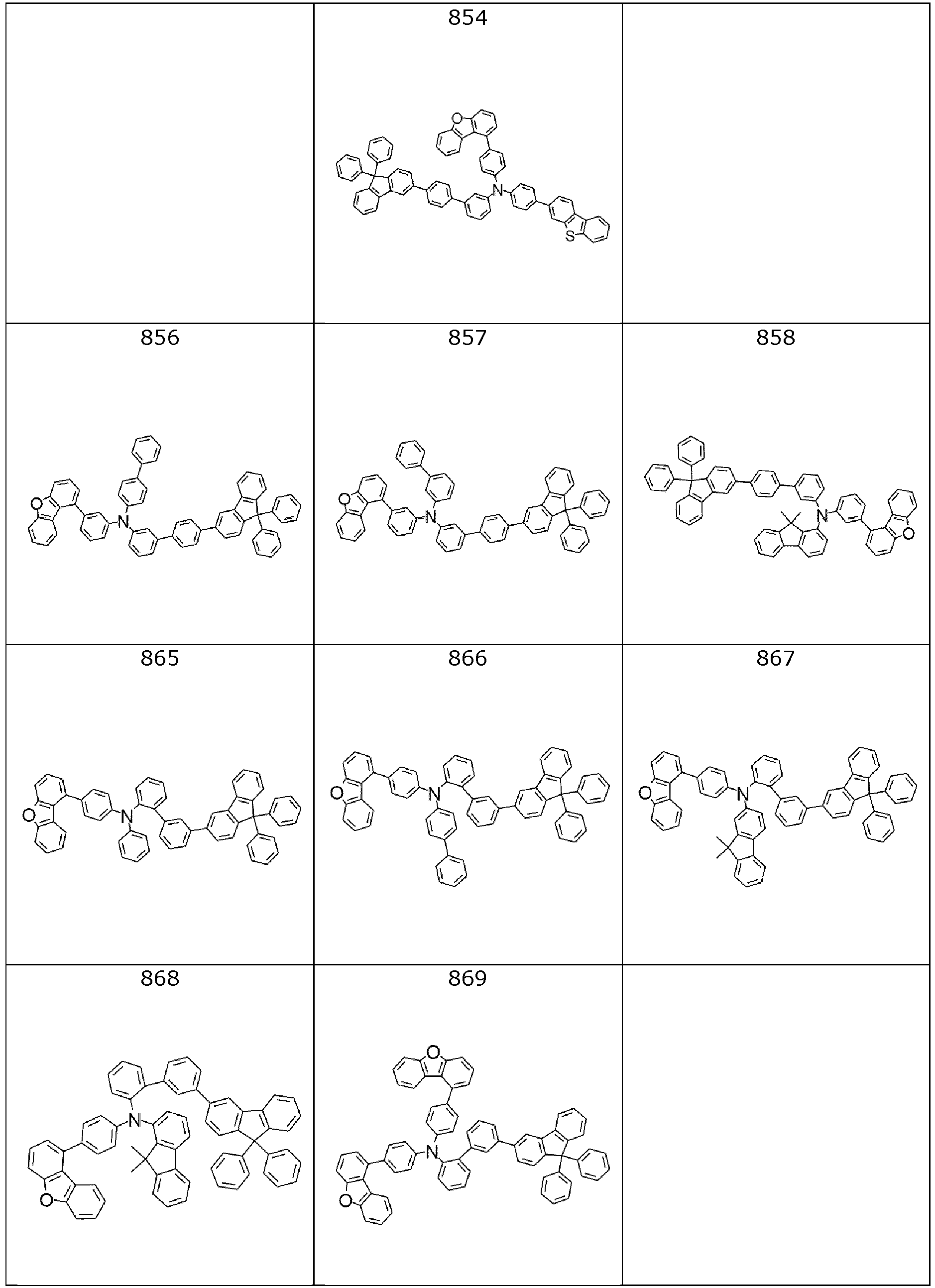

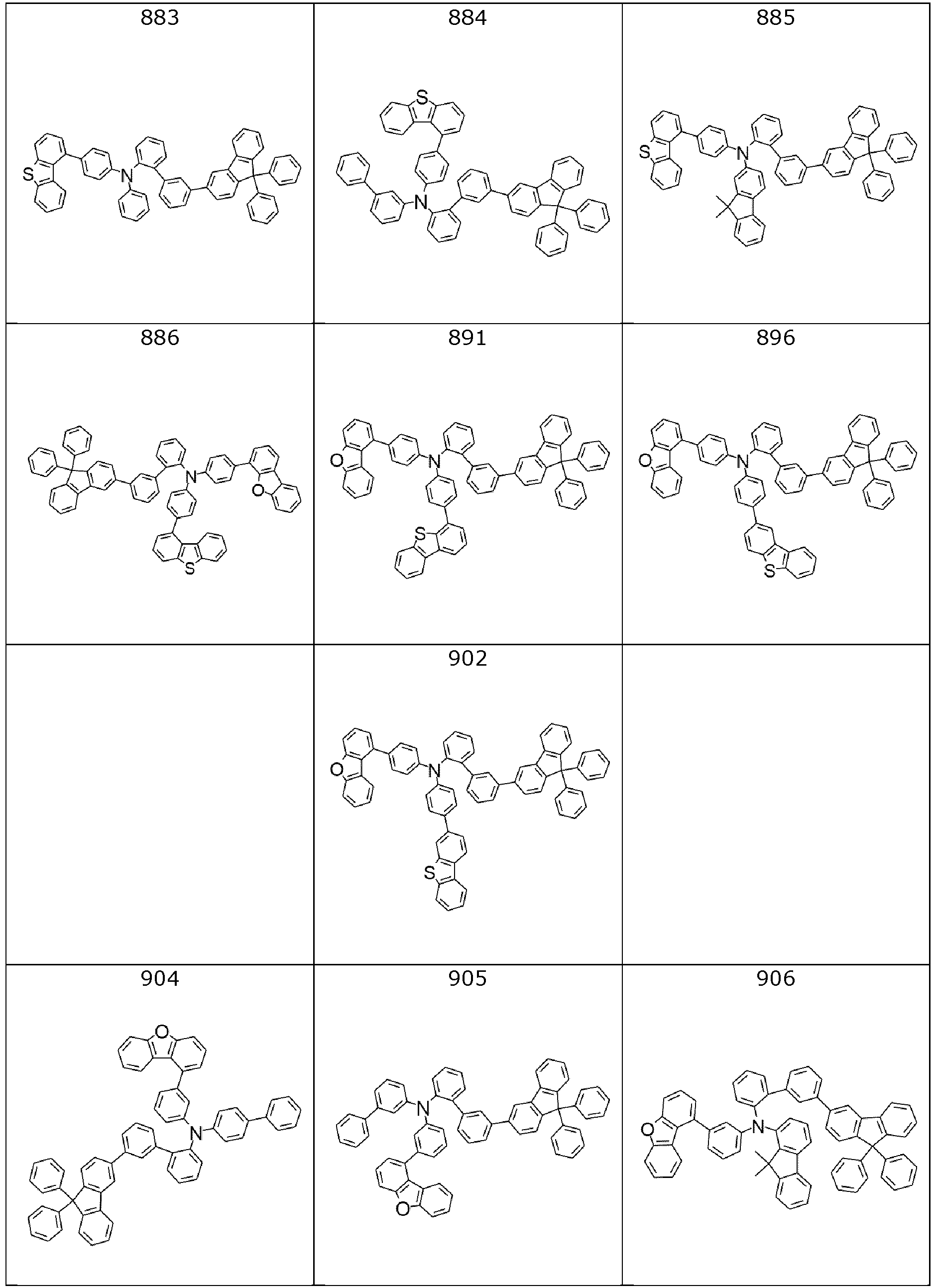

Die Verbindungen der Formel (3) können mittels üblicher Synthesemethoden der organischen Chemie hergestellt werden, beispielsweise Buchwald-Kupplungsreaktionen und Suzuki-Kupplungsreaktionen. Ganz grundsätzlich erfolgt die Synthese der erfindungsgemäßen Verbindungen also mit Verfahren, die dem Fachmann auf dem Gebiet sehr gut bekannt sind. Zunächst wird die das Fluoren mittels Suzuki Kupplung umgesetzt. In einem zweiten Schritt wird das Produkt der ersten Reaktion mittels einer Buchwald Reaktion zum endgültigen Produkt umgesetzt.

Im Folgenden ist ein bevorzugter Syntheseweg für die Verbindungen gemäß der vorliegenden Anmeldung gezeigt. Der Fachmann kann diesen Syntheseweg im Rahmen seiner allgemeinen Fachkenntnisse abwandeln.

Gegenstand der Anmeldung ist damit ein Verfahren zur Herstellung einer Verbindung der Formel (3) mittels Suzuki- und Buchwald-Kupplung.

Weiterer Gegenstand der Erfindung sind daher Oligomere, Polymere oder Dendrimere enthaltend eine oder mehrere Verbindungen gemäß Formel (3), wobei die Bindung(en) zum Polymer, Oligomer oder Dendrimer an beliebigen, in Formel (3) mit R¹, R², R³, R⁴, R⁵, R⁶, R⁷, R⁸, R¹¹ oder R¹² substituierten Positionen lokalisiert sein können. Je nach Verknüpfung der Verbindung gemäß Formel (3) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (3) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (3) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (3) in Oligomeren, Dendrimeren und Polymeren gelten dieselben Bevorzugungen wie oben für Verbindungen gemäß Formel (3) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen, Spirobifluorenen, Paraphenylenen, Carbazolen, Thiophenen, Dihydrophenanthrenen, cis- und trans-Indenofluorenen, Ketonen, Phenanthrenen oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine oder phosphoreszierende Metallkomplexe, und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (3) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur im Detail beschrieben.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, alpha-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (3) oder mindestens ein Polymer, Oligomer oder Dendrimer enthaltend mindestens eine Einheit gemäß Formel (3) sowie mindestens ein Lösungsmittel, bevorzugt ein organisches Lösungsmittel. Wie solche Lösungen hergestellt werden können, ist dem Fachmann bekannt.

Die Verbindung gemäß Formel (3) eignet sich für den Einsatz in einer elektronischen Vorrichtung, insbesondere einer organischen Elektrolumineszenzvorrichtung (OLED). Abhängig von der Substitution kann die Verbindung der Formel (3) in unterschiedlichen Funktionen und Schichten eingesetzt werden. Bevorzugt ist die Verwendung als lochtransportierendes Material in einer lochtransportierenden Schicht und/oder in einer Elektronenblockierschicht und/oder als Matrixmaterial in einer emittierenden Schicht, besonders bevorzugt in Kombination mit einem phosphoreszierenden Emitter.

Weiterer Gegenstand der Erfindung ist daher die Verwendung einer Verbindung gemäß Formel (3) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen lichtemittierenden Dioden (OLEDs).

Die erfindungsgemäßen Verbindungen eigenen sich also besonders für den Einsatz in organischen Elektrolumineszenzvorrichtungen, zu denen auch die OLEDs, OLECs, OLETs, QFQDs und O-Laser gehören.

Gegenstand der Erfindung ist weiterhin eine elektronische Vorrichtung, enthaltend mindestens eine Verbindung gemäß Formel (3). Dabei ist die elektronische Vorrichtung bevorzugt ausgewählt aus den oben genannten Vorrichtungen.

Besonders bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung enthalten ist, welche mindestens eine Verbindung gemäß Formel (3) enthält. Bevorzugt ist eine organische Elektrolumineszenzvorrichtung, enthaltend Anode, Kathode und mindestens eine emittierende Schicht, dadurch gekennzeichnet, dass mindestens eine organische Schicht in der Vorrichtung, gewählt aus lochtransportierenden und emittierenden Schichten, mindestens eine Verbindung gemäß Formel (3) enthält.

Unter einer lochtransportierenden Schicht werden dabei alle Schichten verstanden, die zwischen Anode und emittierender Schicht angeordnet sind, bevorzugt Lochinjektionsschicht, Lochtransportschicht, und Elektronenblockierschicht. Unter eine Lochinjektionsschicht wird dabei eine Schicht verstanden, die direkt an die Anode angrenzt. Unter einer Lochtransportschicht wird dabei eine Schicht verstanden, die zwischen Anode und emittierender Schicht vorliegt, aber nicht direkt an die Anode angrenzt, bevorzugt auch nicht direkt an die emittierende Schicht angrenzt. Unter einer Elektronenblockierschicht wird dabei eine Schicht verstanden, die zwischen Anode und emittierender Schicht vorliegt und direkt an die emittierende Schicht angrenzt. Eine Elektronenblockierschicht weist bevorzugt ein energetisch hoch liegendes LUMO auf und hält dadurch Elektronen von Austritt aus der emittierenden Schicht ab.

Außer Kathode, Anode und emittierender Schicht kann die elektronische Vorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der elektronischen Vorrichtung ist bevorzugt wie folgt:
- Anode-
- Lochinjektionsschicht-
- Lochtransportschicht-
- optional weitere Lochtransportschichten-
- emittierende Schicht-
- optional Lochblockierschicht-
- Elektronentransportschicht-
- Elektroneninjektionsschicht-
- Kathode-.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei jeweils eine der drei Schichten blaue, jeweils eine der drei Schichten grüne und jeweils eine der drei Schichten orangefarbene oder rote Emission zeigt. Die erfindungsgemäßen Verbindungen sind dabei bevorzugt in einer lochtransportierenden Schicht oder in der emittierenden Schicht vorhanden. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist bevorzugt, dass die Verbindung der Formel (3) als Lochtransportmaterial verwendet wird. Dabei kann die emittierende Schicht eine fluoreszierende emittierende Schicht sein, oder sie kann eine phosphoreszierende emittierende Schicht sein. Bevorzugt ist die emittierende Schicht eine blau fluoreszierende Schicht oder eine grün phosphoreszierende Schicht. Besonders bevorzugt ist die Verwendung der erfindungsgemäßen Verbindungen als lochleitendes Elektronenblockiermaterial in einer Elektronenblockierschicht.

Wenn die Vorrichtung enthaltend die Verbindung der Formel (3) eine phosphoreszierende emittierende Schicht enthält, ist es bevorzugt, dass diese Schicht zwei oder mehr, bevorzugt genau zwei, verschiedene Matrixmaterialien enthält (mixed-Matrix-System). Bevorzugte Ausführungsformen von mixed-Matrix-Systemen sind weiter unten näher beschrieben.

Wird die Verbindung gemäß Formel (3) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden.

Gemäß einer bevorzugten Ausführungsform enthält eine lochtransportierende Schicht oder eine Elektronenblockierschicht enthaltend die Verbindung der Formel (3) zusätzlich eine oder mehrere weitere lochtransportierende Verbindungen. Diese weiteren lochtransportierenden Verbindungen sind bevorzugt gewählt aus Triarylamin-Verbindungen, besonders bevorzugt aus Mono-Triarylaminverbindungen. Ganz besonders bevorzugt sind sie gewählt aus den weiter unten angegebenen bevorzugten Ausführungsformen von Lochtransportmaterialien. In der beschriebenen bevorzugten Ausführungsform sind die Verbindung der Formel (3) und die eine oder mehrere weiteren lochtransportierenden Verbindungen bevorzugt jeweils in einem Anteil von mindestens 10% vorhanden, besonders bevorzugt jeweils in einem Anteil von mindestens 20% vorhanden.

Gemäß einer bevorzugten Ausführungsform enthält eine lochtransportierende Schicht oder eine Elektronenblockierschicht enthaltend die Verbindung der Formel (3) zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoOs, WO₃ und ReO₃. Nochmals weiterhin bevorzugt sind Komplexe von Bismut in der Oxidationsstufe (III), insbesondere Bismut(III)-Komplexe mit elektronenarmen Liganden, insbesondere Carboxylat-Liganden.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden. Der p-Dotand liegt bevorzugt in einem Anteil von 1 bis 10 % in der p-dotierten Schicht vor.

Bevorzugt sind als p-Dotanden insbesondere die folgenden Verbindungen:

| | | | |
|---|---|---|---|
| 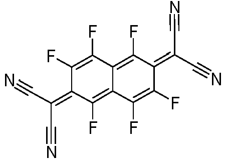 | 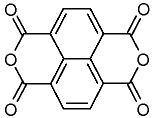 | | 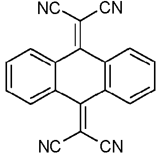 |
| (D-1) | (D-2) | | (D-3) |
| 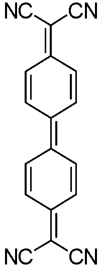 | 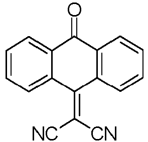 | | 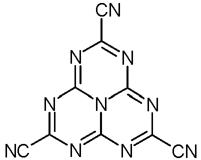 |
| (D-4) | (D-5) | | (D-6) |
| 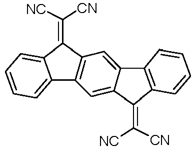 | 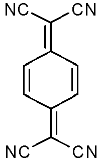 | | 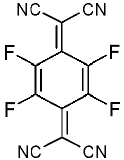 |
| (D-7) | (D-8) | | (D-9) |
| 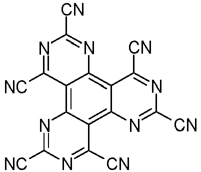 | 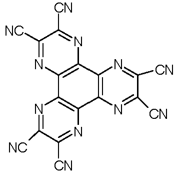 | | 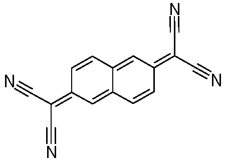 |
| (D-10) | (D-11) | | (D-12) |
| 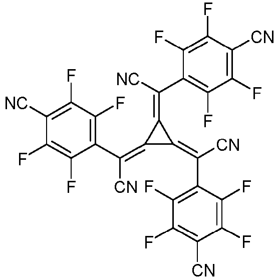 | | 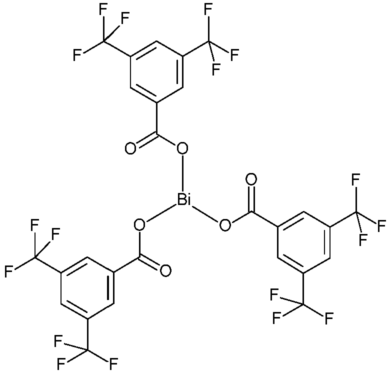 | |
| (D-13) | | (D-14) | |

Gemäß einer bevorzugten Ausführungsform ist in der Vorrichtung eine Lochinjektionsschicht vorhanden, die einer der folgenden Ausführungsformen entspricht: a) sie enthält ein Triarylamin und einen p-Dotanden; oder b) sie enthält ein einzelnes elektronenarmes Material (Elektronenakzeptor). Gemäß einer bevorzugten Ausführungsform der Ausführungsform a) ist das Triarylamin ein Mono-Triarylamin, insbesondere eines der weiter unten genannten bevorzugten Triarylamin-Derivate. Gemäß einer bevorzugten Ausführungsform der Ausführungsform b) ist das elektronenarme Material ein Hexaazatriphenylenderivat, wie in US 2007/0092755 beschrieben.

Die Verbindung der Formel (3) kann in einer Lochinjektionsschicht, in einer Lochtransportschicht, und/oder in einer Elektronenblockierschicht der Vorrichtung enthalten sein. Wenn die Verbindung in einer Lochinjektionschicht oder in einer Lochtransportschicht vorliegt, ist sie bevorzugt p-dotiert, das heißt sie liegt gemischt mit einem p-Dotanden, wie oben beschrieben, in der Schicht vor.

Besonders bevorzugt ist die Verbindung der Formel (3) in einer Elektronenblockierschicht enthalten. Bevorzugt ist sie in diesem Fall nicht p-dotiert. Weiterhin bevorzugt liegt sie in diesem Fall bevorzugt als Einzelverbindung in der Schicht vor, ohne Beimischung einer weiteren Verbindung.

Vorzugsweise wird die Verbindung der Formel (3) in der Elektronenblockierschicht der Vorrichtunge eingesetzt, wobei die Vorrichtung eine grüne Emission aufweist. Die Vorrichtung enthält dann bevorzugt wenigstens einen fluoreszierenden oder phosphoreszierenden Emitter, der grünes Licht emittiert, wobei grün emittierende phosphoreszierende Emitter bevorzugt sind.

Gemäß einer alternativen bevorzugten Ausführungsform liegt die Verbindung der Formel (3) in einer emittierenden Schicht als Matrixmaterial in Kombination mit einer oder mehreren emittierenden Verbindungen, vorzugsweise phosphoreszierenden emittierenden Verbindungen, eingesetzt. Die phosphoreszierenden emittierenden Verbindungen sind dabei bevorzugt gewählt aus rot phosphoreszierenden und grün phosphoreszierenden Verbindungen.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (3) als eine Komponente von Mixed-Matrix-Systemen, bevorzugt für phosphoreszierende Emitter, verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Bevorzugt ist weiterhin, wenn eines der Materialien gewählt ist aus Verbindungen mit großer Energiedifferenz zwischen HOMO und LUMO (Wide-Bandgap-Materialien). Die Verbindung der Formel (3) stellt in einem mixed-Matrix-System bevorzugt das Matrixmaterial mit lochtransportierenden Eigenschaften dar. Entsprechend ist, wenn die Verbindung der Formel (3) als Matrixmaterial für einen phosphoreszierenden Emitter in der emittierenden Schicht einer OLED eingesetzt wird, eine zweite Matrixverbindung in der emittierenden Schicht vorhanden, die elektronentransportierende Eigenschaften aufweist. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen.

Die erfiindungsgemäßen Verbindungen der Formel (3) können auch zusammen mit anderen Materialien als Feststoffmischung eingesetzt werden, um aus einer Quelle zur Herstellung einer Schicht einer organischen elektronischen Vorrichtung aufgedampft zu werden. Bei der zusätzlichen Komponente handelt es sich vorzugsweise um ein Lochtransportmaterial, ein Elektronenblockiermaterial oder um ein Matrixmaterial. Solche Feststoffmischungen werden auch als Premixed-Systeme bezeichnet.

Die vorliegende Anmeldung betrifft daher auch Feststoffmischungen enthaltend eine Verbindung der Formel (3) und wenigstens ein weiteres Lochtransportmaterial, ein Elektronenblockiermaterial oder ein Matrixmaterial.

Die vorliegende Anmeldung betrifft auch ein Verfahren zur Herstellung einer Schicht einer organischen elektronischen Vorrichtung durch Verdampfen einer Feststoffmischungen enthaltend eine Verbindung der Formel (3) und wenigstens ein weiteres Lochtransportmaterial, ein Elektronenblockiermaterial oder ein Matrixmaterial.

Ein weiterer Gegenstand der vorliegenden Anmeldung sind Mischungen oder Zusammensetzungen enthaltend eine oder mehrere Verbindungen der Formel (3) sowie wenigstens ein weiteres Material, das ausgewählt ist aus der Gruppe bestehend aus den Lochtransportmaterialien, Lochinjektionsmaterialien, p-Dotanden, Elektronenblockiermaterialien, Matrixmaterialien, Emittern und Elektronentransportmaterialien. Die eingestzten Materialien sind dem Fachman gut bekannt und können grundsätzlich alle zu diesem Zweck eingesetzt werden. Dazu eignen sich insbesondere die Materialien, die an anderer Stelle in der vorliegenden Anmeldung genannt wurden. Der Emitter kann ein fluoreszierender oder phosphoreszierender Emitter sein, wobei phosphoreszierende Emitter bevorzugt sind. Die Matrixmaterialien umfassen typischerweise die lochleitenden und die elektronenleitenden Matrixmaterialien, aber auch die bipolaren Matrixmaterialien sowie die sogenannten Wide Band Gap Materialien, also solche Materialien, die als Matrixmaterial eingesetzt werden und eine große Bandlücke (d.h. HOMO-LUMO Abstand) aufweisen, die vorzugsweise größer oder gleich 3,0 eV beträgt.

In den oben genannten Schichten der Vorrichtung werden bevorzugt die folgenden Materialklassen eingesetzt:

### Phosphoreszierende Emitter:

Vom Begriff phosphoreszierende Emitter sind typischerweise Verbindungen umfasst, bei denen die Lichtemission durch einen Spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Weitere Beispiele für geeignete phosphoreszierende Emitter sind in der folgenden Tabelle gezeigt:

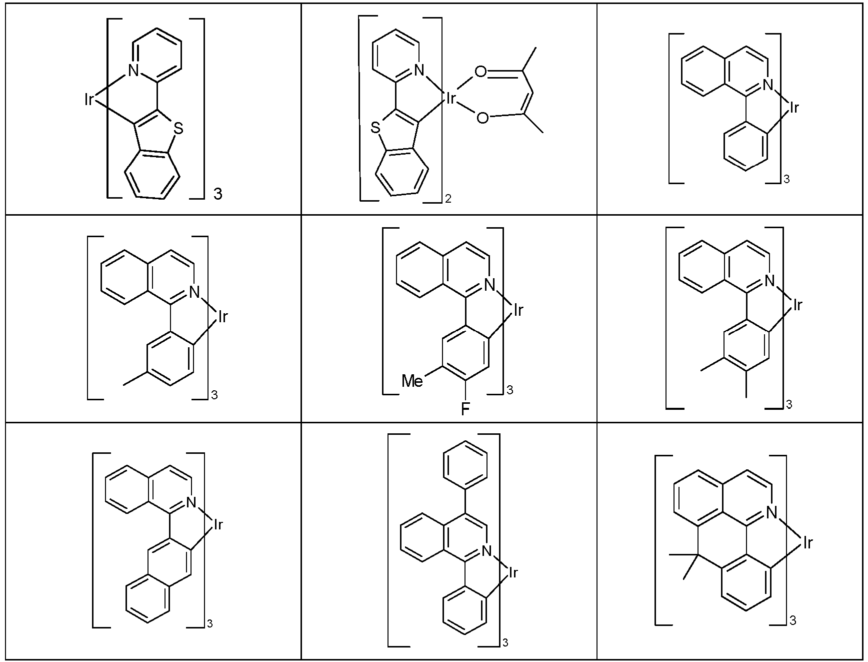

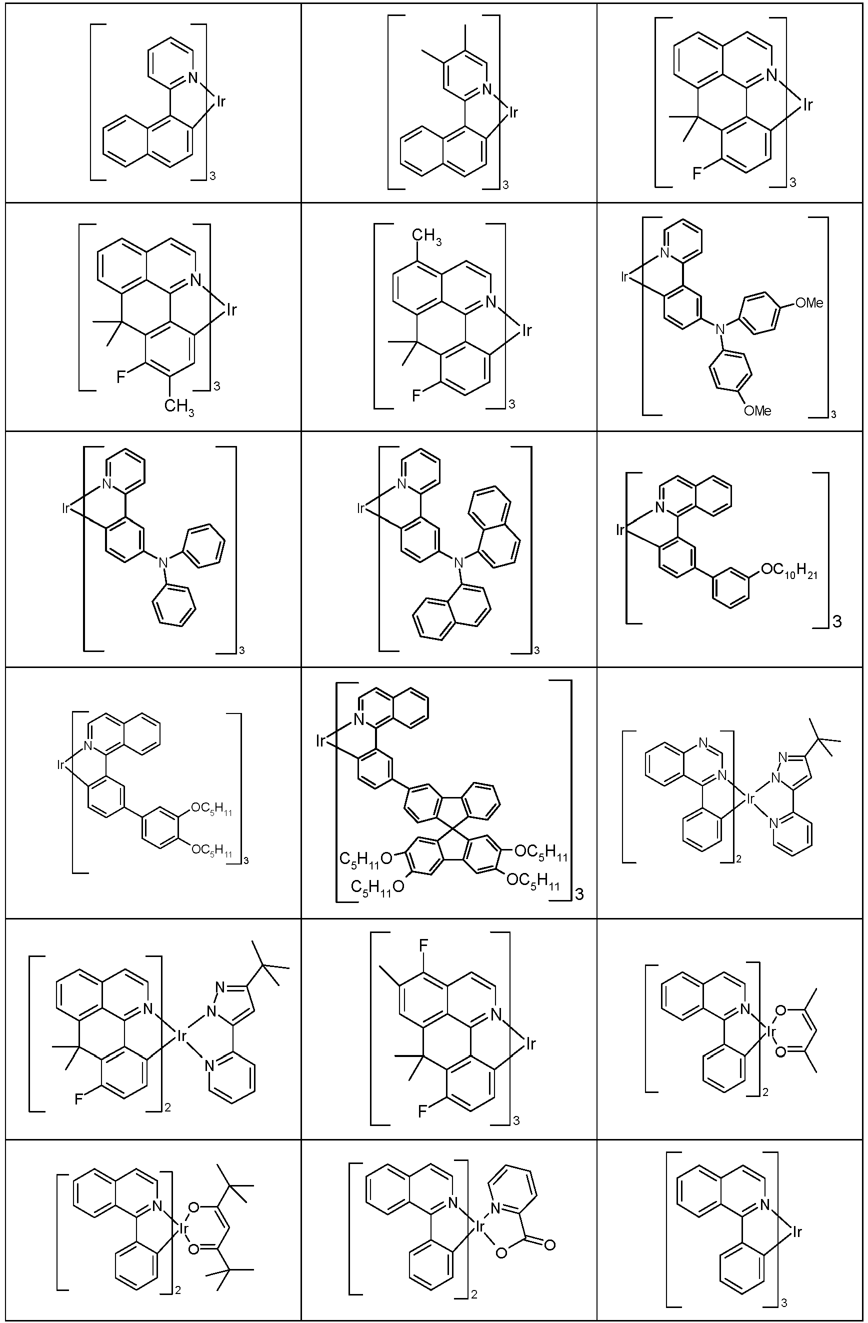

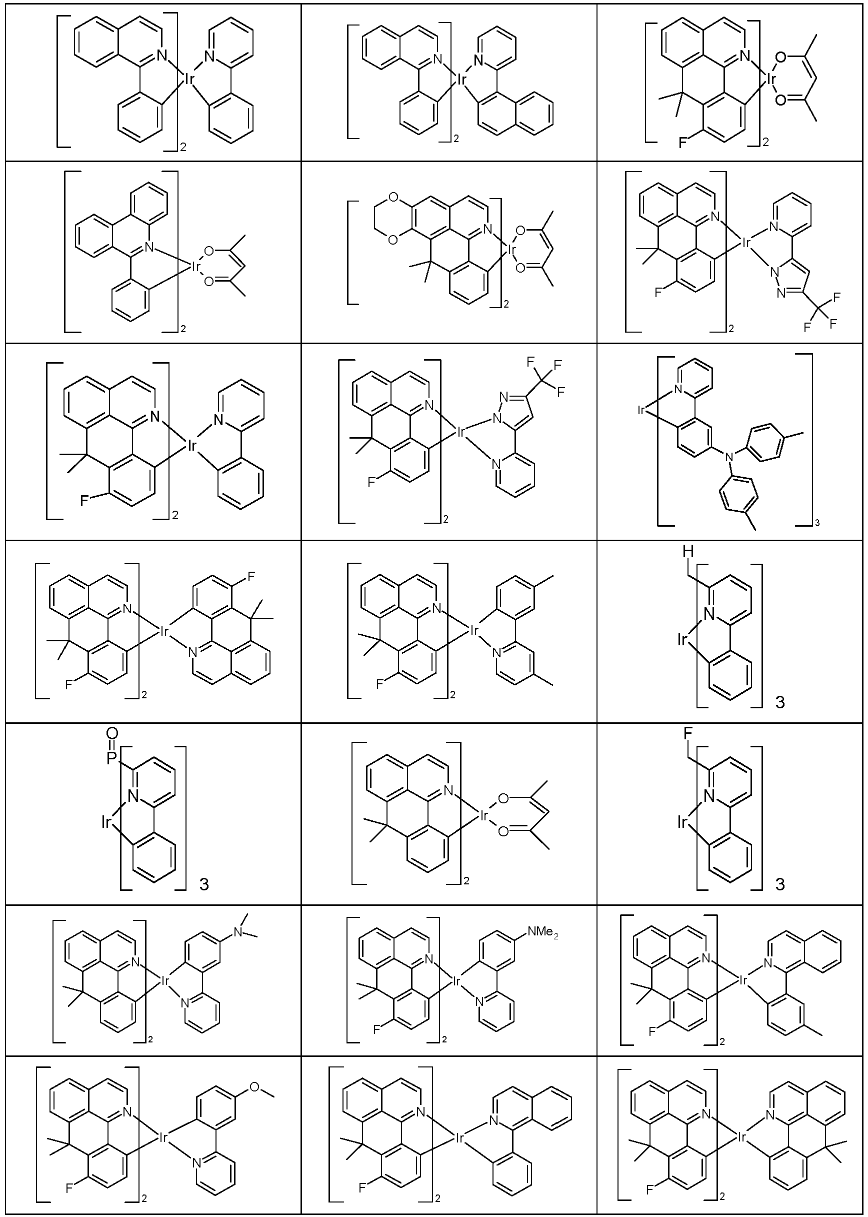

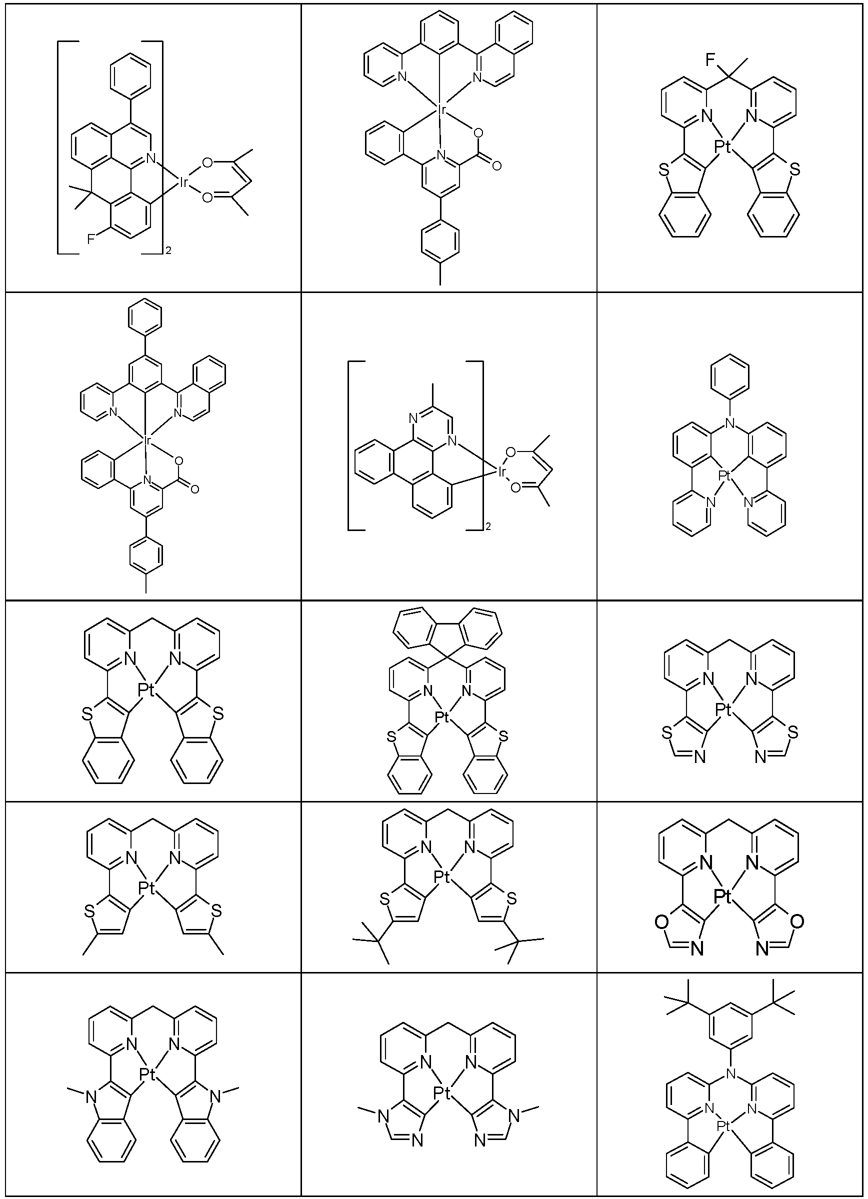

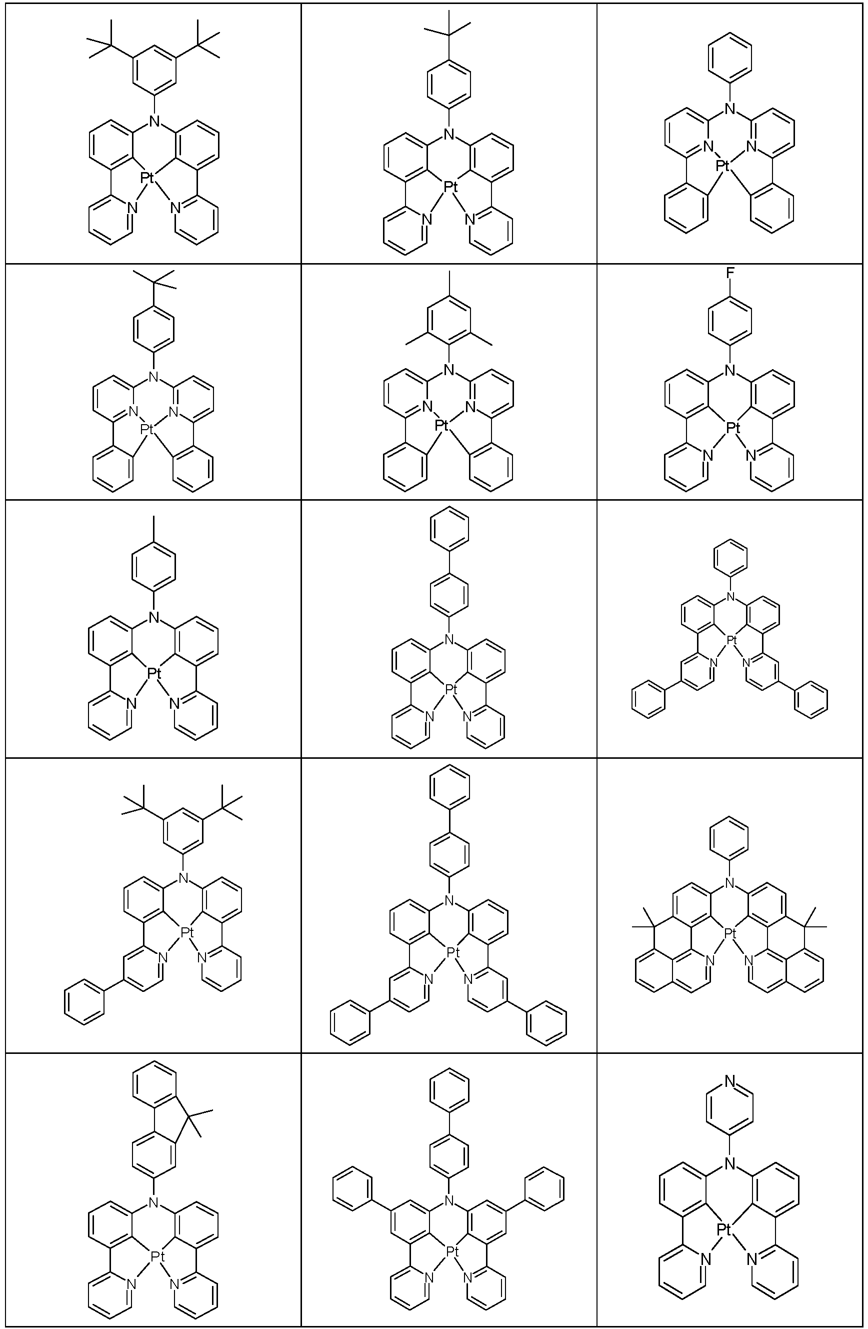

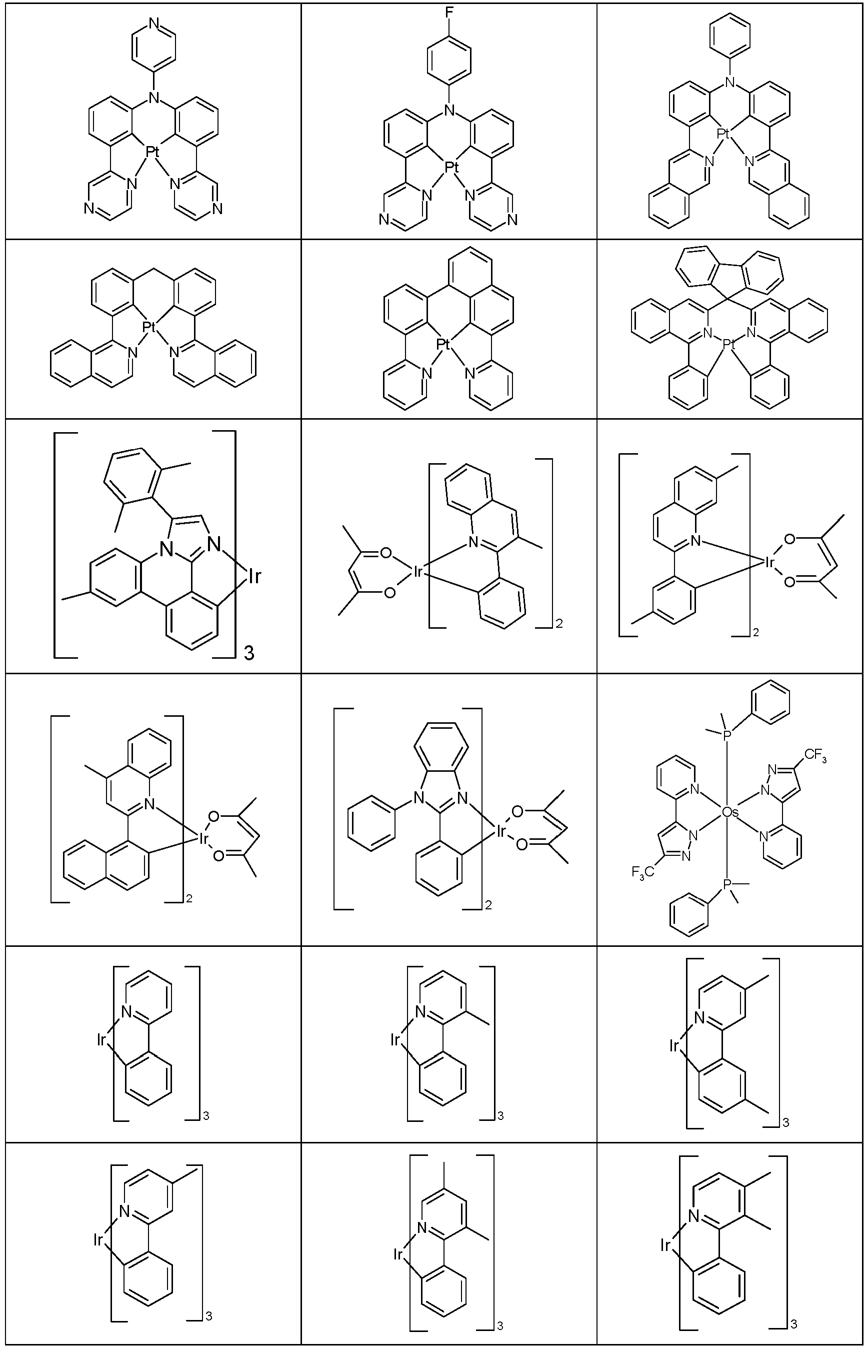

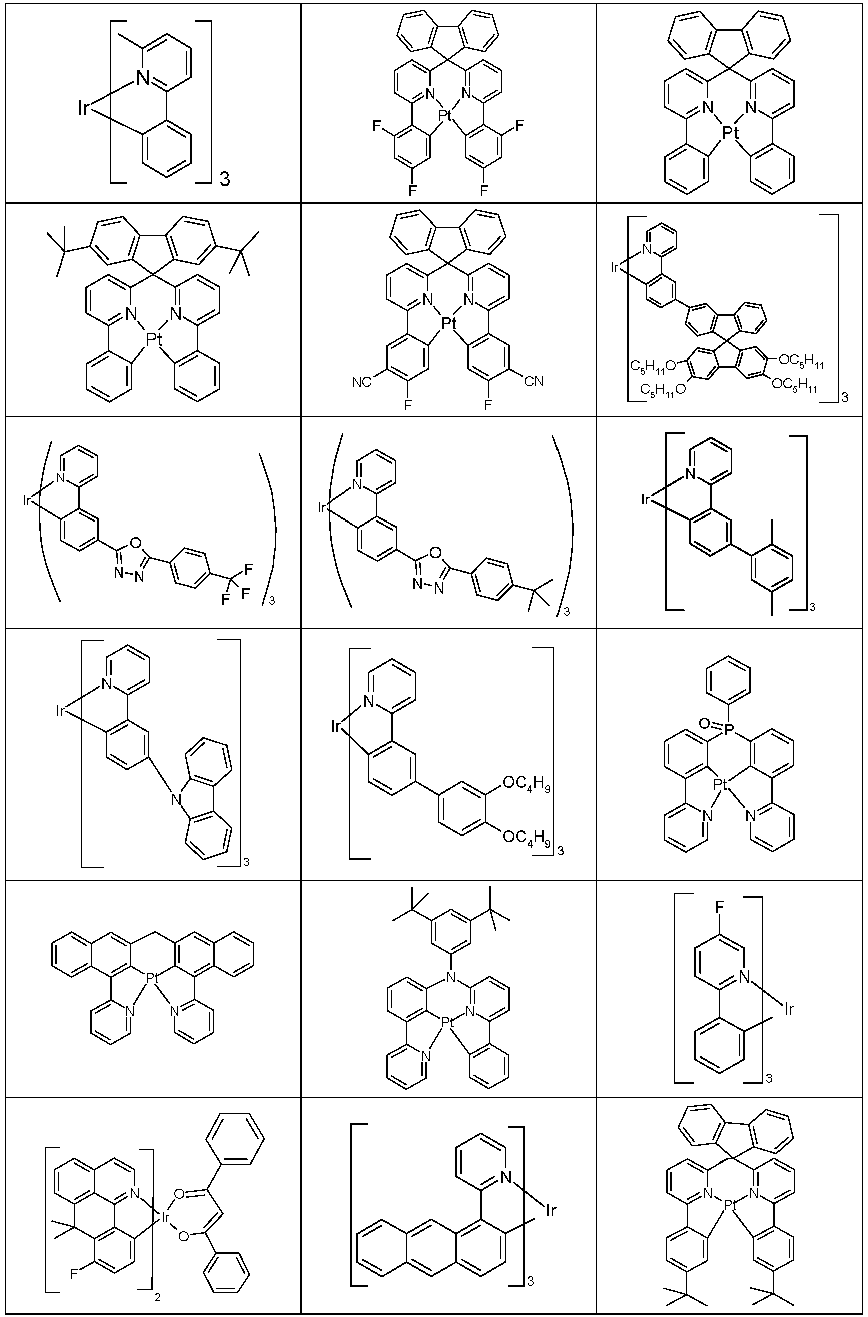

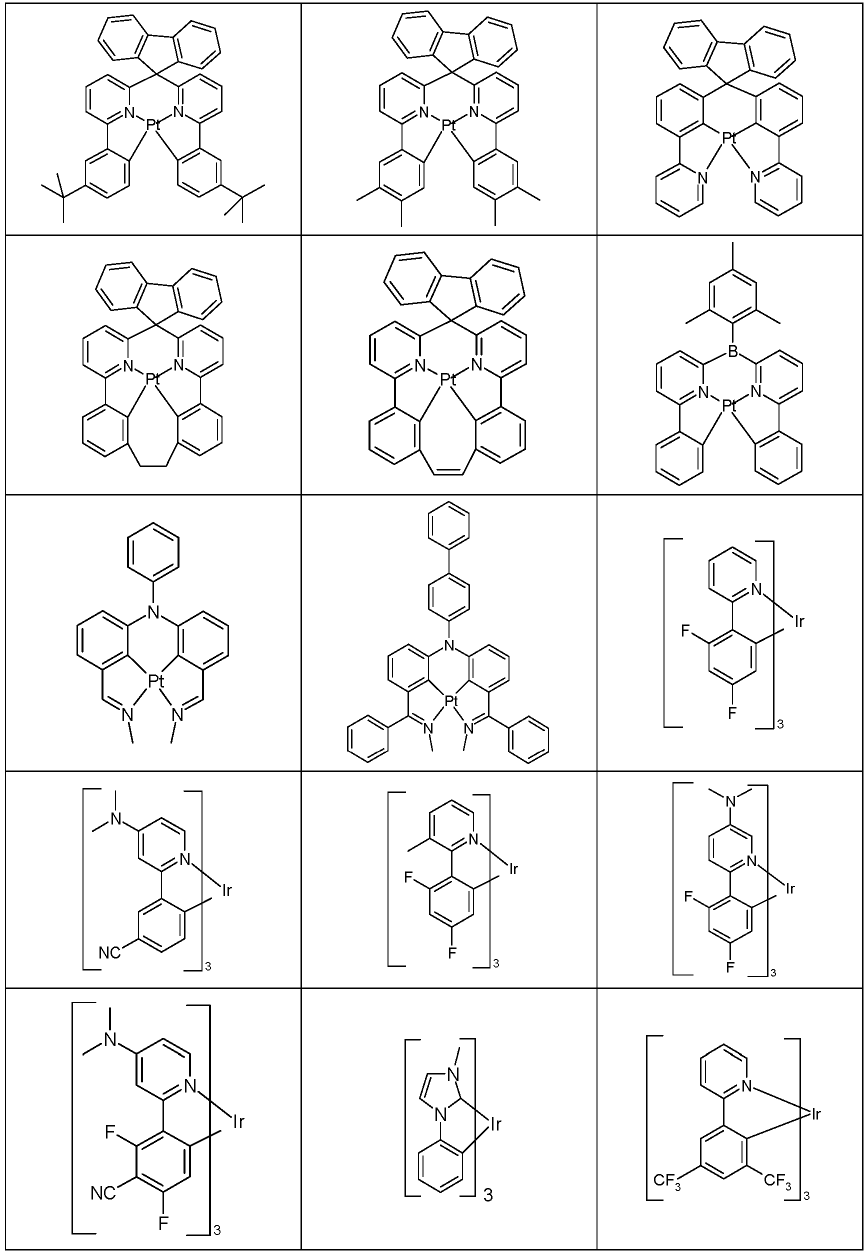

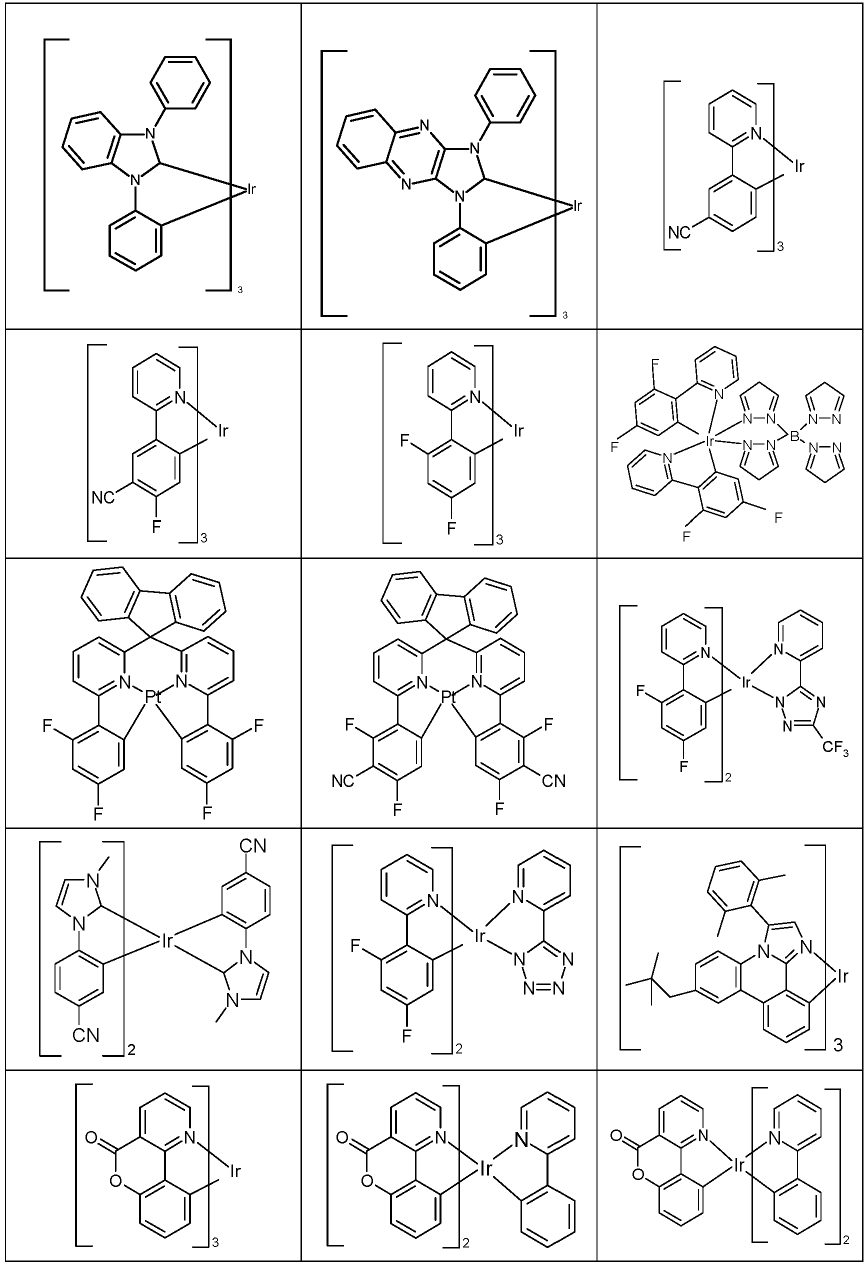

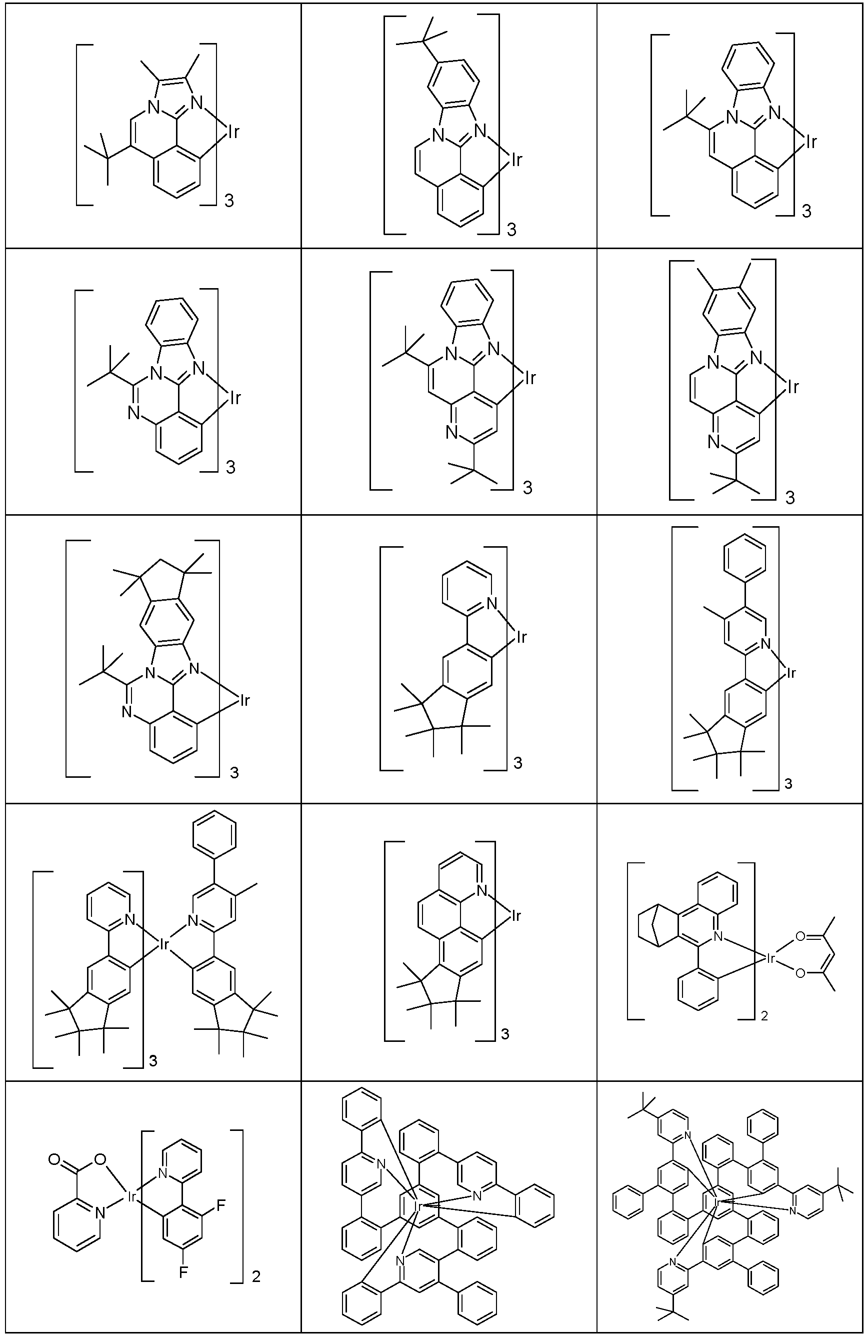

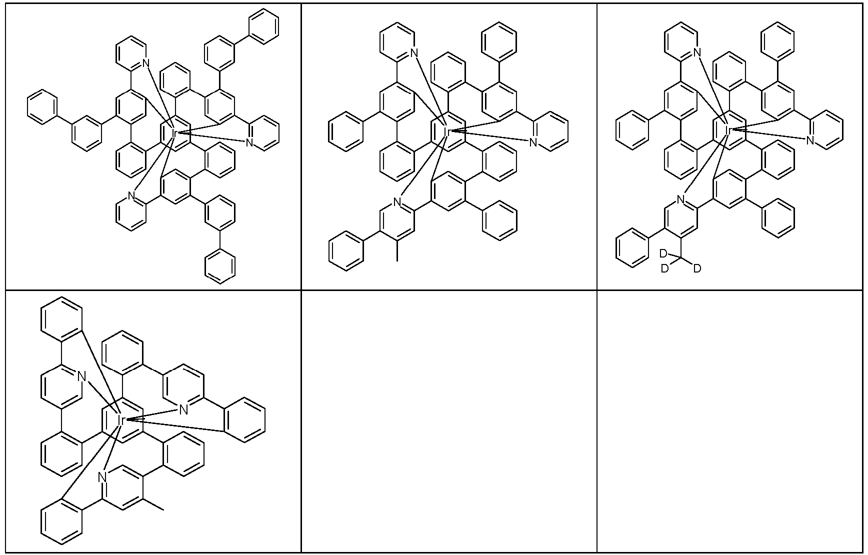

### Fluoreszierende Emitter:

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position.

Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, Benzoindenofluorenamine bzw. -diamine, und Dibenzoindenofluorenamine bzw. -diamine, sowie Indenofluorenderivate mit kondensierten Arylgruppen. Ebenfalls bevorzugt sind Pyren-Arylamine. Ebenfalls bevorzugt sind Benzoindenofluoren-Amine, Benzofluoren-Amine, erweiterte Benzoindenofluorene, Phenoxazine, und Fluoren-Derivate, die mit Furan-Einheiten oder mit Thiophen-Einheiten verbunden sind. Beispiele für fluoreszierende Emitter sind in der folgenden Tabelle abgebildet:

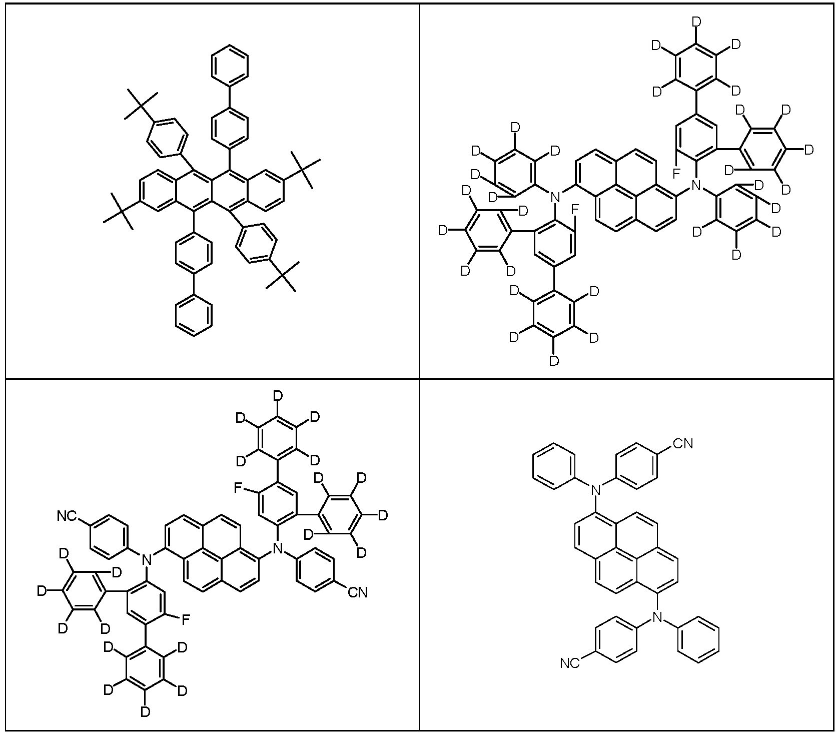

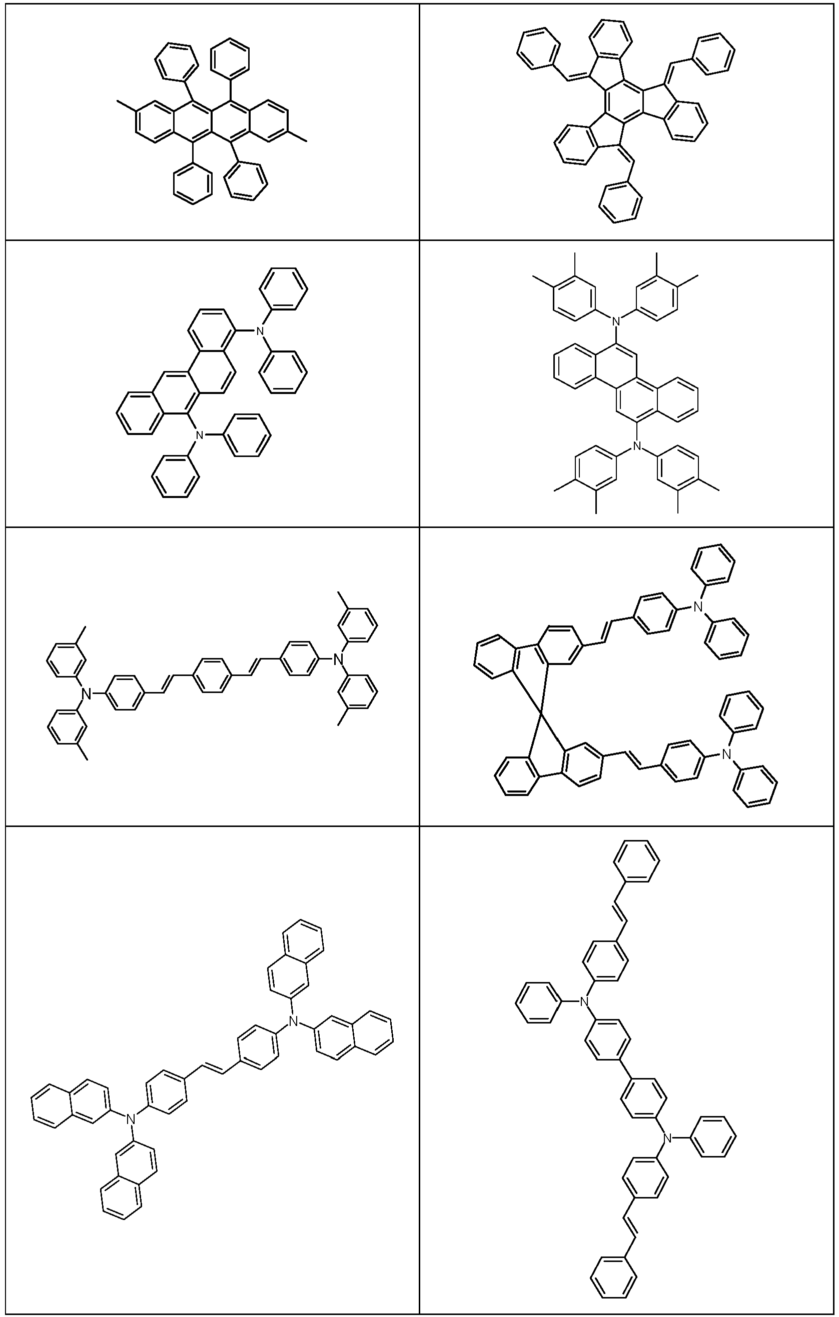

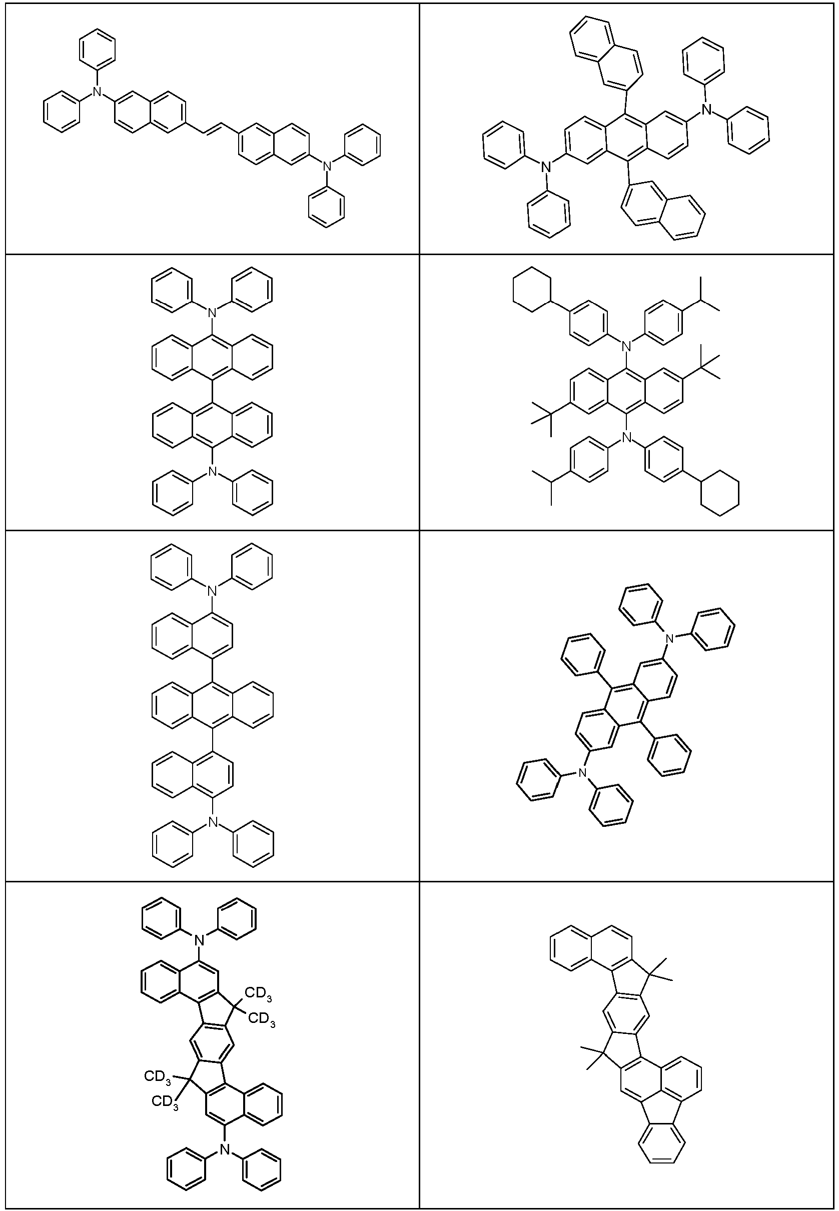

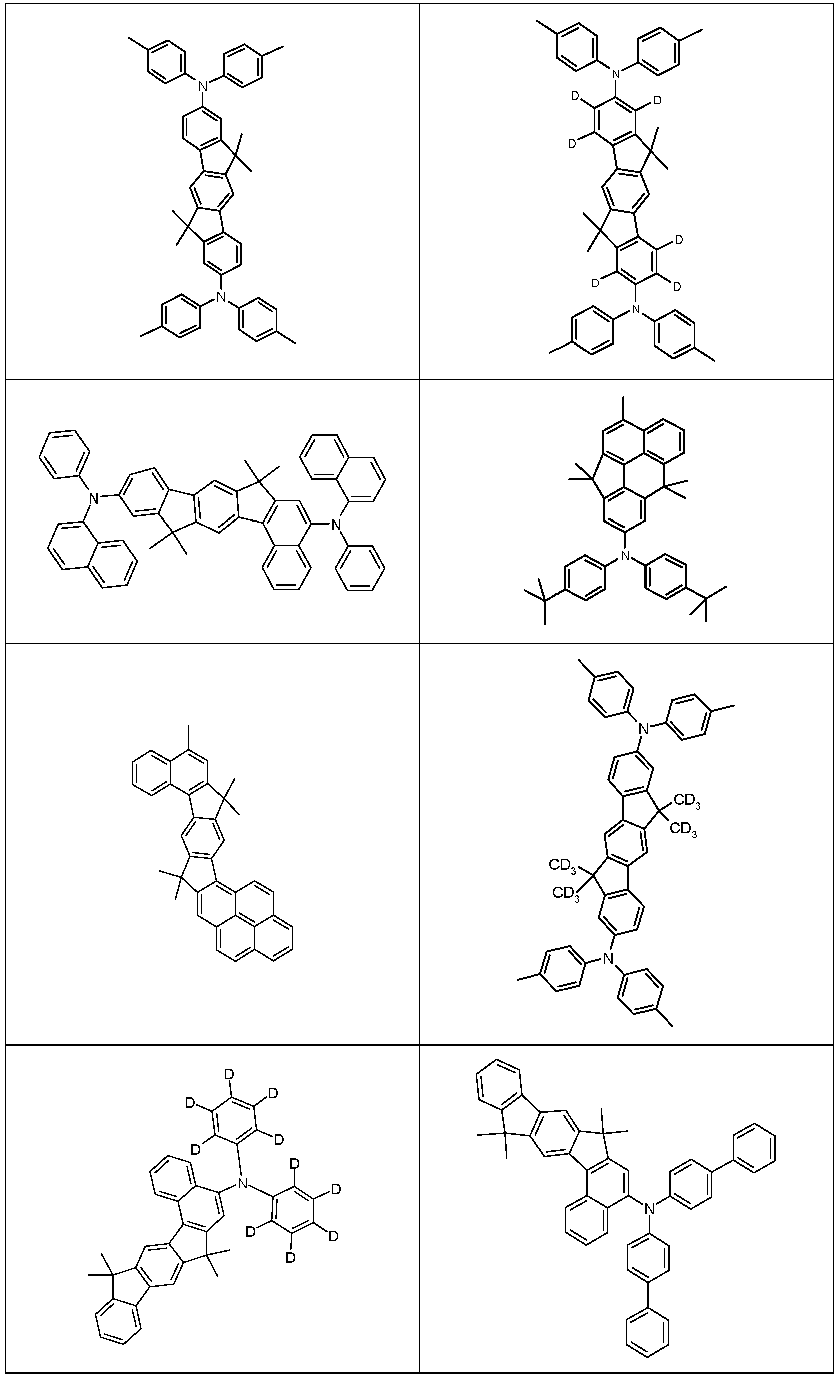

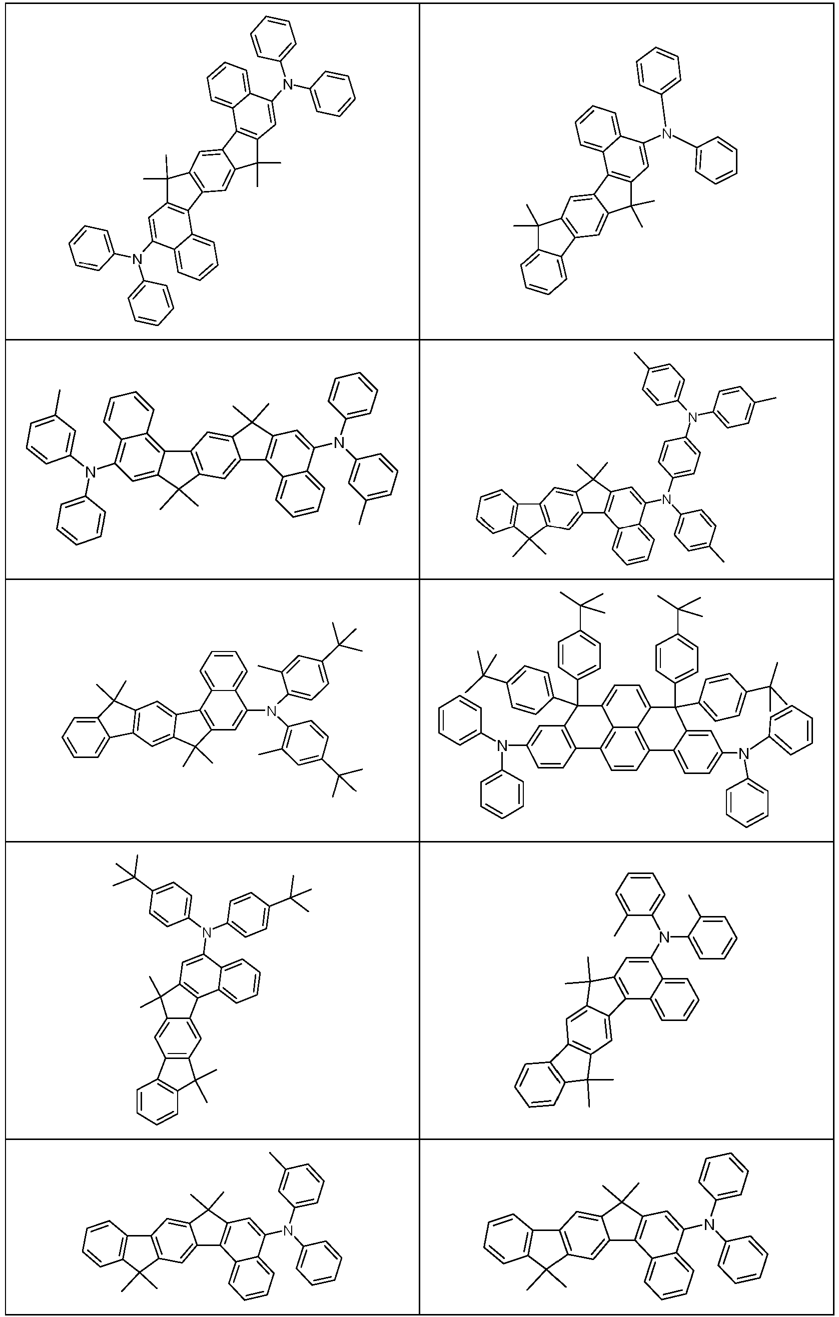

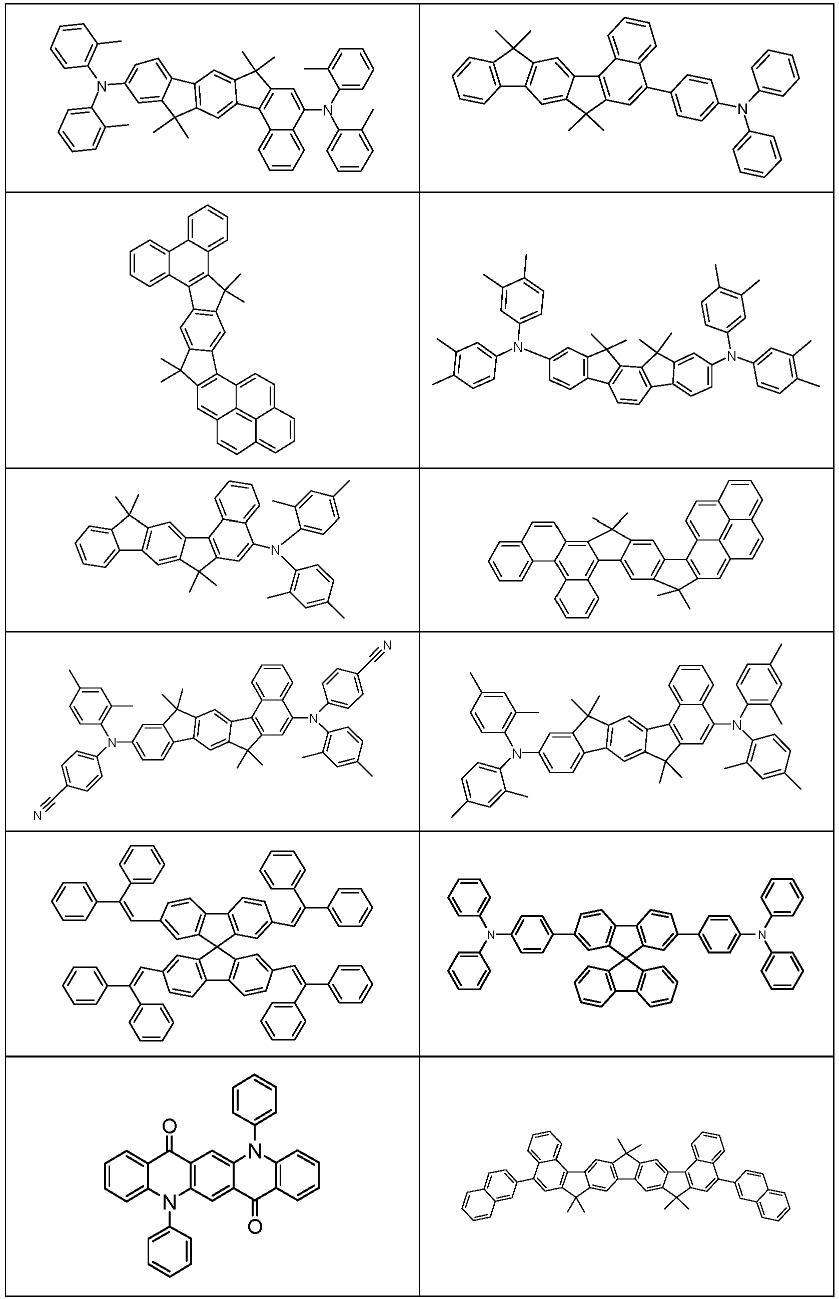

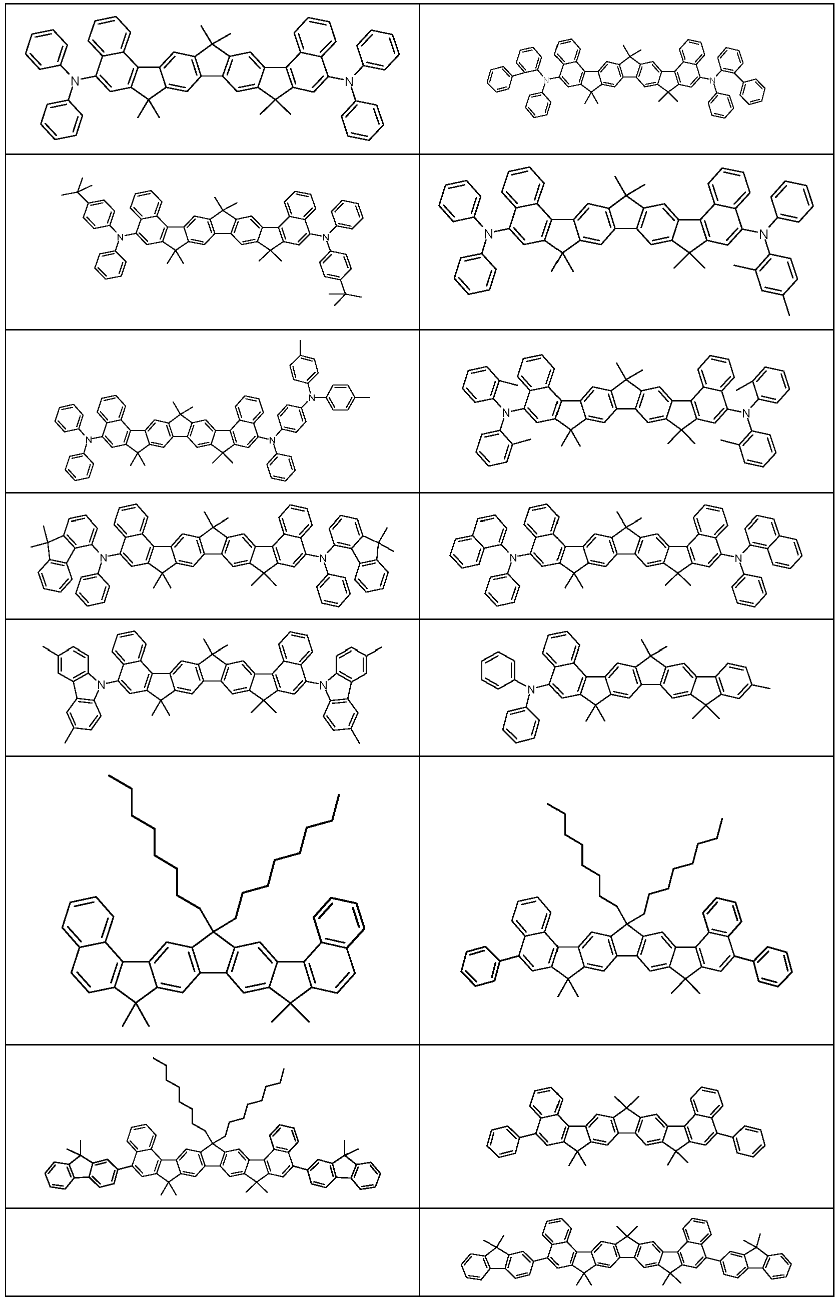

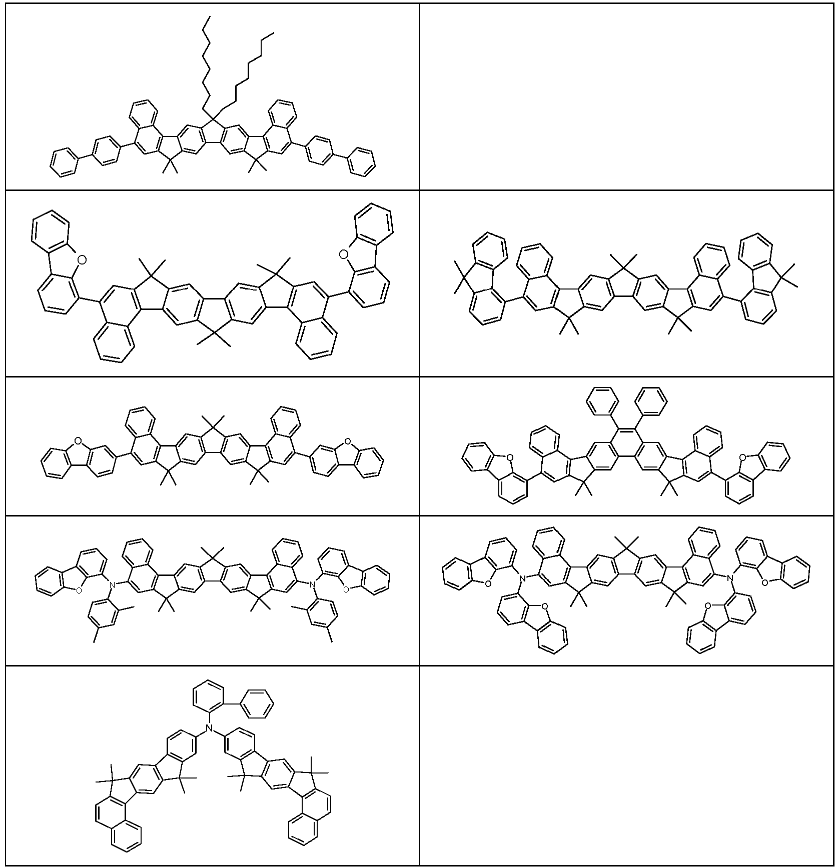

### Matrixmaterialien für fluoreszierende Emitter:

Bevorzugte Matrixmaterialien für fluoreszierende Emitter sind ausgewählt aus den Klassen der Oligoarylene (z. B. 2,2',7,7'-Tetraphenylspirobifluoren), insbesondere der Oligoarylene enthaltend kondensierte aromatische Gruppen, der Oligoarylenvinylene, der polypodalen Metallkomplexe, der lochleitenden Verbindungen, der elektronenleitenden Verbindungen, insbesondere Ketone, Phosphinoxide, und Sulfoxide; der Atropisomere, der Boronsäurederivate oder der Benzanthracene. Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugte Matrixmaterialien für fluoreszierende Emitter sind in der folgenden Tabelle abgebildet:

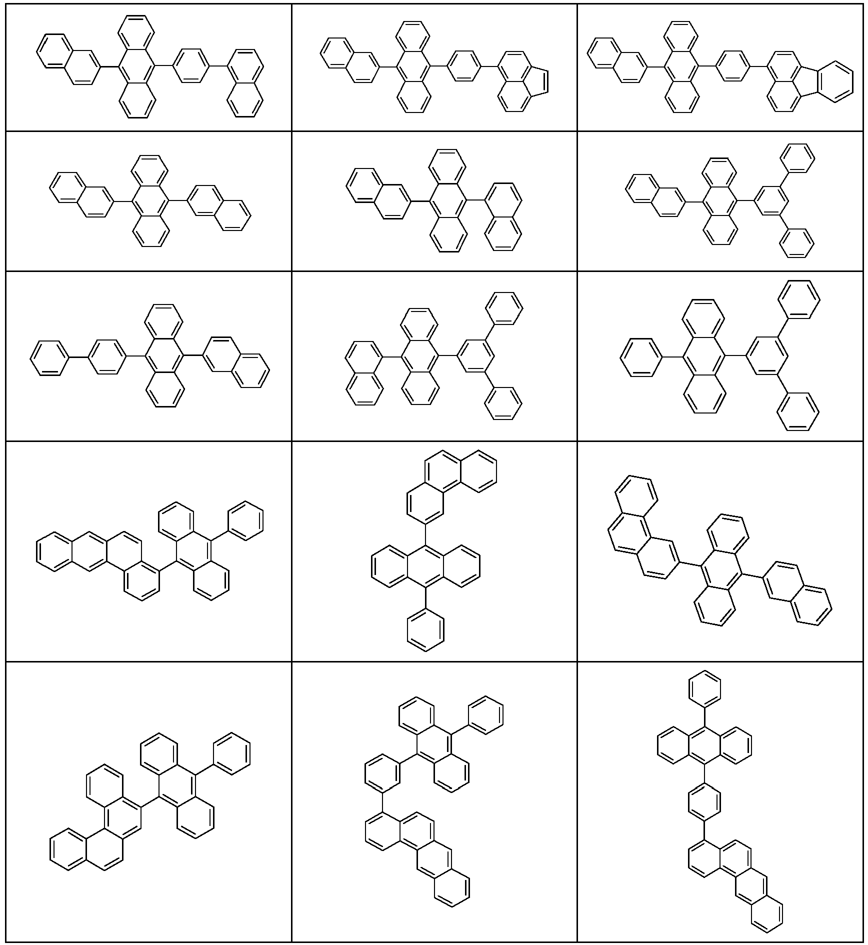

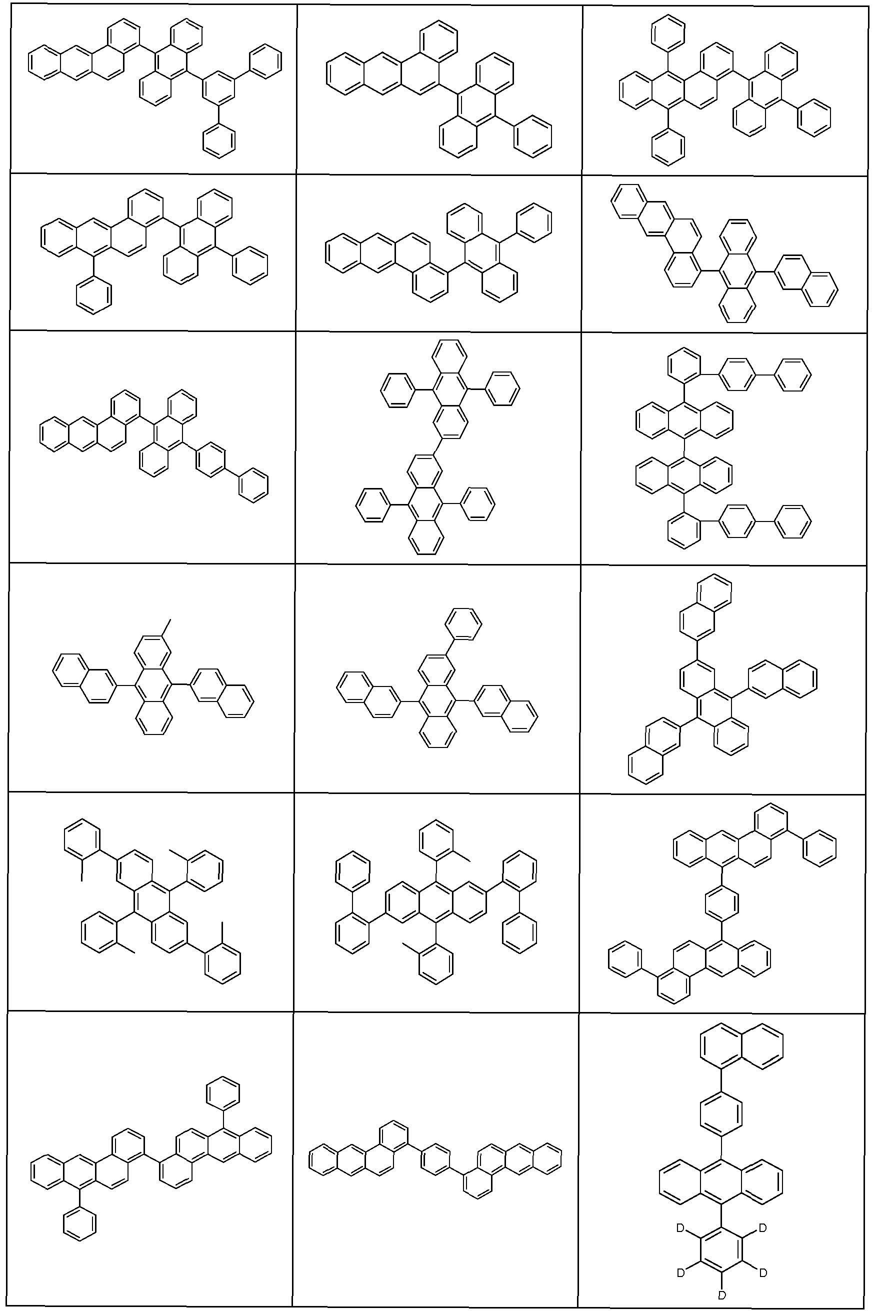

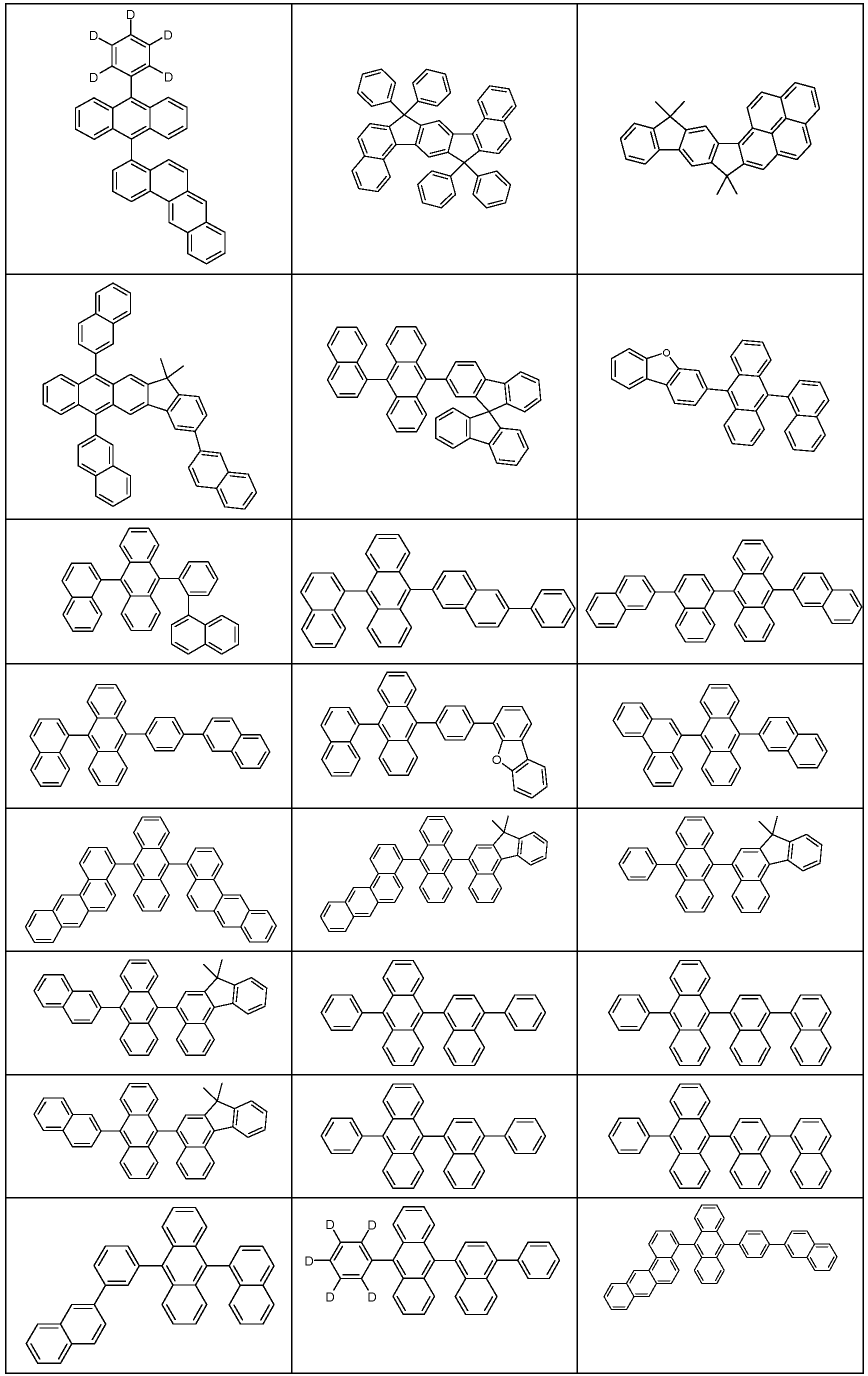

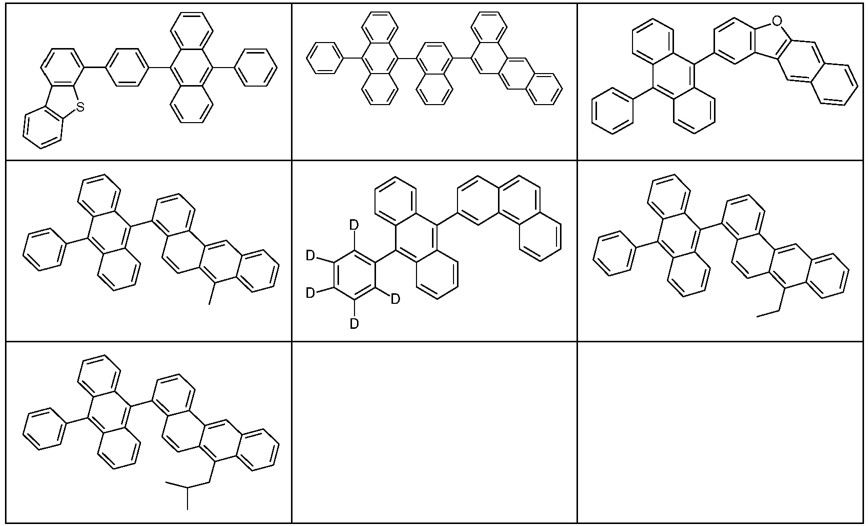

### Matrixmaterialien für phosphoreszierende Emitter:

Bevorzugte Matrixmaterialien für phosphoreszierende Emitter sind neben den Verbindungen der Formel (3) aromatische Ketone, aromatische Phosphinoxide oder aromatische Sulfoxide oder Sulfone, Triarylamine, Carbazolderivate, z. B. CBP (N,N-Biscarbazolylbiphenyl) oder Carbazol-derivate, Indolocarbazolderivate, Indenocarbazolderivate, Azacarbazolderivate, bipolare Matrixmaterialien, Silane, Azaborole oder Boronester, Triazinderivate, Zinkkomplexe, Diazasilol- bzw. Tetraazasilol-Derivate, Diazaphosphol-Derivate, überbrückte Carbazol-Derivate, Triphenylenderivate, oder Lactame.

Geeignete Matrixmaterialien für phosphoreszierende emittierende Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarba-zolylbiphenyl), m-CBP oder die in WO 2005/039246, US 2005/0069729, JP 2004/288381, EP 1205527, WO 2008/086851 oder US 2009/0134784 offenbarten Carbazolderivate, verbrückte Carbazolderivate, z. B. gemäß US 2009/0136779, WO 2010/050778, WO 2011/042107 oder WO 2011/088877, Biscarbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015, Dibenzothiophenderivate oder Triphenylenderivate, oder Imidazo-imidazolderivate, z. B. gemäß US 2016/0190480, US 2019/0273211 oder WO 2011/160757.

Beispiele für geeignete Matrixmaterialien sind die nachfolgend abgebildeten Verbindungen:

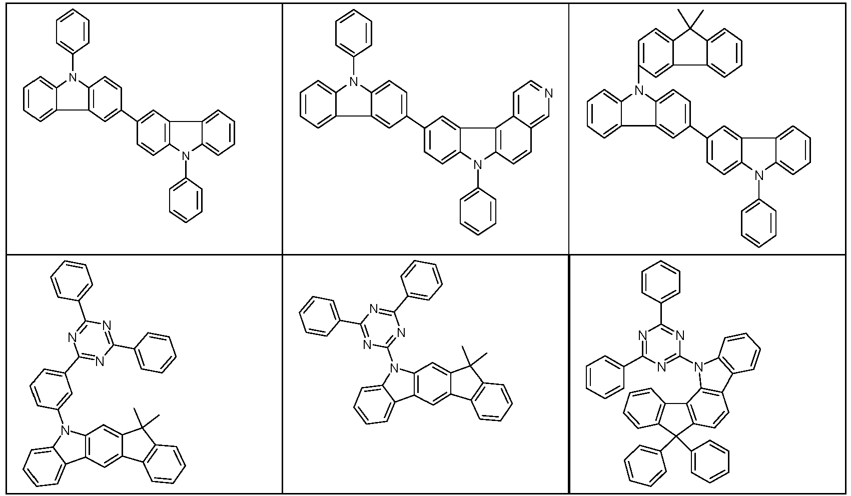

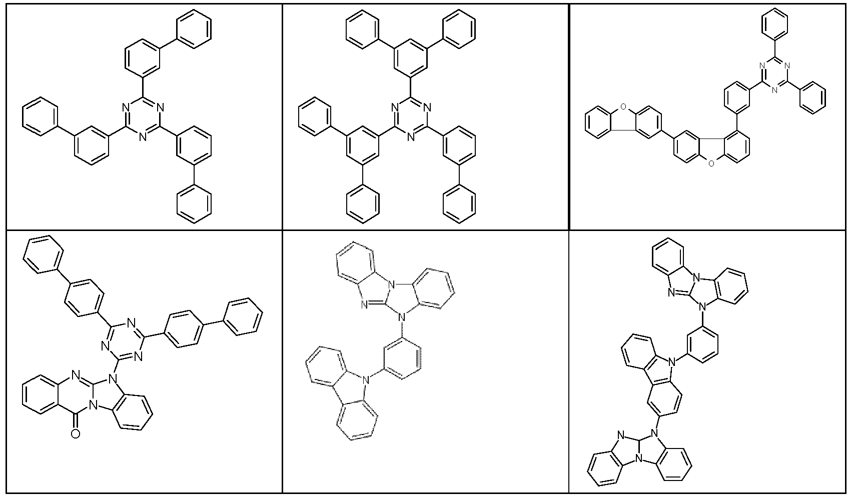

### Elektronentransportierende Materialien:

Geeignete elektronentransportierende Materialien sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, die gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Bevorzugte elektronentransportierende Verbindungen sind in der folgenden Tabelle gezeigt:

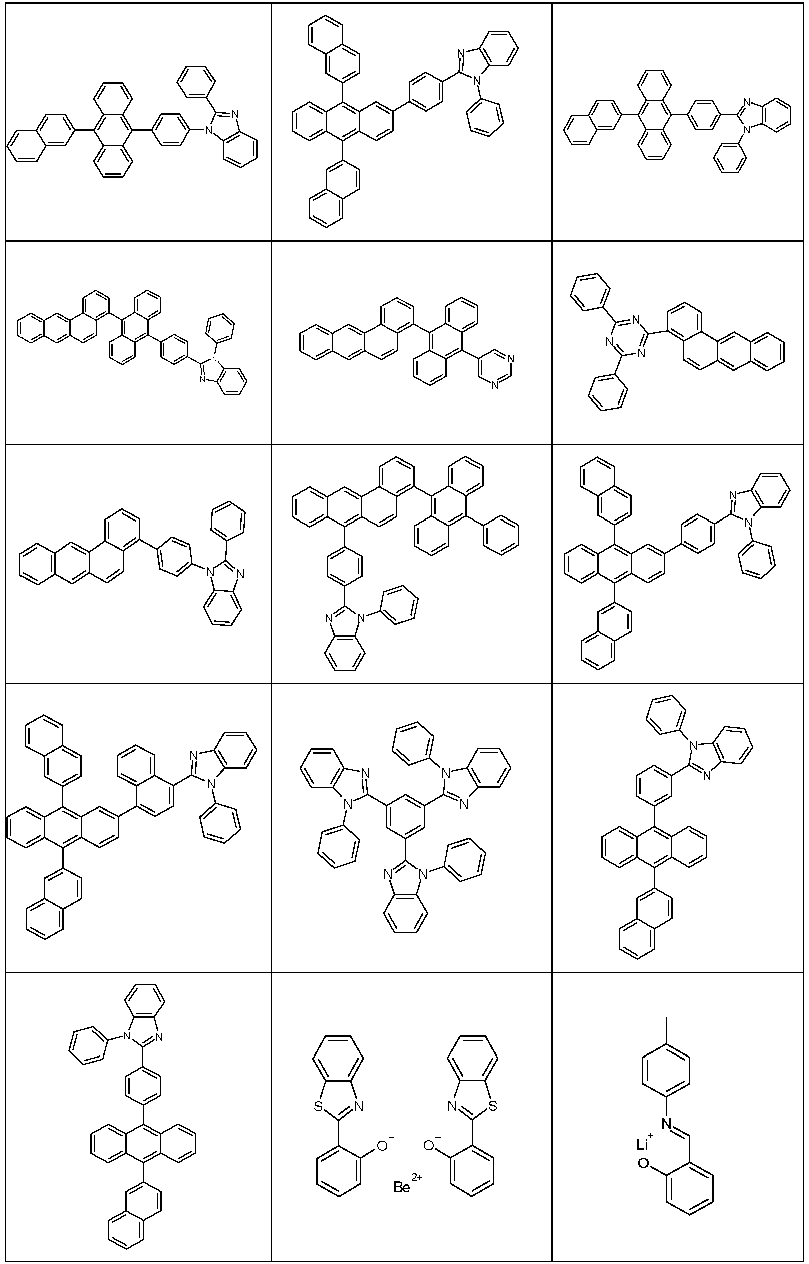

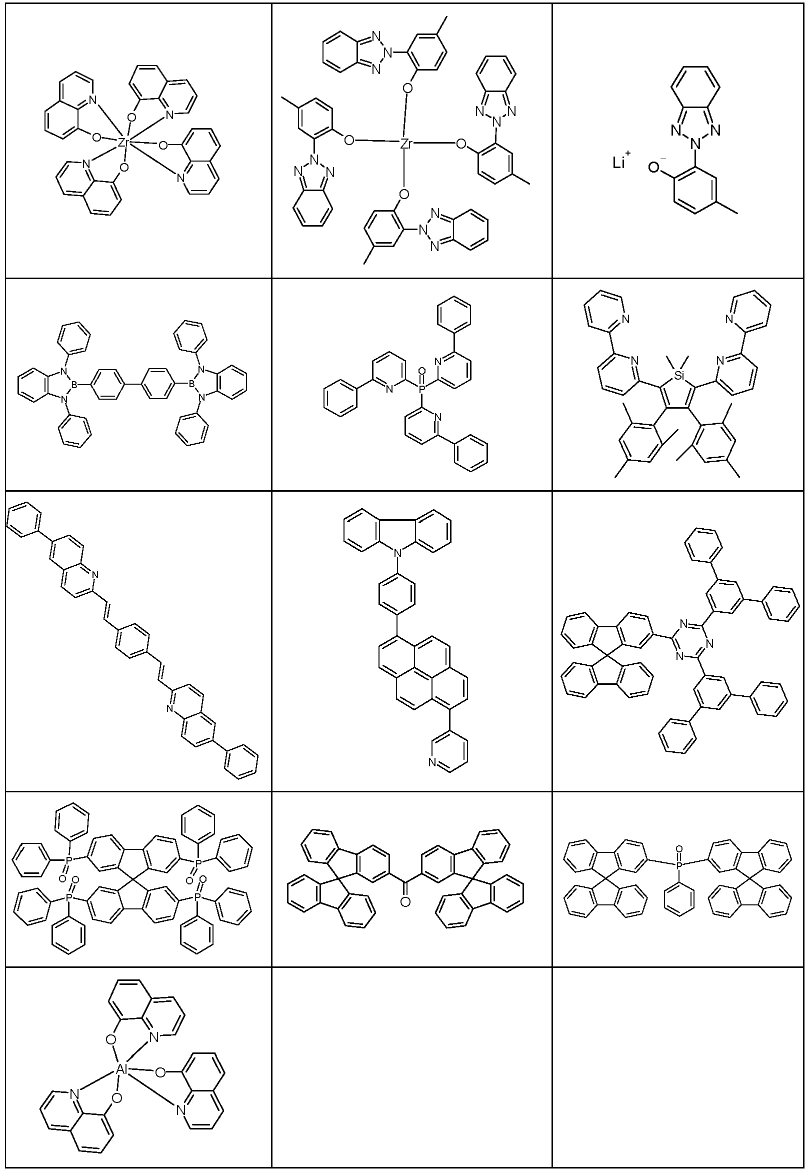

### Lochtransportierende Materialien:

Weitere Verbindungen, die neben den Verbindungen der Formel (3) bevorzugt in lochtransportierenden Schichten der erfindungsgemäßen OLEDs eingesetzt werden, sind Indenofluorenamin-Derivate, Aminderivate, Hexaazatriphenylenderivate, Aminderivate mit kondensierten Aromaten, Monobenzoindenofluorenamine, Dibenzoindenofluorenamine, Spirobifluoren-Amine, Fluoren-Amine, Spiro-Dibenzopyran-Amine, Dihydroacridin-Derivate, Spirodibenzofurane und Spirodibenzothiophene, Phenanthren-Diarylamine, Spiro-Tribenzotropolone, Spirobifluorene mit meta-Phenyldiamingruppen, Spiro-Bisacridine, Xanthen-Diarylamine, und 9,10-Dihydroanthracen-Spiroverbindungen mit Diarylaminogruppen. Bevorzugte Lochtransportierende Verbindungen sind in der folgenden Tabelle gezeigt:

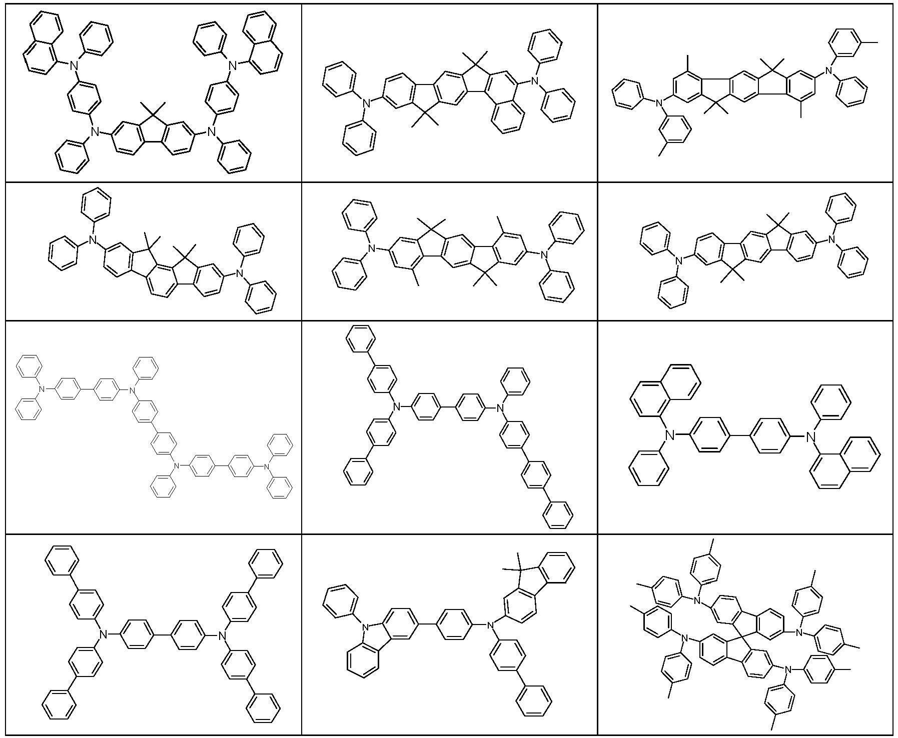

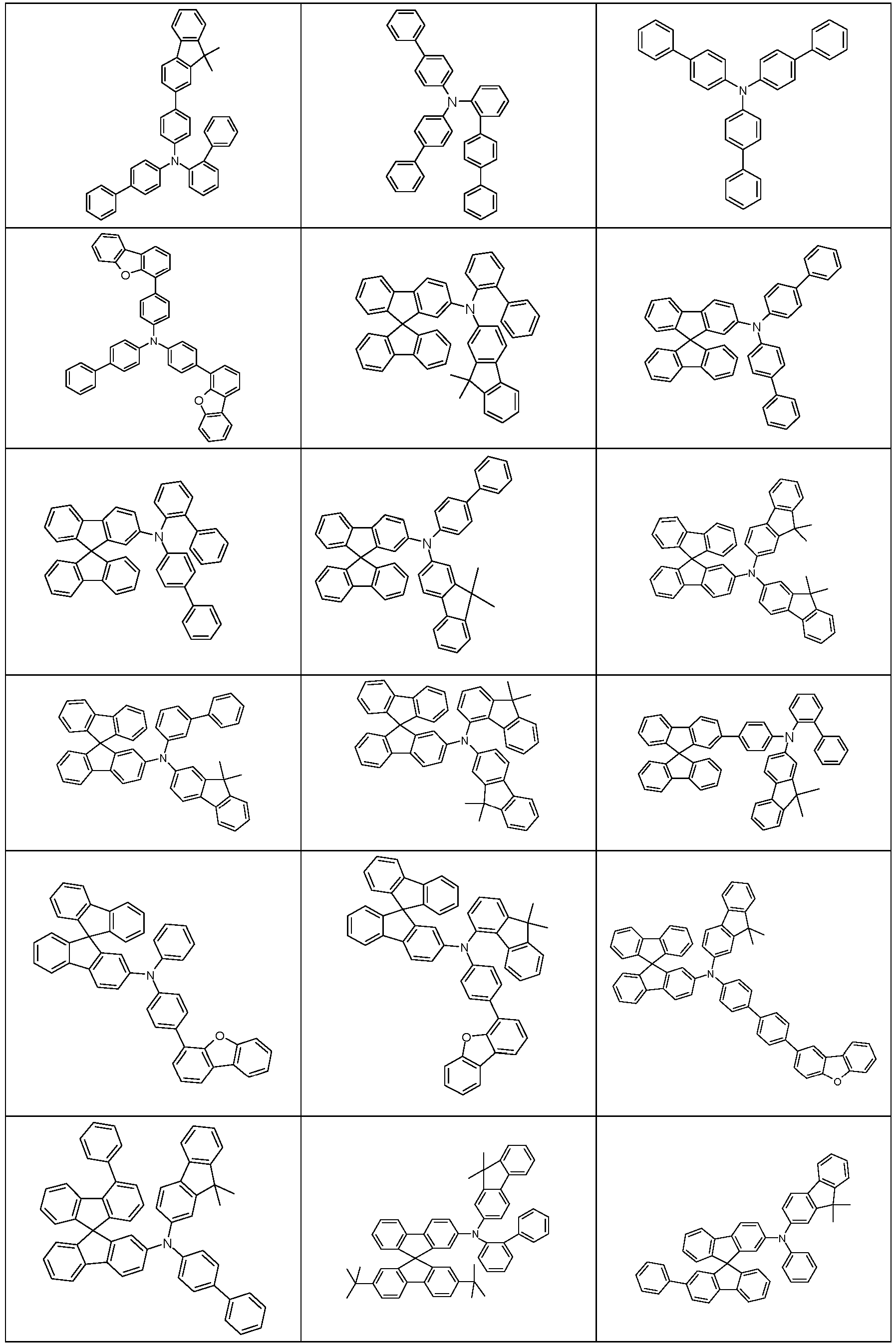

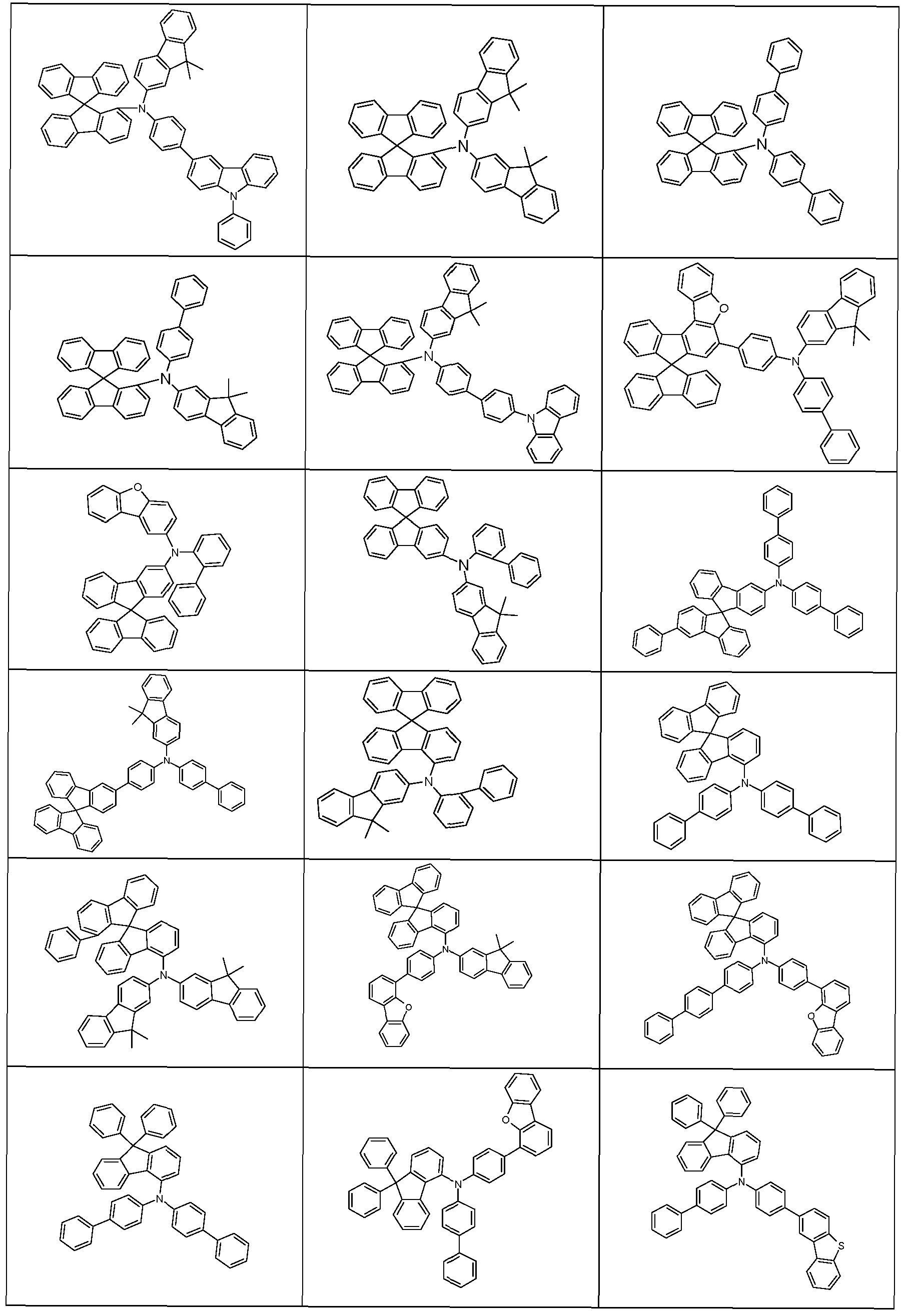

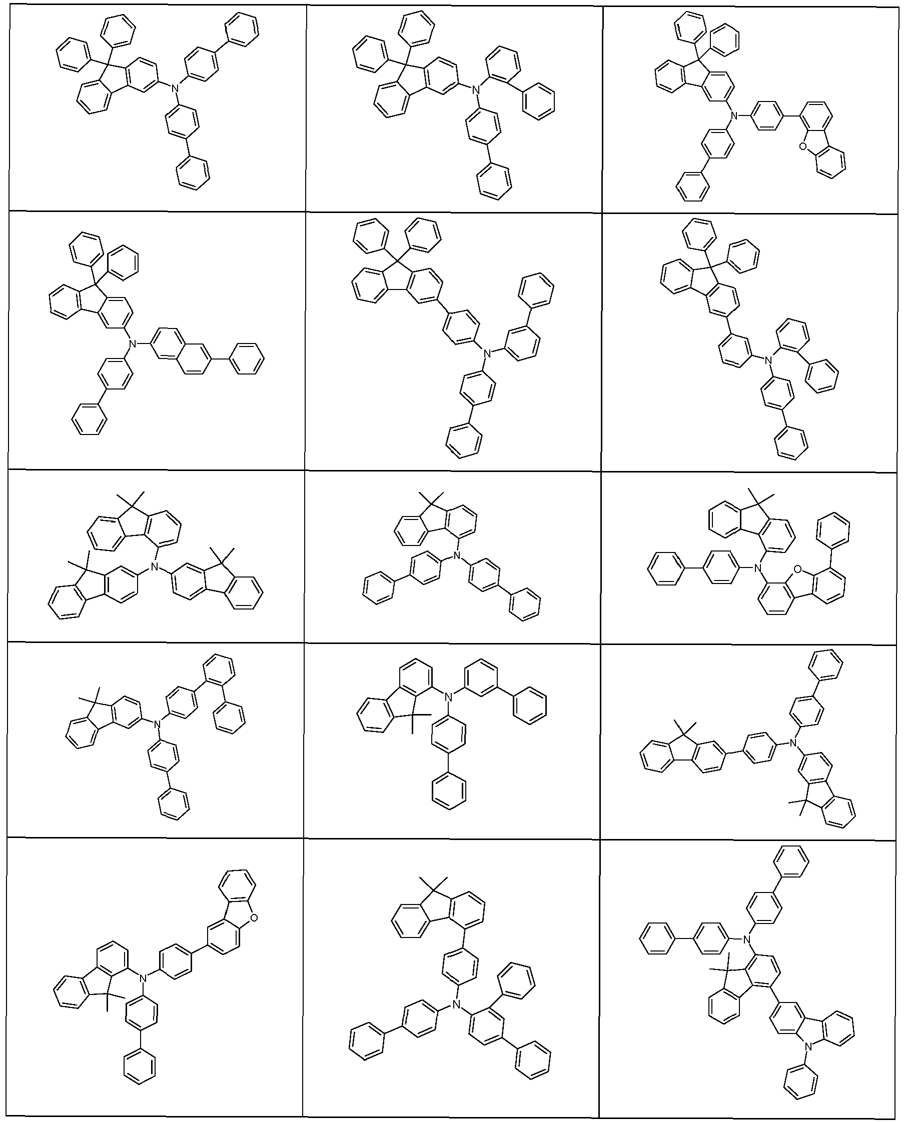

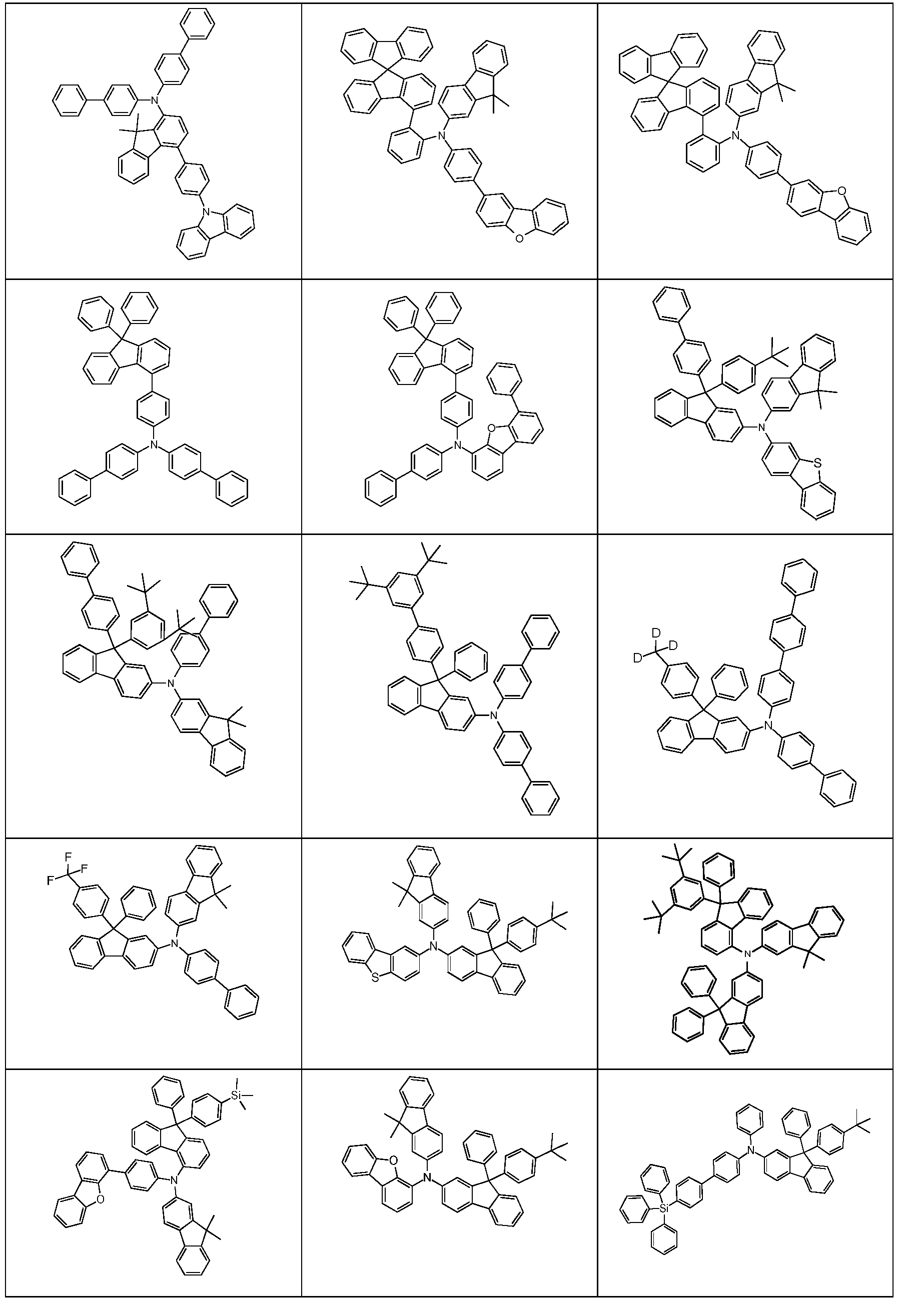

Die folgenden Verbindungen HT-1 bis HT-68 sind geeignet zur Verwendung in einer Schicht mit lochtransportierender Funktion, insbesondere in einer Lochinjektionsschicht, einer Lochtransportschicht und/oder einer Elektronenblockierschicht, oder zur Verwendung in einer emittierenden Schicht als Matrixmaterial, insbesondere als Matrixmaterial in einer emittierenden Schicht enthaltend einen oder mehrere phosphoreszierende Emitter. Am meisten bevorzugt ist die Verwendung in einer Lochinjektionsschicht, Lochtransportschicht und/oder Elektronenblockierschicht. Die Verbindungen können dabei entweder in Kombination mit einer erfindungsgemäßen Verbindung in einer Schicht oder in einer separaten Schicht eingesetzt werden, da die Verbindungen selbst bereits sehr gute Lochinjektionseigenschaften sowie lochtransportierende und elektronenblockierende Eigenschaften aufweisen und die Leistungsdaten (z.B. Effizient, Lebensdauer und Spannung) organischer Elektrolumineszenzvorrichtungen erheblich verbessern können.

| | | |
|---|---|---|
| 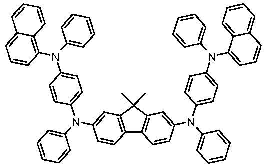 | 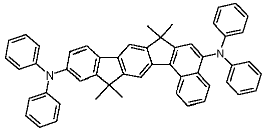 | 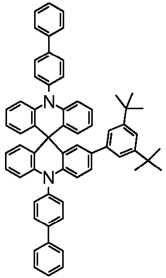 |
| HT-1 | HT-2 | HT-3 |
| 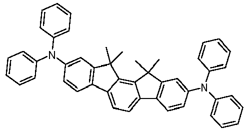 | 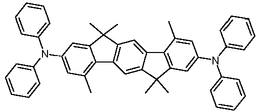 | 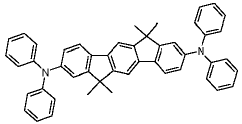 |
| HT-4 | HT-5 | HT-6 |
| 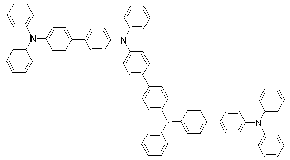 | 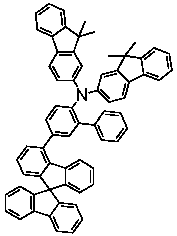 | 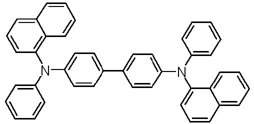 |
| HT-7 | HT-8 | HT-9 |
| 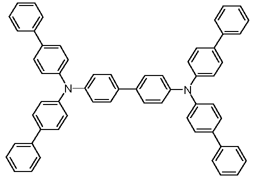 | 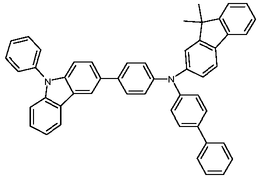 | 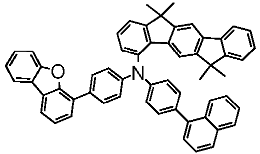 |
| HT-10 | HT-11 | HT-12 |
| 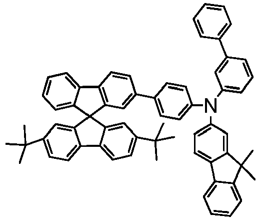 | 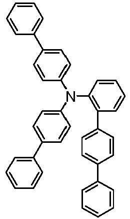 | 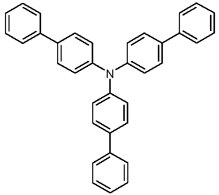 |
| HT-13 | HT-14 | HT-15 |
| 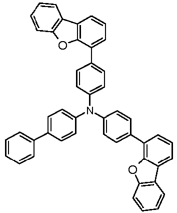 | 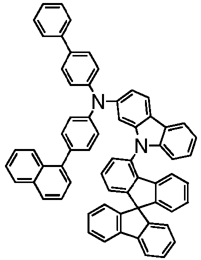 | 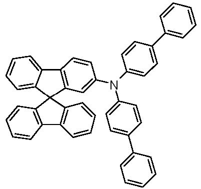 |
| HT-16 | HT-17 | HT-18 |
| 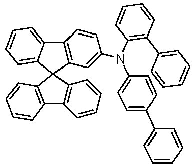 | 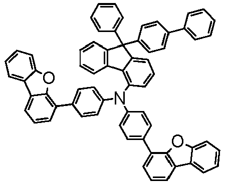 | 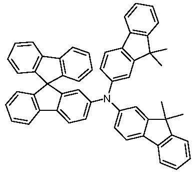 |
| HT-19 | HT-20 | HT-21 |
| 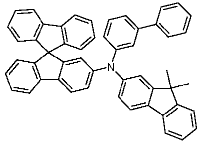 | 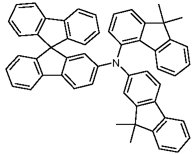 | 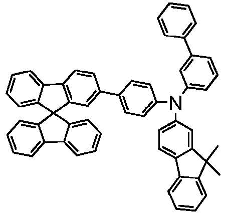 |
| HT-22 | HT-23 | HT-24 |
| 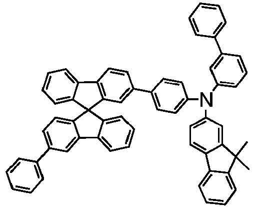 | 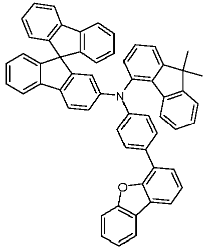 | 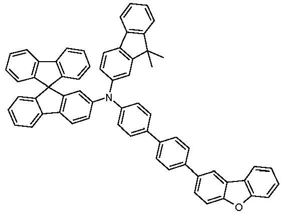 |
| HT-25 | HT-26 | HT-27 |
| 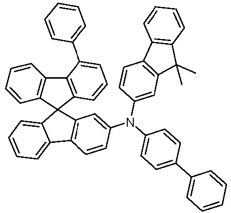 | 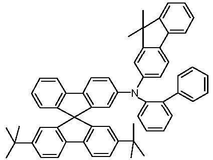 | 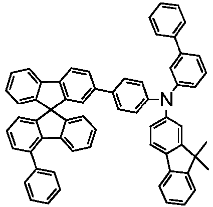 |
| HT-28 | HT-29 | HT-30 |
| 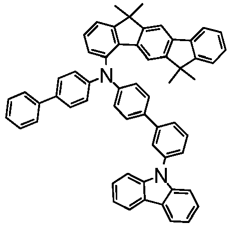 | 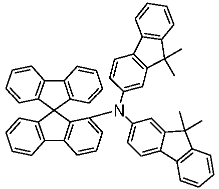 | 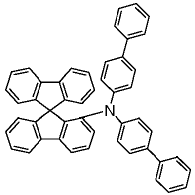 |
| HT-31 | HT-32 | HT-33 |
| 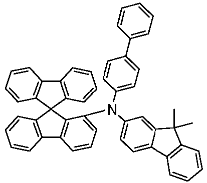 | 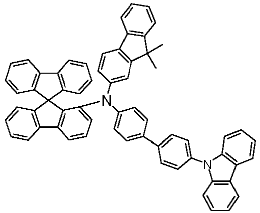 | 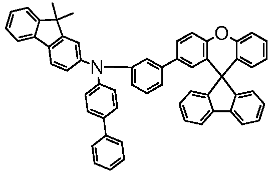 |
| HT-34 | HT-35 | HT-36 |
| 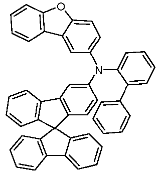 | 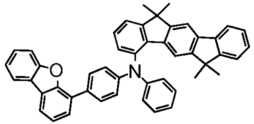 | 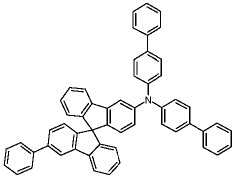 |
| HT-37 | HT-38 | HT-39 |
| 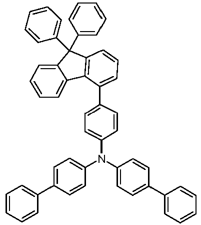 | 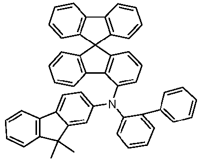 | 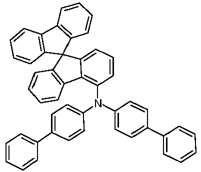 |
| HT-40 | HT-41 | HT-42 |
| 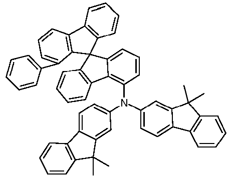 | 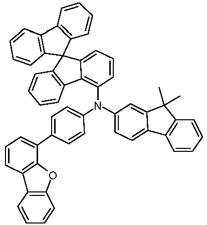 | 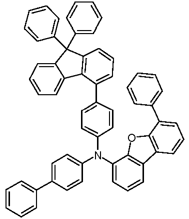 |
| HT-43 | HT-44 | HT-45 |
| 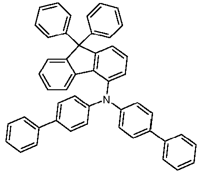 | 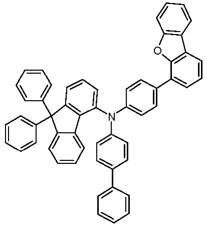 | 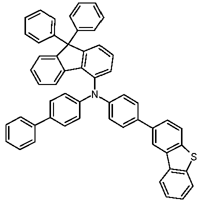 |
| HT-46 | HT-47 | HT-48 |
| 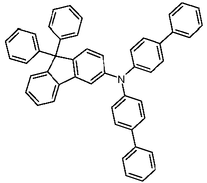 | 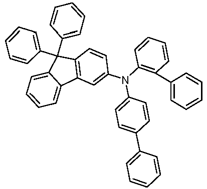 | 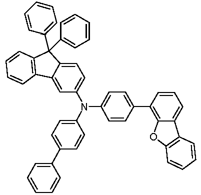 |
| HT-49 | HT-50 | HT-51 |
| 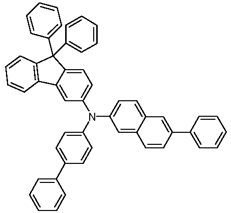 | 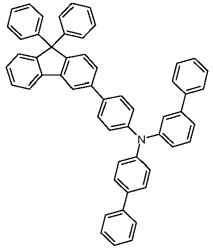 | 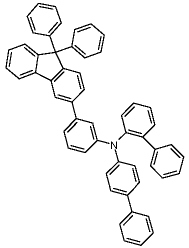 |
| HT-52 | HT-53 | HT-54 |
| 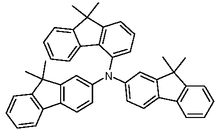 | 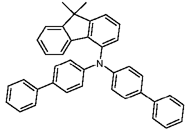 | 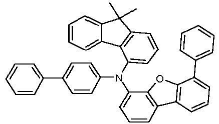 |
| HT-55 | HT-56 | HT-57 |
| 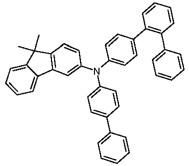 | 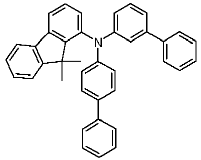 | 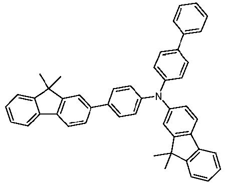 |
| HT-58 | HT-59 | HT-60 |
| 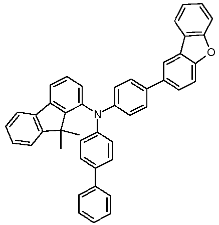 | 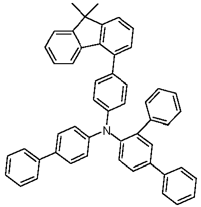 | 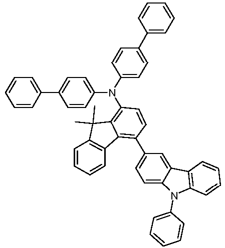 |
| HT-61 | HT-62 | HT-63 |
| 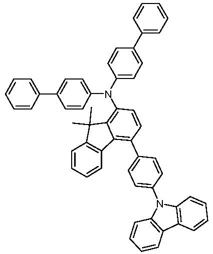 | 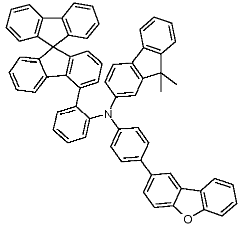 | 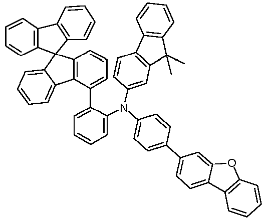 |
| HT-64 | HT-65 | HT-66 |
| 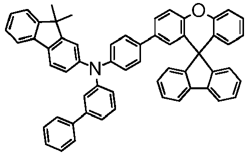 | 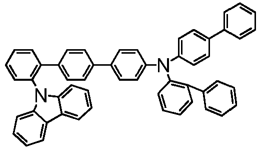 | |
| HT-67 | HT-68 | |

Die Verbindungen HT-1 bis HT-68 sind daher allgemein hervorragend geeignet für die oben genannten Verwendungen in OLEDs jeglicher Bauart und Zusammensetzung, nicht nur in OLEDs gemäß der vorliegenden Anmeldung. Verfahren zur Herstellung dieser Verbindungen und weitere relevante Offenbarung zur Verwendung dieser Verbindungen sind in den folgenden Offenlegungsschriften offenbart: WO 2021/074106, WO 2018/069167, WO 2020/127145, WO 2019/048443, WO 2012/034627, WO 2019/020654, WO 2014/079527, WO 2013/120577, and WO 2015/158411. Die Verbindungen zeigen in OLEDs gute Leistungsdaten, insbesondere gute Lebensdauer und gute Effizienz.

### Kathode:

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

### Anode:

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (3) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Die Vorrichtung wird nach Aufbringen der Schichten, je nach Anwendung, strukturiert, kontaktiert und schließlich versiegelt, um schädigende Effekte von Wasser und Luft auszuschließen.

Erfindungsgemäß können die elektronischen Vorrichtungen enthaltend eine oder mehrere Verbindungen gemäß Formel (3) in Displays, als Lichtquellen in Beleuchtungsanwendungen sowie als Lichtquellen in medizinischen und/oder kosmetischen Anwendungen eingesetzt werden.

Die erfindungsgemäßen Verbindungen bzw. die erfindungsgemäßen organischen Elektrolumineszenzvorrichtungen zeichnen sich durch folgende Vorteile gegenüber dem Stand der Technik aus:
1. Die erfindungsgemäßen Verbindungen eignen sich sehr gut für den Einsatz in der Elektronenblockierschicht (EBL) einer organischen elektronischen Vorrichtung. Vorrichtungen enthaltend die erfindungsgemäßen Verbindungen zu diesem Zwecke zeichnen sich durch sehr gute Lebensdauern aus. Ferner sind die Effizienzen und Betriebsspannungen solcher Vorrichtungen auf einem sehr guten Niveau. Besonders gute Leistungsdaten werden erreicht, wenn die elektronische Vorrichtung eine grüne Emission aufweist, die Vorrichtung also aufgrund von fluoreszenter oder phosphoreszenter Emission, vorzugsweise phosphoreszenter Emission, grünes Licht abstrahlt.
2. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit.
3. Die erfindungsgemäßen Verbindungen weisen eine relativ niedrige Sublimationstemperatur, eine hohe Temperaturstabilität sowie eine hohe Oxidationsstabilität und eine hohe Glasübergangtemperatur auf, was sowohl für die Prozessierbarkeit, beispielweise aus Lösung oder aus der Gasphase, als auch für die Verbendung in elektronischen Vorrichtungen vorteilhaft ist. Die Verbindungen eignen sich aufgrund ihrer Leistungsdaten und der anderen genannten physikalischen Eigenschaften vorzüglich für die kommerzielle Verwendung, für eine kommerzielle Produktion und die Massenproduktion.
4. Die Verwendung der erfindungsgemäßen Verbindungen in elektronischen Vorrichtungen als lochleitende Matrix, als Lochtransport- oder Lochinjektionsmaterial führt zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### A) Synthesebeispiele

Ganz allgemein erffolgt die Synthese der erfindungsgemäßen Verbindungen mit Verfahren, die dem Fachmann auf dem Gebiet sehr gut bekannt sind. Zunächst wird die das Fluoren mittels Suzuki Kupplung umgesetzt. In einem zweiten Schritt wird das Produkt der ersten Reaktion mittels einer Buchwald Reaktion zum endgültigen Produkt umgesetzt.

### a)3-(4-Chlorphenyl)-9,9-dimethyl-9H-fluoren

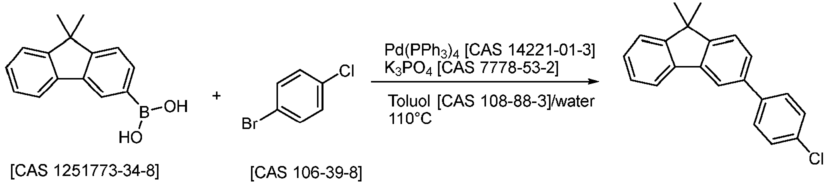

39,5 g (166 mmol, 1,10 eq) (9,9-Dimethyl-9H-fluoren-3-yl)boronsäure [CAS 1251773-34-8], 30,6 g (160 mmol, 1,00 eq) 1-Brom-4-chlorbenzen [CAS 106-39-8] und 102 g (480 mmol; 3,00 eq) Kaliumphosphate [CAS 7778-53-2] werden in 2000 mL Toluol [CAS 108-88-3] und 200 mL Wasser gelöst. Nach Intertisieren im Argonstrom für 45 Minuten werden 3,70 g (3,20 mmol, 2,00 mol%) Tetrakis(triphenylphosphin)palladium hinzugefügt und für 16 Stunden zum Rückfluss erhitzt. Nach Abkühlen auf Raumtemperatur wird die organische Phase abgetrennt und die wässrige Phase mit Ethylacetat extrahiert. Die vereinten organischen Phasen werden mit Wasser gewaschen und über Na₂SO₄ getrocknet. Nach Entfernen des Lösemittels im Vakuum wird der erhaltene Feststoff in Methylenchlorid aufgenommen und Zugabe von Ethanol gefällt. Nach mehrmaligem Durchführen dieses Vorgangs werden 40,5 g (133 mmol, 83% der Theorie) des Produkts erhalten.

Analog werden erhalten:

| **Nr.** | **Edukt** 1 | **Edukt** 2 | **Produkt** | **Ausbeute** |
|---|---|---|---|---|
| **1a** | 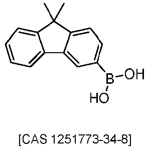 | 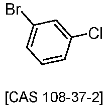 | 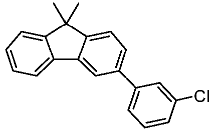 | 80% |
| **2a** | 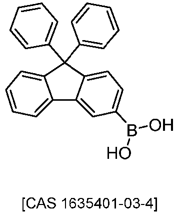 | 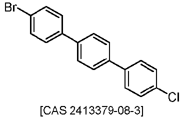 | 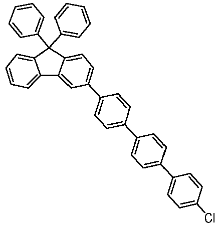 | 68% |
| **3a** | 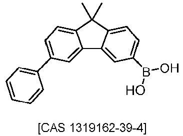 | 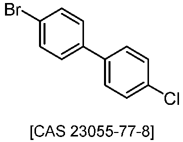 | 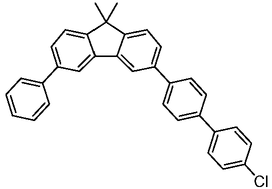 | 72% |
| **4a** | 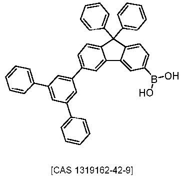 | 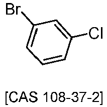 | 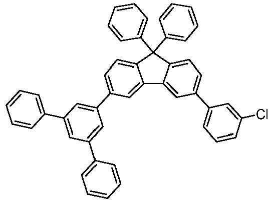 | 55% |
| **5a** | 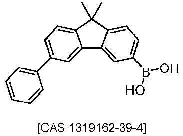 | 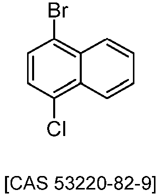 | 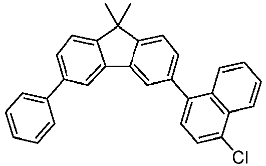 | 61% |
| **6a** | 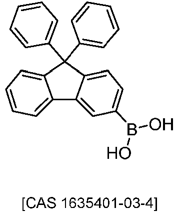 | 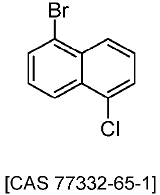 | 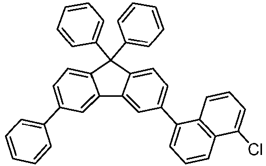 | 64% |
| **7a** | 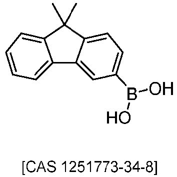 | 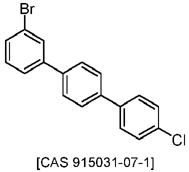 | 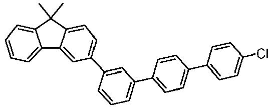 | 70% |
| **8a** | 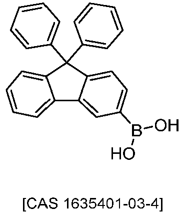 | 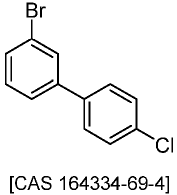 | 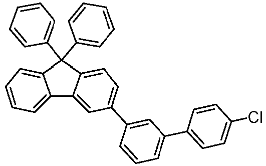 | 79% |
| **9a** | 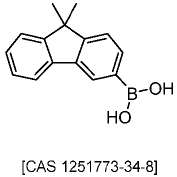 | 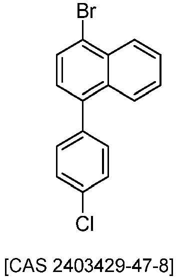 | 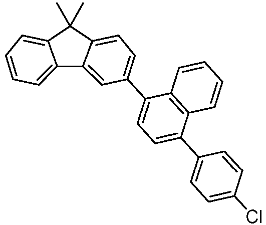 | 69% |
| **10a** | 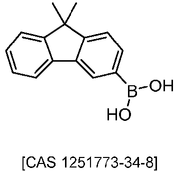 | 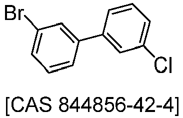 | 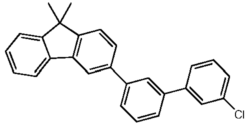 | 71% |

### b) N-[4-(9,9-dimethyl-9H-fluoren-3-yl)phenyl]-N-(4-{8-oxatricyclo[7.4.0.02,7]trideca1(9),2(7),3,5,10,12-hexaen-6-yl}phenyl)-[1,1'-biphenyl]-4-amine (nicht erfindungsgemäß)

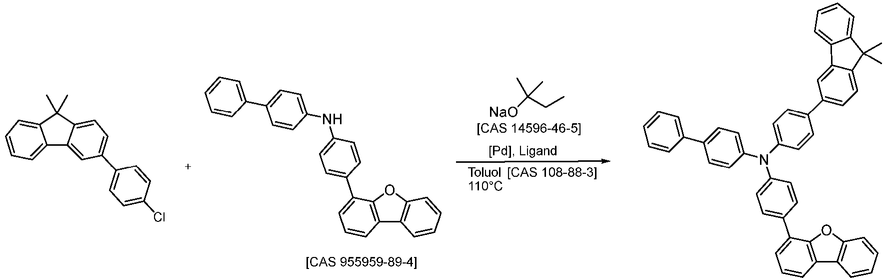

40,0 g (131 mmol; 1,00 eq.) 3-(4-Chlorphenyl)-9,9-dimethyl-9H-fluoren, 54.0 g (131 mmol; 1,00 eq.) N-(4-{8-Oxatricyclo[7.4.0.0^{2,7}]trideca-1(9),2(7),3,5,10,12hexaen6-yl}phenyl)-[1,1'-biphenyl]-4-amin [CAS 955959-89-4] und 15,9 g (144 mmol; 1,10 eq.) Natrium-tert-pentoxid [CAS 14593-46-5] werden in 2000 mL Toluol [CAS 108-88-3] vorgelegt und 30 Minuten im Argonstrom inertisiert. Im Anschluss werden 1,62 mg (3,94 mmol; 3 mol%) Dicyclohexyl-(2',6'-dimethoxy-biphenyl-2-yl)-phosphane (SPhos) [CAS 657408-07-6] und 886 mg (3,94 mmol; 3 mol%) Palladiumacetat [CAS 3375-31-3] zugegeben und für 18 Stunden zum Rückfluss erhitzt. Nach vollständigem Umsatz und Erkalten auf Raumtemperatur wird die Reaktion mit Wasser versetzt. Nach Trennung der Phasen und Extraktion der wässrigen Phase mit Toluol [CAS 108-88-3] werden die vereinten organischen Phasen eingeengt und mit Heptan versetzt. Der ausgefallen Feststoff wird isoliert. Aufreinigung mittels Soxhlet-Extraktion, Umkristallisation und Vakuumsublimation ergibt das gewünschte Produkt (52,0 g; 77,1 mmol; 59% der Theorie).

Analog werden erhalten:

| **Nr.** | **Edukt 1** | **Edukt 2** | **Produkt** | **Ausbeute** |
|---|---|---|---|---|
| **1b*** | 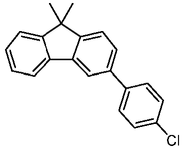 | 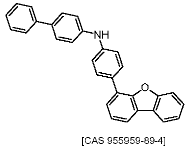 | 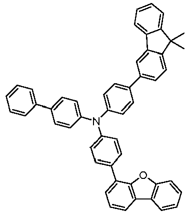 | 56% |
| **2b*** | 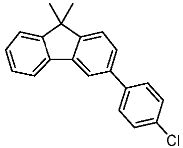 | 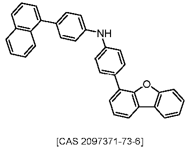 | 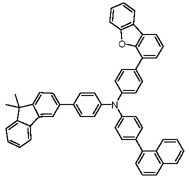 | 62% |
| **3b*** | 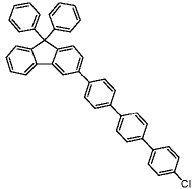 | 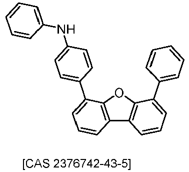 | 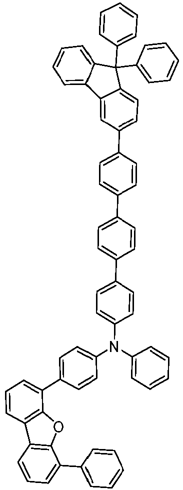 | 48% |
| **4b*** | 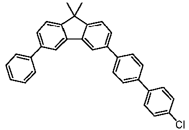 | 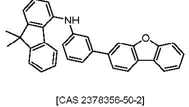 | 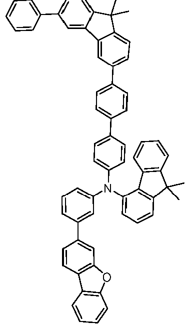 | 42% |
| **5b** | 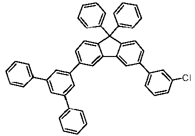 | 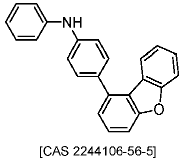 | 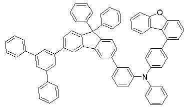 | 55% |
| **6b*** | 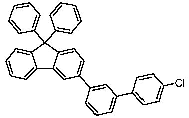 | 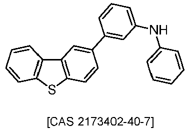 | 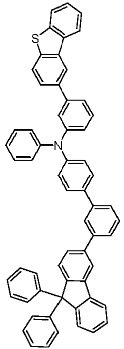 | 58% |
| **7b*** | 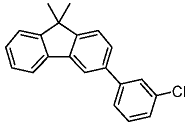 | 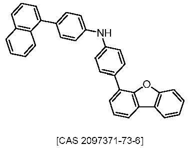 | 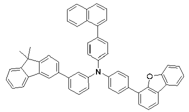 | 63% |
| **8b*** | 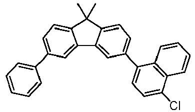 | 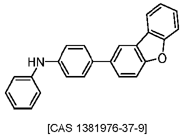 | 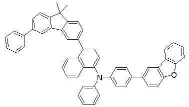 | 51% |
| **9b*** | 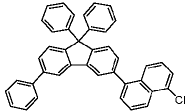 | 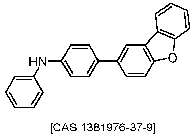 | 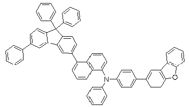 | 57% |
| **10 b*** | 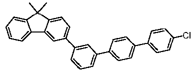 | 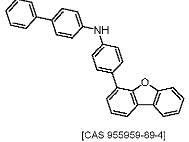 | 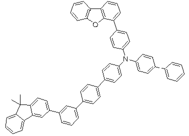 | 45% |
| **11 b*** | 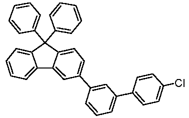 | 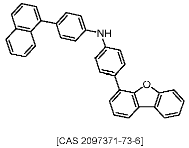 | 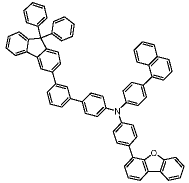 | 60% |
| **13 b*** | 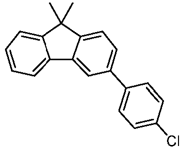 | 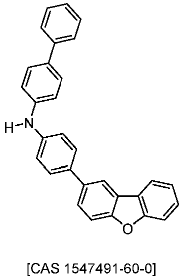 | 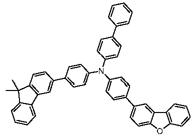 | 55% |
| **14 b** | 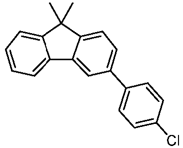 | 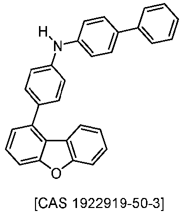 | 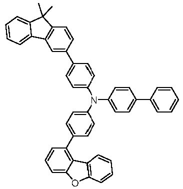 | 52% |
| **15 b** | 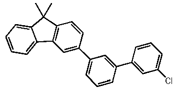 | 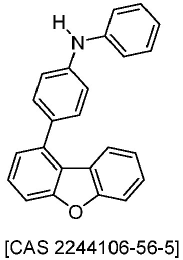 | 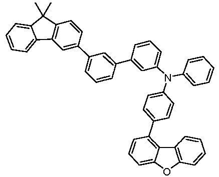 | 63% |
| **16 b** | 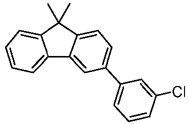 | 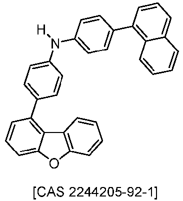 | 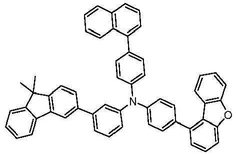 | 60% |

| | | | | |
|---|---|---|---|---|
| *nicht erfindungsgemäß | | | | |

Die Synthese der Vergleichsverbindung, FIMA1, ist in WO2014/015935 A2 offenbart (Verbindung 2-8 in Beispiel 2).

### B) Device-Beispiele

In den folgenden Beispielen E1 bis E5 (siehe Tabelle 1) wird der Einsatz der erfindungsgemäßen Materialien in OLEDs vorgestellt.

Vorbehandlung für die Beispiele E1-E5 Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt. Die Daten der OLEDs sind in Tabelle 3 aufgelistet.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:IC2:TEG1 (59%:29%:12%) bedeutet hierbei, dass das Material IC1 in einem Volumenanteil von 49%, IC2 in einem Anteil von 44% und TEG1 in einem Anteil von 7% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (SE, gemessen in cd/A) und die externe Quanteneffizienz (EQE, gemessen in %) in Abhängigkeit der Leuchtdichte, berechnet aus Strom-Spannungs-Leuchtdichte-Kennlinien unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 3 bezeichnet die Spannung, die für eine Leuchtdichte von 1000 cd/m² benötigt wird. SE1000 und EQE1000 bezeichnen die Stromeffizienz bzw. die externe Quanteneffizienz, die bei 1000cd/m² erreicht werden. Als Lebensdauer LD wird die Zeit definiert, nach der die Leuchtdichte bei Betrieb mit konstanter Stromdichte j0 von der Startleuchtdichte auf einen gewissen Anteil L1 absinkt. Eine Angabe L1=80% in Tabelle 3 bedeutet, dass die in Spalte LD angegebene Lebensdauer der Zeit entspricht, nach der die Leuchtdichte auf 80% ihres Anfangswertes absinkt.

Die erfindungsgemäßen Verbindungen EG1, EG2, EG3, EG4 werden in den Beispielen E2, E3, E4 und E5 als Elektronenblockiermaterial in phosphoreszenten grünen OLEDs eingesetzt werden. Die Ergebnisse werden dem Vergleichsbeispiel E1 gegenüber gestellt. Tabelle 3 fasst die Leistunsgdaten der OLEDs zusammen.

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| E1 | HATCN 5nm | SpMA1 230nm | FIMA1 10nm | IC1:IC2:TEG1 (59%:29%:12%) 30nm | ST2 10nm | ST2:LiQ (50%:50%) 30nm | LiQ 1nm |
| E2* | HATCN 5nm | SpMA1 230nm | EG1 10nm | IC1:IC2:TEG1 (59%:29%:12%) 30nm | ST2 10nm | ST2:LiQ (50%:50%) 30nm | LiQ 1nm |
| E3 | HATCN 5nm | SpMA1 230nm | EG2 10nm | IC1:IC2:TEG1 (59%:29%:12%) 30nm | ST2 10nm | ST2:LiQ (50%:50%) 30nm | LiQ 1nm |
| E4 | HATCN 5nm | SpMA1 230nm | EG3 10nm | IC1:IC2:TEG1 (59%:29%:12%) 30nm | ST2 10nm | ST2:LiQ (50%:50%) 30nm | LiQ 1nm |
| E5 | HATCN 5nm | SpMA1 230nm | EG4 10nm | IC1:IC2:TEG1 (59%:29%:12%) 30nm | ST2 10nm | ST2:LiQ (50%:50%) 30nm | LiQ 1nm |

| | | | | | | | |
|---|---|---|---|---|---|---|---|
| *nicht erfindungsgemäß | | | | | | | |

**Tabelle 2: Strukturformeln der Materialien für die OLEDs**

| | |
|---|---|
| 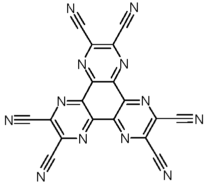 | 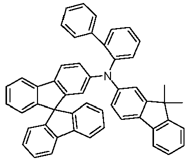 |
| HATCN | SpMA1 |
| 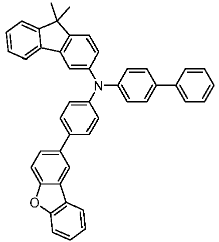 | 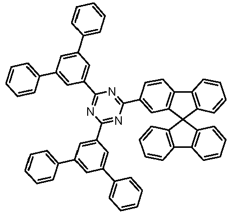 |
| FIMA1 | ST2 |
| 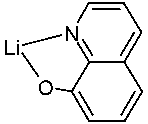 | 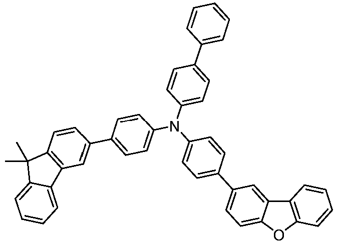 |
| LiQ | EG1 |
| 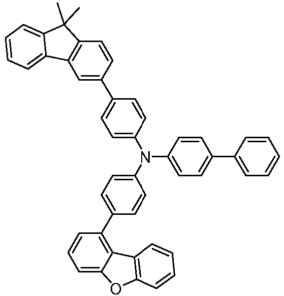 | 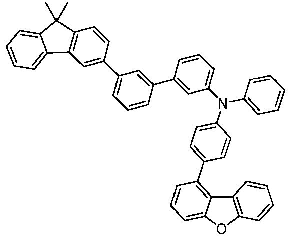 |
| EG2 | EG3 |
| 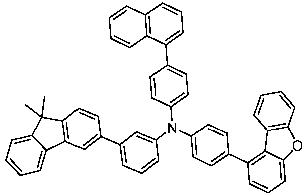 | 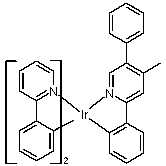 |
| EG4 | TEG1 |
| 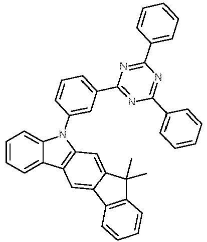 | 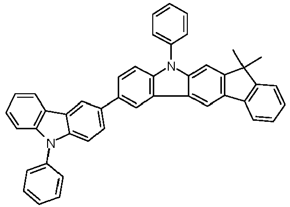 |
| IC1 | IC2 |

**Tabelle 3: Daten der OLEDs**

| Bsp. | U1000 (V) | SE1000 (cd/A) | EQE 1000 (%) | CIE x/y bei 1000 cd/m² | j₀ (mA/cm²) | L1 (%) | LD (h) |
|---|---|---|---|---|---|---|---|
| E1 | 3.3 | 69 | 19.8 | 0.36/0.61 | 20 | 80 | 310 |
| E2* | 3.3 | 71 | 20.8 | 0.36/0.62 | 20 | 80 | 328 |
| E3 | 3.3 | 77 | 21.0 | 0.36/0.62 | 20 | 80 | 316 |
| E4 | 3.3 | 80 | 21.5 | 0.36/0.62 | 20 | 80 | 298 |
| E5 | 3.3 | 81 | 21.5 | 0.36/0.62 | 20 | 80 | 305 |

| | | | | | | | |
|---|---|---|---|---|---|---|---|
| *nicht erfindungsgemäß | | | | | | | |

Es zeigt sich, dass OLEDs enthaltend die erfindungsgemäßen Verbindungen sehr gute Leistungsdaten aufweisen, insbesondere zeigen sie deutlich verbesserte Effizienzen gegenüber dem Stand der Technik. Außerdem sind die Spannungen und Lebensdauern auf einem sehr hohen Niveu.

## Patentansprüche

1. Verbindung gemäß Formel (3) 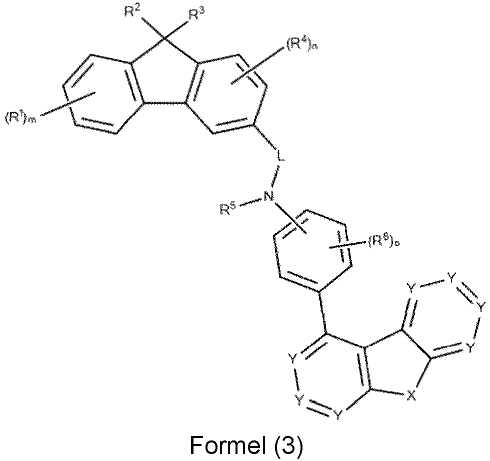 wobei für die verwendeten Symbole gilt:
X ist gleich O oder S, vorzugsweise O;
Y ist gleich oder verschieden bei jedem Auftreten CR⁷ oder N, bevorzugt ist, wenn Y gleich CR⁷ ist;
L ist ein divalentes aromatisches Ringsystem mit 6 bis 40 aromatischen Ringatomen;
R¹, R⁴, R⁶ und R⁷ sind bei jedem Auftreten gleich oder verschieden gewählt aus H, D, F, Cl, Br, I, C(=O)R¹¹, CN, Si(R¹¹)₃, N(R¹¹)₂, P(=O)(R¹¹)₂. OR¹¹. S(=O)R¹¹. S(=O)₂R¹¹. geradkettigen Alkvl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ keinen Ring miteinander bilden, zwei oder mehr Reste R⁴ miteinander verknüpft sein können und einen Ring bilden können und/oder zwei oder mehr Reste R⁶ miteinander verknüpft sein können und einen Ring bilden können und/oder zwei oder mehr Reste R⁷ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; vorzugsweise bilden zwei oder mehr Reste R⁴ keinen Ring miteinander, und/oder zwei oder mehr Reste R⁶ keinen Ring miteinander und/oder zwei oder mehr Reste R⁷ keinen Ring miteinander;
R² und R³ sind bei jedem Auftreten gleich oder verschieden gewählt aus geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die beiden Reste R² and R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C=C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden;
R⁵ ist ein aromatisches Ringsystem mit 6 bis 40 aromatischen Ringatomen, das mit einem oder mehreren Resten R¹¹ substituiert sei kann, oder ein heteroaromatisches Ringsystem mit 5 bis 40 aromatischen Ringatomen, das mit einem oder meheren Resten R¹¹ substituiert sein kann, wobei der Rest R⁵ kein kondensiertes heteroaromatisches Ringsystem mit mehr als 12 aromatischen Kohlenstoffatomen als Ringatome im heteroaromatischen Ringsystem enthält;
R¹¹ ist bei jedem Auftreten gleich oder verschieden gewählt aus H, D, F, Cl, Br, I, C(=O)R¹², CN, Si(R¹²)₃, N(R¹²)₂, P(=O)(R¹²)₂, OR¹², S(=O)R¹²,
S(=O)₂R¹², geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹² substituiert sind; und wobei eine oder mehrere CH₂-Gruppen in den genannten Alkyl-, Alkoxy-, Alkenyl- und Alkinylgruppen durch -R¹²C=CR¹²-, -C=C-, Si(R¹²)₂, C=O, C=NR¹², -C(=O)O-, -C(=O)NR¹²-, NR¹², P(=O)(R¹²), -O-, -S-, SO oder SO₂ ersetzt sein können; wobei zwei oder mehr Reste R¹¹ miteinander keinen Ring bilden können
R¹² ist bei jedem Auftreten gleich oder verschieden gewählt aus H, D, F, Cl, Br, I, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei wobei die genannten Alkyl-, Alkoxy-, Alkenyl- und Alkinylgruppen, aromatischen Ringsysteme und heteroaromatischen Ringsysteme mit einem oder mehreren Resten gewählt aus F und CN substituiert sein können;
m ist 0, 1, 2, 3 oder 4, bevorzugt ist, wenn m gleich 0 oder 1 ist, ganz bevorzugt ist, wenn m gleich 1 ist und ganz besonders bevorzugt ist, wenn m gleich 0 ist;
n ist 0,1, 2 oder 3, bevorzugt ist, wenn n gleich 0 oder 1 ist, ganz bevorzugt ist, wenn n gleich 1 ist und ganz besonders bevorzugt ist, wenn n gleich 0 ist;
o ist 0,1, 2 oder 3, bevorzugt ist, wenn o gleich 0 oder 1 ist, ganz bevorzugt ist, wenn o gleich 1 ist und ganz besonders bevorzugt ist, wenn o gleich 0 ist.

2. Die Verbindung gemäß Anspruch 1, **dadurch gekennzeichnet, dass** L ausgewählt ist aus den folgenden Formeln 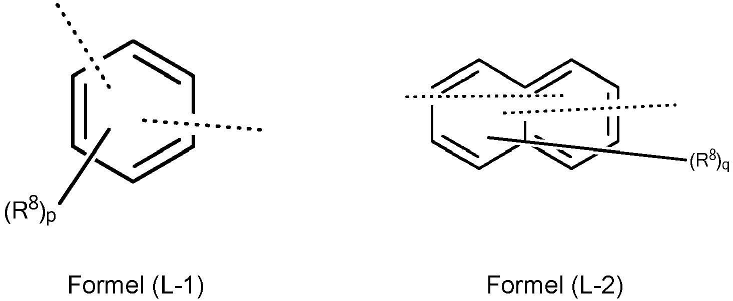 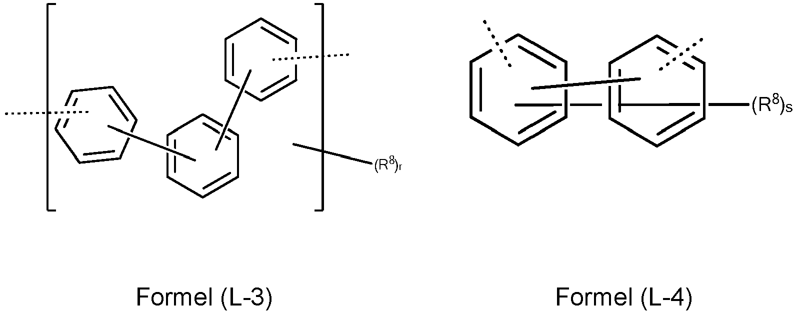 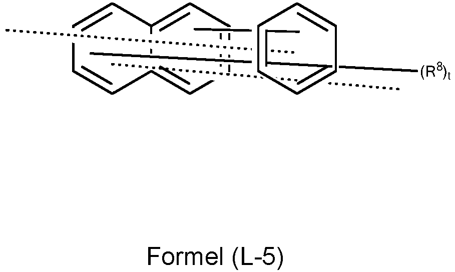 wobei gilt:
R⁸ ist bei jedem Auftreten gleich oder verschieden gewählt aus H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R⁸ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit Resten R¹¹ substituiert sein können; und wobei eine oder mehrere CH₂-Gruppen in den genannten Alkyl-, Alkoxy-, Alkenyl- und Alkinylgruppen durch -R¹¹C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO oder SO₂ ersetzt sein können; vorzugsweise bilden zwei oder mehr Reste R⁸ keinen Ring miteinander;
und wobei R¹¹ wie in Anspruch 1 angegeben definiert ist; p ist 0,1, 2, 3 oder 4, bevorzugt ist, wenn p gleich 0 oder 1 ist, ganz bevorzugt ist, wenn p gleich 1 ist und ganz besonders bevorzugt ist, wenn p gleich 0 ist;
q ist 0, 1, 2, 3, 4, 5 oder 6, bevorzugt ist, wenn q gleich 0 oder 1 ist, ganz bevorzugt ist, wenn q gleich 1 ist und ganz besonders bevorzugt ist, wenn q gleich 0 ist;
r ist 0, 1, 2, 3, 4, 5, 7, 8, 9, 10, 11 oder 12, bevorzugt ist, wenn r gleich 0 oder 1 ist, ganz bevorzugt ist, wenn r gleich 1 ist und ganz besonders bevorzugt ist, wenn r gleich 0 ist;
s ist 0, 1, 2, 3, 4, 5, 7 oder 8, bevorzugt ist, wenn s gleich 0 oder 1 ist, ganz bevorzugt ist, wenn s gleich 1 ist und ganz besonders bevorzugt ist, wenn s gleich 0 ist;
t ist 0, 1, 2, 3, 4, 5, 7, 8, 9, oder 10 , bevorzugt ist, wenn t gleich 0 oder 1 ist, ganz bevorzugt ist, wenn t gleich 1 ist und ganz besonders bevorzugt ist, wenn t gleich 0 ist.

3. Die Verbindung gemäß Anspruch 1 oder 2, **dadurch gekennzeichnet, dass** L ausgewählt ist aus den folgenden Formeln 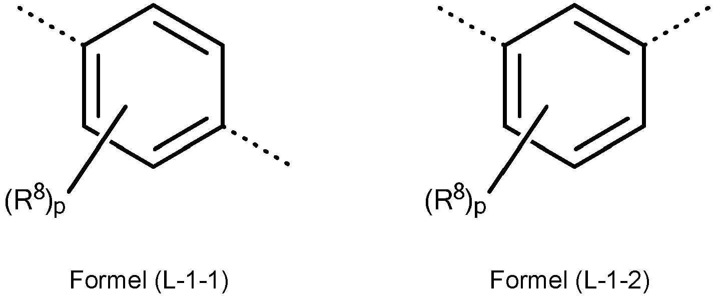 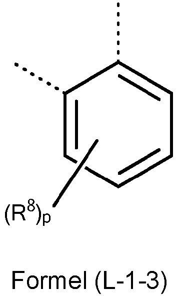 wobei für die verwendeten Symbole die Definitionen aus den obigen Ansprüchen gelten.

4. Die Verbindung gemäß Anspruch 1 oder 2, **dadurch gekennzeichnet, dass** L ausgewählt ist aus den folgenden Formeln 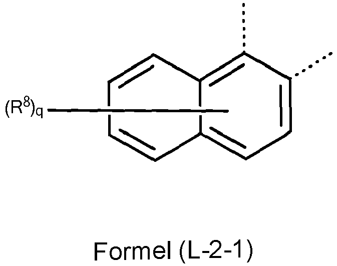 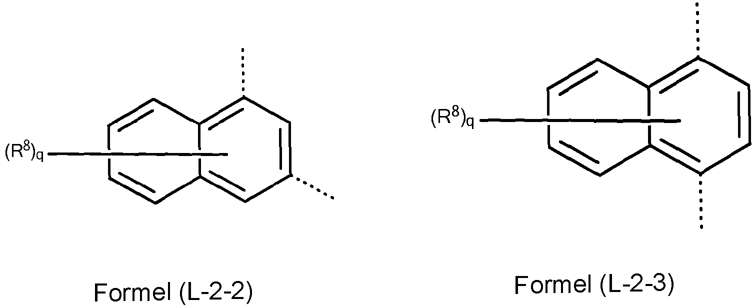 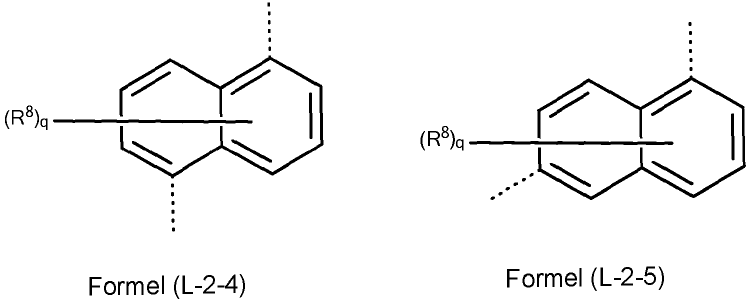 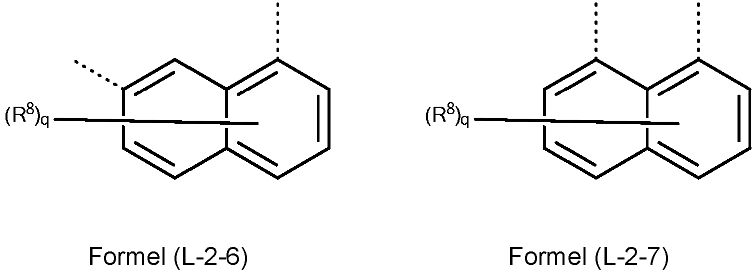 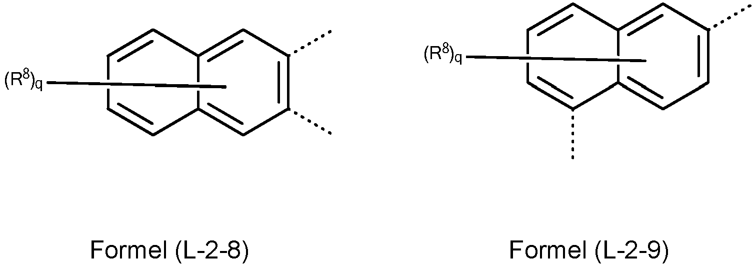 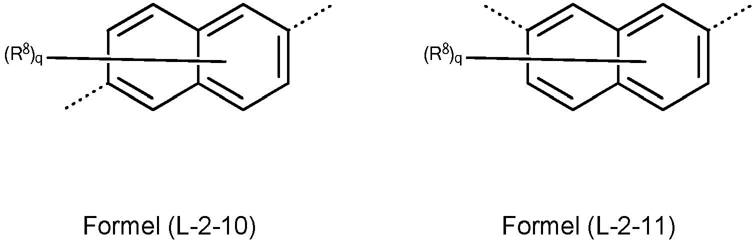 wobei für die verwendeten Symbole die Definitionen aus den obigen Ansprüchen gelten.

5. Die Verbindung gemäß Anspruch 1 oder 2, **dadurch gekennzeichnet, dass** L ausgewählt ist aus den folgenden Formeln 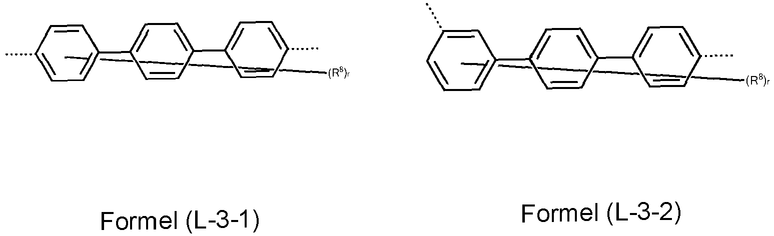 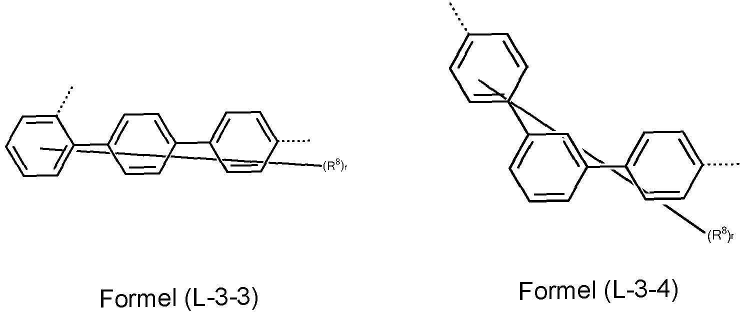 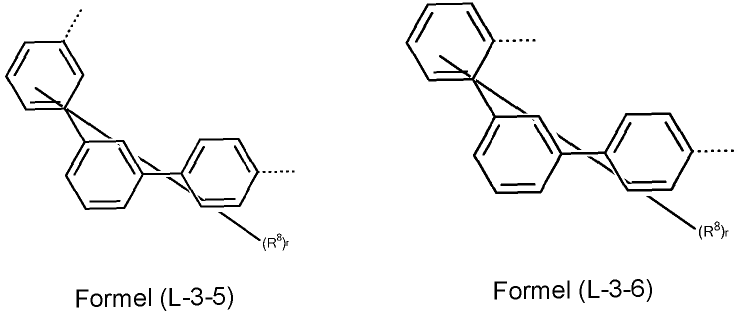 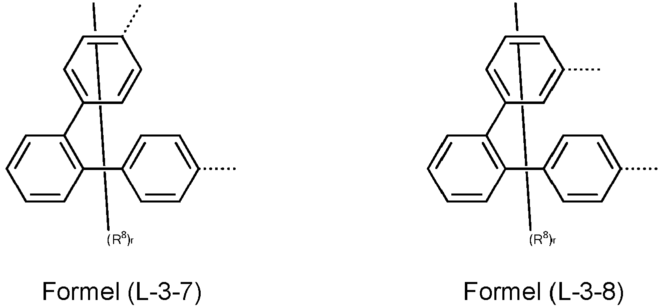 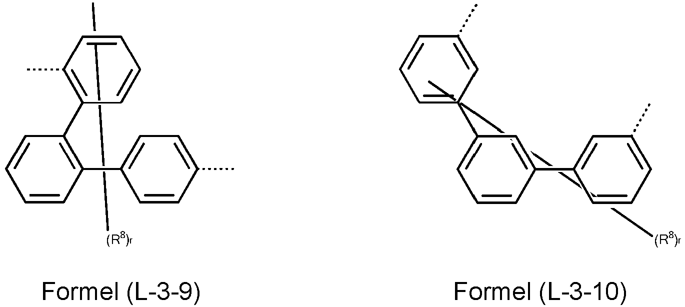 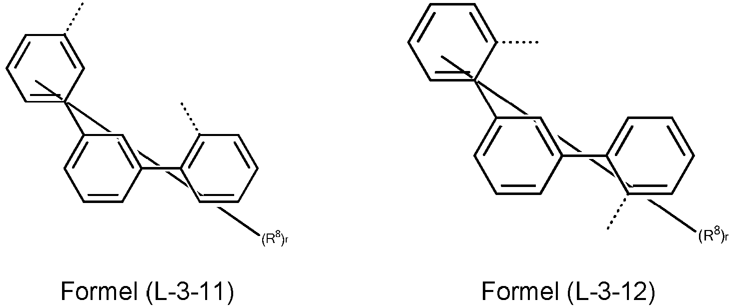 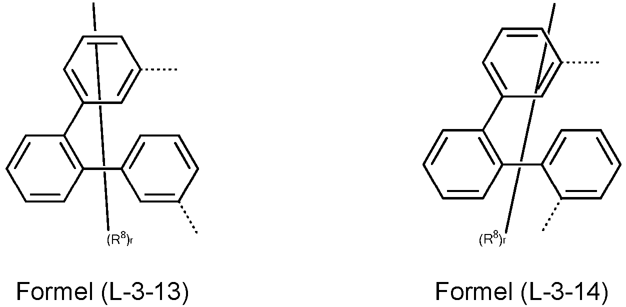 wobei für die verwendeten Symbole die Definitionen aus den obigen Ansprüchen gelten.

6. Die Verbindung gemäß Anspruch 1 oder 2, **dadurch gekennzeichnet, dass** L ausgewählt ist aus den folgenden Formeln 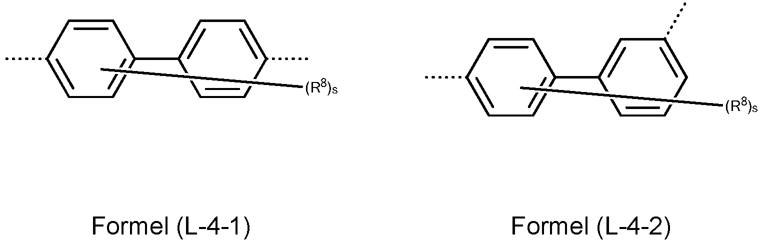 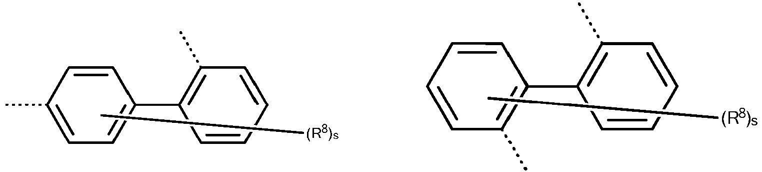 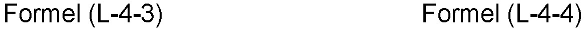 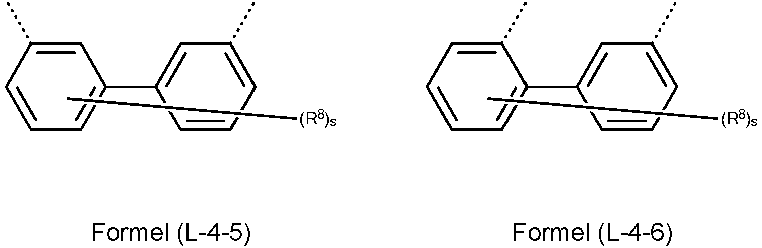 wobei für die verwendeten Symbole die Definitionen aus den obigen Ansprüchen gelten.

7. Die Verbindung gemäß einem oder mehreren der Ansprüche 1 bis 6, **dadurch gekennzeichnet, dass** es sich um eine Monoaminverbindung handelt.

8. Die Verbindung gemäß einem oder mehreren der Ansprüche 1 bis 7, **dadurch gekennzeichnet, dass** R² und R³ gleich oder verschieden bei jedem Auftreten ausgewählt sind aus geradkettigen Alkylgruppen mit 1 bis 10 C-Atomen, verzweigten oder cyclischen Alkylgruppen mit 3 bis 10 C-Atomen oder aromatischen Ringsystemen mit 6 bis 18 aromatischen Ringatomen; wobei die beiden Reste R² and R³ miteinander verknüpft sein können und einen Ring bilden können; wobei die genannten Alkylgruppen und die genannten aromatischen Ringsysteme jeweils mit Resten R¹¹ substituiert sein können; sofern die beiden Reste R² and R³ einen Ring bilden, entsteht eine Spiroverbindung, vorzugsweise ein Spirobifluoren; besonders bevorzugt ist, wenn die beiden Reste R² und R³ keinen Ring miteinander bilden.

9. Die Verbindung gemäß einem oder mehreren der Ansprüche 1 bis 8, **dadurch gekennzeichnet, dass** die Reste R⁵ ausgewählt sind aus den folgenden Formeln 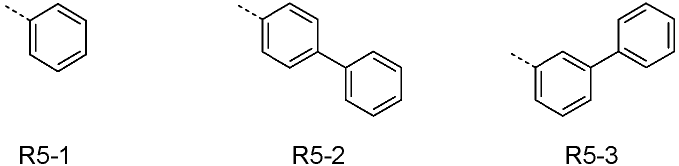 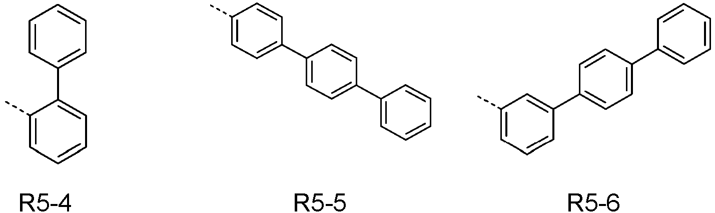 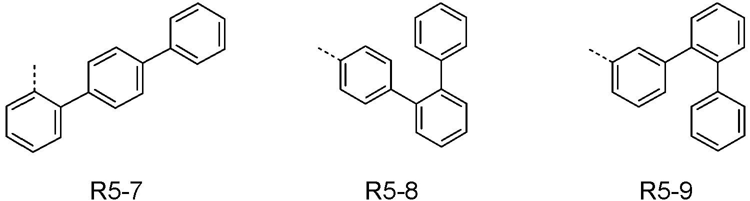 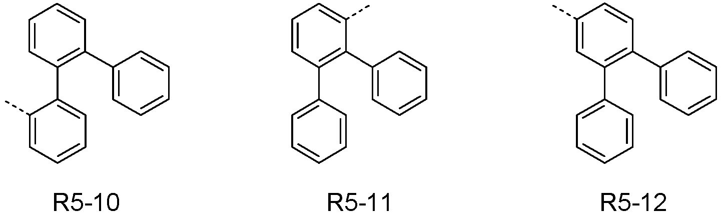 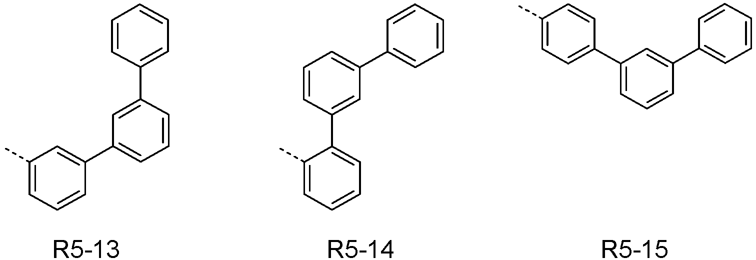 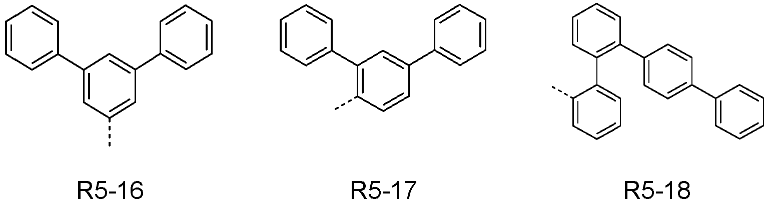 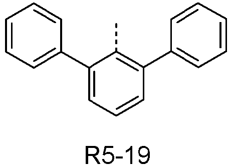 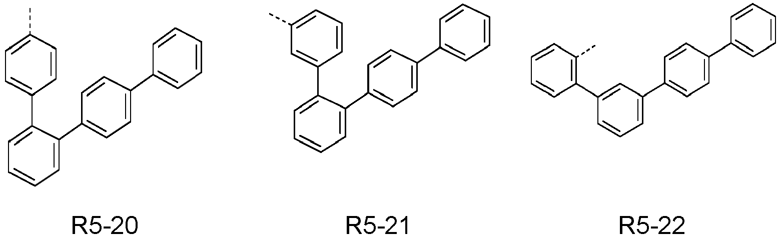 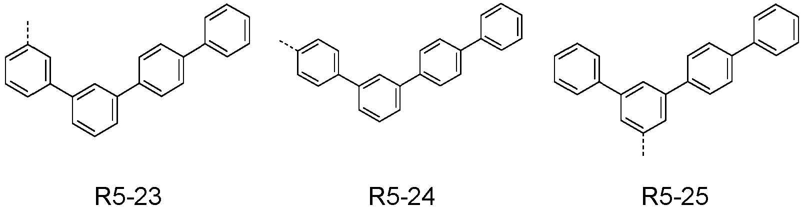 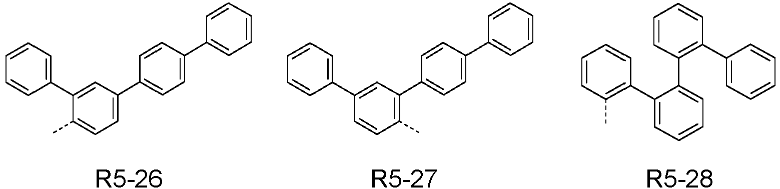 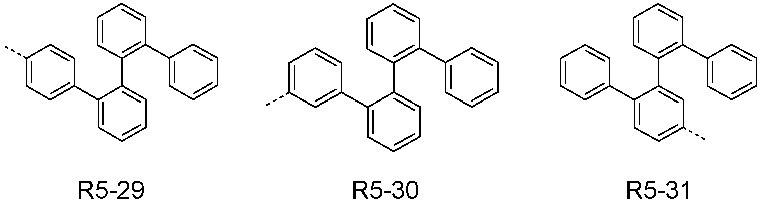 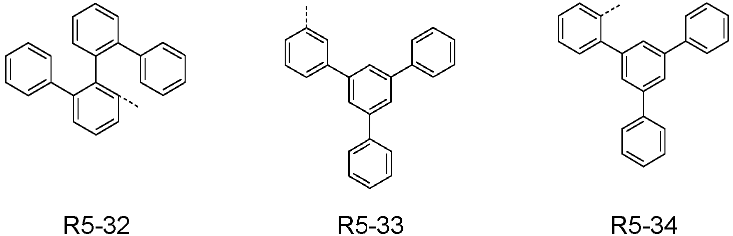 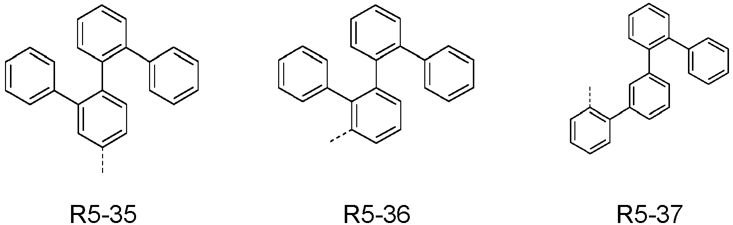 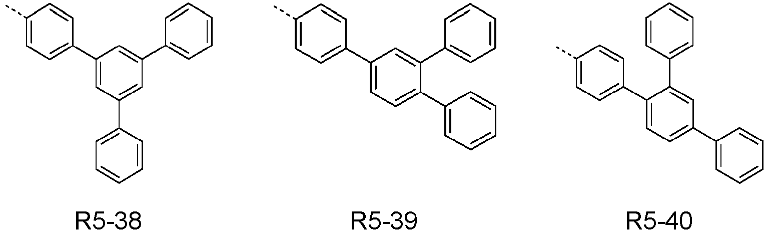 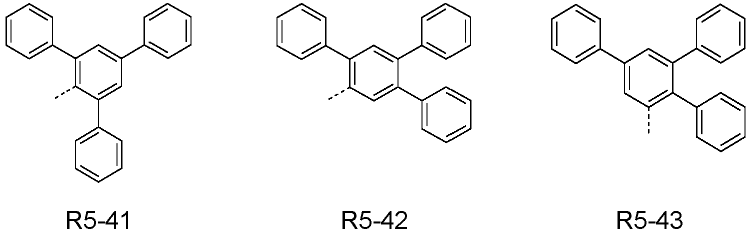 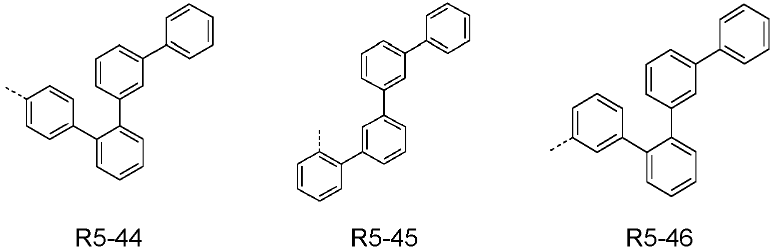 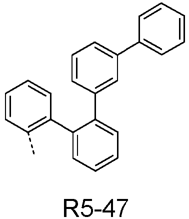 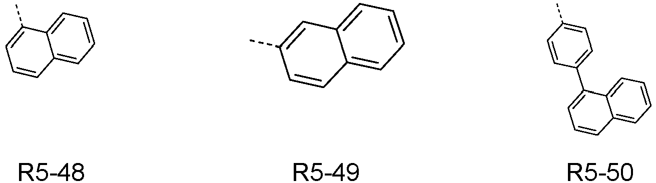 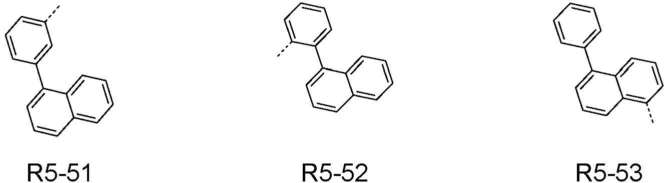 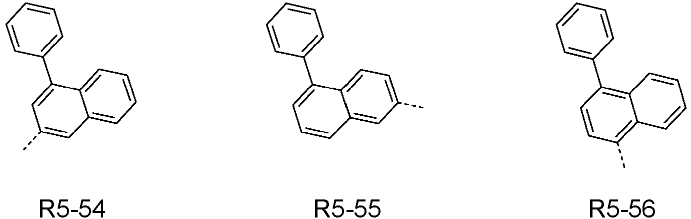 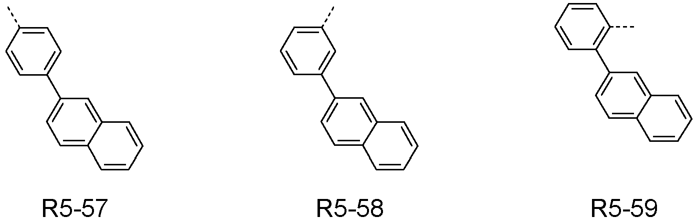 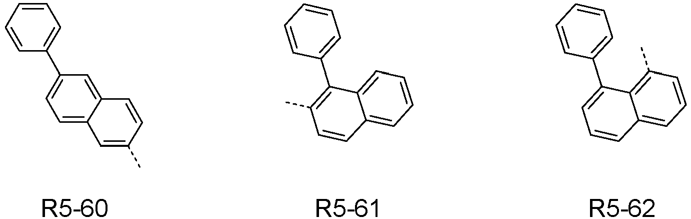 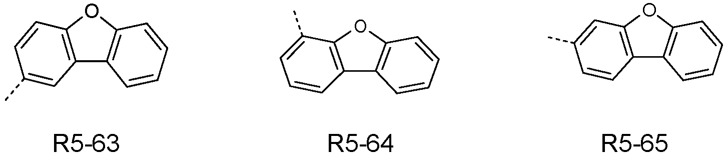 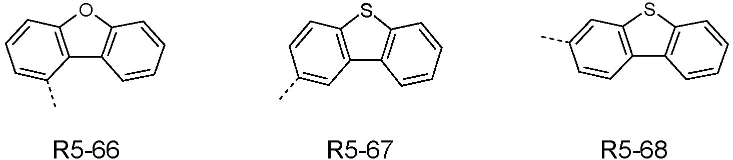 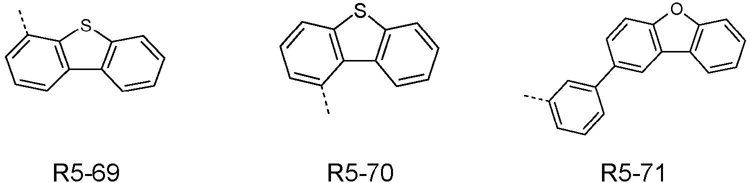 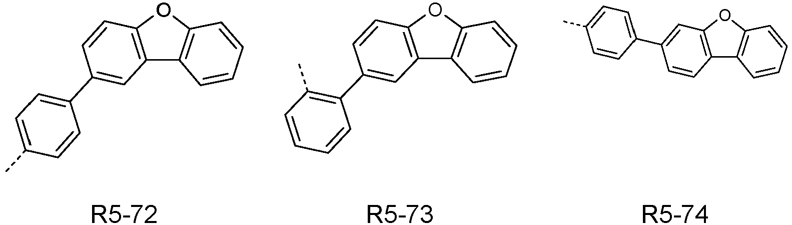 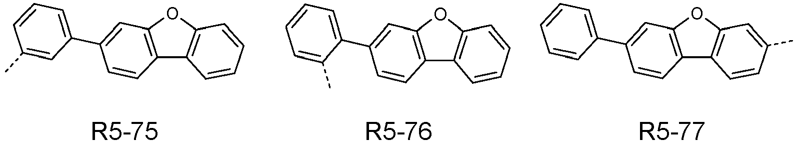 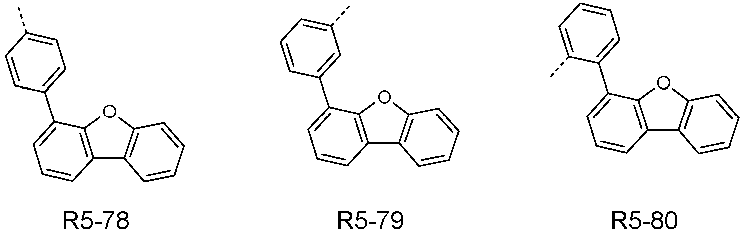 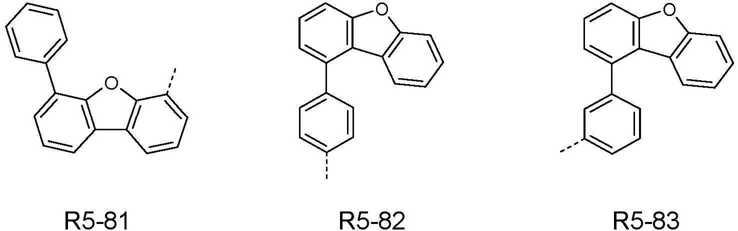 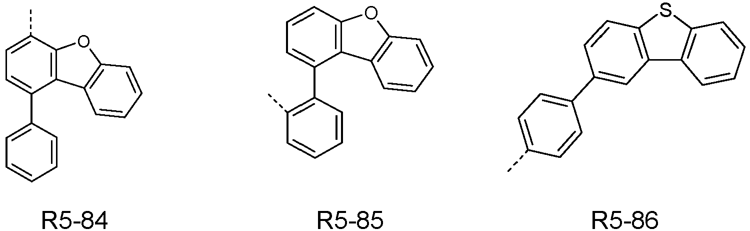 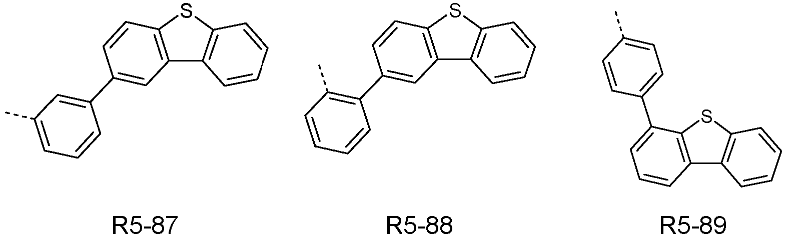 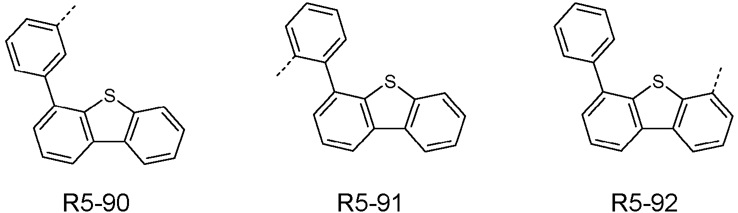 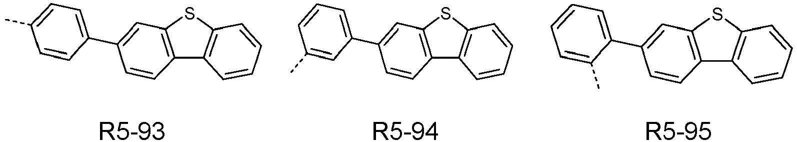 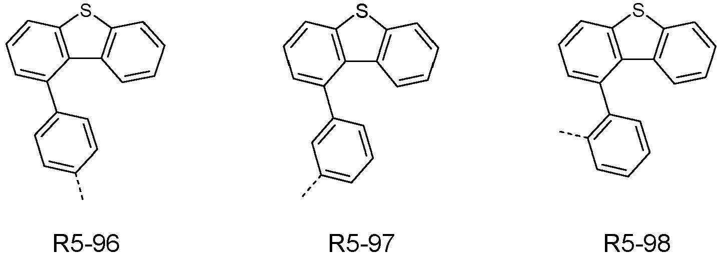 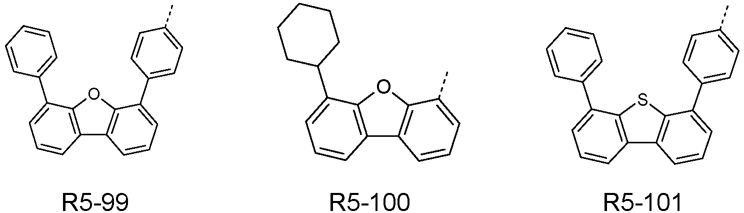 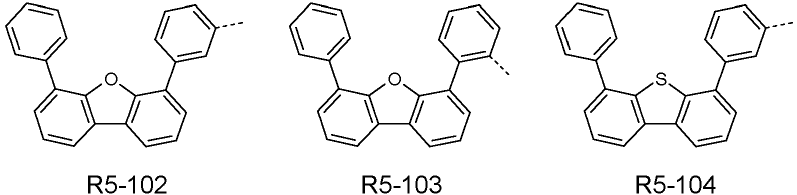 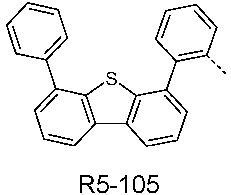 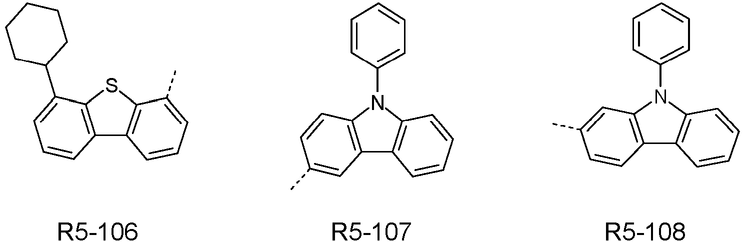 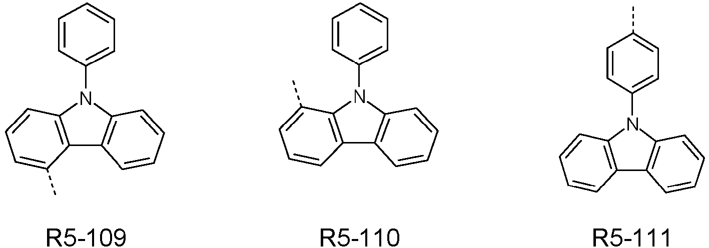 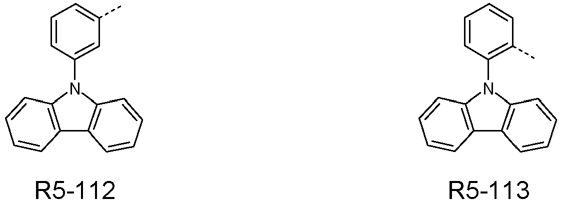 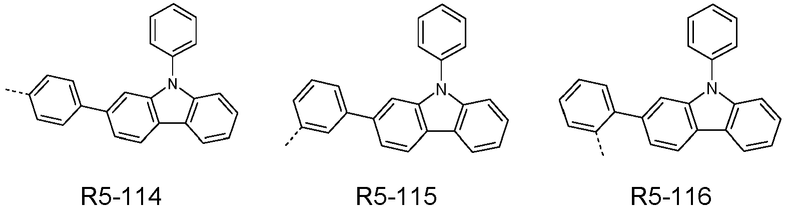 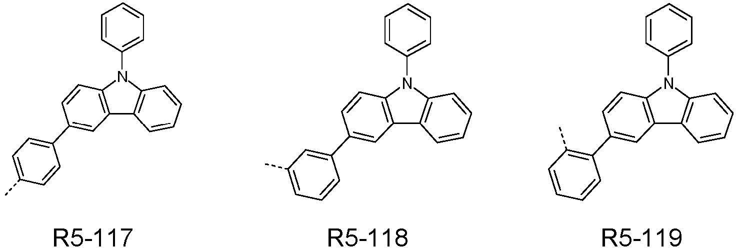 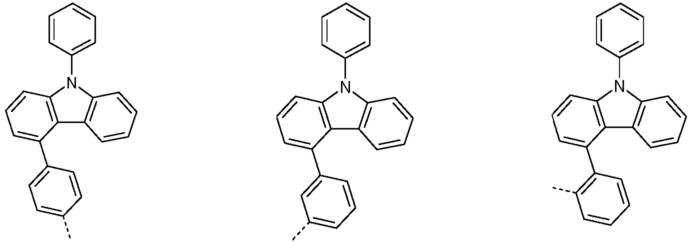 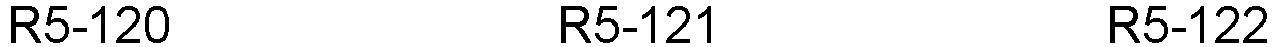 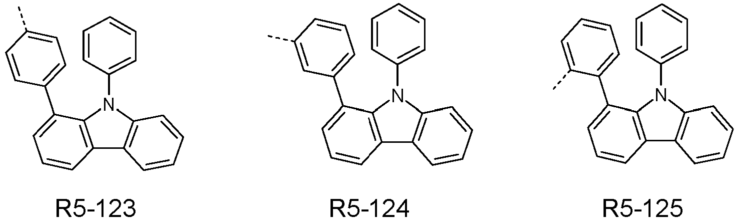 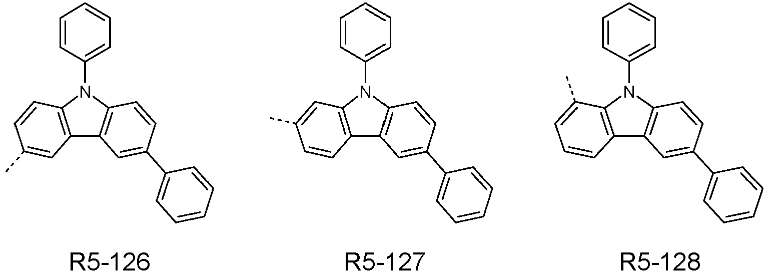 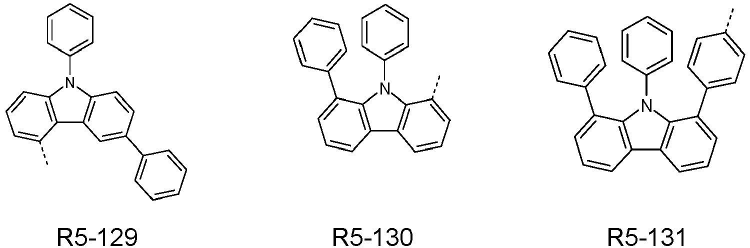 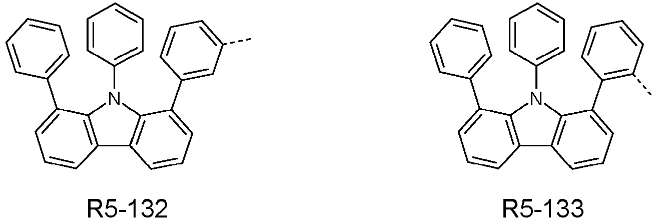 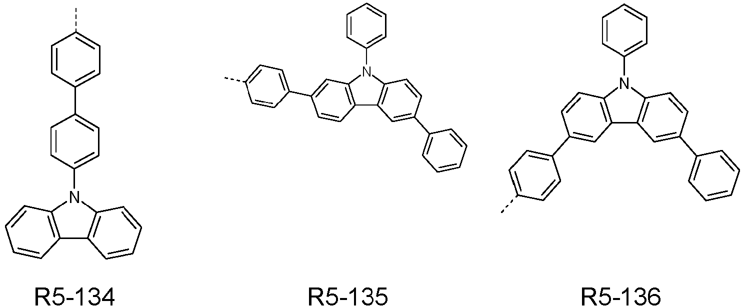 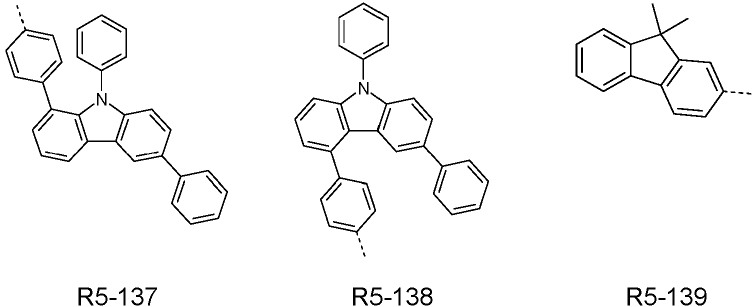 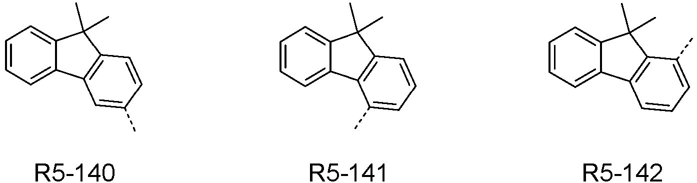 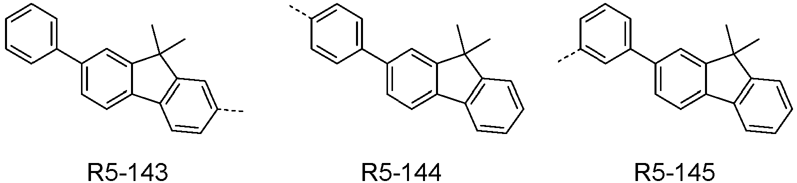 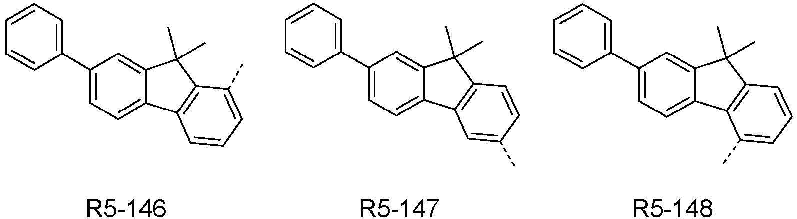 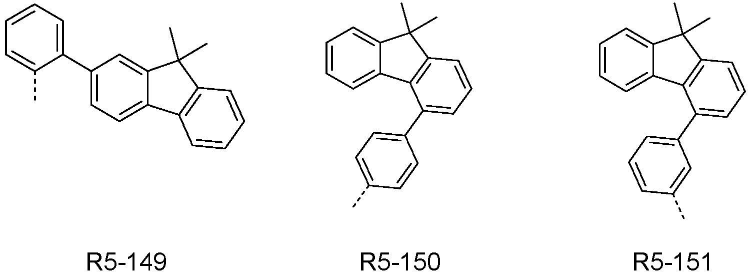 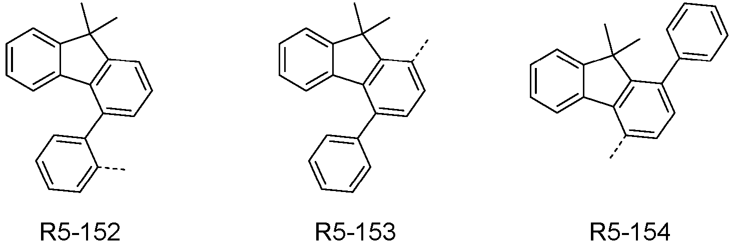 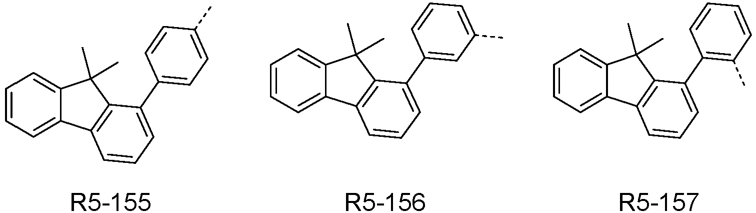 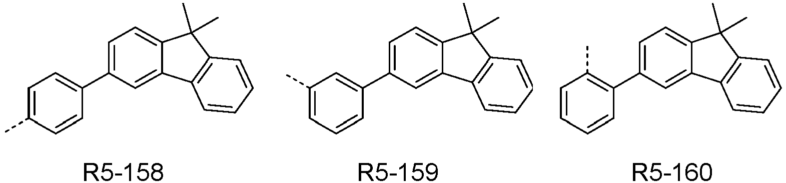 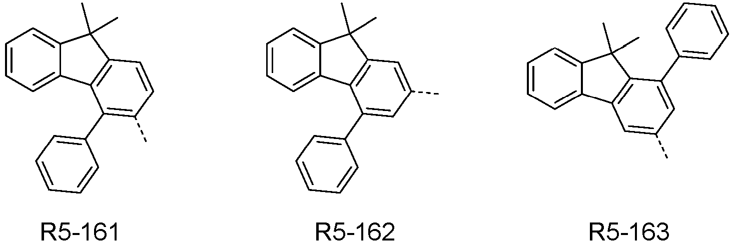 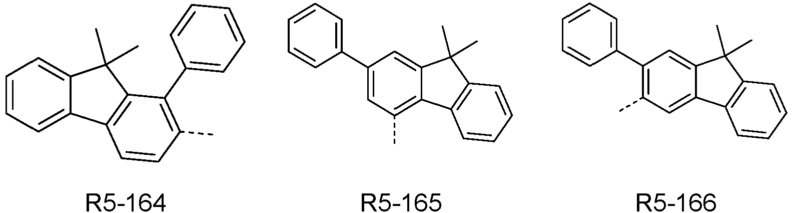 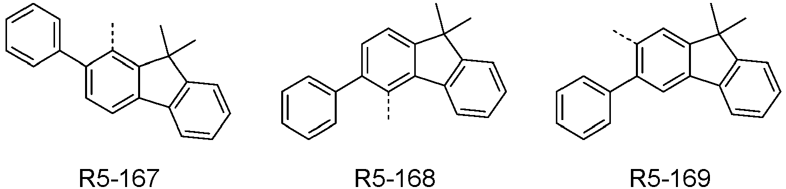 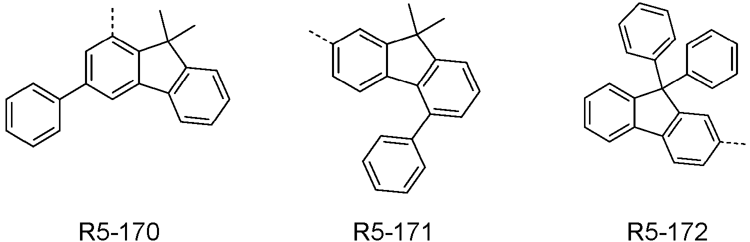 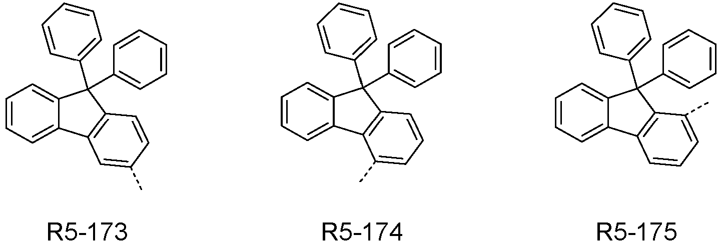 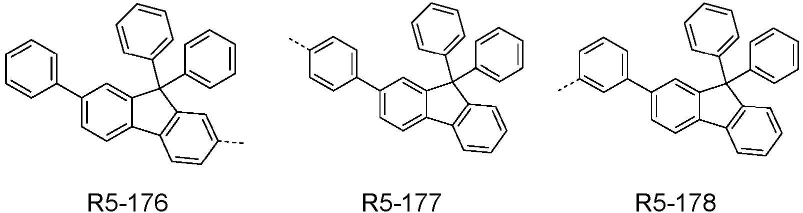 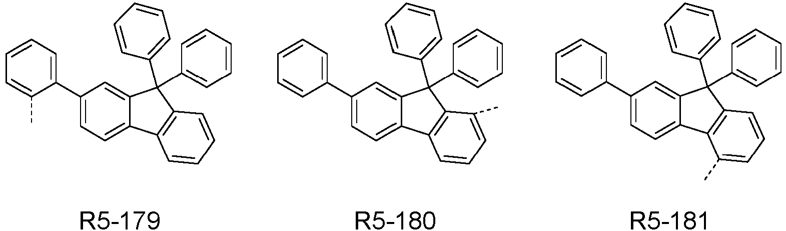 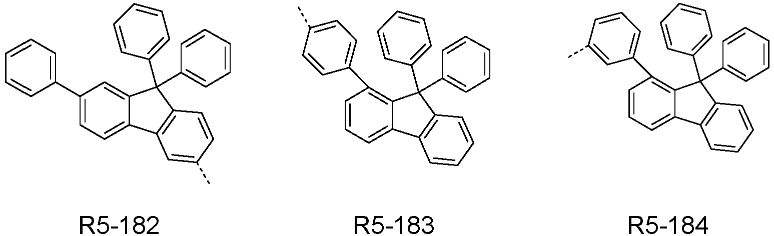 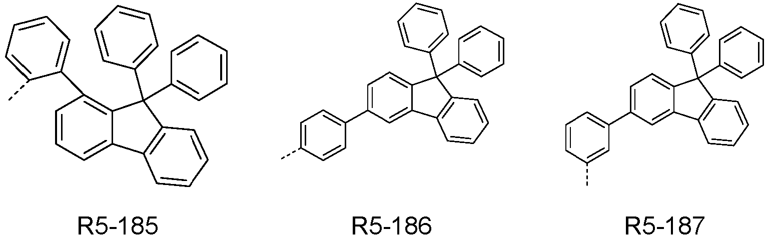 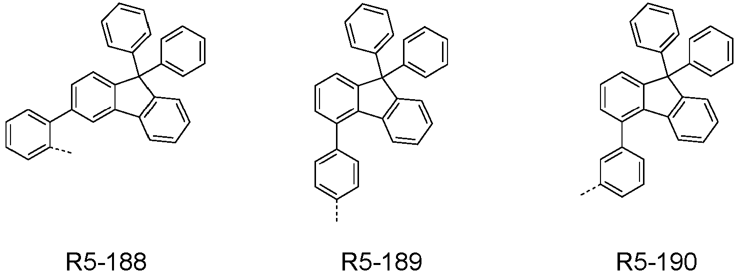 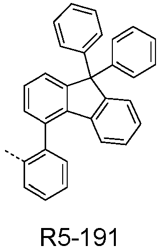 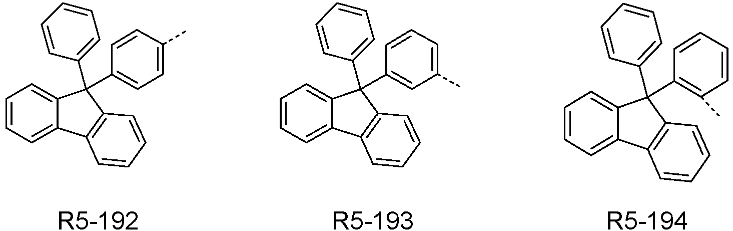 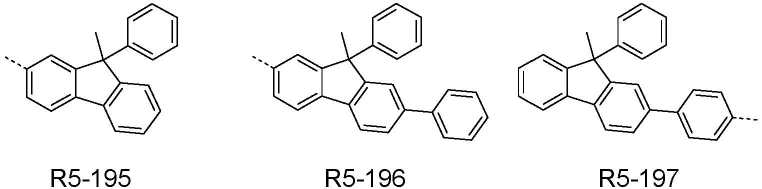 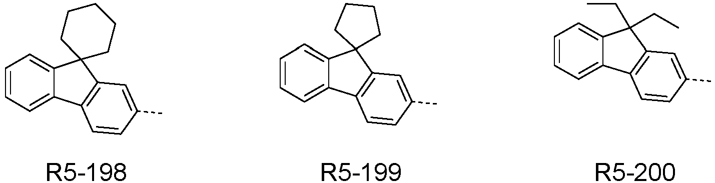 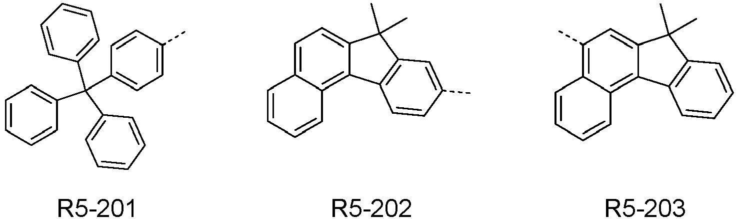 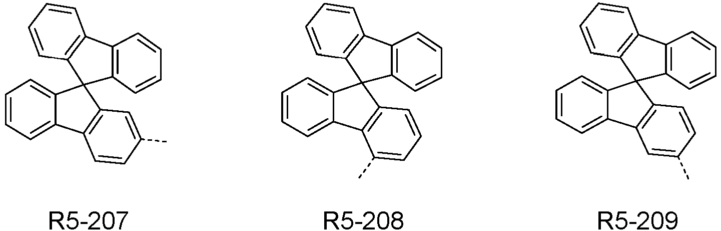 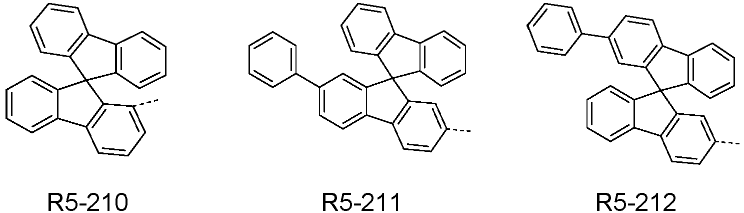 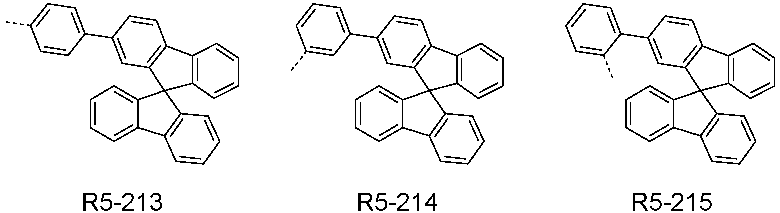 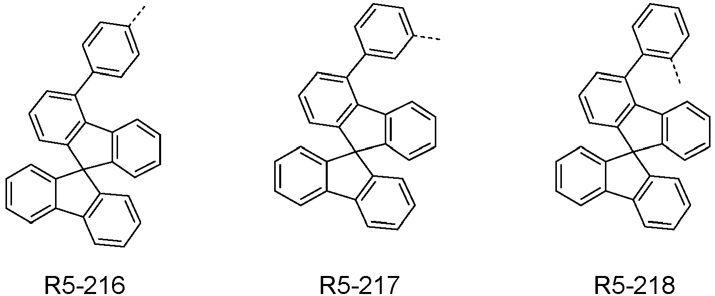 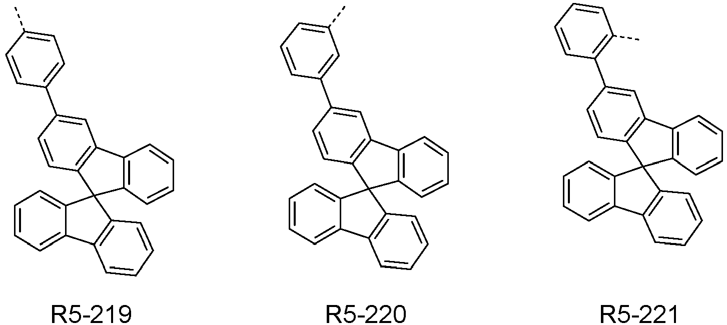 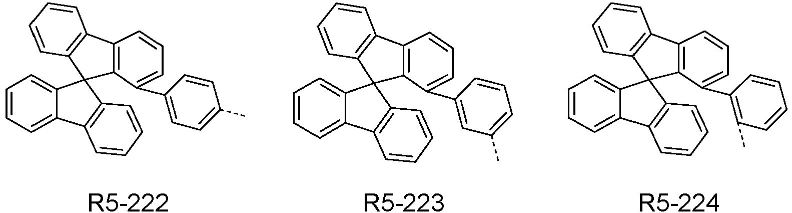 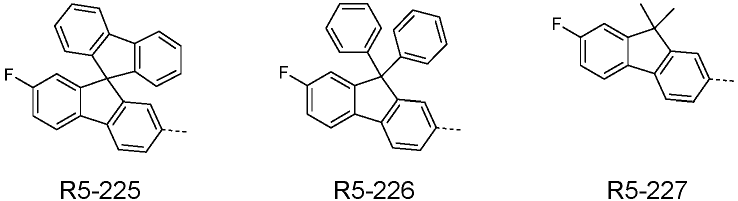 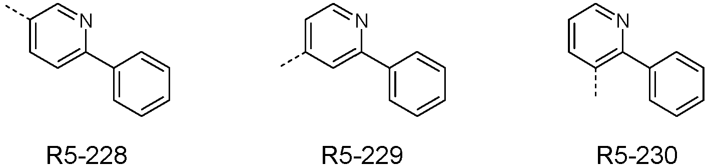 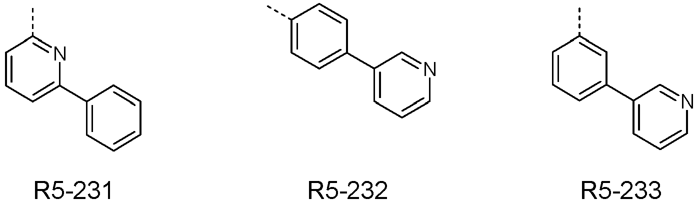 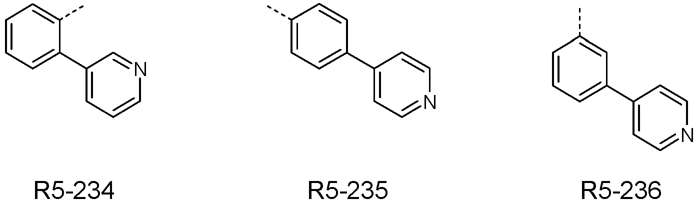 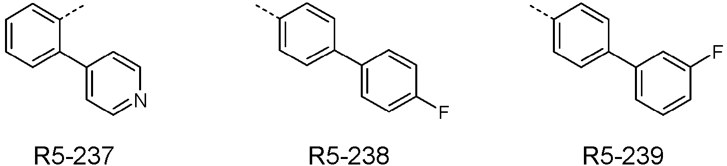 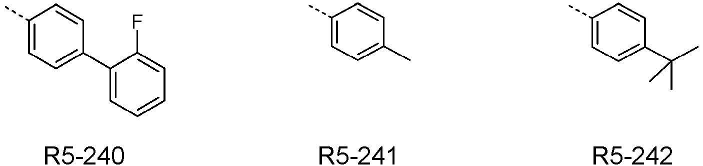 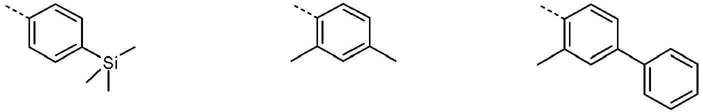 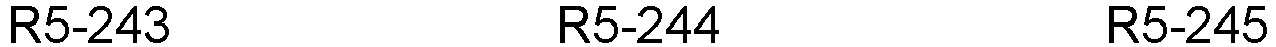 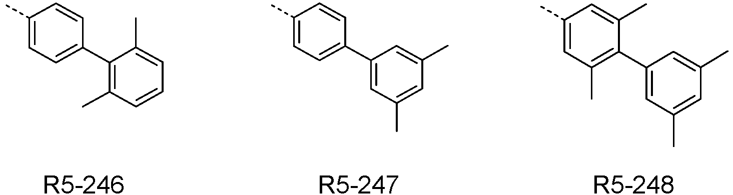 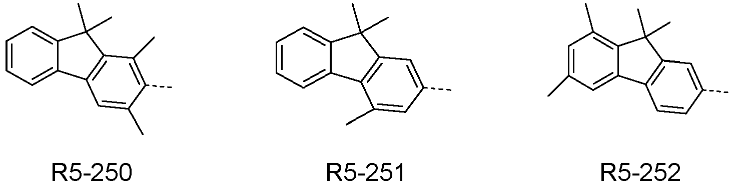 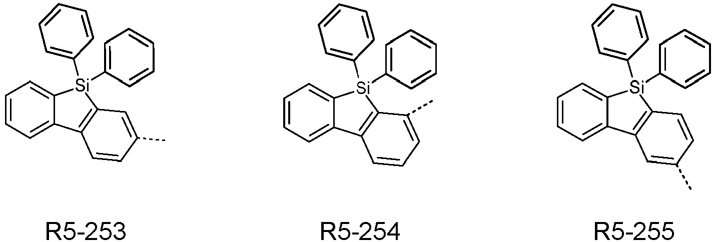 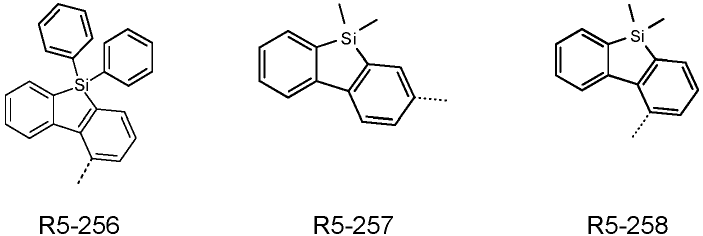 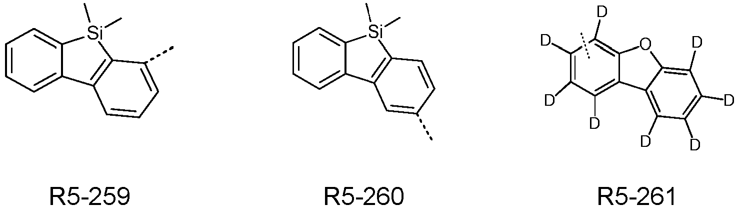 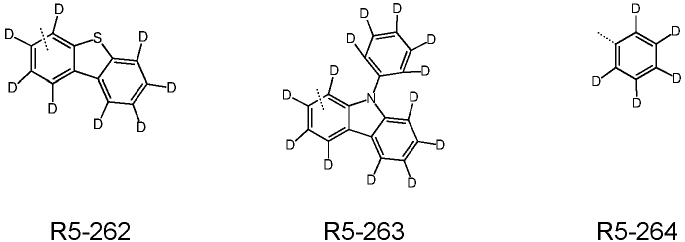 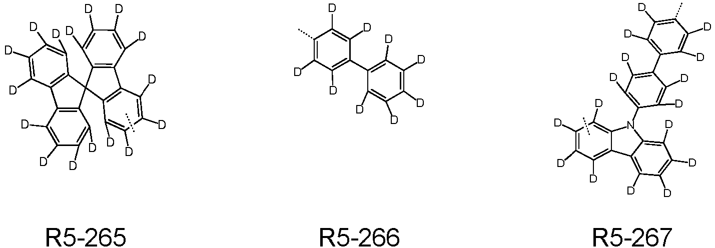 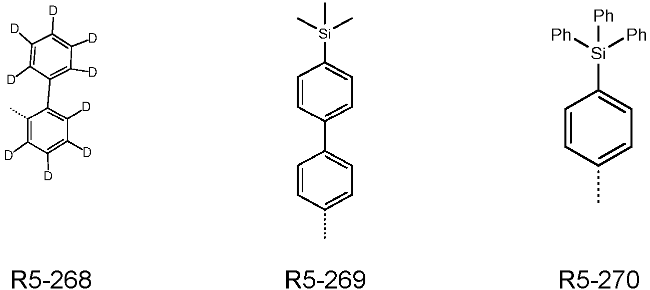 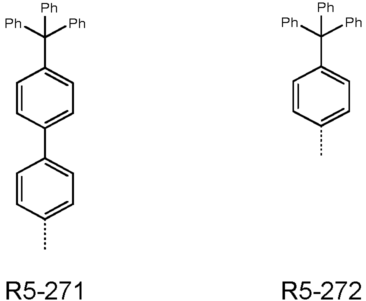 wobei die genannten Reste R⁵ an den unsubstituiert dargestellten Positionen mit Resten R¹¹ substituiert sein können, wobei R¹¹ in diesen Positionen bevorzugt H ist, und wobei die gestrichelte Bindung die Bindung an das Amin-Stickstoffatom ist; wobei für die verwendeten Symbole die Definitionen aus den obigen Ansprüchen gelten.

10. Die Verbindung gemäß einem oder mehreren der Ansprüche 1 bis 9, **dadurch gekennzeichnet, dass** m, n und o gleich 0 sind.

11. Verfahren zur Herstellung einer Verbindung gemäß einem oder mehreren der Ansprüche 1 bis 10 mit Hilfe der Suzuki Kupplung oder der Buchwald Reaktion.

12. Oligomer, Polymer oder Dendrimer, enthaltend eine oder mehrere Verbindungen gemäß einem oder mehreren der Ansprüche 1 bis 10, wobei die Bindung(en) zum Polymer, Oligomer oder Dendrimer an beliebigen, in Formel (3) mit R¹, R², R³, R⁴, R⁵, R⁶, R⁷, R⁸, R¹¹ oder R¹² substituierten Positionen lokalisiert sein können.

13. Zusammensetzung enthaltend eine oder mehrere Verbindungen der Formel (3) sowie wenigstens ein weiteres Material, das ausgewählt ist aus der Gruppe bestehend aus den Lochtransportmaterialien, Lochinjektionsmaterialien, p-Dotanden, Elektronenblockiermaterialien, Matrixmaterialien, Emittern und Elektronentransportmaterialien.

14. Formulierung, enthaltend mindestens eine Verbindung nach einem oder mehreren der Ansprüche 1 bis 10 oder mindestens ein Polymer, Oligomer oder Dendrimer gemäß Anspruch 12 oder mindestens eine Zusammensetzung gemäß Anspruch 13, sowie mindestens ein Lösungsmittel.

15. Elektronische Vorrichtung, enthaltend mindestens eine Verbindung gemäß einem oder mehreren der Ansprüche 1 bis 10, oder mindestens ein Polymer, Oligomer oder Dendrimer gemäß Anspruch 12 oder mindestens eine Zusammensetzung gemäß Anspruch 13.

16. Elektronische Vorrichtung gemäß Anspruch 15, **dadurch gekennzeichnet, dass** sie eine organische Elektrolumineszenzvorrichtung ist und Anode, Kathode und mindestens eine emittierende Schicht enthält, und dass die Verbindung in einer elektronenblockirenden Schicht oder in einer lochtransportierenden Schicht oder in einer emittierenden Schicht der Vorrichtung enthalten ist.

17. Organische Elektrolumineszenzvorrichtung gemäß Anspruch 16, **dadurch gekennzeichnet, dass** die Verbindung in einer lochtransportierenden Schicht enthalten ist, die eine Lochtransportschicht oder eine Elektronenblockierschicht ist.

18. Verwendung einer Verbindung gemäß einem oder mehreren der Ansprüche 1 bis 10 in einer elektronischen Vorrichtung.

## Claims

1. Compound of the formula (3) 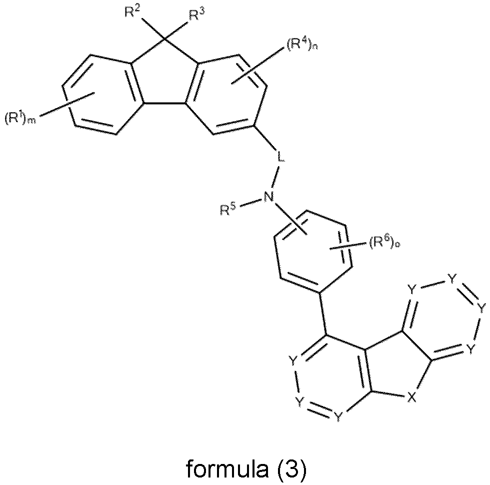 where the following applies to the symbols used:
X is equal to O or S, preferably O;
Y is, identically or differently on each occurrence, CR⁷ or N, it is preferred if Y is equal to CR⁷;
L is a divalent aromatic ring system having 6 to 40 aromatic ring atoms;
R¹, R⁴, R⁶ and R⁷ are selected on each occurrence, identically or differently, from H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ do not form a ring with one another, two or more radicals R⁴ may be linked to one another and may form a ring and/or two or more radicals R⁶ may be linked to one another and may form a ring and/or two or more radicals R⁷ may be linked to one another and may form a ring; where the said alkyl, alkoxy, alkenyl and alkynyl groups and the said aromatic ring systems and heteroaromatic ring systems may in each case be substituted by radicals R¹¹; and where one or more CH₂ groups in the said alkyl, alkoxy, alkenyl and alkynyl groups may be replaced by -R¹¹ C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO or SO₂; preferably, two or more radicals R⁴ do not form a ring with one another, and/or two or more radicals R⁶ do not form a ring with one another and/or two or more radicals R⁷ do not form a ring with one another;
R² and R³ are selected on each occurrence, identically or differently, from straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where the two radicals R² and R³ may be linked to one another and may form a ring; where the said alkyl, alkoxy, alkenyl and alkynyl groups and the said aromatic ring systems and heteroaromatic ring systems may in each case be substituted by radicals R¹¹; and where one or more CH₂ groups in the said alkyl, alkoxy, alkenyl and alkynyl groups may be replaced by -R¹¹C=CR¹¹- -C≡C- Si(R¹¹)₂, C=O, C=NR¹¹, -C(=O)O-, -C(=O)NR¹¹-, NR¹¹, P(=O)(R¹¹), -O-, -S-, SO or SO₂; if the two radicals R² and R³ form a ring, a spiro compound forms, preferably a spirobifluorene; it is particularly preferred if the two radicals R² and R³ do not form a ring with one another;
R⁵ is an aromatic ring system having 6 to 40 aromatic ring atoms, which may be substituted by one or more radicals R¹¹, or a heteroaromatic ring system having 5 to 40 aromatic ring atoms, which may be substituted by one or more radicals R¹¹, where the radical R⁵ does not contain a condensed heteroaromatic ring system having more than 12 aromatic carbon atoms as ring atoms in the heteroaromatic ring system;
R¹¹ is selected on each occurrence, identically or differently, from H, D, F, Cl, Br, I, C(=O)R¹², CN, Si(R¹²)₃, N(R¹²)₂, P(=O)(R¹²)₂, OR¹², S(=O)R¹², S(=O)₂R¹², straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹¹ may be linked to one another and may form a ring; where the said alkyl, alkoxy, alkenyl and alkynyl groups and the said aromatic ring systems and heteroaromatic ring systems may in each case be substituted by radicals R¹²; and where one or more CH₂ groups in the said alkyl, alkoxy, alkenyl and alkynyl groups may be replaced by -R¹²C=CR¹²-, -C≡C-, Si(R¹²)₂, C=O, C=NR¹², -C(=O)O-, -C(=O)NR¹²-, NR¹², P(=O)(R¹²), -O-, -S-, SO or SO₂; where two or more radicals R¹¹ cannot form a ring with one another
R¹² is selected on each occurrence, identically or differently, from H, D, F, Cl, Br, I, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where the said alkyl, alkoxy, alkenyl and alkynyl groups, aromatic ring systems and heteroaromatic ring systems may be substituted by one or more radicals selected from F and CN;
m is 0, 1, 2, 3 or 4, it is preferred if m is equal to 0 or 1, it is very preferred if m is equal to 1 and it is very particularly preferred if m is equal to 0;
n is 0, 1, 2 or 3, it is preferred if n is equal to 0 or 1, it is very preferred if n is equal to 1 and it is very particularly preferred if n is equal to 0;
o is 0, 1, 2 or 3, it is preferred if o is equal to 0 or 1, it is very preferred if o is equal to 1 and it is very particularly preferred if o is equal to 0.

2. Compound according to Claim 1, **characterised in that** L is selected from the following formulae 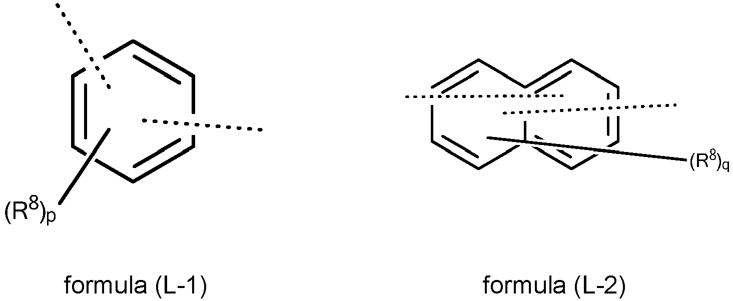 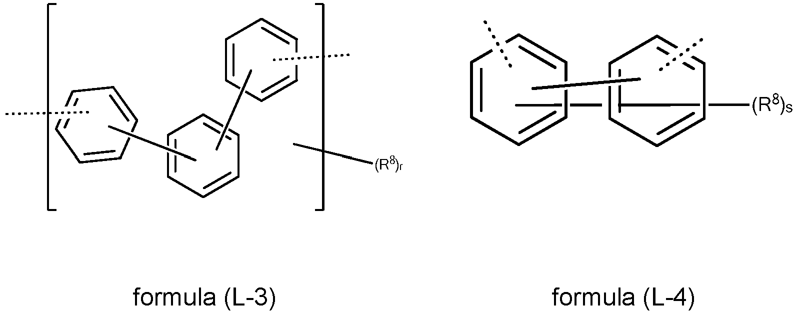 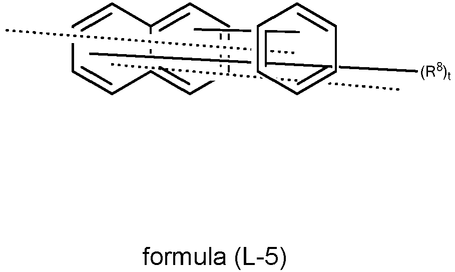 where the following applies:
R⁸ is selected on each occurrence, identically or differently, from H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R⁸ may be linked to one another and may form a ring; where the said alkyl, alkoxy, alkenyl and alkynyl groups and the said aromatic ring systems and heteroaromatic ring systems may in each case be substituted by radicals R¹¹; and where one or more CH₂ groups in the said alkyl, alkoxy, alkenyl and alkynyl groups may be replaced by -R¹¹C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO or SO₂; preferably, two or more radicals R⁸ do not form a ring with one another;
and where R¹¹ is defined as indicated in Claim 1;
p is 0, 1, 2, 3 or 4, it is preferred if p is equal to 0 or 1, it is very preferred if p is equal to 1 and it is very particularly preferred if p is equal to 0;
q is 0, 1, 2, 3, 4, 5 or 6, it is preferred if q is equal to 0 or 1, it is very preferred if q is equal to 1 and it is very particularly preferred if q is equal to 0;
r is 0, 1, 2, 3, 4, 5, 7, 8, 9, 10, 11 or 12, it is preferred if r is equal to 0 or 1, it is very preferred if r is equal to 1 and it is very particularly preferred if r is equal to 0;
s is 0, 1, 2, 3, 4, 5, 7 or 8, it is preferred if s is equal to 0 or 1, it is very preferred if s is equal to 1 and it is very particularly preferred if s is equal to 0;
t is 0, 1, 2, 3, 4, 5, 7, 8, 9 or 10, it is preferred if t is equal to 0 or 1, it is very preferred if t is equal to 1 and it is very particularly preferred if t is equal to 0.

3. Compound according to Claim 1 or 2, **characterised in that** L is selected from the following formulae 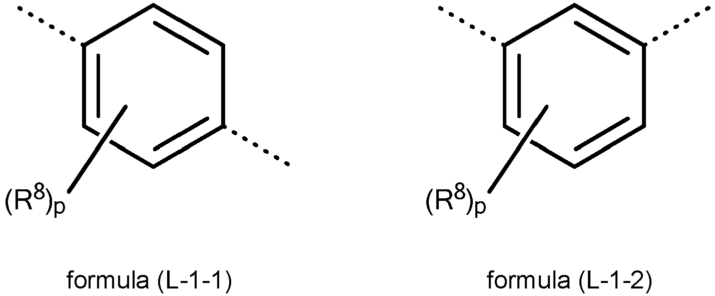 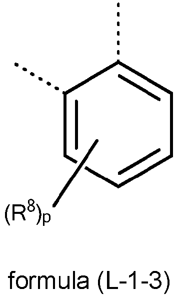 where the definitions from the above claims apply to the symbols used.

4. Compound according to Claim 1 or 2, **characterised in that** L is selected from the following formulae 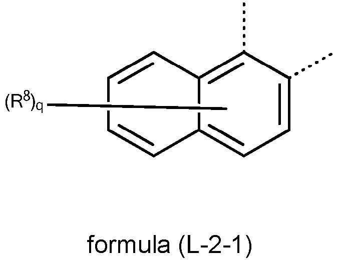 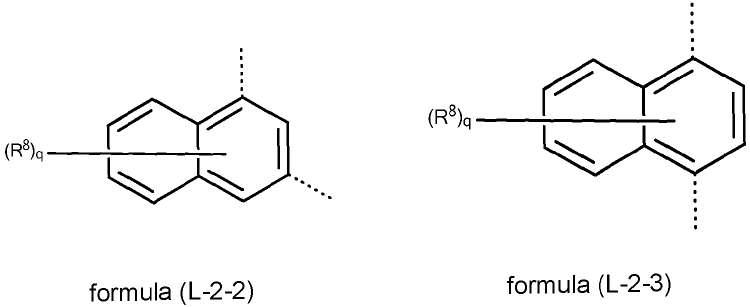 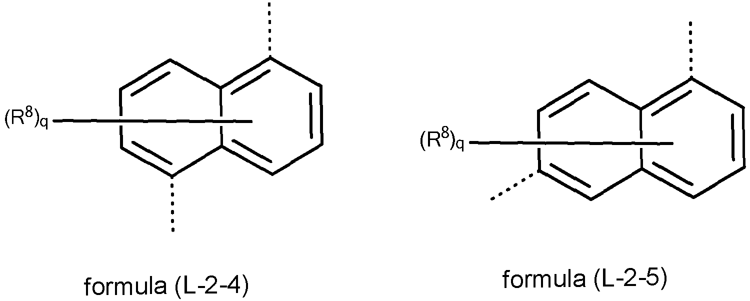 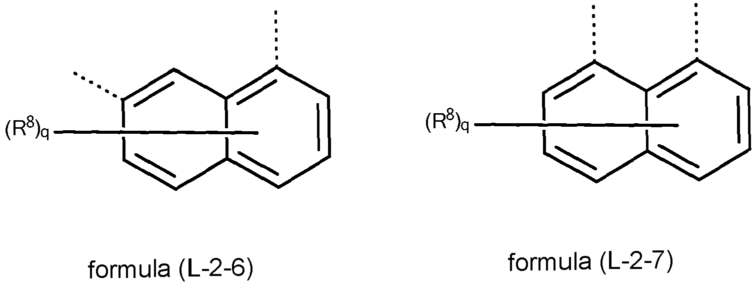 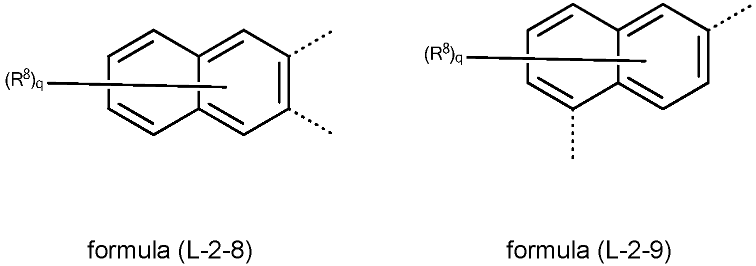 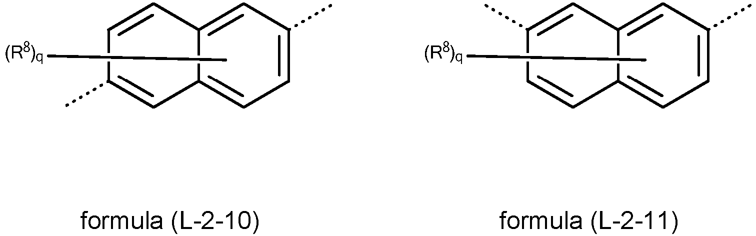 where the definitions from the above claims apply to the symbols used.

5. Compound according to Claim 1 or 2, **characterised in that** L is selected from the following formulae 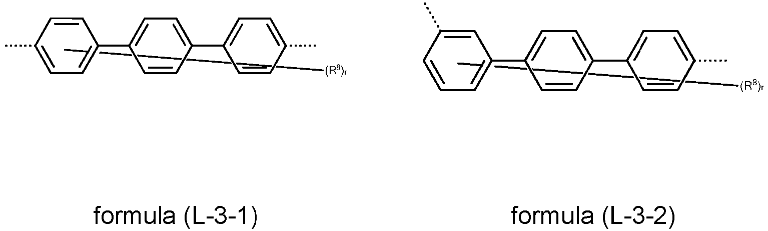 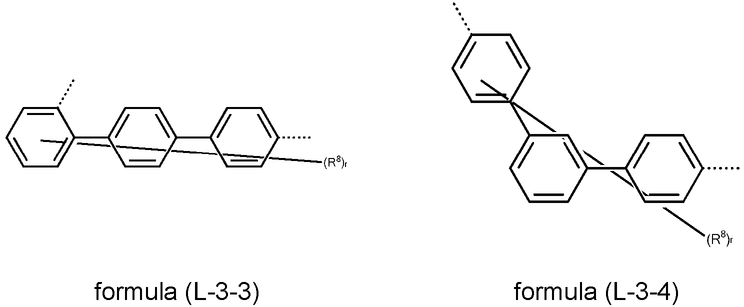 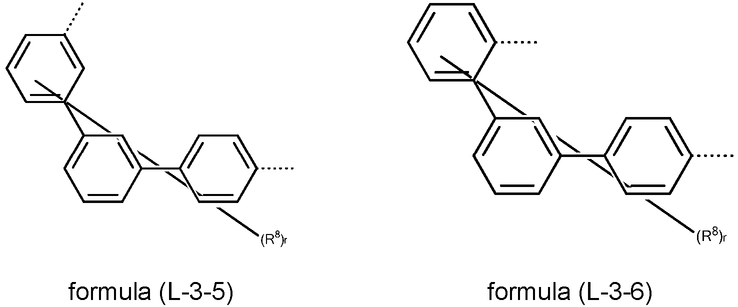 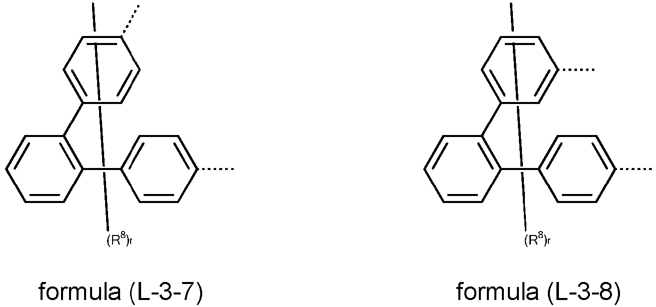 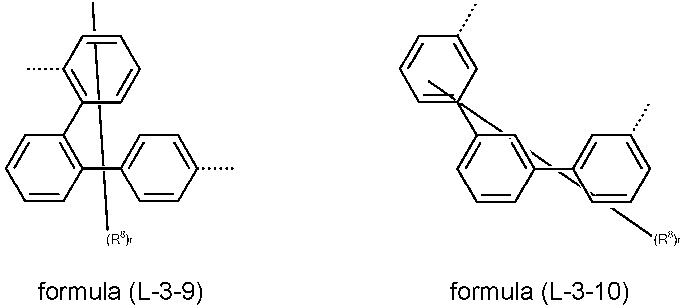 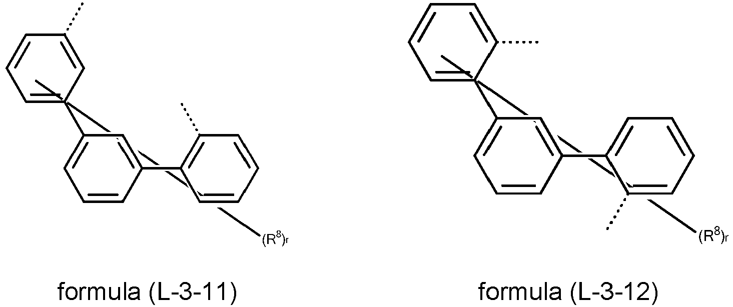 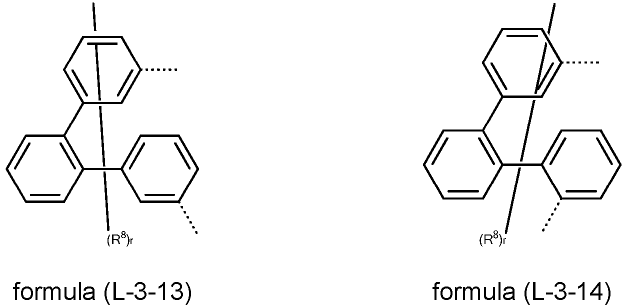 where the definitions from the above claims apply to the symbols used.

6. Compound according to Claim 1 or 2, **characterised in that** L is selected from the following formulae 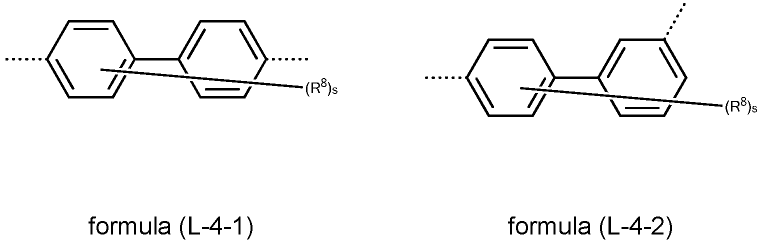 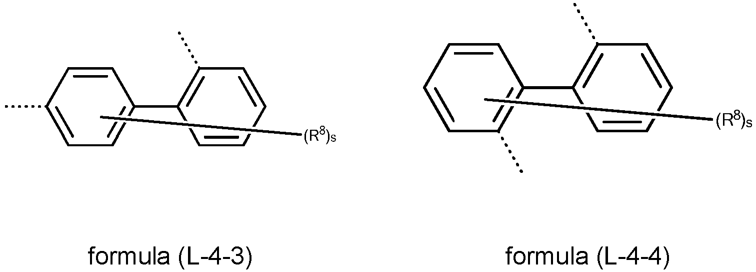 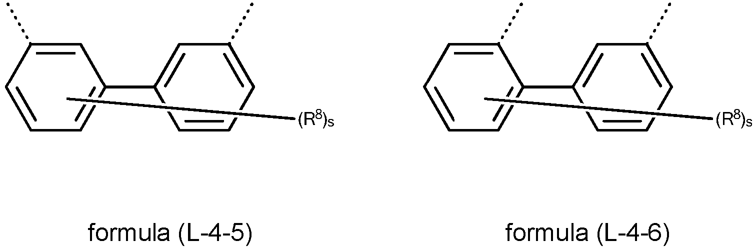 where the definitions from the above claims apply to the symbols used.

7. Compound according to one or more of Claims 1 to 6, **characterised in that** it is a monoamine compound.

8. Compound according to one or more of Claims 1 to 7, **characterised in that** R² and R³ are selected, identically or differently on each occurrence, from straight-chain alkyl groups having 1 to 10 C atoms, branched or cyclic alkyl groups having 3 to 10 C atoms or aromatic ring systems having 6 to 18 aromatic ring atoms; where the two radicals R² and R³ may be linked to one another and may form a ring; where the said alkyl groups and the said aromatic ring systems may in each case be substituted by radicals R¹¹; if the two radicals R² and R³ form a ring, a spiro compound forms, preferably a spirobifluorene; it is particularly preferred if the two radicals R² and R³ do not form a ring with one another.

9. Compound according to one or more of Claims 1 to 8, **characterised in that** the radicals R⁵ are selected from the following formulae 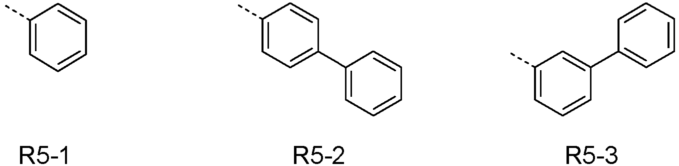 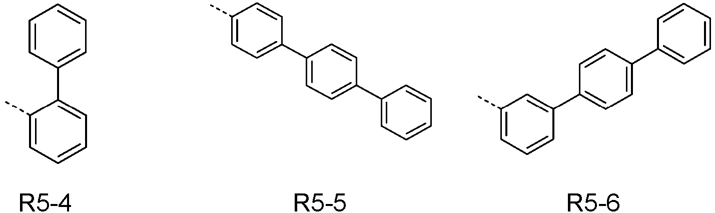 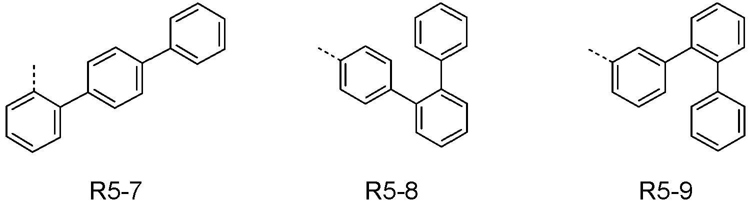 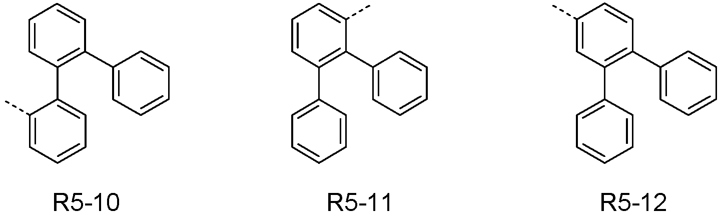 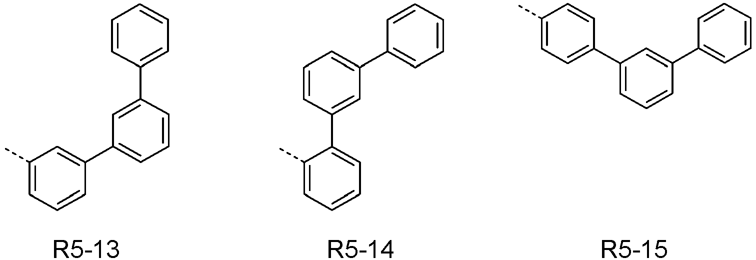 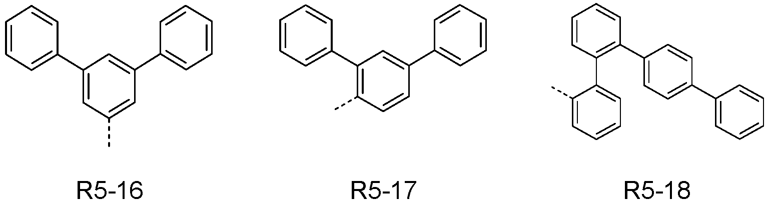 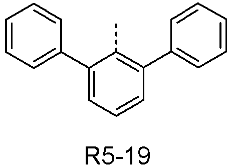 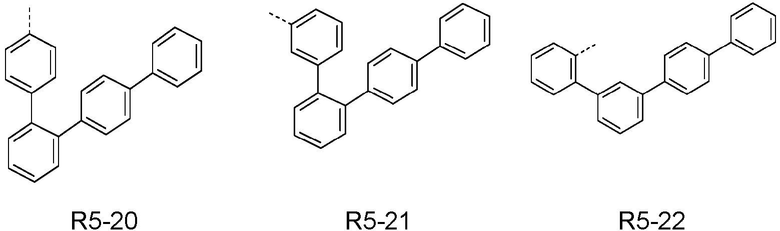 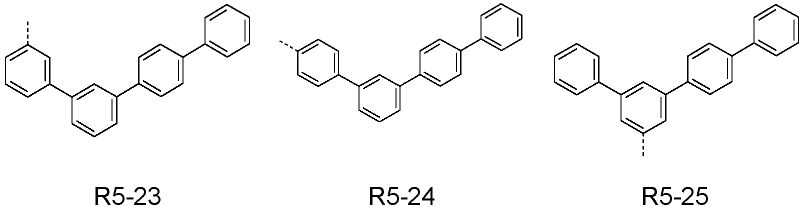 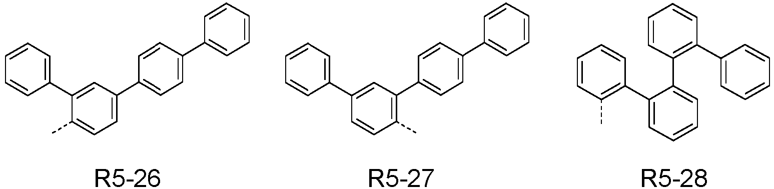 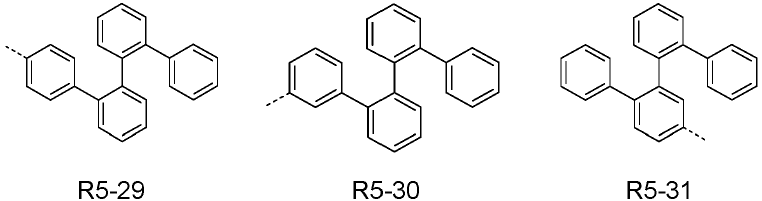 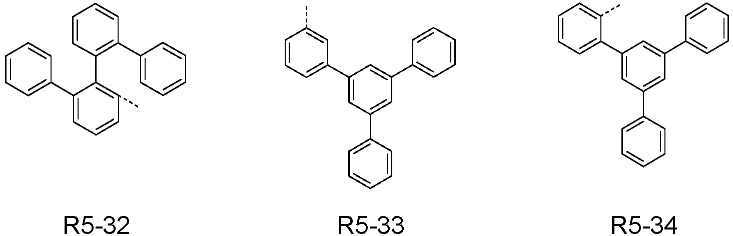 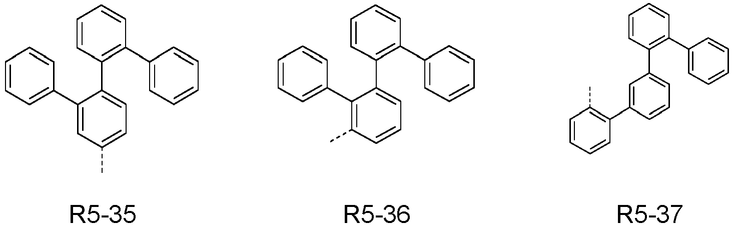 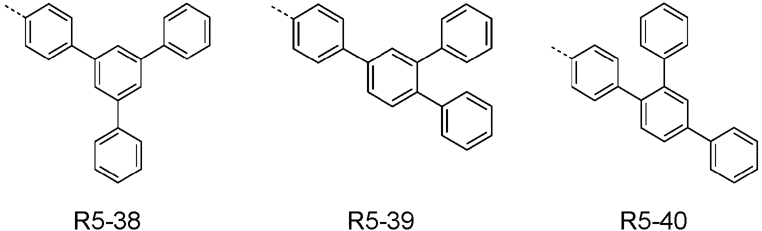 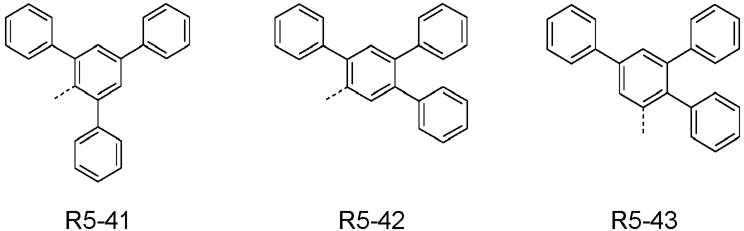 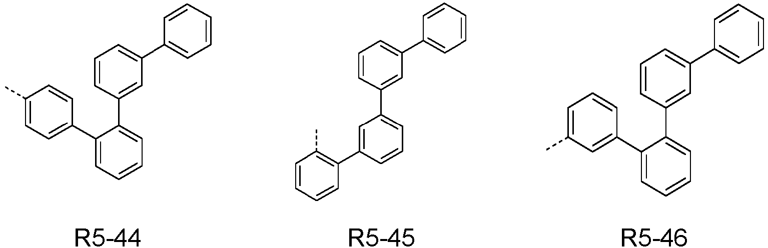 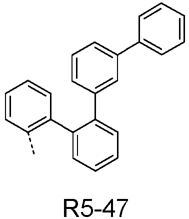 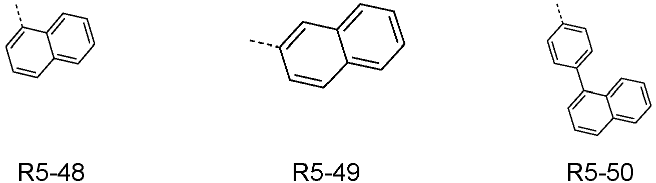 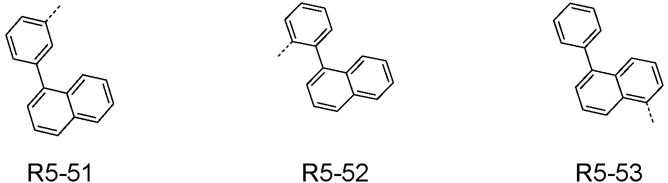 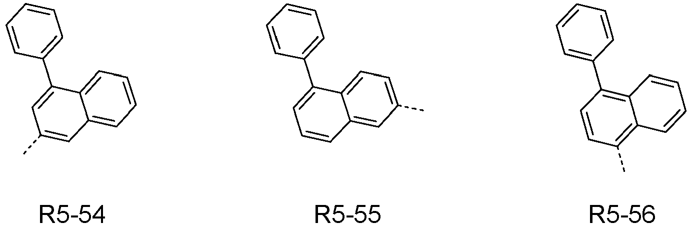 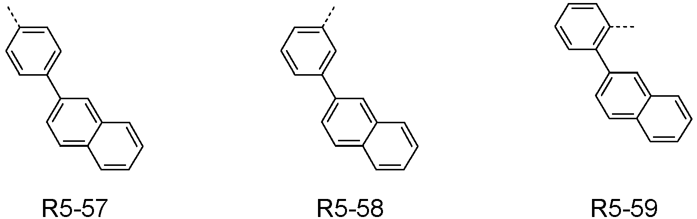 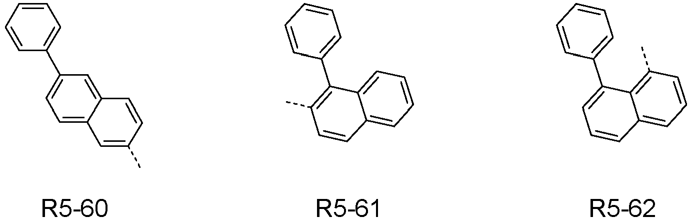 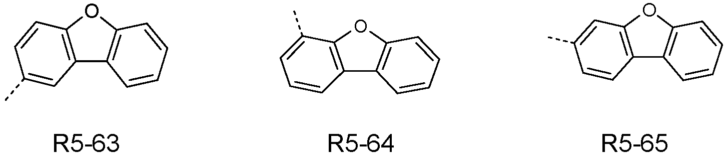 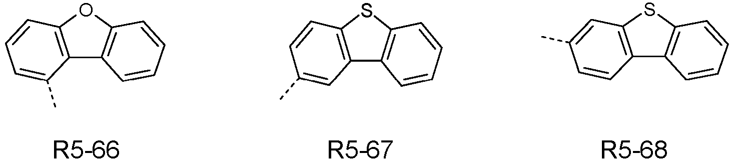 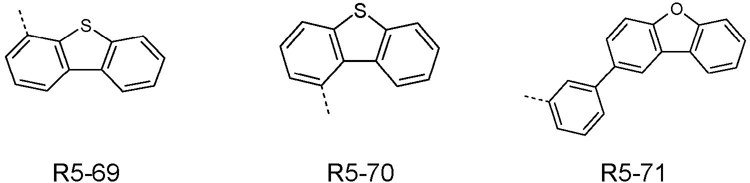 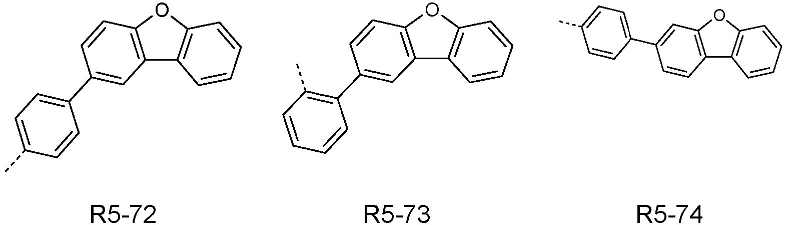 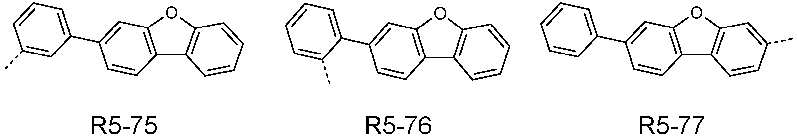 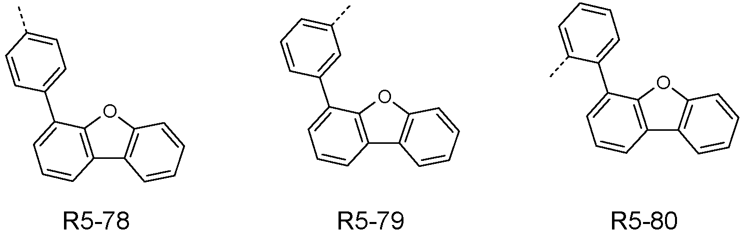 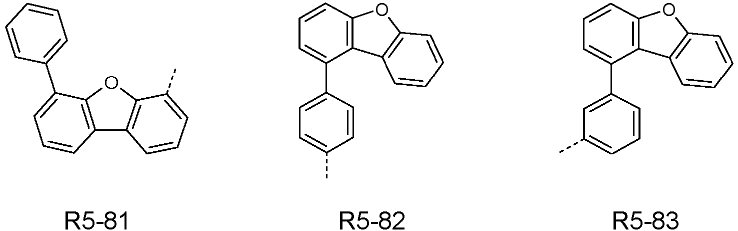 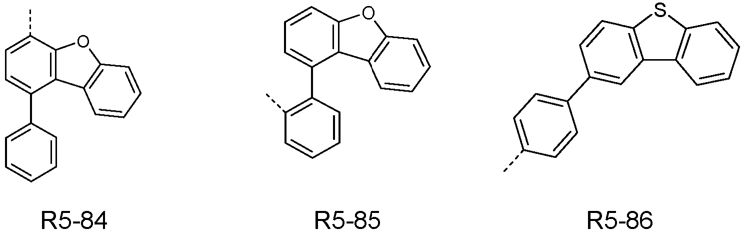 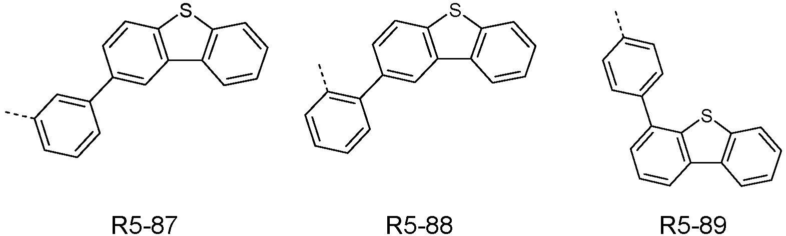 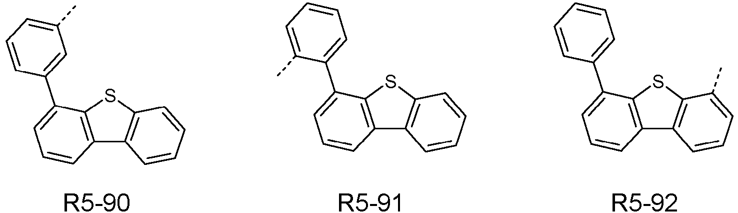 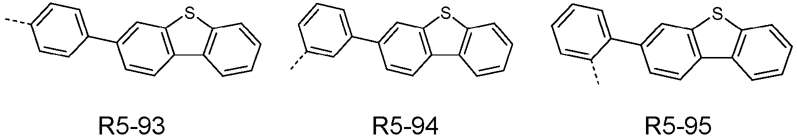 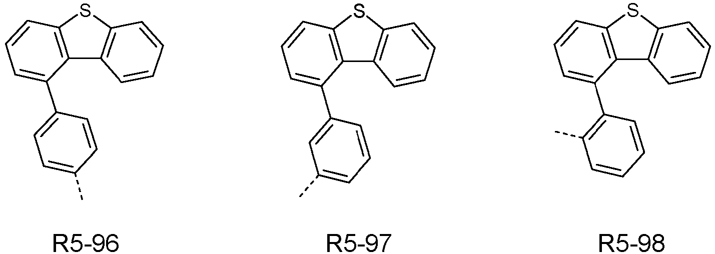 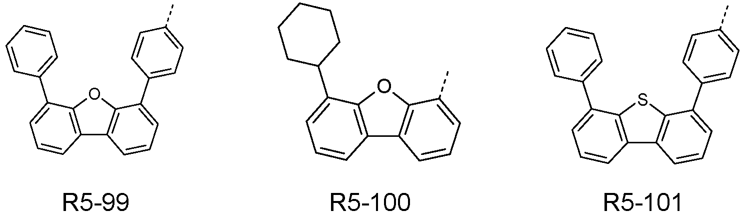 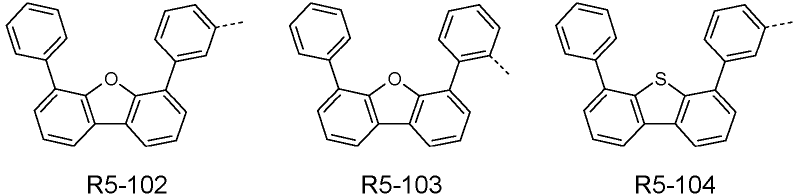 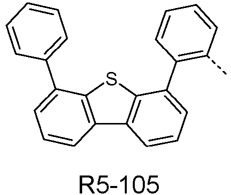 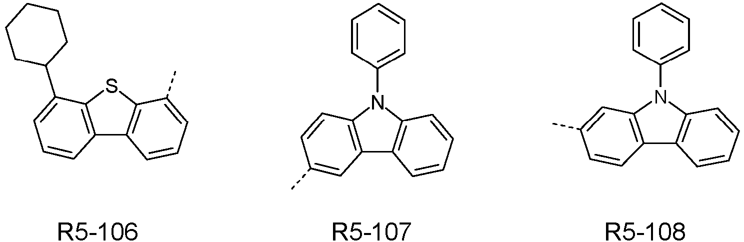 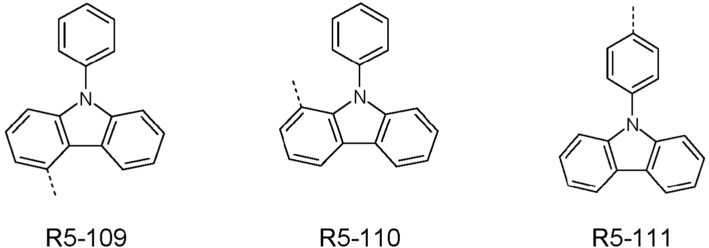 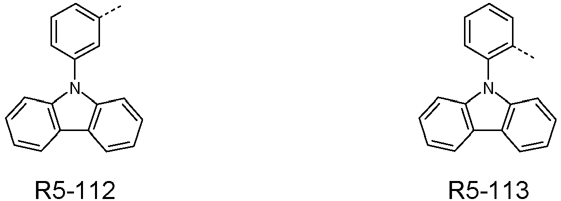 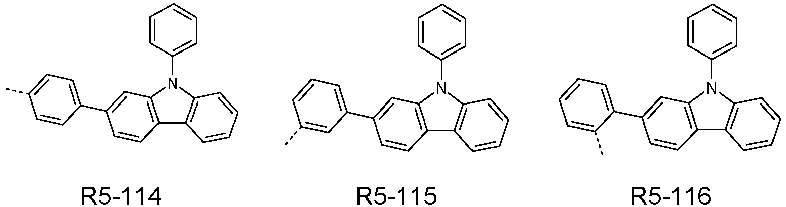 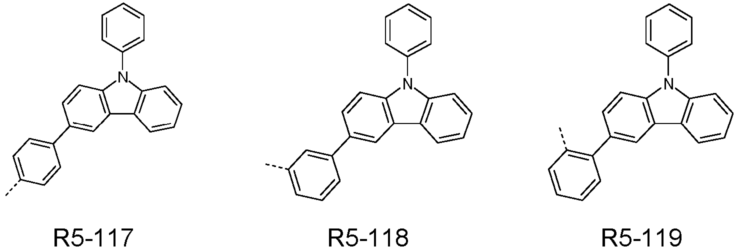 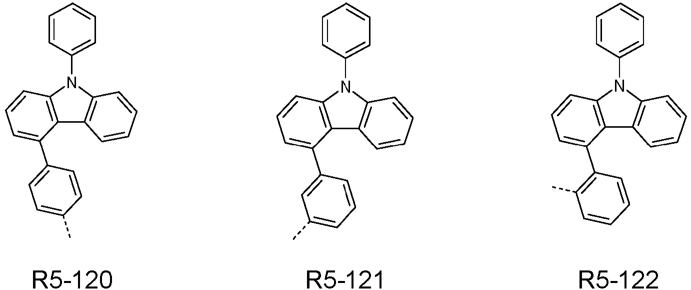 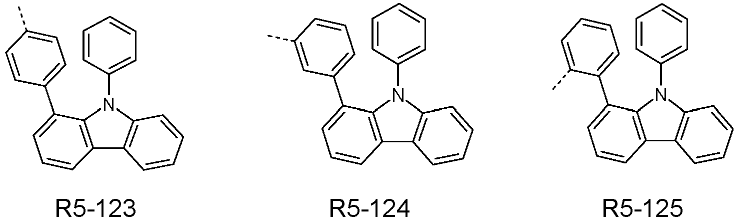 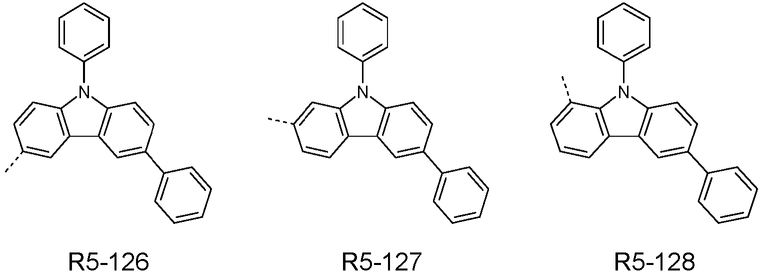 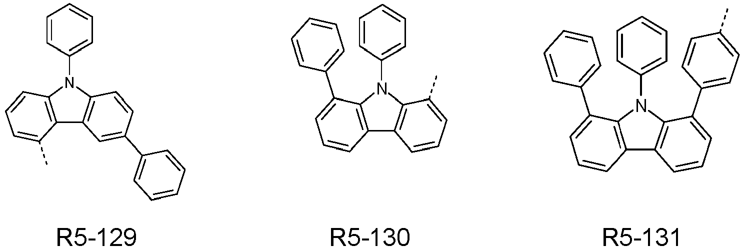 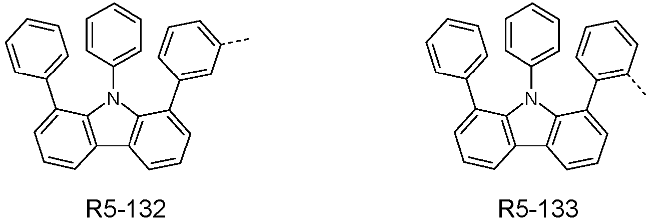 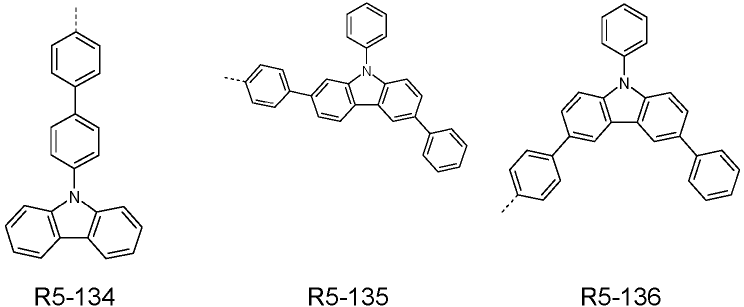 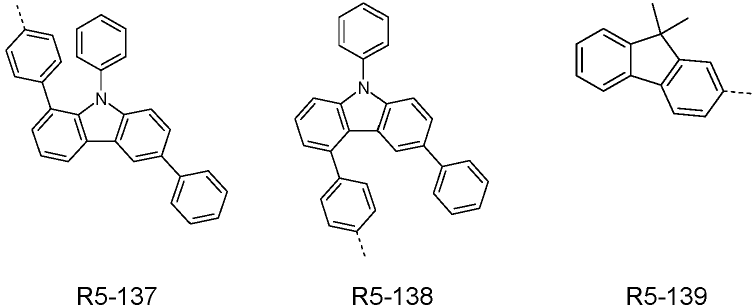 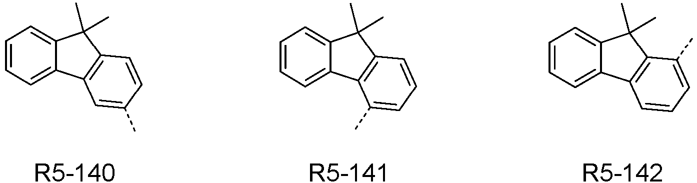 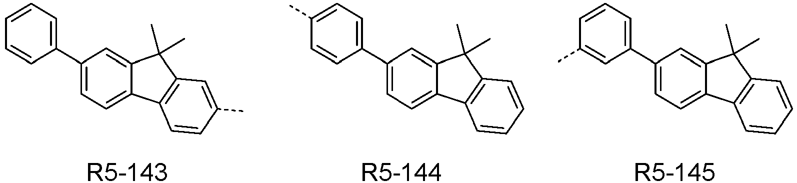 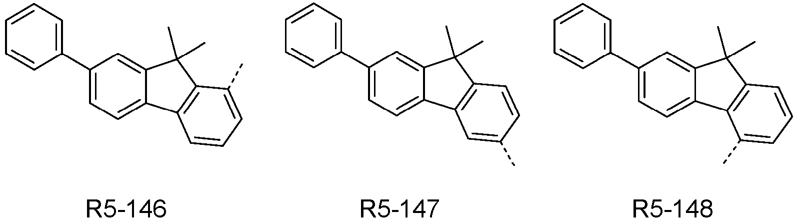 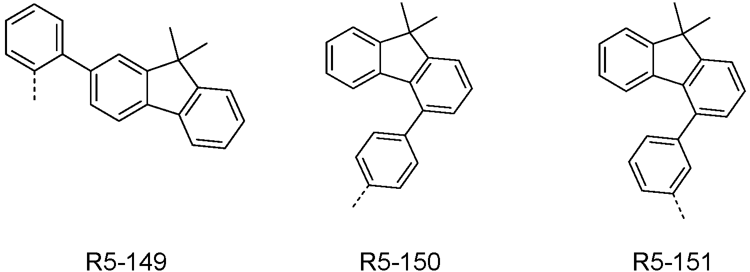 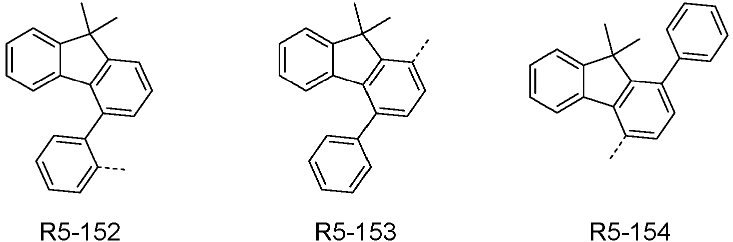 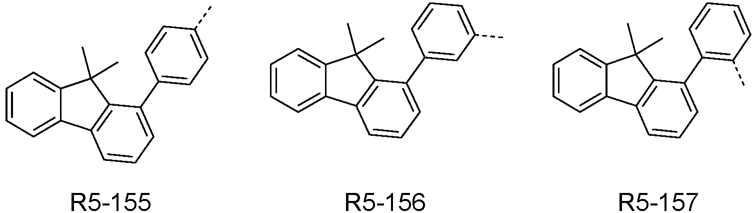 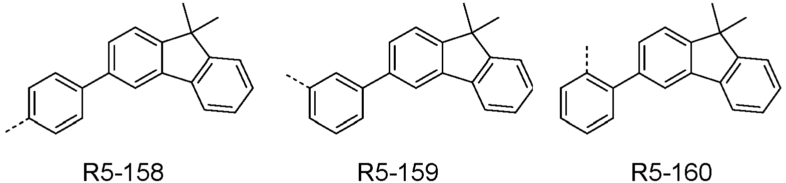 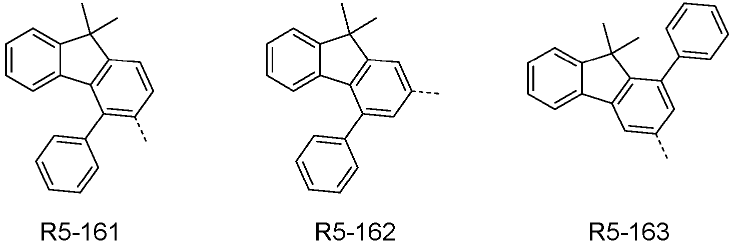 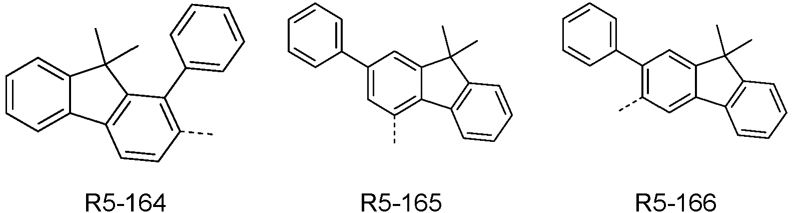 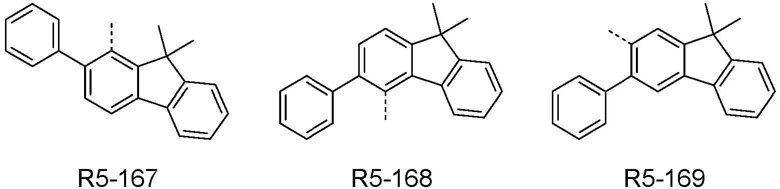 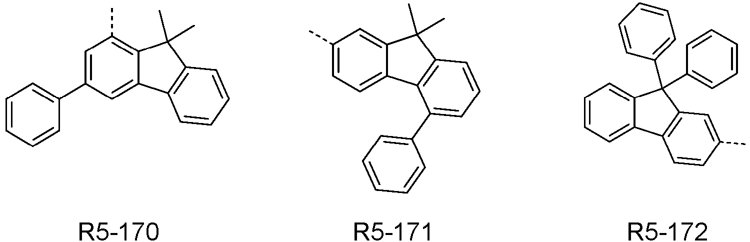 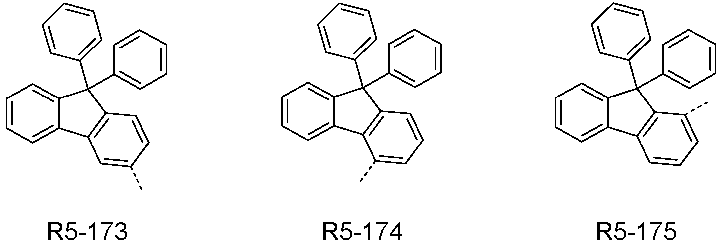 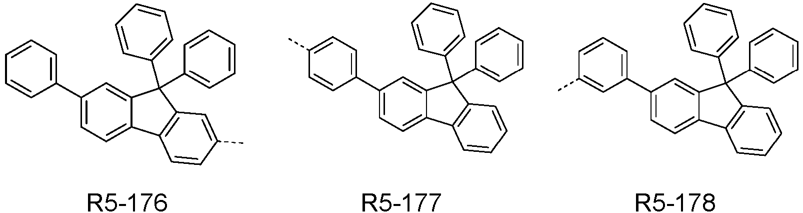 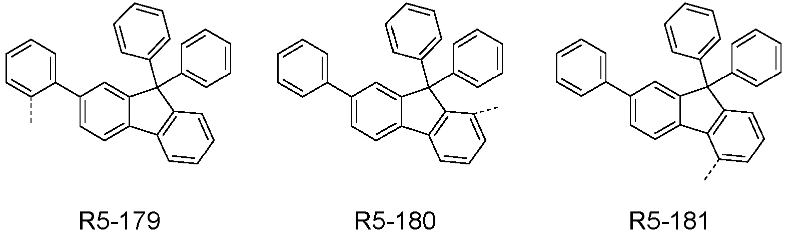 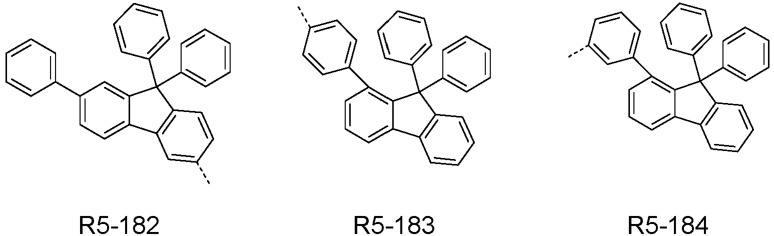 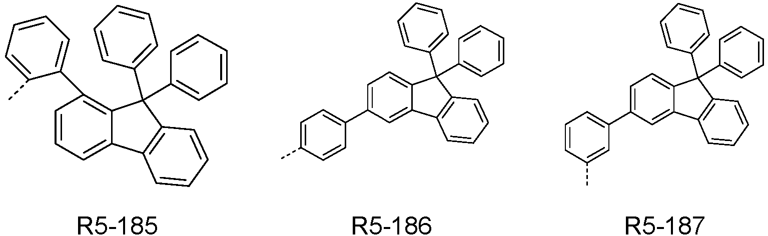 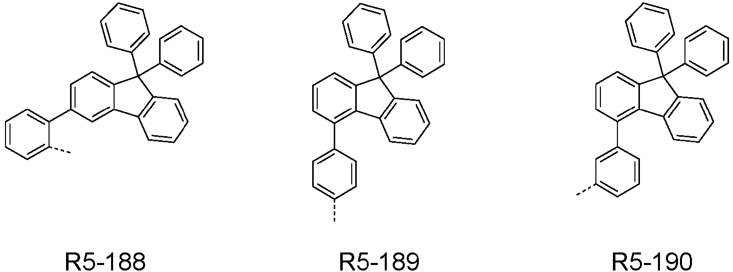 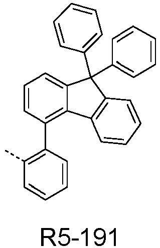 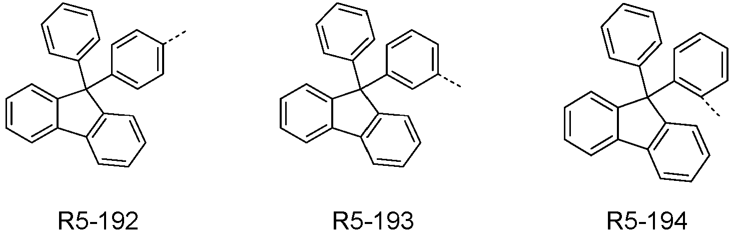 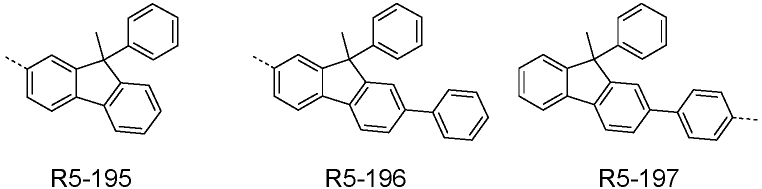 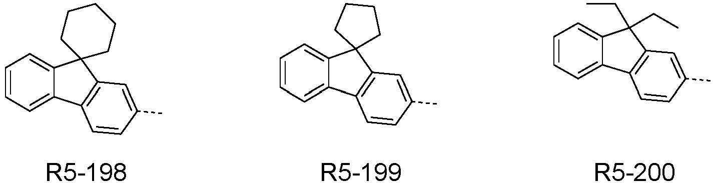 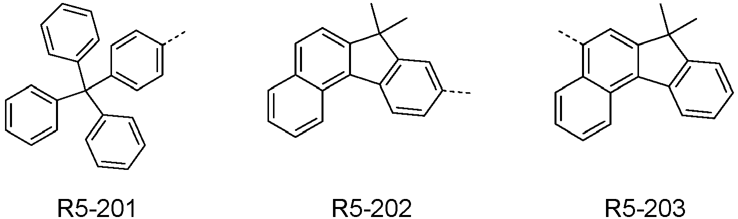 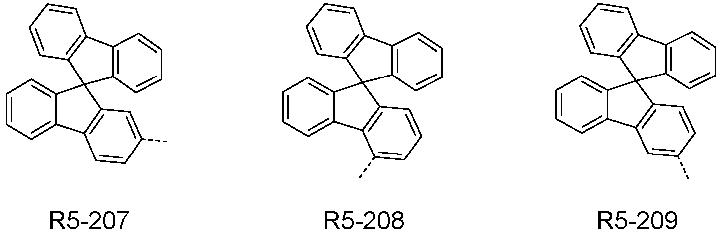 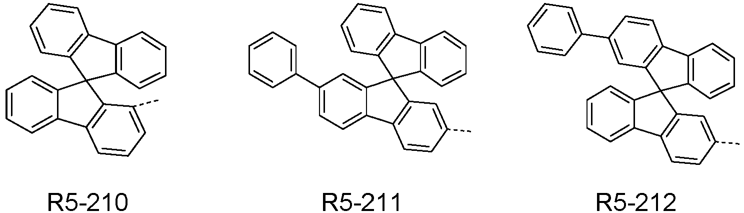 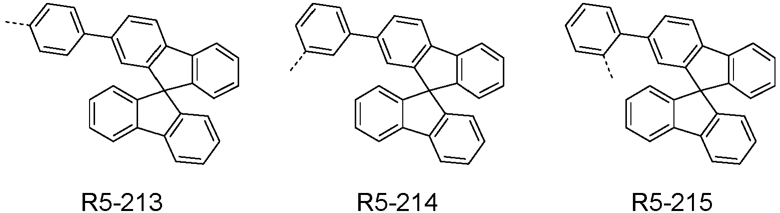 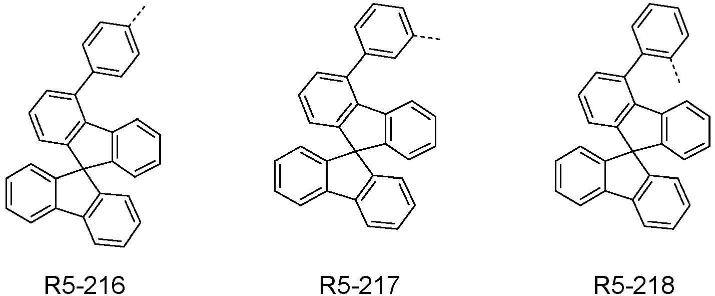 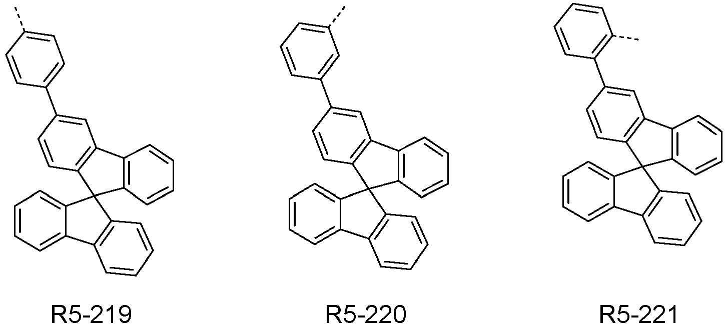 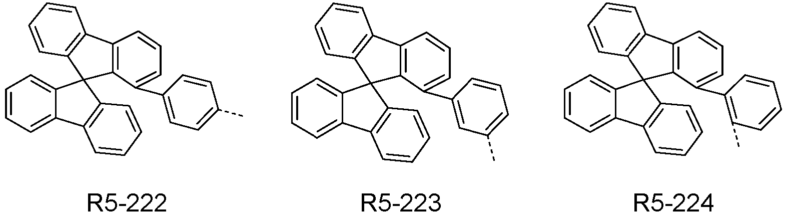 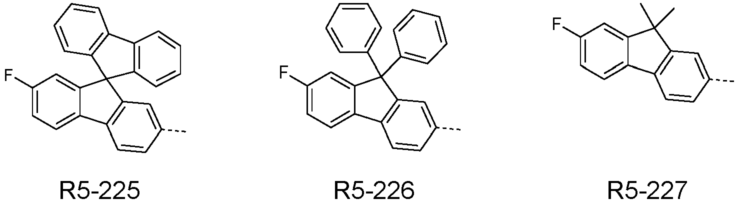 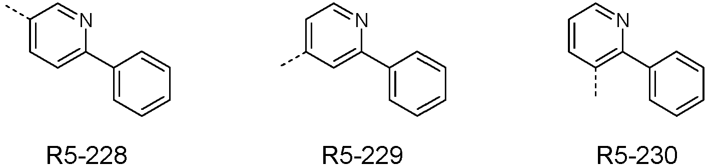 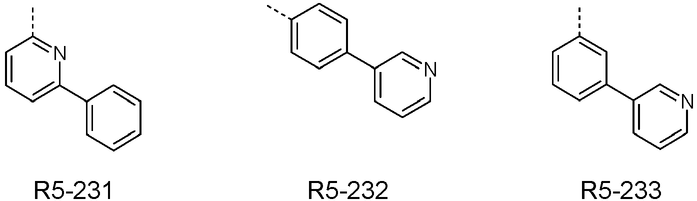 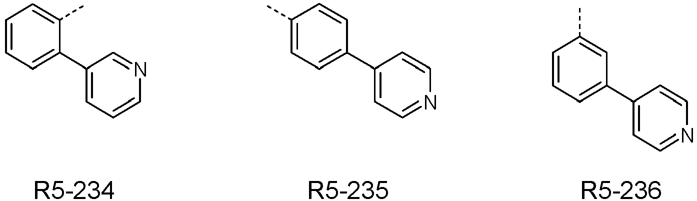 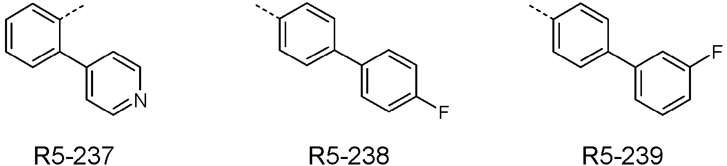 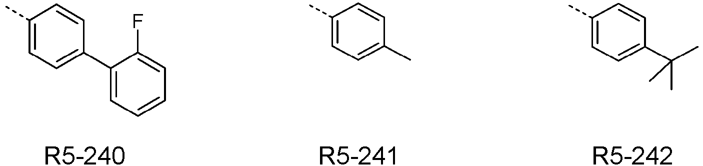 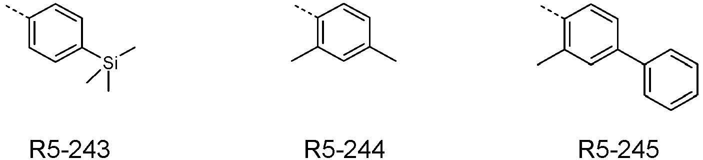 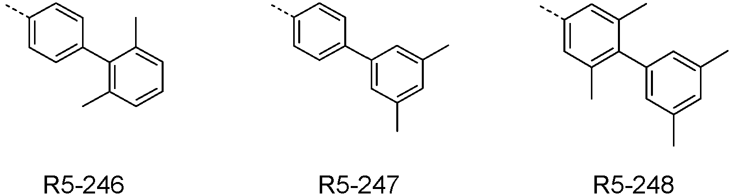 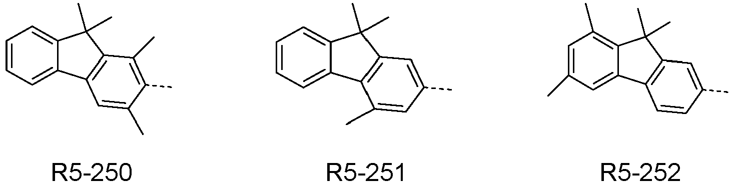 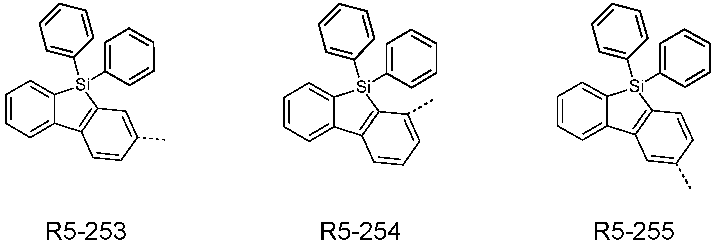 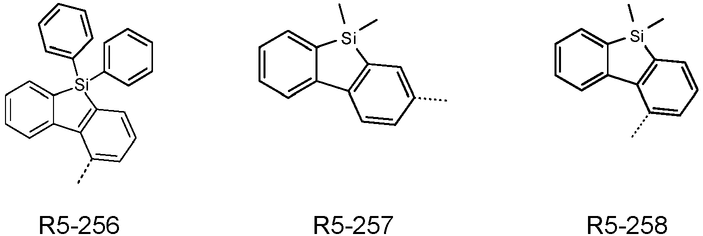 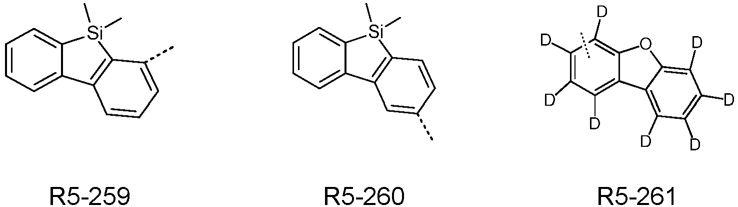 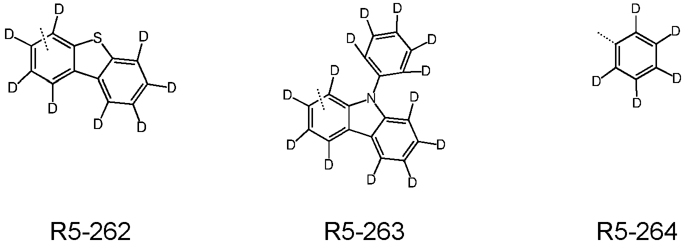 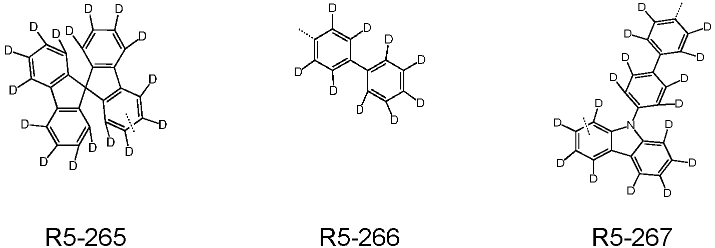 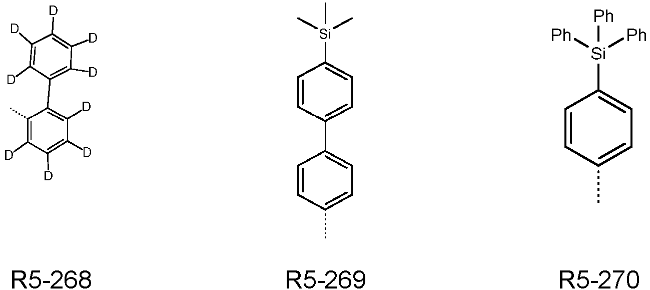 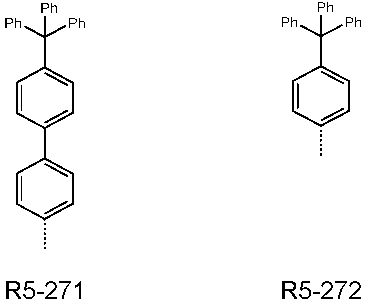 where the said radicals R⁵ may be substituted by radicals R¹¹ at the positions depicted as unsubstituted, where R¹¹ is preferably H in these positions, and where the dashed bond is the bond to the amine nitrogen atom; where the definitions from the above claims apply to the symbols used.

10. Compound according to one or more of Claims 1 to 9, **characterised in that** m, n and o are equal to 0.

11. Process for the preparation of a compound according to one or more of Claims 1 to 10 with the aid of Suzuki coupling or the Buchwald reaction.

12. Oligomer, polymer or dendrimer containing one or more compounds according to one or more of Claims 1 to 10, where the bond(s) to the polymer, oligomer or dendrimer may be localised at any desired positions in formula (3) that are substituted by R¹, R², R³, R⁴, R⁵, R⁶, R⁷, R⁸, R¹¹ or R¹².

13. Composition comprising one or more compounds of the formula (3) and at least one further material which is selected from the group consisting of hole-transport materials, hole-injection materials, p-dopants, electron-blocking materials, matrix materials, emitters and electrontransport materials.

14. Formulation comprising at least one compound according to one or more of Claims 1 to 10 or at least one polymer, oligomer or dendrimer according to Claim 12 or at least one composition according to Claim 13, and at least one solvent.

15. Electronic device containing at least one compound according to one or more of Claims 1 to 10, or at least one polymer, oligomer or dendrimer according to Claim 12 or at least one composition according to Claim 13.

16. Electronic device according to Claim 15, **characterised in that** it is an organic electroluminescent device and contains anode, cathode and at least one emitting layer, and **in that** the compound is present in an electron-blocking layer or in a hole-transporting layer or in an emitting layer of the device.

17. Organic electroluminescent device according to Claim 16, **characterised in that** the compound is present in a hole-transporting layer, which is a hole-transport layer or an electron-blocking layer.

18. Use of a compound according to one or more of Claims 1 to 10 in an electronic device.

## Revendications

1. Composé de formule (3) 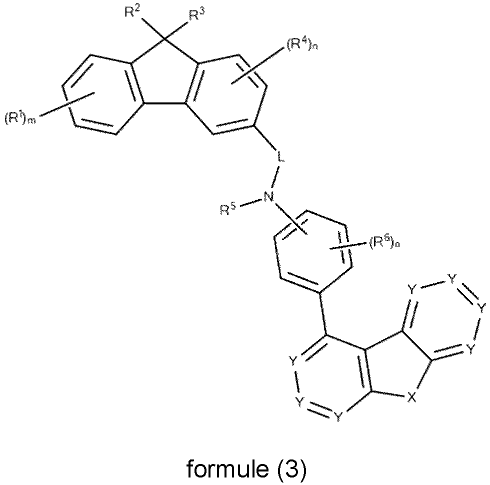 dans laquelle ce qui suit s'applique aux symboles utilisés :
X est égal à O ou S, préférablement O ;
Y est, de manière identique ou différente à chaque occurrence, CR⁷ ou N, on préfère que Y soit égal à CR⁷ ;
L est un noyau aromatique divalent ayant de 6 à 40 atomes de cycle aromatique ;
R¹, R⁴, R⁶ et R⁷ sont choisis à chaque occurrence, de manière identique ou différente, parmi H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ ne forment pas de cycle les uns avec les autres, deux, ou plus, radicaux R⁴ peuvent être liés les uns aux autres et peuvent former un cycle et/ou deux, ou plus, radicaux R⁶ peuvent être liés les uns aux autres et peuvent former un cycle et/ou deux, ou plus, radicaux R⁷ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par des radicaux R¹¹ ; et où un ou plusieurs groupements CH₂ dans lesdits groupements alkyle, alcoxy, alcényle et alcynyle peuvent être remplacés par -R¹¹C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO ou SO₂ ; préférablement, deux, ou plus, radicaux R⁴ ne forment pas de cycle les uns avec les autres, et/ou deux, ou plus, radicaux R⁶ ne forment pas de cycle les uns avec les autres et/ou deux, ou plus, radicaux R⁷ ne forment pas de cycle les uns avec les autres ;
R² et R³ sont choisis à chaque occurrence, de manière identique ou différente, parmi des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où les deux radicaux R² et R³ peuvent être liés l'un avec l'autre et peuvent former un cycle ; où lesdits groupements alkyle, alcoxy, alcényle et alcynyle et lesdits noyaux aromatiques et noyaux hétéroaromatiques peuvent dans chaque cas être substitués par des radicaux R¹¹ ; et où un ou plusieurs groupements CH₂ dans lesdits groupements alkyle, alcoxy, alcényle et alcynyle peuvent être remplacés par -R¹¹C=CR¹¹-, -C≡C- Si(R¹¹)₂, C=O, C=NR¹¹, -C(=O)O-, -C(=O)NR¹¹-, NR¹¹, P(=O)(R¹¹), -O-, -S-, SO ou SO₂ ; si les deux radicaux R² et R³ forment un cycle, un composé spiro se forme, préférablement un spirobifluorène ; on préfère particulièrement que les deux radicaux R² et R³ ne forment pas de cycle l'un avec l'autre ;
R⁵ est un noyau aromatique ayant de 6 à 40 atomes de cycle aromatique, qui peut être substitué par un ou plusieurs radicaux R¹¹, ou un noyau hétéroaromatique ayant de 5 à 40 atomes de cycle aromatique, qui peut être substitué par un ou plusieurs radicaux R¹¹, où le radical R⁵ ne contient pas de noyau hétéroaromatique condensé ayant plus de 12 atomes de carbone aromatiques comme atomes de cycle dans le noyau hétéroaromatique ;
R¹¹ est choisi à chaque occurrence, de manière identique ou différente, parmi H, D, F, Cl, Br, I, C(=O)R¹², CN, Si(R¹²)₃, N(R¹²)₂, P(=O)(R¹²)₂, OR¹², S(=O)R¹², S(=O)₂R¹², des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹¹ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par des radicaux R¹² ; et où un ou plusieurs groupements CH₂ dans lesdits groupements alkyle, alcoxy, alcényle et alcynyle peuvent être remplacés par -R¹²C=CR¹²-, -C≡C-, Si(R¹²)₂, C=O, C=NR¹², -C(=O)O-, -C(=O)NR¹²-, NR¹², P(=O)(R¹²), -O-, -S-, SO ou SO₂ ; où deux, ou plus, radicaux R¹¹ ne peuvent pas former de cycle les uns avec les autres ;
R¹² est choisi à chaque occurrence, de manière identique ou différente, parmi H, D, F, Cl, Br, I, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où lesdits groupements alkyle, alcoxy, alcényle et alcynyle, noyaux aromatiques et noyaux hétéroaromatiques peuvent être substitués par un ou plusieurs radicaux choisis parmi F et CN ;
m vaut 0, 1, 2, 3 ou 4, on préfère que m soit égal à 0 ou 1, on préfère particulièrement que m soit égal à 1 et on préfère très particulièrement que m soit égal à 0 ;
n vaut 0, 1, 2 ou 3, on préfère que n soit égal à 0 ou 1, on préfère particulièrement que n soit égal à 1 et on préfère très particulièrement que n soit égal à 0 ;
o vaut 0, 1, 2 ou 3, on préfère que o soit égal à 0 ou 1, on préfère particulièrement que o soit égal à 1 et on préfère très particulièrement que o soit égal à 0.

2. Composé selon la revendication 1, **caractérisé en ce que** L est choisi parmi les formules suivantes 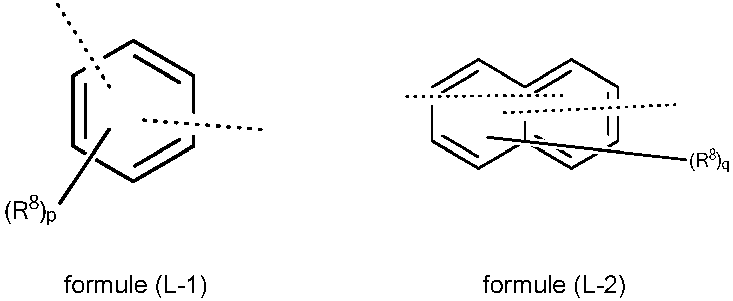 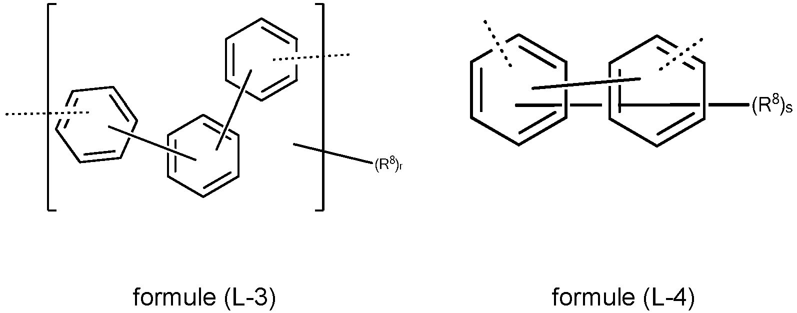 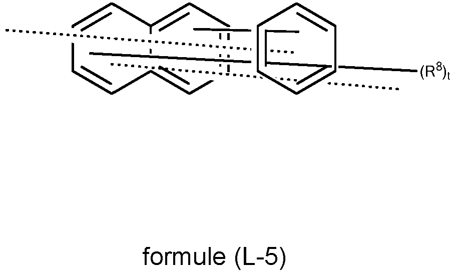 dans lesquelles ce qui suit s'applique :
R⁸ est choisi à chaque occurrence, de manière identique ou différente, parmi H, D, F, Cl, Br, I, C(=O)R¹¹, CN, Si(R¹¹)₃, N(R¹¹)₂, P(=O)(R¹¹)₂, OR¹¹, S(=O)R¹¹, S(=O)₂R¹¹, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R⁸ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par des radicaux R¹¹ ; et où un ou plusieurs groupements CH₂ dans lesdits groupements alkyle, alcoxy, alcényle et alcynyle peuvent être remplacés par -R¹¹C=CR¹¹-, -C≡C-, Si(R¹¹)₂, C=O, C=NR¹¹, -C(=O)O-, -C(=O)NR¹¹-, NR¹¹, P(=O)(R¹¹), -O-, -S-, SO ou SO₂ ; préférablement, deux, ou plus, radicaux R⁸ ne forment pas de cycle les uns avec les autres ;
et où R¹¹ est défini tel qu'indiqué selon la revendication 1 ;
p vaut 0, 1, 2, 3 ou 4, on préfère que p soit égal à 0 ou 1, on préfère particulièrement que p soit égal à 1 et on préfère très particulièrement que p soit égal à 0 ;
q vaut 0, 1, 2, 3, 4, 5 ou 6, on préfère que q soit égal à 0 ou 1, on préfère particulièrement que q soit égal à 1 et on préfère très particulièrement que q soit égal à 0 ;
r vaut 0, 1, 2, 3, 4, 5, 7, 8, 9, 10, 11 ou 12, on préfère que r soit égal à 0 ou 1, on préfère particulièrement que r soit égal à 1 et on préfère très particulièrement que r soit égal à 0 ;
s vaut 0, 1, 2, 3, 4, 5, 7 ou 8, on préfère que s soit égal à 0 ou 1, on préfère particulièrement que s soit égal à 1 et on préfère très particulièrement que s soit égal à 0 ;
t vaut 0, 1, 2, 3, 4, 5, 7, 8, 9 ou 10, on préfère que t soit égal à 0 ou 1, on préfère particulièrement que t soit égal à 1 et on préfère très particulièrement que t soit égal à 0.

3. Composé selon la revendication 1 ou 2, **caractérisé en ce que** L est choisi parmi les formules suivantes 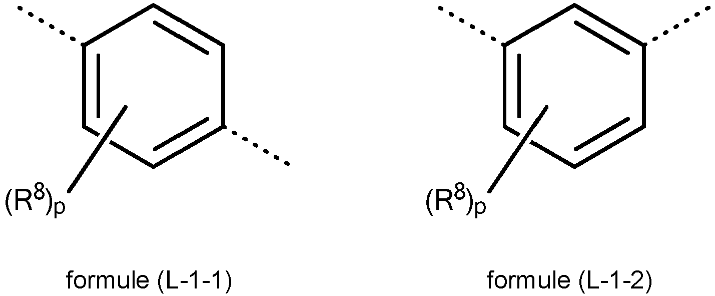 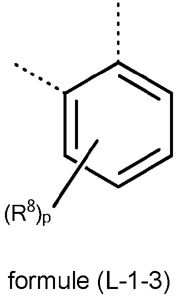 dans lesquelles les définitions provenant des revendications ci-dessus s'appliquent aux symboles utilisés.

4. Composé selon la revendication 1 ou 2, **caractérisé en ce que** L est choisi parmi les formules suivantes 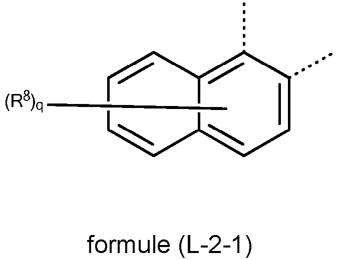 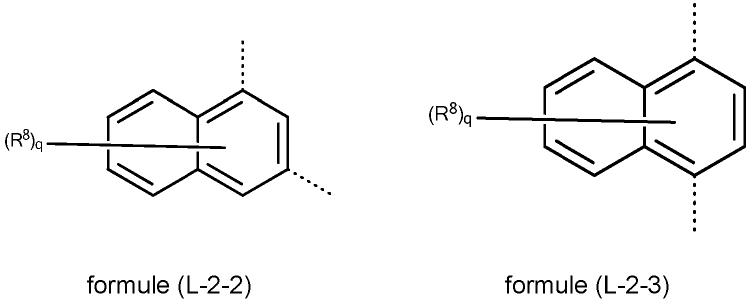 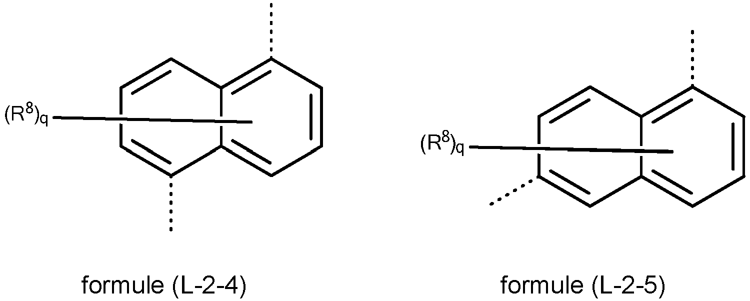 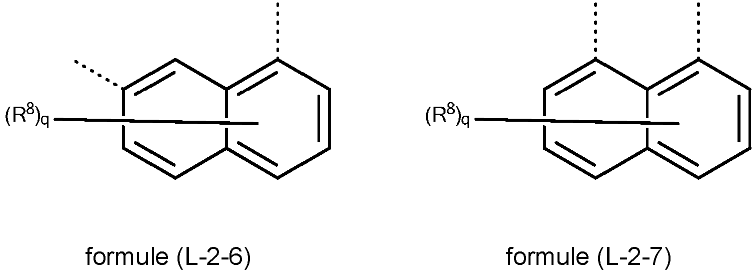 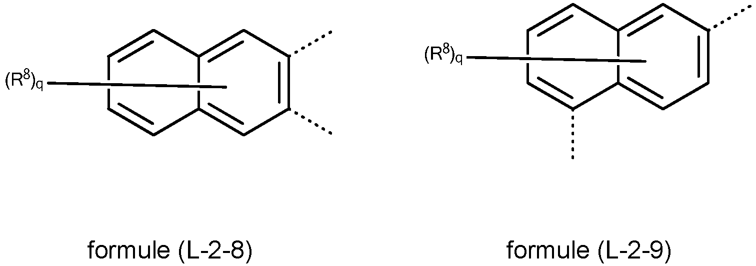 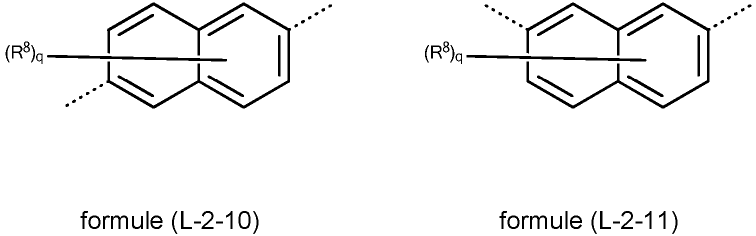 dans lesquelles les définitions provenant des revendications ci-dessus s'appliquent aux symboles utilisés.

5. Composé selon la revendication 1 ou 2, **caractérisé en ce que** L est choisi parmi les formules suivantes 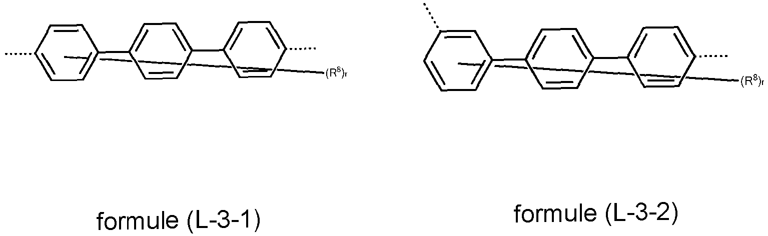 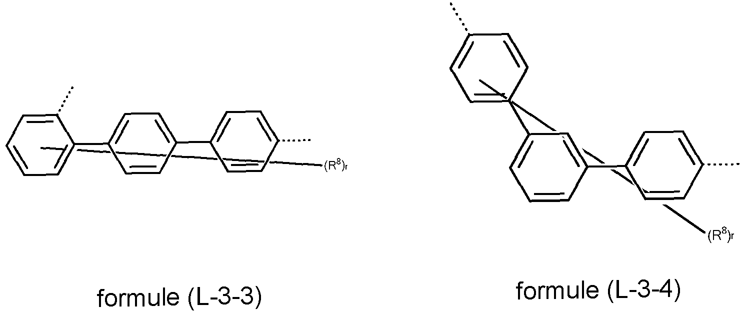 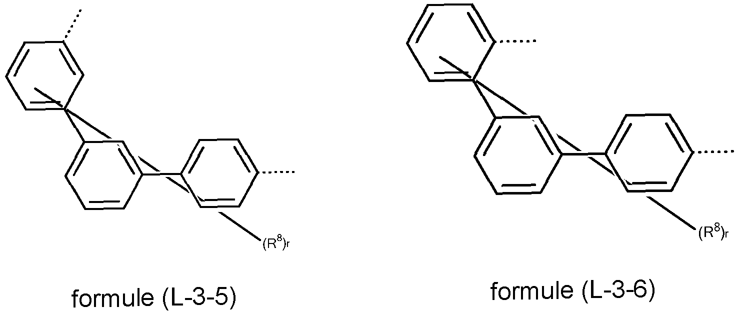 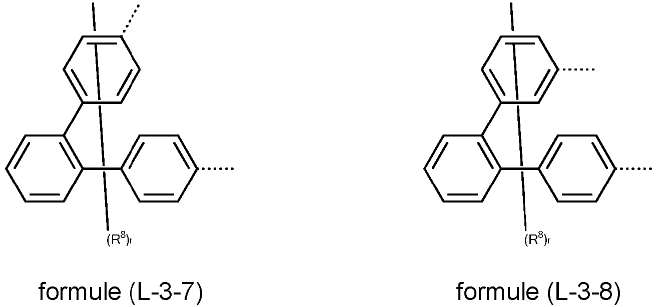 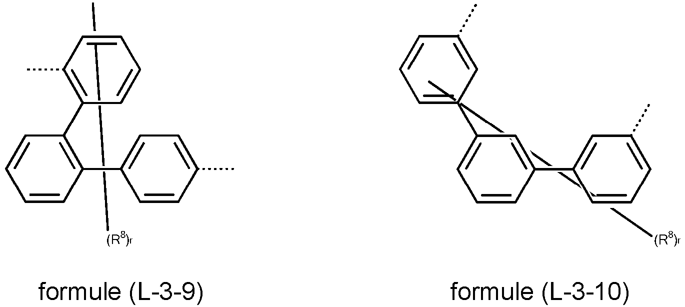 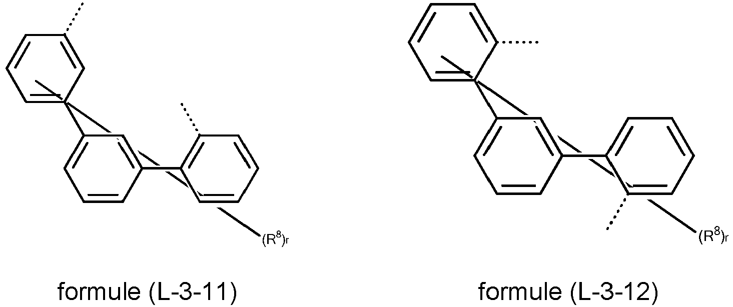 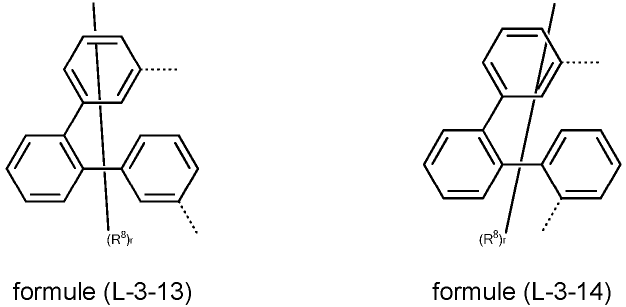 dans lesquelles les définitions provenant des revendications ci-dessus s'appliquent aux symboles utilisés.

6. Composé selon la revendication 1 ou 2, **caractérisé en ce que** L est choisi parmi les formules suivantes 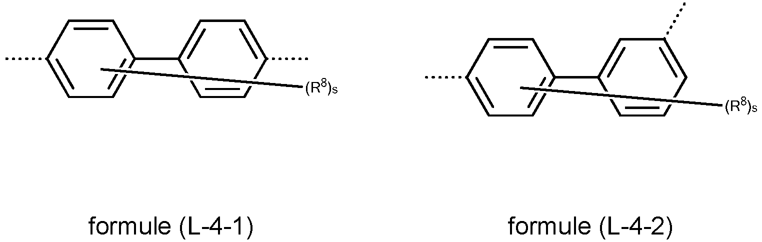 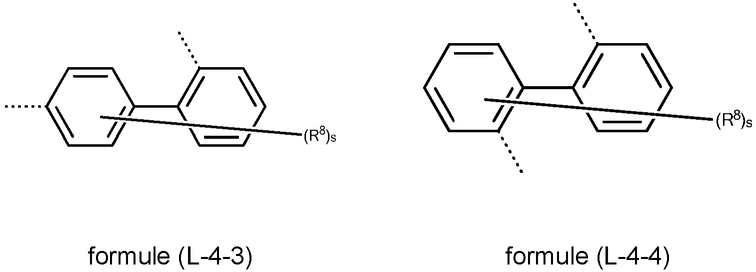 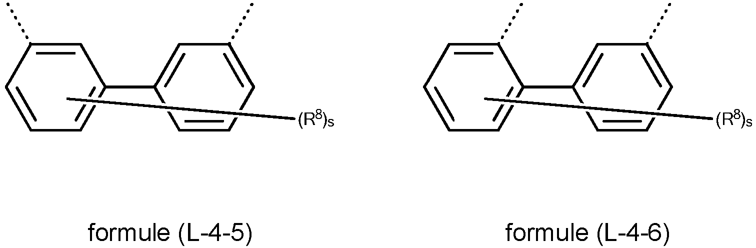 dans lesquelles les définitions provenant des revendications ci-dessus s'appliquent aux symboles utilisés.

7. Composé selon l'une ou plusieurs parmi les revendications 1 à 6, **caractérisé en ce qu'**il s'agit d'un composé de monoamine.

8. Composé selon l'une ou plusieurs parmi les revendications 1 à 7, **caractérisé en ce que** R² et R³ sont choisis, de manière identique ou différente à chaque occurrence, parmi des groupements alkyle à chaîne linéaire ayant de 1 à 10 atomes de C, des groupements alkyle ramifiés ou cycliques ayant de 3 à 10 atomes de C ou des noyaux aromatiques ayant de 6 à 18 atomes de cycle aromatique ; où les deux radicaux R² et R³ peuvent être liés l'un à l'autre et peuvent former un cycle ; où lesdits groupements alkyle et lesdits noyaux aromatiques peuvent dans chaque cas être substitués par des radicaux R¹¹ ; si les deux radicaux R² et R³ forment un cycle, un composé spiro se forme, préférablement un spirobifluorène ; on préfère particulièrement que les deux radicaux R² et R³ ne forment pas de cycle l'un avec l'autre.

9. Composé selon l'une ou plusieurs parmi les revendications 1 à 8, **caractérisé en ce que** les radicaux R⁵ sont choisis parmi les formules suivantes 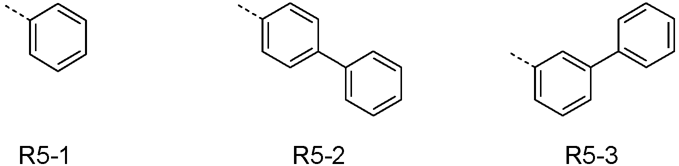 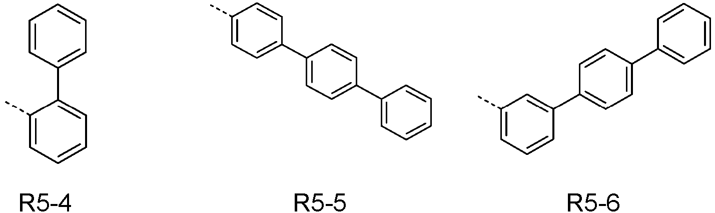 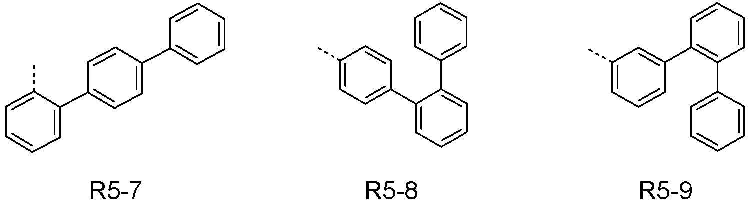 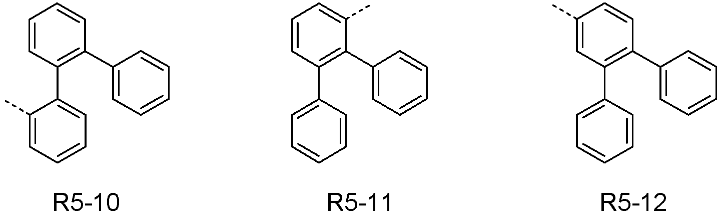 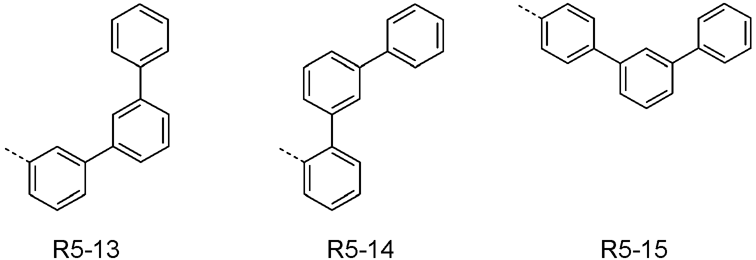 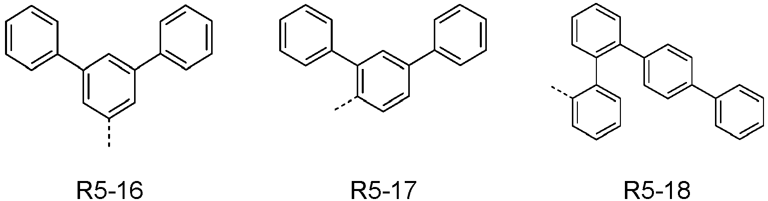 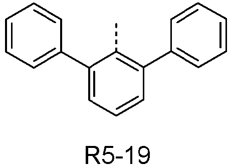 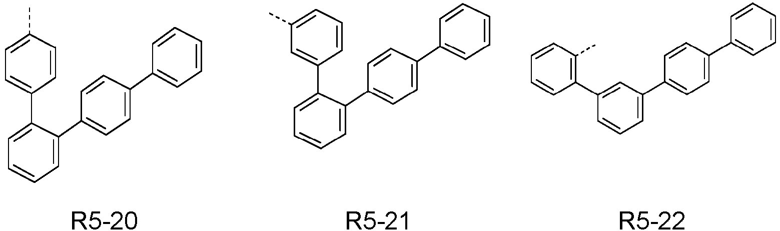 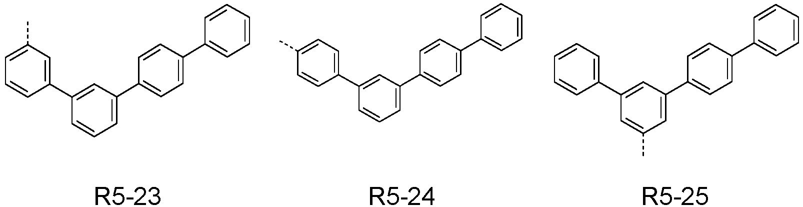 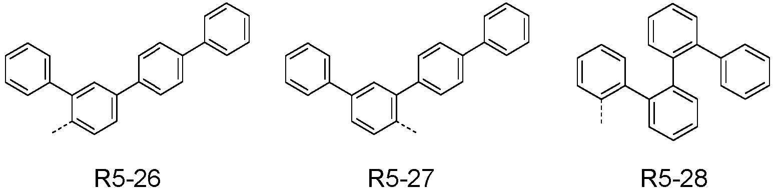 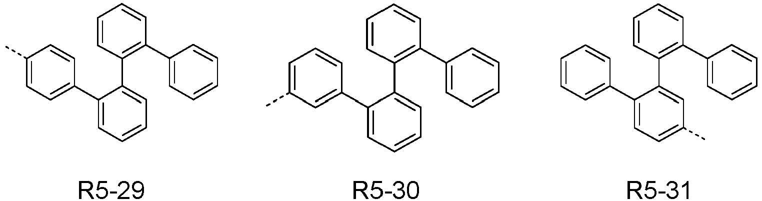 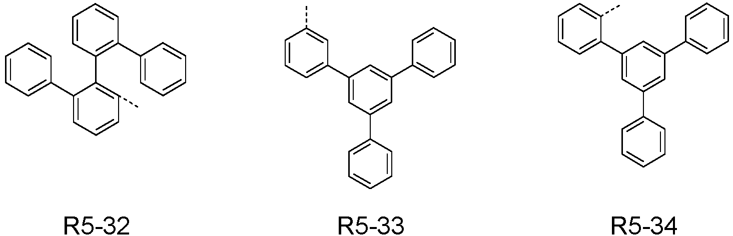 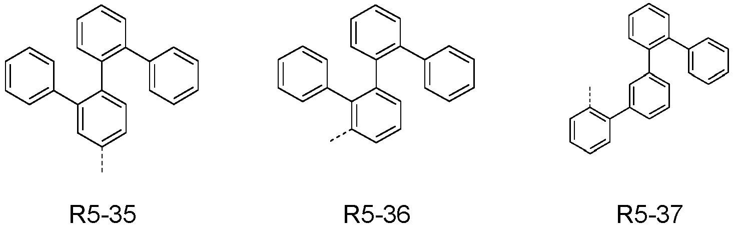 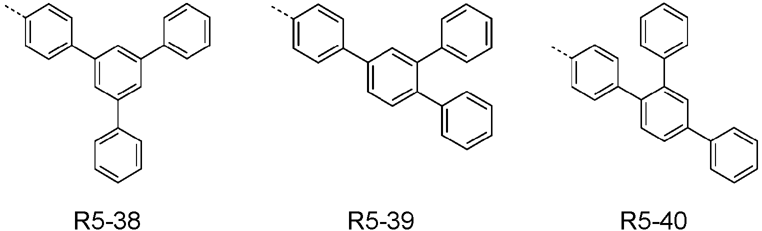 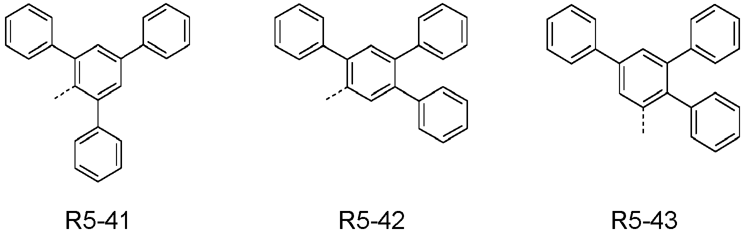 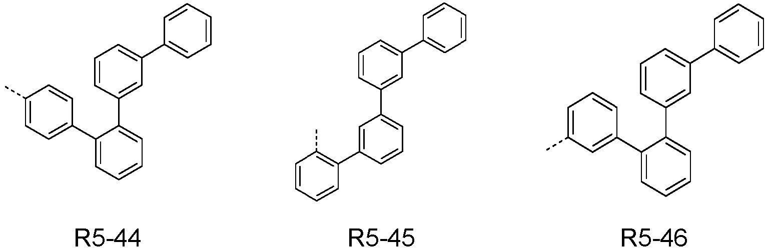 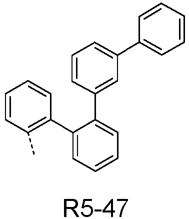 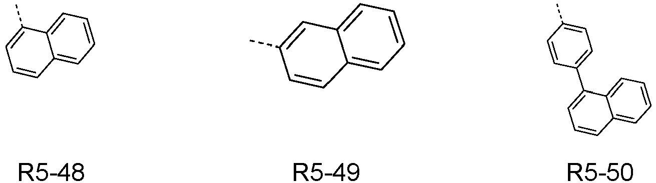 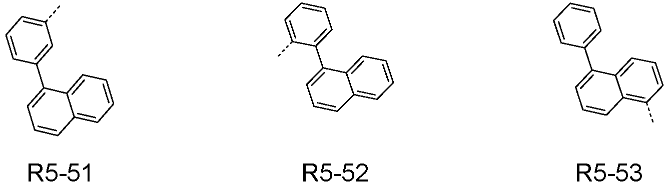 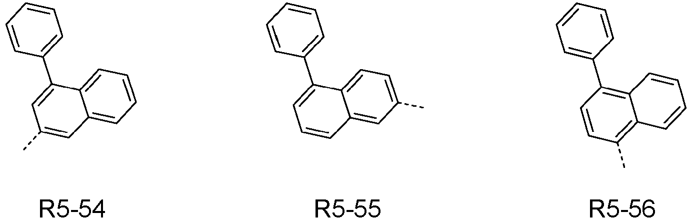 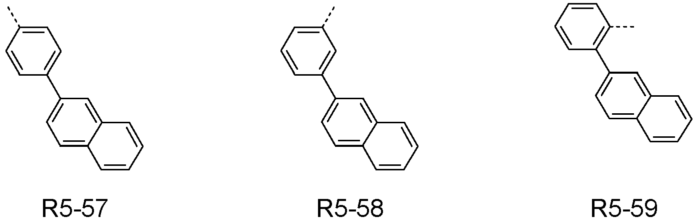 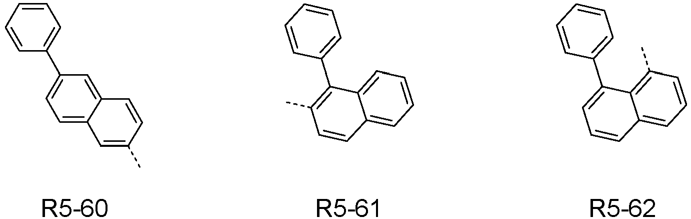 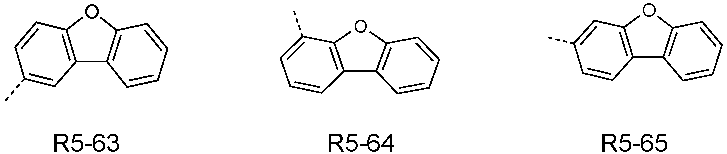 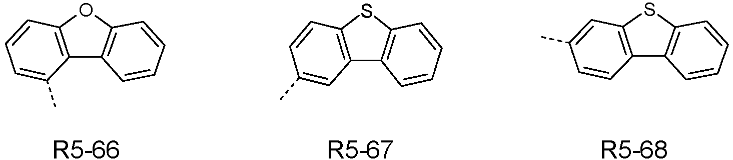 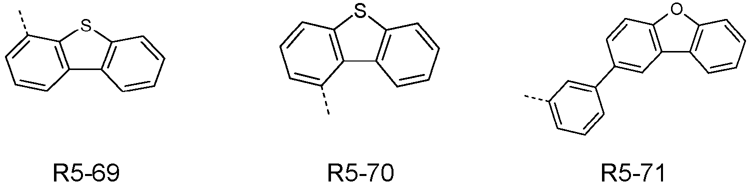 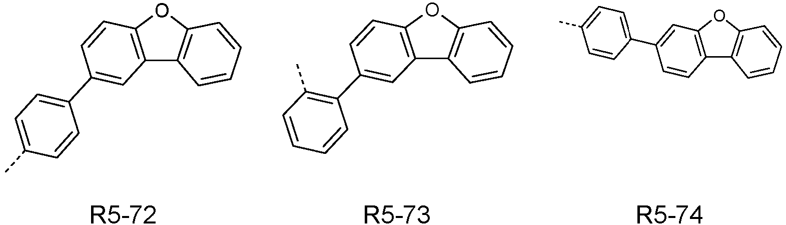 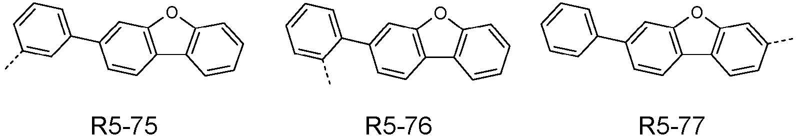 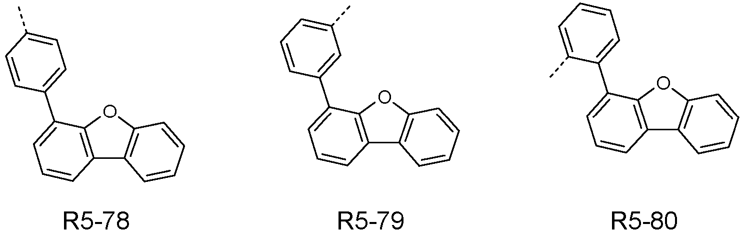 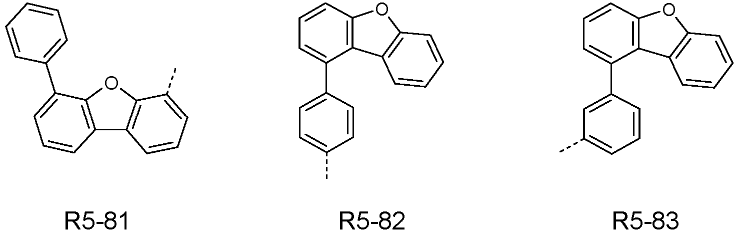 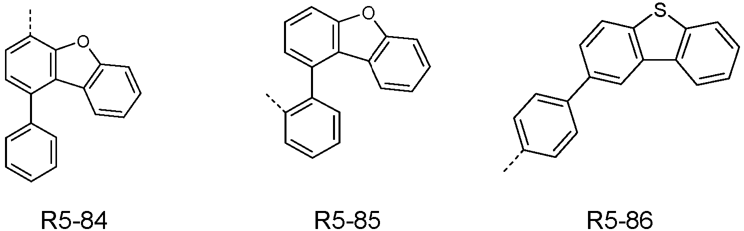 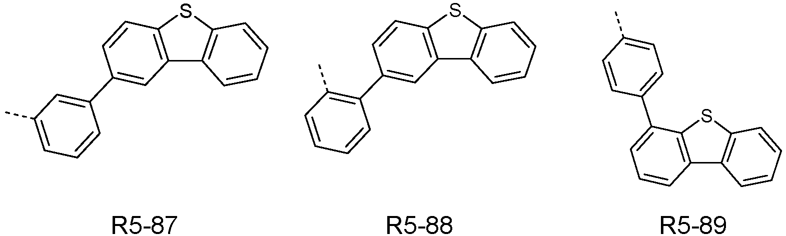 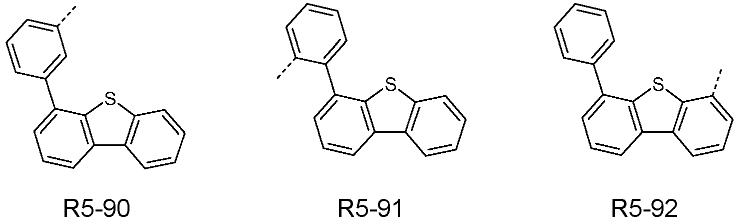 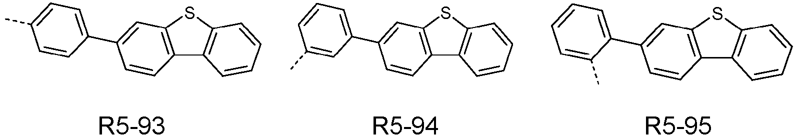 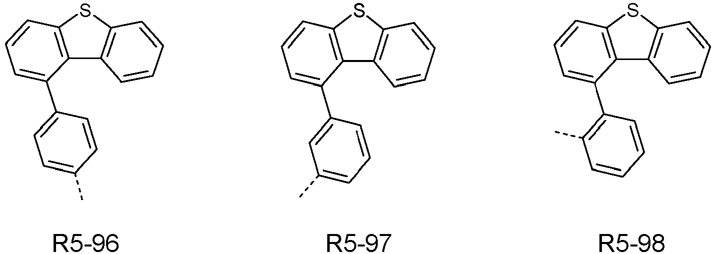 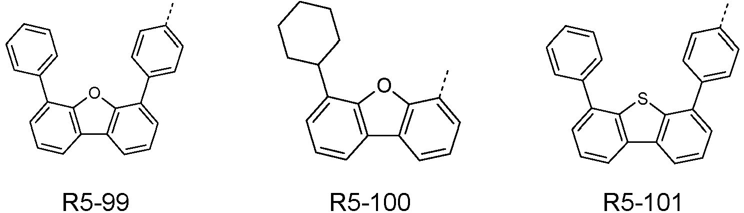 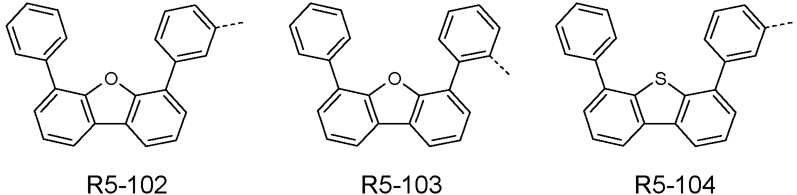 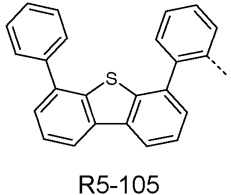 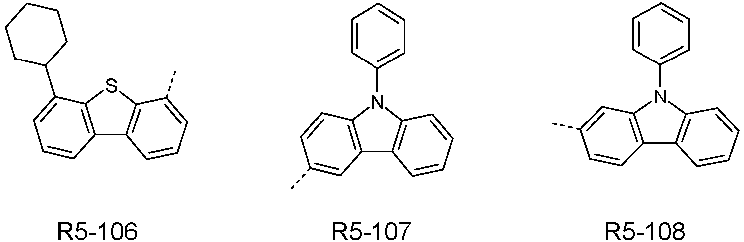 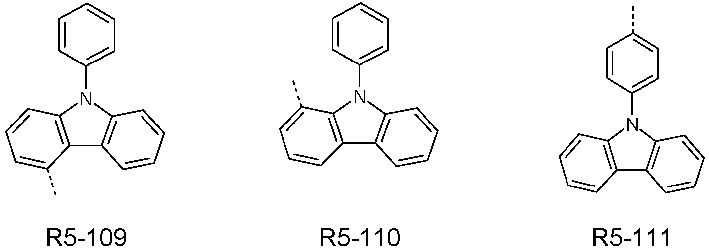 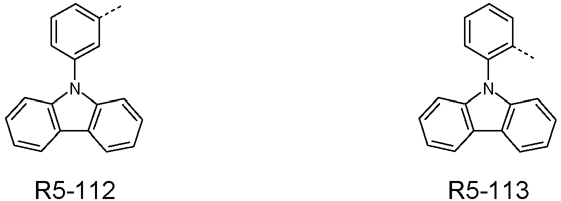 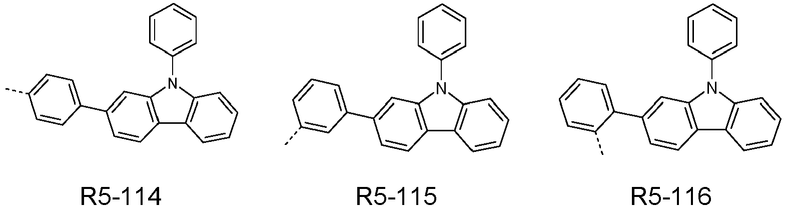 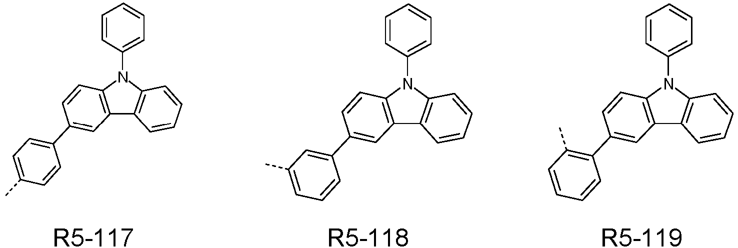 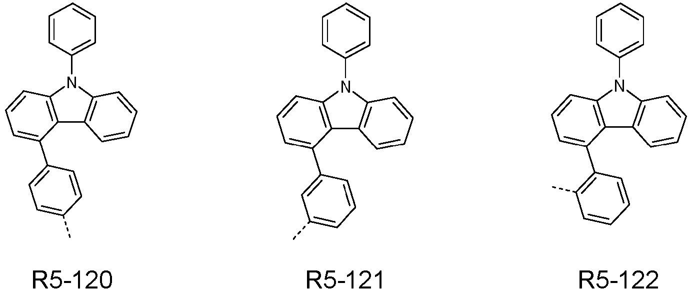 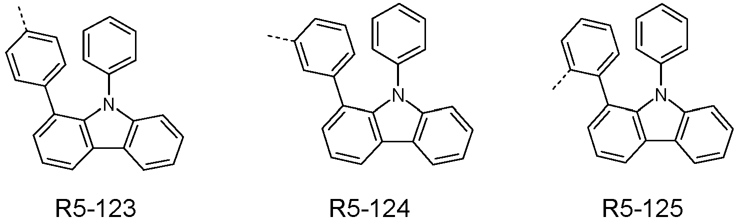 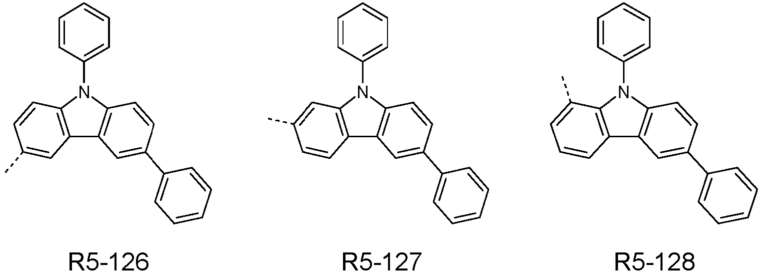 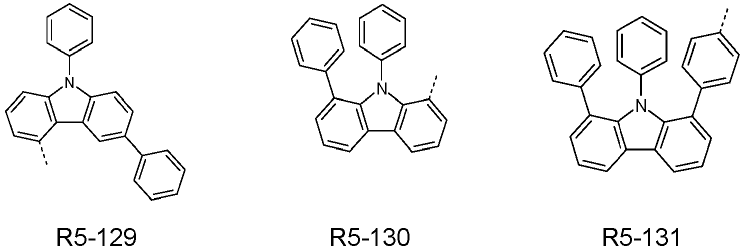 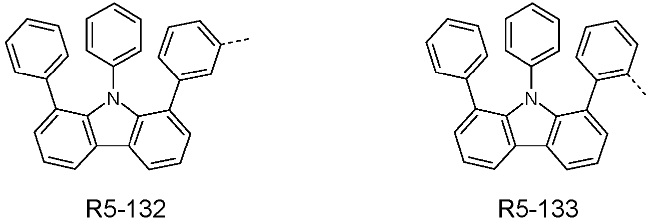 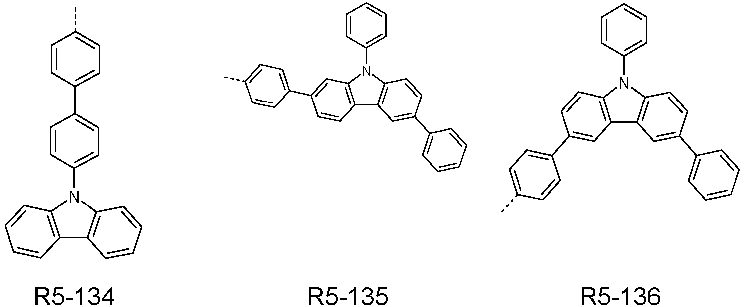 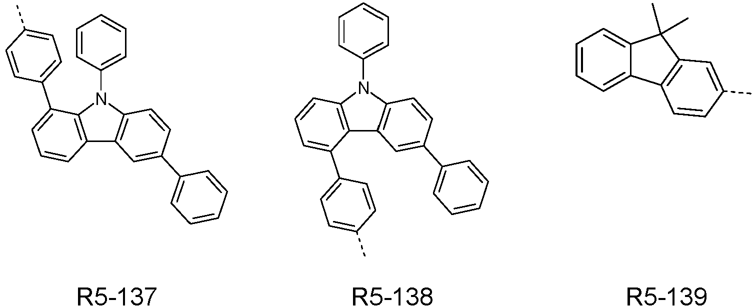 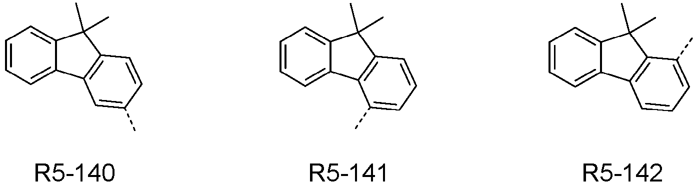 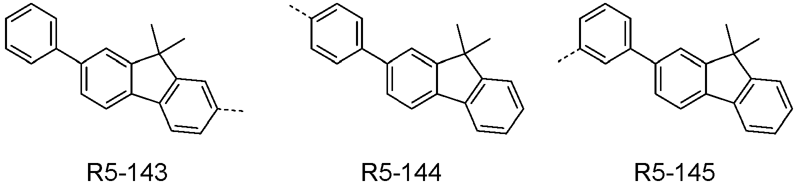 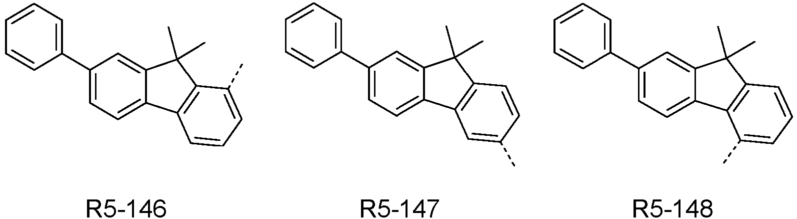 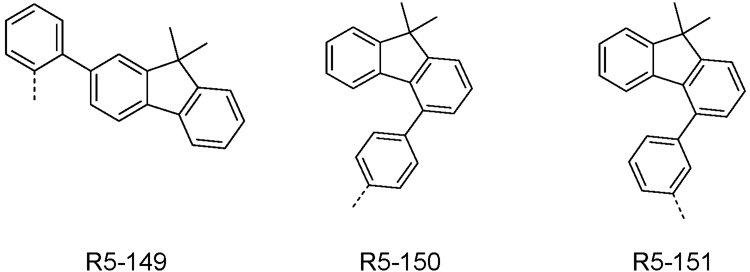 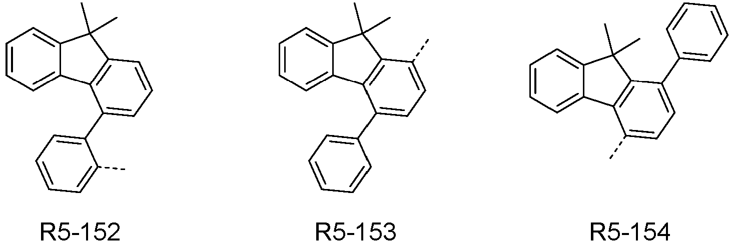 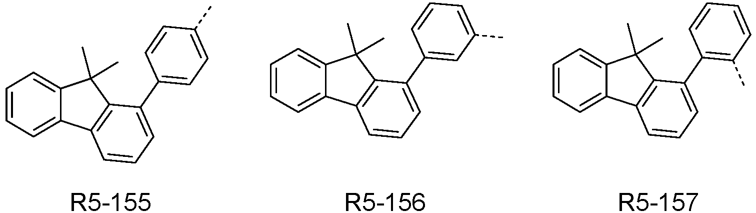 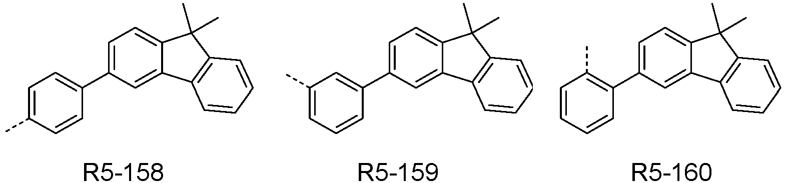 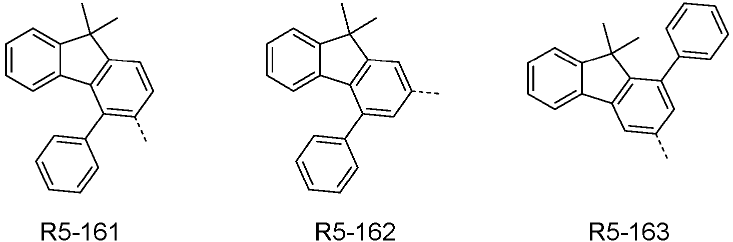 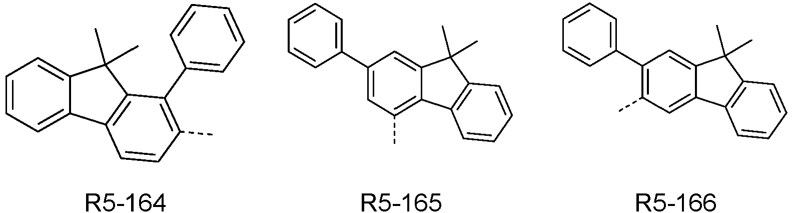 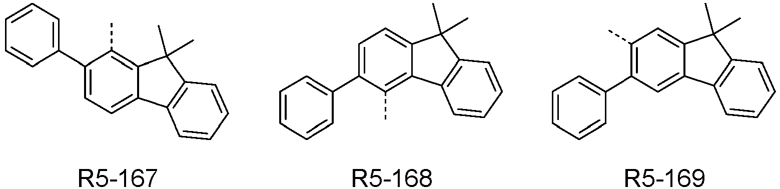 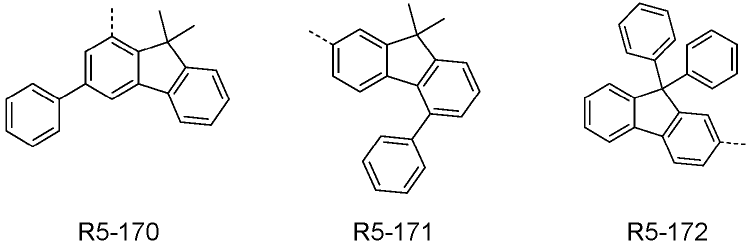 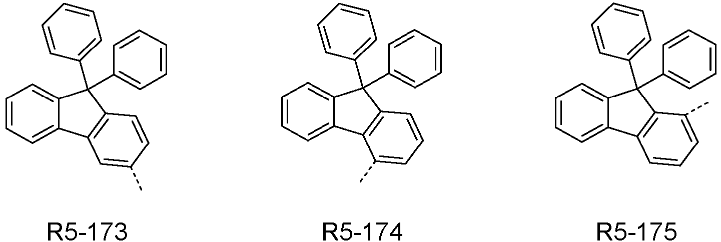 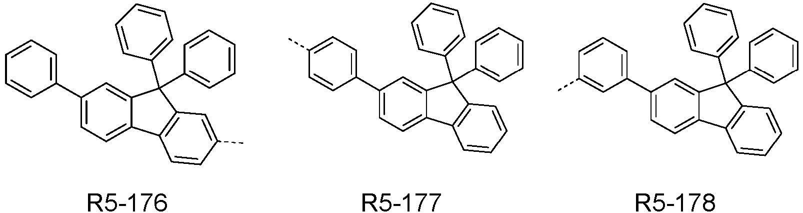 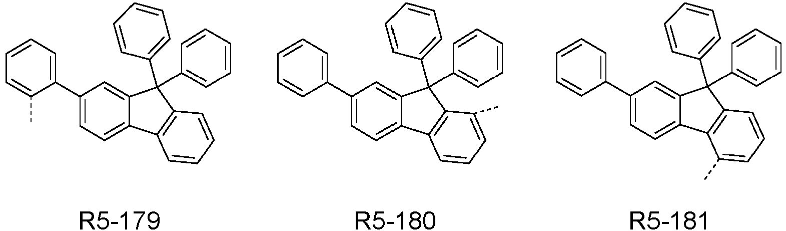 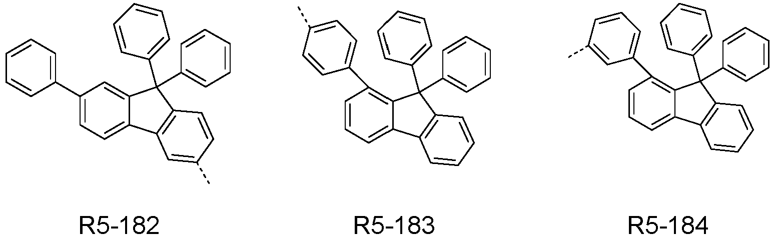 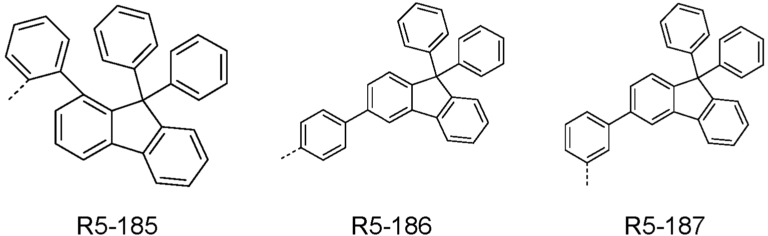 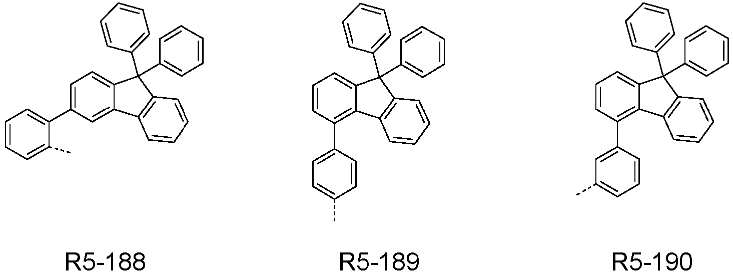 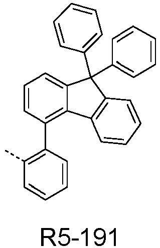 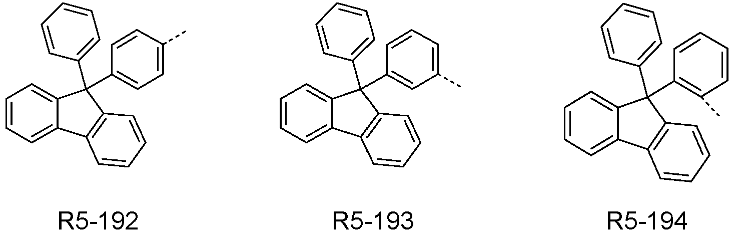 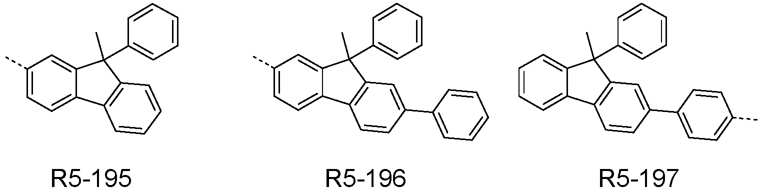 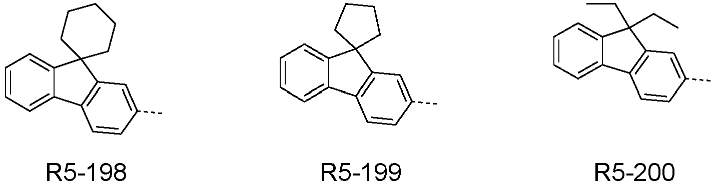 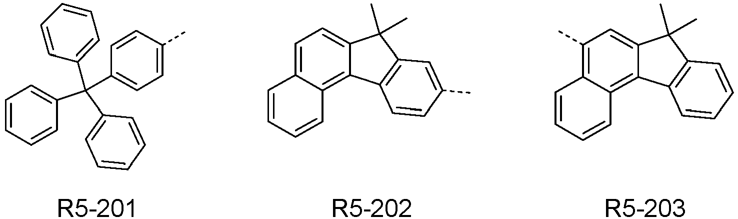 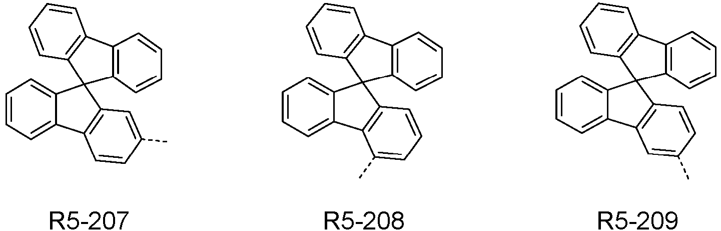 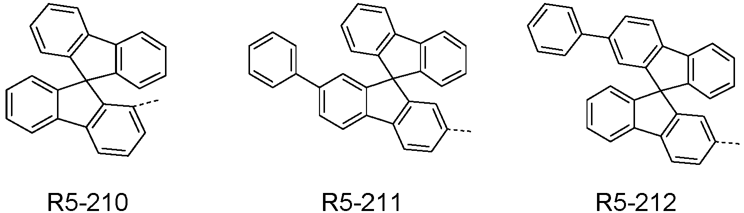 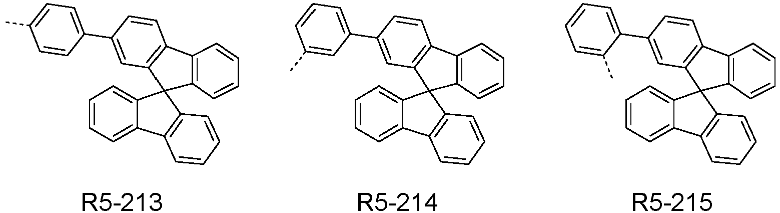 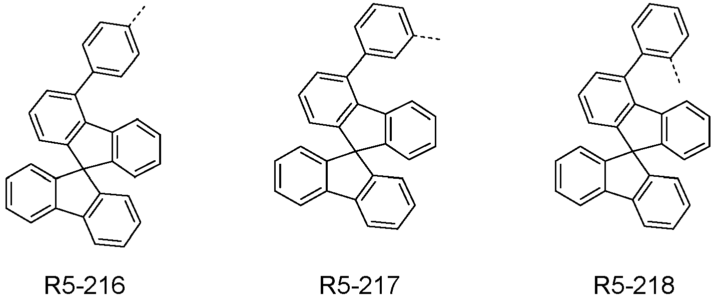 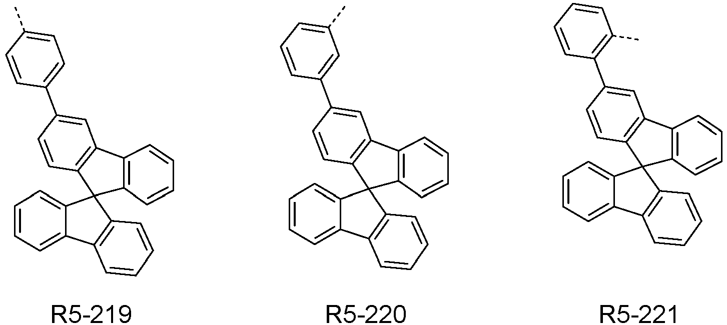 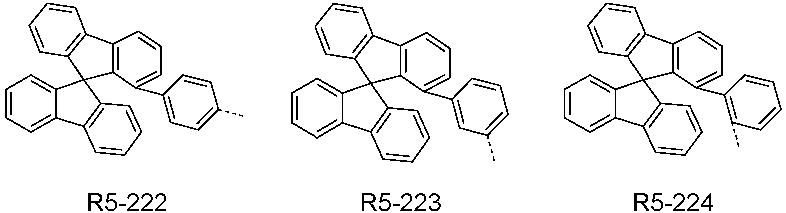 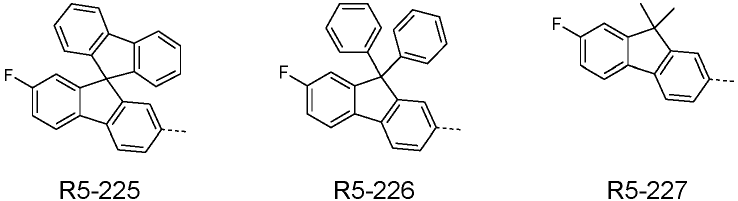 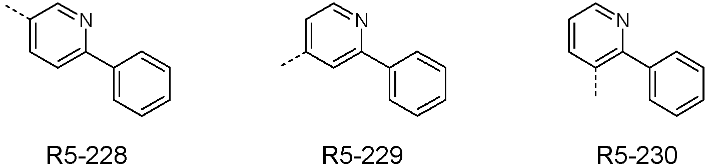 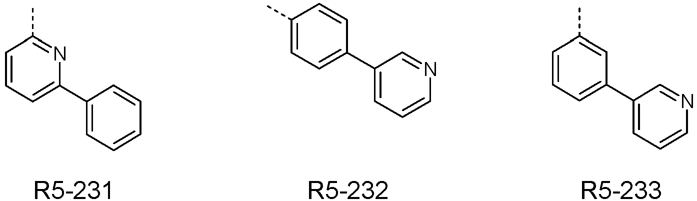 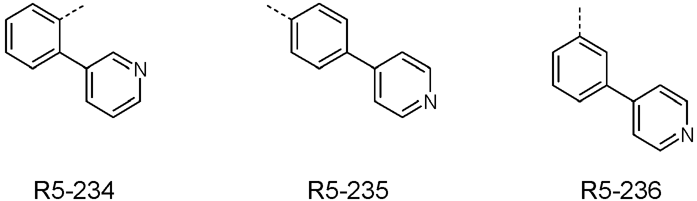 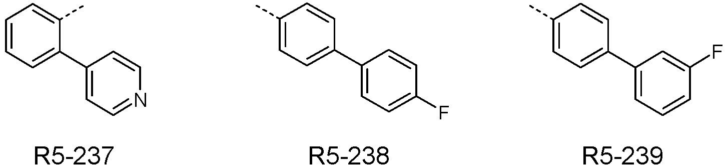 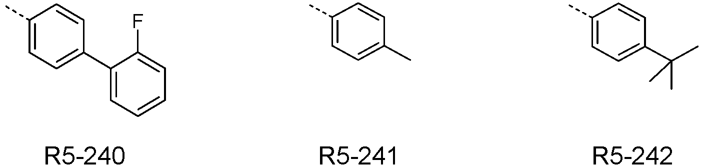 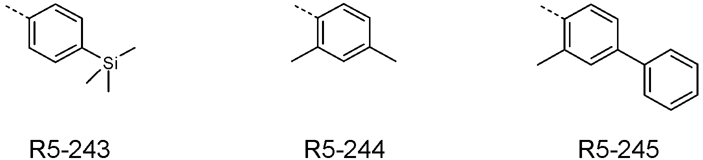 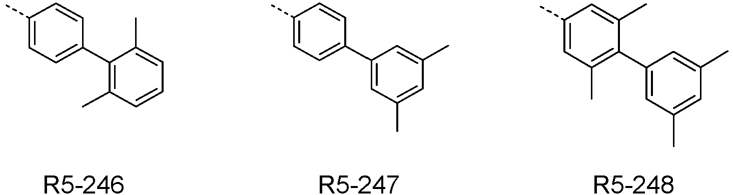 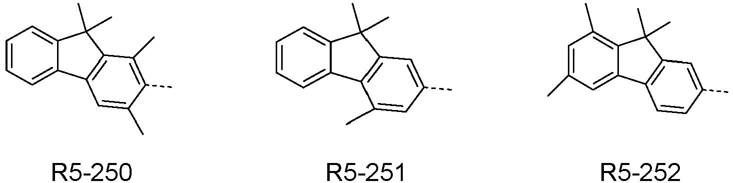 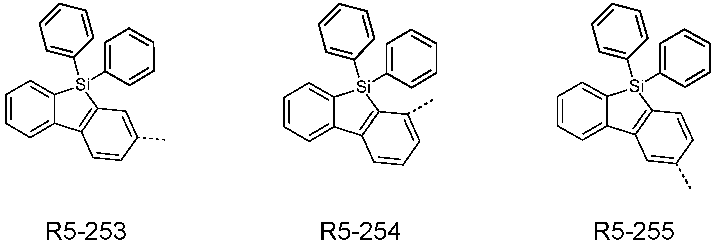 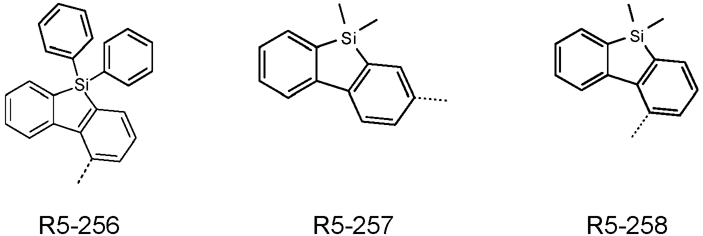 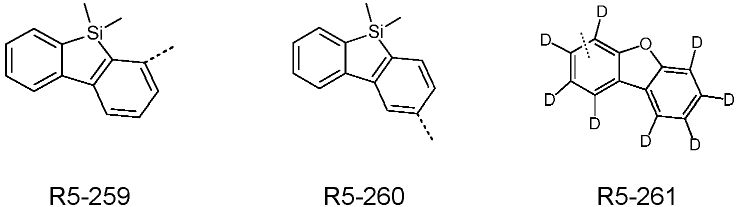 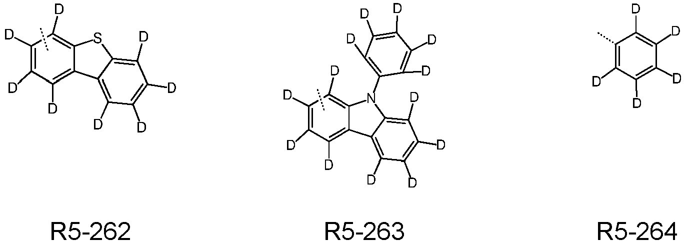 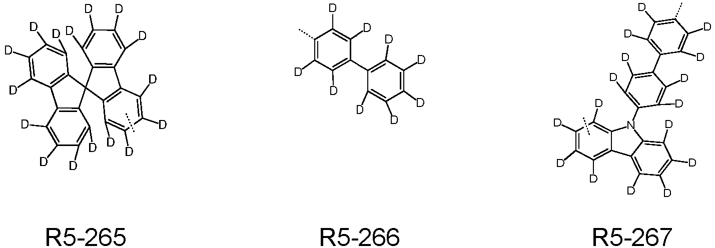 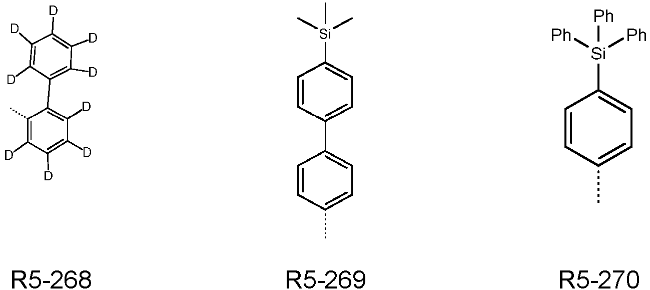 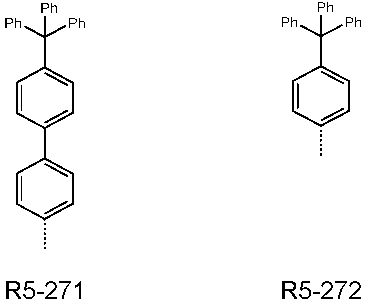 dans lesquelles lesdits radicaux R⁵ peuvent être substitués par des radicaux R¹¹ au niveau des positions illustrées comme étant non substituées, où R¹¹ est préférablement H dans ces positions, et où la liaison en pointillés est la liaison à l'atome d'azote de l'amine ; dans lesquelles les définitions provenant des revendications ci-dessus s'appliquent aux symboles utilisés.

10. Composé selon l'une ou plusieurs parmi les revendications 1 à 9, **caractérisé en ce que** m, n et o sont égaux à 0.

11. Procédé de préparation d'un composé selon l'une ou plusieurs parmi les revendications 1 à 10, à l'aide d'un couplage de Suzuki ou de la réaction de Buchwald.

12. Oligomère, polymère ou dendrimère contenant un ou plusieurs composés selon l'une ou plusieurs parmi les revendications 1 à 10, la ou les liaisons au polymère, à l'oligomère ou au dendrimère pouvant être situées au niveau de positions désirées quelconques dans la formule (3) qui sont substituées par R¹, R², R³, R⁴, R⁵, R⁶, R⁷, R⁸, R¹¹ ou R¹².

13. Composition comprenant un ou plusieurs composés de formule (3) et au moins un autre matériau qui est choisi dans le groupe constitué par les matériaux de transport de trous, les matériaux d'injection de trous, les dopants de type P, les matériaux de blocage d'électrons, les matériaux de matrice, les émetteurs et les matériaux de transport d'électrons.

14. Formulation comprenant au moins un composé selon l'une ou plusieurs parmi les revendications 1 à 10 ou au moins un polymère, oligomère ou dendrimère selon la revendication 12 ou au moins une composition selon la revendication 13, et au moins un solvant.

15. Dispositif électronique contenant au moins un composé selon l'une ou plusieurs parmi les revendications 1 à 10, ou au moins un polymère, oligomère ou dendrimère selon la revendication 12 ou au moins une composition selon la revendication 13.

16. Dispositif électronique selon la revendication 15, **caractérisé en ce qu'**il s'agit d'un dispositif électroluminescent organique et qu'il comprend une anode, une cathode et au moins une couche émettrice, et **en ce que** le composé est présent dans une couche bloquant des électrons ou dans une couche transportant des trous ou dans une couche émettrice du dispositif.

17. Dispositif électroluminescent organique selon la revendication 16, **caractérisé en ce que** le composé est présent dans une couche transportant des trous, qui est une couche de transport de trous ou une couche de blocage d'électrons.

18. Utilisation d'un composé selon l'une ou plusieurs parmi les revendications 1 à 10, dans un dispositif électronique.
